(19) Europäisches Patentamt
European Patent Office
Office européen des brevets

(11) EP 4 426 701 B1

(12) # EUROPÄISCHE PATENTSCHRIFT

(45) Veröffentlichungstag und Bekanntmachung des Hinweises auf die Patenterteilung:
**20.05.2026 Patentblatt 2026/21**

(21) Anmeldenummer: **22813181.9**

(22) Anmeldetag: **31.10.2022**

(51) Internationale Patentklassifikation (IPC):
***C07D 491/048*** (2006.01) ***C07D 495/04*** (2006.01)
***C09K 11/06*** (2006.01) ***H10K 50/12*** (2023.01)
***H10K 85/30*** (2023.01) ***H10K 85/60*** (2023.01)
***H10K 101/10*** (2023.01) ***H10K 101/00*** (2023.01)

(52) Gemeinsame Patentklassifikation (CPC):
**C07D 491/048; C07D 495/04; C09K 11/06; H10K 85/615; H10K 85/633; H10K 85/654; H10K 85/657; H10K 85/6572; H10K 85/6574;**
H10K 50/12; H10K 85/342; H10K 2101/10; H10K 2101/90

(86) Internationale Anmeldenummer:
**PCT/EP2022/080317**

(87) Internationale Veröffentlichungsnummer:
**WO 2023/078812 (11.05.2023 Gazette 2023/19)**

(54) **BENZOFURO[3,2-D]PYRIMIDIN-2,4-DICARBONITRIL-DERIVATE UND ÄHNLICHE VERBINDUNGEN FÜR ORGANISCHE ELEKTROLUMINESZENZVORRICHTUNGEN**

BENZOFURO[3,2-D]PYRIMIDIN-2,4-DICARBONITRILE DERIVATIVES AND SIMILAR COMPOUNDS FOR ORGANIC ELECTROLUMINESCENT DEVICES

DÉRIVÉS DE BENZOFURO[3,2-D]PYRIMIDIN-2,4-DICARBONITRILE ET COMPOSÉS SIMILAIRES POUR DISPOSITIFS ÉLECTROLUMINESCENTS

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **02.11.2021 EP 21205980**

(43) Veröffentlichungstag der Anmeldung:
**11.09.2024 Patentblatt 2024/37**

(73) Patentinhaber: **Merck Patent GmbH**
**64293 Darmstadt (DE)**

(72) Erfinder:
- **PARHAM, Amir Hossain**
  **64293 DARMSTADT (DE)**
- **EHRENREICH, Christian**
  **64293 DARMSTADT (DE)**

(74) Vertreter: **Merck Patent Association**
**Merck Patent GmbH**
**64271 Darmstadt (DE)**

(56) Entgegenhaltungen:
**WO-A1-2018/060218**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 426 701 B1

## Beschreibung

Technisches Gebiet

Die vorliegende Erfindung betrifft dicyano-substituierte Monoaza- oder Diazadibenzofuranderivate und dicyano-substituierte Monoaza- oder Diazadibenzothiophenderivate sowie elektronische Vorrichtungen enthaltend diese Verbindungen, insbesondere organische Elektrolumineszenzvorrichtungen enthaltend diese Verbindungen als Triplettmatrixmaterialien, gegebenenfalls in Kombination mit einem weiteren Triplettmatrixmaterial und geeigneten phosphoreszierenden Emittern, geeignete Mischungen und Formulierungen.

Stand der Technik

In organischen Elektrolumineszenzvorrichtungen (OLEDs) werden häufig phosphoreszierende metallorganische Komplexe eingesetzt. Generell gibt es bei OLEDs immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Carbazolderivate, Dibenzofuranderivate, Indenocarbazolderivate, Indolocarbazolderivate, Azadibenzofuranderivate und Azadibenzothiophenderivate als Matrixmaterialien für phosphoreszierende Emitter verwendet.

In WO2014097866 wird ein cyanosubstituiertes Hostmaterial H1-12 beschrieben, das eine Monoazadibenzothiophen-Einheit und eine Monoazadibenzofuran-Einheit enthält.

In EP2826781 werden speziell substituierte Diazadibenzofuran-, Diazadibenzothiophen- und Diazadibenzoselenophen-derivate beschrieben und deren Verwendung in organischen Elektrolumineszenzverbindungen.

In KR20170068927 werden monocyanosubstituierte Diazadibenzofurane oder - thiophene beschrieben, sowie deren Verwendung als Hostmaterial in Kombination mit Verbindungen der Formel 2, wie beschrieben.

In CN109912610, WO15105313, WO15108301, WO18060307 werden spezielle Azadibenzofuranderivate oder Azadibenzothiophenderivate beschrieben und deren Verwendung in organischen Elektrolumineszenzverbindungen.

In WO2018060218 werden spezielle Diazadibenzofuran- oder Diazadibenzothiophenderivate beschrieben, die mit zwei Cyanogruppen substituiert sind und deren Verwendung in organischen Elektrolumineszenzverbindungen.

In WO2017115608, WO2019160315 und KR20190141598 werden spezielle Dibenzofuran- oder Dibenzothiophenderivate beschrieben, die mit einer Cyanogruppe oder mit zwei Cyanogruppen substituiert sein können und deren Verwendung in organischen Elektrolumineszenzverbindungen.

In US2017186969 wird eine organische lichtemittierende Vorrichtung beschrieben, wobei in der organischen Schicht spezielle Monoarylamine enthalten sind, die unsubstituiert oder teilweise deuteriert sein können, insbesondere enthalten in einer emittierenden Hilfsschicht.

Spezielle Monoarylamine, die unsubstituiert oder teilweise deuteriert sein können, werden in den Offenlegungsschriften WO2015022051, WO2017148564, WO2018083053 CN112375053, WO2019192954, WO2021156323 und WO21107728 beschrieben.

Generell besteht bei diesen Materialien für die Verwendung als Matrixmaterialien noch Verbesserungsbedarf. Aufgabe der vorliegenden Erfindung ist die Bereitstellung von Verbindungen, welche sich insbesondere für den Einsatz als Matrixmaterial in einer phosphoreszierenden OLED eignen. Insbesondere ist es die Aufgabe der vorliegenden Erfindung, Matrixmaterialien bereitzustellen, die zu einer verbesserten Lebensdauer führen. Dies gilt insbesondere für die Verwendung einer geringen bis mittleren Emitterkonzentration, d. h. Emitterkonzentrationen in der Größenordnung von 3 bis 20 %, insbesondere von 3 bis 15 %, da insbesondere hier die Devicelebensdauer limitiert ist.

Es wurde nun gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz der Verbindungen als Matrixmaterial für phosphoreszierende Dotanden.

Es wurde weiterhin gefunden, dass die Kombination mindestens einer Verbindung der Formel (1) als erstes Hostmaterial und mindestens einer lochtransportierenden Verbindung der Formel (2) als zweites Hostmaterial in einer Licht emittierenden Schicht einer organischen elektrolumineszierenden Vorrichtung, diese Aufgabe lösen und die Nachteile aus dem Stand der Technik beseitigen.

Zusammenfassung der Erfindung

Ein erster Gegenstand der vorliegenden Erfindung ist eine Verbindung gemäß Formel (1),

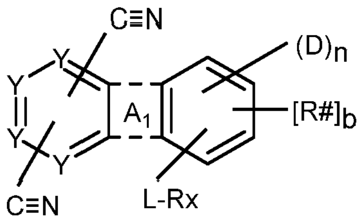

Formel (1),

wobei für die verwendeten Symbole und Indizes gilt:
Ring $A_1$ in Formel (1) entspricht der Formel (1A)

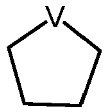

Formel (1A) ;

- Y ist bei jedem Auftreten unabhängig voneinander N, C oder CR+, wobei mindestens ein Y für N steht und mindestens zwei Y für C stehen, an denen die CN-Gruppe gebunden ist; stehen zwei Y für N, so sind diese durch mindestens eine Gruppe C-CN voneinander getrennt;
- V ist O oder S;
- L ist eine Einfachbindung oder ein aromatisches oder heteroaromatisches Ringsystem mit 5 bis 30 Ringatomen, das unsubstituiert oder teilweise oder vollständig mit D substituiert sein kann, mit der Bedingung, dass das heteroaromatische Ringsystem eine über das N-Atom gebundene Carbazolgruppe ausschließt
- Rx ist ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann, mit der Bedingung, dass das Ringsystem und daran gebundene Substituenten keine N-gebundene Carbazolgruppe enthält;
- R+ ist ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann;
- R# ist bei jedem Auftreten gleich oder verschieden ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann, mit der Bedingung, dass das Ringsystem und daran gebundene Substituenten keine N-gebundene Carbazolgruppe enthält;
- $R^2$ ist bei jedem Auftreten gleich oder verschieden ausgewählt aus der Gruppe bestehend aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^2$ miteinander ein mono- oder polycyclisches, aliphatisches Ringsystem bilden;
- b ist 0 oder 1;
- n ist 0, 1, 2 oder 3.

Ein weiterer Gegenstand der Erfindung ist eine Mischung, enthaltend mindestens eine Verbindung nach Formel (1) wie zuvor beschrieben oder später bevorzugt beschrieben und mindestens eine weitere Verbindung, ausgewählt aus der Gruppe der Matrixmaterialien, der phosphoreszierenden Emitter, der fluoreszierenden Emitter und/oder der Emitter, die TADF (thermally activated delayed fluorescence) zeigen.

Ein weiterer Gegenstand der Erfindung ist eine Formulierung, enthaltend mindestens eine Verbindung nach Formel (1), wie zuvor beschrieben oder später bevorzugt beschrieben, oder eine Mischung, wie zuvor beschrieben, und mindestens ein Lösemittel.

Ein weiterer Gegenstand der Erfindung ist eine organische elektrolumineszierende Vorrichtung umfassend eine Anode, eine Kathode und mindestens eine organische Schicht, enthaltend mindestens eine Verbindung nach Formel (1), wie zuvor beschrieben oder später bevorzugt beschrieben.

Ein weiterer Gegenstand der Erfindung ist ein Verfahren zur Herstellung einer organischen elektrolumineszierenden Vorrichtung, wie zuvor beschrieben oder nachfolgend bevorzugt beschrieben, dadurch gekennzeichnet, dass die

organische Schicht durch Gasphasenabscheidung oder aus Lösung aufgebracht wird.

Beschreibung der Erfindung

In der vorliegenden Patentanmeldung bezeichnet "D" oder "D-Atom" Deuterium.

Eine Arylgruppe im Sinne dieser Erfindung enthält 6 bis 40 Ringatome, bevorzugt C-Atome. Eine Heteroarylgruppe im Sinne dieser Erfindung enthält 5 bis 40 Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise abgeleitet von Naphthalin, Anthracen, Phenanthren, Chinolin oder Isochinolin, verstanden. Eine Arylgruppe mit 6 bis 18 C-Atomen ist daher vorzugsweise Phenyl, Naphthyl, Phenanthryl oder Triphenylenyl, wobei die Anbindung der Arylgruppe als Substituent dabei nicht eingeschränkt ist. Die Aryl- oder Heteroarylgruppe im Sinne dieser Erfindung kann einen oder mehrere Reste tragen, wobei der geeignete Rest nachfolgend beschrieben wird. Ist kein derartiger Rest beschrieben, so ist die Arylgruppe oder Heteroarylgruppe nicht substituiert.

Ein aromatisches Ringsystem im Sinne dieser Erfindung enthält 6 bis 40 C-Atome im Ringsystem. Das aromatisches Ringsystem umfasst auch Arylgruppen, wie zuvor beschrieben.

Ein aromatisches Ringsystem mit 6 bis 18 C-Atomen wird vorzugsweise aus Phenyl, vollständig deuteriertes Phenyl, Biphenyl, Naphthyl, Phenanthryl und Triphenylenyl ausgewählt.

Ein heteroaromatisches Ringsystem im Sinne dieser Erfindung enthält 5 bis 40 Ringatome und mindestens ein Heteroatom. Ein bevorzugtes heteroaromatisches Ringsystem hat 9 bis 40 Ringatome und mindestens ein Heteroatom. Das heteroaromatische Ringsystem umfasst auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C- oder O-Atom oder eine Carbonylgruppe, unterbrochen sein können. So sollen beispielsweise auch Systeme wie 9,9'-Spirobifluoren, 9,9-Dialkylfluoren, 9,9-Diarylfluoren, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls von der Definition des aromatischen bzw. heteroaromatischen Ringsystems umfasst.

Unter einem aromatischen oder heteroaromatischen Ringsystem mit 5 - 40 Ringatomen, welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Abkürzung Ar bedeutet bei jedem Auftreten gleich oder verschieden ein aromatisches oder heteroaromatisches Ringsystem mit 5 bis 40 Ringatomen, welches mit einem oder mehreren Resten $R^7$ substituiert sein kann, wobei der Rest $R^7$ oder die Substituenten $R^7$ eine Bedeutung hat/haben, wie zuvor oder nachfolgend beschrieben. Eine bevorzugte Bedeutung von Ar wird nachfolgend beschrieben.

Die Abkürzung $Ar_1$ bedeutet bei jedem Auftreten gleich oder verschieden ein aromatisches oder heteroaromatisches Ringsystem mit 5 bis 30 Ringatomen, das mit einem oder mehreren nicht-aromatischen Resten $R^5$ substituiert sein kann; dabei können zwei Reste $Ar_1$, welche an dasselbe N-Atom, P-Atom oder B-Atom binden, auch durch eine

Einfachbindung oder eine Brücke, ausgewählt aus $C(R^5)_2$, O oder S, miteinander verbrückt sein, wobei der Rest $R^5$ oder die Substituenten $R^5$ eine Bedeutung hat/haben, wie zuvor oder nachfolgend beschrieben. Eine bevorzugte Bedeutung von $Ar_1$ wird nachfolgend beschrieben.

Die Abkürzung $Ar_5$ steht gleich oder verschieden bei jedem Auftreten für ein aromatisches oder heteroaromatisches Ringsystem mit 5 bis 40 Ringatomen, welches mit einem oder mehreren Resten $R^7$ substituiert sein kann, wobei der Rest $R^7$ oder die Substituenten $R^7$ eine Bedeutung hat/haben, wie zuvor oder nachfolgend beschrieben. Eine bevorzugte Bedeutung von $Ar_5$ wird nachfolgend beschrieben.

Unter einer cyclischen Alkyl-, Alkoxy- oder Thioalkylgruppe im Sinne dieser Erfindung wird eine monocyclische, eine bicyclische oder eine polycyclische Gruppe verstanden.

Im Rahmen der vorliegenden Erfindung werden unter einer geradkettigen, verzweigten oder cyclischen $C_1$- bis $C_{20}$-Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden.

Unter einer geradkettigen oder verzweigten $C_1$- bis $C_{20}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einer geradkettigen $C_1$- bis $C_{20}$-Thioalkylgruppe werden beispielsweise S-Alkylgruppen verstanden, beispielsweise Thiomethyl, 1-Thioethyl, 1-Thio-i-propyl, 1-Thio-n-propoyl, 1-Thio-i-butyl, 1-Thio-n-butyl oder 1-Thio-t-butyl.

Eine Aryloxy- oder Heteroaryloxygruppe mit 5 bis 40 aromatischen Ringatomen bedeutet O-Aryl oder O-Heteroaryl und bedeutet, dass die Aryl- bzw. Heteroarylgruppe über ein Sauerstoffatom gebunden wird, wobei die Aryl- bzw. Heteroarylgruppe eine Bedeutung hat, wie zuvor beschrieben.

Unter der Formulierung, dass zwei oder mehr Reste miteinander ein Ringsystem bilden können, wird die Bildung eines aliphatischen, heteroaliphatischen, aromatischen oder heteroaromatischen Ringsystems verstanden, und es soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

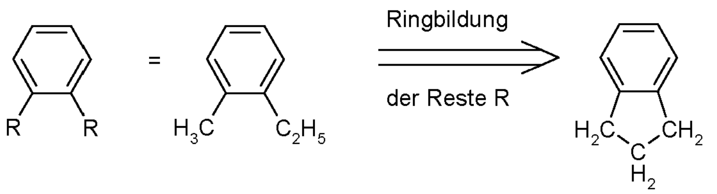


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

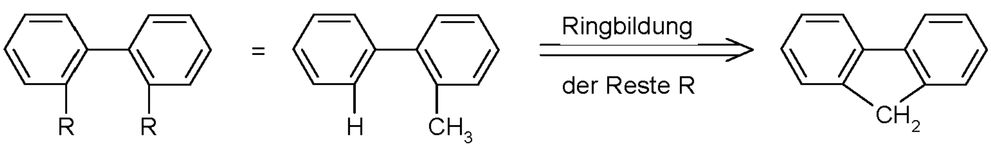


Im Folgenden werden die Verbindungen der Formel (1) und deren bevorzugte Ausführungsformen beschrieben. Die bevorzugten Ausführungsformen gelten auch für die erfindungsgemäße Mischung, erfindungsgemäße Formulierung und erfindungsgemäße organische elektrolumineszierende Vorrichtung.

In Verbindungen der Formel (1) ist Y bei jedem Auftreten unabhängig voneinander N, C oder CR+, wobei mindestens ein Y für N steht und mindestens zwei Y für C stehen, an denen die CN-Gruppe gebunden ist; stehen zwei Y für N, so sind diese durch mindestens eine Gruppe C-CN voneinander getrennt.

Bevorzugte Ausführungsformen der Verbindungen der Formel (1) sind Verbindungen der Formeln (1a), (1b),

(1c), (1d), (1e) oder (1f), wobei die verwendeten Symbole V, L, R+, b, Rx, R# und n eine zuvor angegebene oder nachfolgend bevorzugt genannte Bedeutung haben,

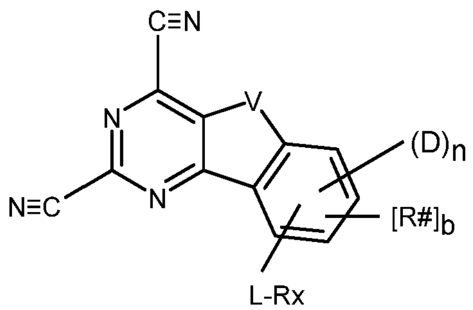

Formel (1a),

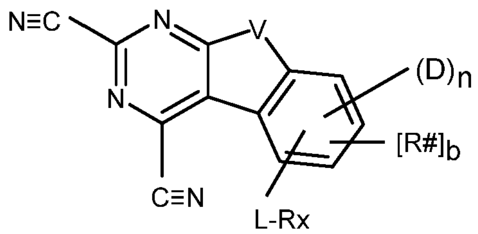

Formel (1b),

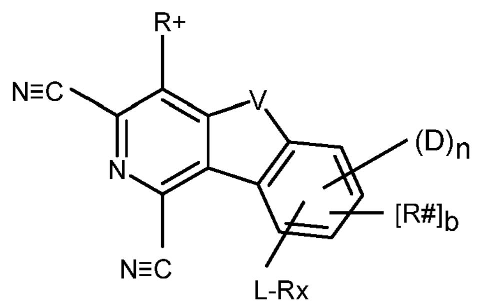

Formel (1c),

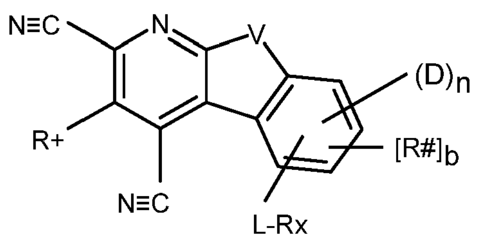

Formel (1d),

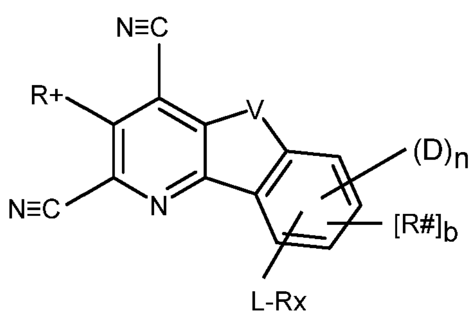

Formel (1e),

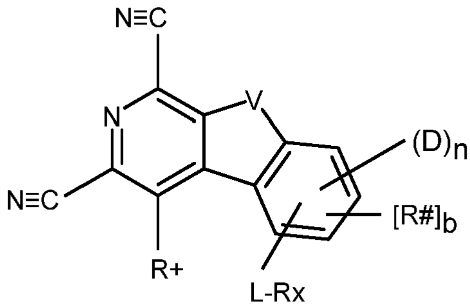

Formel (1f).

Ein weiterer Gegenstand der Erfindung sind demzufolge Verbindungen der Formeln (1a), (1b), (1c), (1d), (1e) oder (1f), wie zuvor beschrieben oder nachfolgend bevorzugt beschrieben.

Bevorzugte Verbindungen der Formel (1) entsprechen den Formeln (1a), (1b), (1d) und (1e).

Besonders bevorzugte Verbindungen der Formel (1) entsprechen den Formeln (1a) oder (1b). Ganz besonders bevorzugte Verbindungen der Formel (1) entsprechen der Formel (1a).

In Verbindungen der Formeln (1), (1a), (1b), (1c), (1d), (1e) und (1f) steht V bevorzugt für O.

Ein weiterer Gegenstand der Erfindung sind demzufolge Verbindungen der Formeln (1), (1a), (1b), (1c), (1d), (1e) oder (1f), wie zuvor beschrieben oder nachfolgend bevorzugt beschrieben, in denen V O bedeutet.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist n 0, 1, 2 oder 3, bevorzugt 0, 1 oder 2, besonders bevorzugt 0 oder 1 und ganz besonders bevorzugt 0.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R+ bevorzugt ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann, mit der Bedingung, dass das Ringsystem und daran gebundene Substituenten keine N-gebundene Carbazolgruppe enthält und wobei der Substituent $R^2$ eine zuvor genannte oder nachfolgend bevorzugt genannte Bedeutung hat.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R+ bei Auftreten bevorzugt Phenyl, Dibenzofuranyl, Carbazol-N-yl oder N-Aryl-carbazolyl, die mit einem oder mehreren Resten $R^2$ substituiert sein kann/können, wobei die Abkürzung "Aryl" ein aromatisches oder heteroaromatisches Ringsystem mit 5 bis 30 Ringatomen bedeutet, das mit einem oder mehreren Resten $R^2$ substituiert sein kann, wobei der Substituent $R^2$ eine zuvor genannte oder nachfolgend bevorzugt genannte Bedeutung hat. Bevorzugt ist "Aryl" Phenyl, 1,3-Biphenyl, 1-4-Biphenyl, Dibenzofuranyl oder Dibenzothiophenyl. Besonders bevorzugt ist "Aryl" Phenyl.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R+ bei Auftreten besonders bevorzugt Phenyl, Dibenzofuranyl oder N-Aryl-carbazolyl, die mit einem oder mehreren Resten $R^2$ substituiert sein kann/können, wobei die Abkürzung "Aryl" ein aromatisches oder heteroaromatisches Ringsystem mit 5 bis 30 Ringatomen bedeutet, das mit einem oder mehreren Resten $R^2$ substituiert sein kann, wobei der Substituent $R^2$ eine zuvor genannte oder nachfolgend bevorzugt genannte Bedeutung hat, mit der Bedingung, dass an "Aryl" gebundene Substituenten $R^2$ keine N-gebundene Carbazolgruppe bedeuten. Bevorzugt ist "Aryl" Phenyl, 1,3-Biphenyl, 1-4-Biphenyl, Dibenzofuranyl oder Dibenzothiophenyl. Besonders bevorzugt ist "Aryl" Phenyl.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R+ bei Auftreten ganz besonders bevorzugt Phenyl, Dibenzofuran-1-yl, Dibenzofuran-3-yl oder N-Phenyl-carbazol-3-yl.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R# bei Auftreten bevorzugt ein aromatisches Ringsystem mit 6 bis 18 Ringatomen, das mit einem oder mehreren Resten $R^2$ substituiert sein kann oder ein heteroaromatisches Ringsystem mit 9 bis 13 Ringatomen, das mit einem oder mehreren Resten $R^2$ substituiert sein kann, mit der Bedingung, dass das Ringsystem und daran gebundene Substituenten keine N-gebundene Carbazolgruppe enthält, wobei der Substituent $R^2$ eine zuvor genannte oder nachfolgend bevorzugt genannte Bedeutung hat. In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R# bei Auftreten besonders bevorzugt Phenyl, 1,3-Biphenyl, 1,4-Biphenyl, Dibenzothiophenyl oder Dibenzofuranyl, die mit einem oder mehreren Resten $R^2$ substituiert sein kann/können. In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) ist R# bei Auftreten ganz besonders bevorzugt 1,3-Biphenyl oder Dibenzofuran-1-yl.

In Verbindungen der Formeln (1), (1a), (1b), (1c), (1d), (1e) und (1f) oder bevorzugt genannten Verbindungen der

Formeln (1), (1a), (1b), (1c), (1d), (1e) und (1f), (1g) ist b bevorzugt 0.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) steht der Substituent Rx bevorzugt für ein aromatisches Ringsystem mit 6 bis 20 Ringatomen oder für ein heteroaromatisches Ringsystem mit 6 bis 21 Ringatomen, das jeweils mit einem oder mehreren Resten $R^2$ substituiert sein kann, wobei der Rest $R^2$ eine zuvor genannte oder nachfolgend bevorzugt genannte Bedeutung hat, mit der Bedingung, dass das Ringsystem und daran gebundene Substituenten keine N-gebundene Carbazolgruppe enthält.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) steht der Substituent Rx besonders bevorzugt für ein aromatisches Ringsystem mit 6 bis 20 Ringatomen, das jeweils mit einem oder mehreren Resten $R^2$ substituiert sein kann, oder für Di-Aryl-Pyridin, Di-Aryl-Pyrimidin, Di-Aryl-Triazin, Chinazolin, Dibenzofuran, Dibenzothiophen oder N-Aryl-Carbazol, die jeweils mit einem Rest $R^2$ oder mehreren Resten $R^2$ substituiert sein können, wobei der Rest $R^2$ eine zuvor genannte oder nachfolgend bevorzugt genannte Bedeutung hat und wobei die Abkürzung "Aryl" ein aromatisches oder heteroaromatisches Ringsystem mit 5 bis 30 Ringatomen bedeutet, das mit einem oder mehreren Resten $R^2$ substituiert sein kann, wobei der Substituent $R^2$ eine zuvor genannte oder nachfolgend bevorzugt genannte Bedeutung hat, mit der Bedingung, dass an "Aryl" gebundene Substituenten $R^2$ keine N-gebundene Carbazolgruppe bedeuten. Bevorzugt ist "Aryl" in Rx Phenyl, 1,3-Biphenyl, 1,4-Biphenyl, Dibenzofuranyl, Dibenzothiopenyl, 9,9-Dimethylfluorenyl oder Triphenylenyl, wobei die Anbindung von "Aryl" an das entsprechende N-Atom des N-Aryl-carbazols nicht eingeschränkt ist, sofern nicht anders bezeichnet.

Ist der Substituent Rx, wie zuvor beschrieben, mit einem oder mehreren Resten $R^2$ substituiert, so wird $R^2$ bevorzugt jeweils unabhängig aus der Gruppe D, CN, eine geradkettige oder verzweigte Alkylgruppe mit 1 bis 20 C-Atomen, Phenyl, 1,4-Biphenyl, 1,3-Biphenyl, N-Aryl-carbazolyl und Dibenzofuranyl ausgewählt, wobei "Aryl" in N-Aryl-carbazolyl eine zuvor genannte Bedeutung oder zuvor genannte bevorzugte Bedeutung hat.

Ist der Substituent Rx, wie zuvor beschrieben, mit einem oder mehreren Resten $R^2$ substituiert, so ist $R^2$ bevorzugt jeweils unabhängig voneinander D, Phenyl oder tert-Butyl. In einer Ausführungsform des Substituenten Rx, wie zuvor beschrieben oder bevorzugt beschrieben, ist dieser Substituent deuteriert. In einer bevorzugten Ausführungsform des Substituenten Rx, wie zuvor beschrieben oder bevorzugt beschrieben, hat der Substituent Rx einen Rest $R^2$ oder zwei Reste $R^2$ oder er ist nicht substituiert, wobei der Rest $R^2$ eine zuvor genannte oder bevorzugt genannte Bedeutung hat. Ein bevorzugtes aromatisches Ringsystem als Rx ist beispielsweise Phenyl, 1,3-Biphenyl, 1,4-Biphenyl, Terphenyl, Spirobifluorenyl, 9,9-Dimethylfluorenyl, 9-Phenyl-9-Methylfluorenyl, Triphenylenyl oder Fluoranthenyl, die mit einem oder mehreren Substituenten $R^2$ substituiert sein können, wobei $R^2$ eine zuvor genannte oder bevorzugt genannte Bedeutung hat.

Rx ist besonders bevorzugt Phenyl, 1,3-Biphenyl, Terphenyl, Spirobifluorenyl, 9,9-Dimethylfluorenyl, Triphenylenyl, Fluoranthenyl, Triphenyleno[1,12-bcd]furanyl, Di-Aryl-Triazinyl, Dibenzofuranyl, Dibenzothiophenyl oder N-Aryl-Carbazolyl, die mit einem oder mehreren Substituenten $R^2$ substituiert sein können, wobei "Aryl" und $R^2$ eine zuvor genannte oder bevorzugt genannte Bedeutung haben.

Rx ist ganz besonders bevorzugt Spirobifluorenyl, Triphenylenyl, Di-Aryl-Triazinyl, Dibenzofuranyl oder Dibenzothiophenyl, die mit einem oder mehreren Substituenten $R^2$ substituiert sein können, wobei $R^2$ eine zuvor genannte oder bevorzugt genannte Bedeutung haben.

Rx ist ganz besonders bevorzugt Triphenylenyl, Di-Aryl-Triazinyl oder Dibenzofuranyl, die mit einem oder mehreren Substituenten $R^2$ substituiert sein können, wobei $R^2$ eine zuvor genannte oder bevorzugt genannte Bedeutung haben.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) steht das Symbol L als Linker für eine Einfachbindung oder ein aromatisches oder heteroaromatisches Ringsystem mit 5 bis 30 Ringatomen, das unsubstituiert oder teilweise oder vollständig mit D substituiert sein kann, mit der Bedingung, dass das heteroaromatische Ringsystem eine über das N-Atom gebundene monovalente Carbazolgruppe ausschließt.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) steht das Symbol L bevorzugt für eine Einfachbindung oder einen Linker ausgewählt aus der Gruppe L-1 bis L-33,

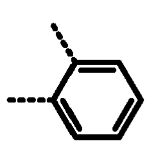 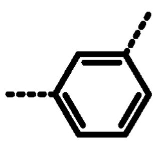 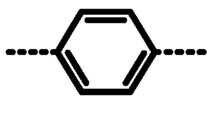 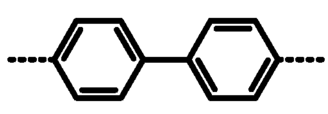

L-1 L-2 L-3 L-4

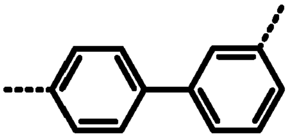
L-5

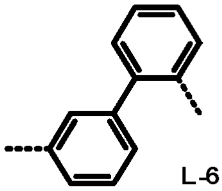
L-6

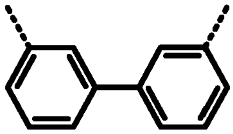
L-7

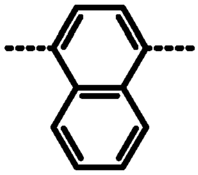
L-8

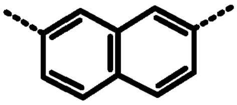
L-9

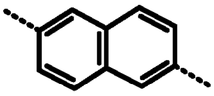
L-10

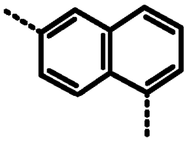
L-11

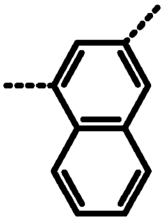
L-12

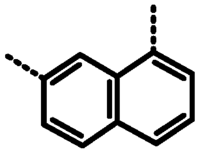
L-13

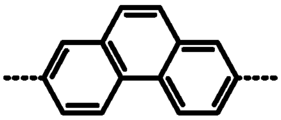
L-14

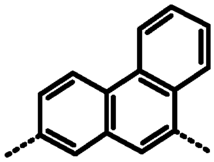
L-15

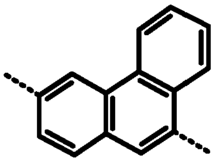
L-16

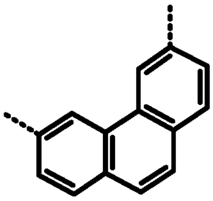
L-17

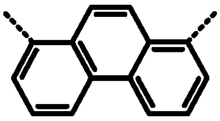
L-18

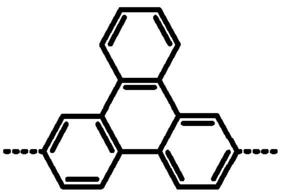
L-19

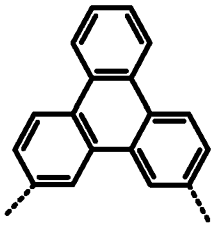
L-20

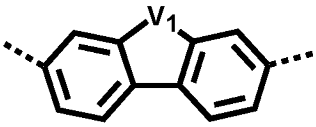
L-21

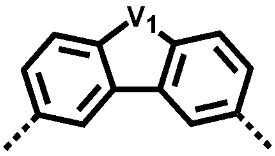
L-22

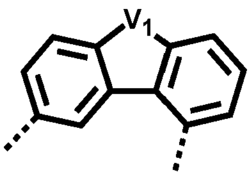
L-23

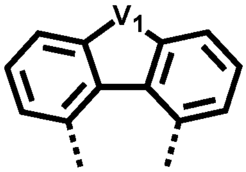
L-24

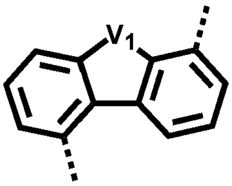
L-25

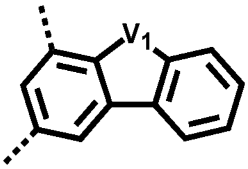
L-26

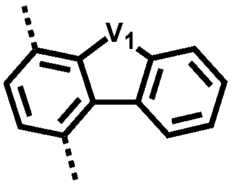
L-27

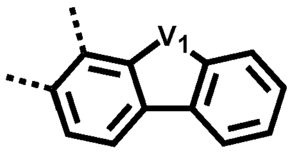
L-28

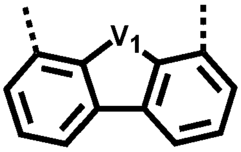

L-29

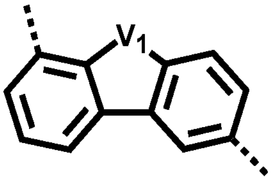

L-30

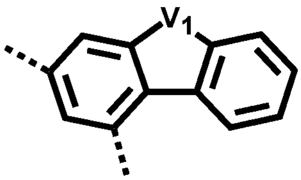

L-31

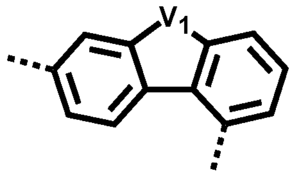

L-32

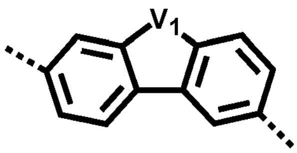

L-33

,

wobei $V_1$ jeweils unabhängig voneinander O, S, $C(R^1)_2$ oder N-Aryl bedeutet, $R^1$ jeweils unabhängig voneinander ausgewählt ist aus Methyl oder Phenyl und "Aryl" eine zuvor angegebene oder bevorzugt angegebene Bedeutung hat und die gestrichelten Linien die Anbindung an Rx und Rest der Formeln (1), (1a), (1b), (1c), (1d), (1e) und (1f) bedeutet. Die Linker L-1 bis L-33 können teilweise oder vollständig deuteriert sein. Bevorzugt ist $V_1$ O oder N-Aryl. Besonders bevorzugt ist $V_1$ O.

In Verbindungen der Formeln (1), (1a), (1b), (1c), (1d), (1e) und (1f) oder bevorzugt genannten Verbindungen der Formeln (1), (1a), (1b), (1c), (1d), (1e) und (1f) steht das Symbol L bevorzugt für eine Einfachbindung oder einen Linker ausgewählt aus der Gruppe L-2, L-3, L-22 und L-23, wie zuvor beschrieben oder bevorzugt beschrieben, besonders bevorzugt für eine Einfachbindung oder L-2, ganz besonders bevorzugt für eine Einfachbindung.

Die Verbindungen der Formeln (1), (1a), (1b), (1c), (1d), (1e) und (1f) oder bevorzugt genannten Verbindungen der Formeln (1), (1a), (1b), (1c), (1d), (1e) und (1f), wie zuvor beschrieben, sind in einer bevorzugten Ausführungsform teilweise oder vollständig deuteriert.

Beispiele für geeignete Hostmaterialien der Formeln (1), (1a), (1b), (1c), (1d), (1e) und (1f) sind die nachstehend genannten Strukturen der Tabelle 1.

Tabelle 1:

| | | |
|---|---|---|
| 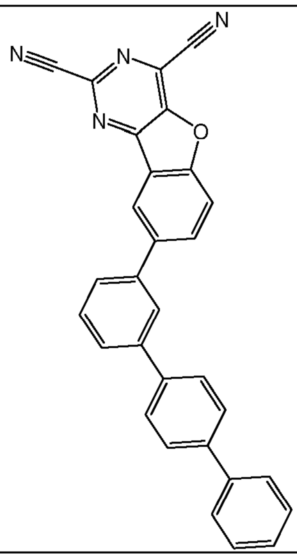 | 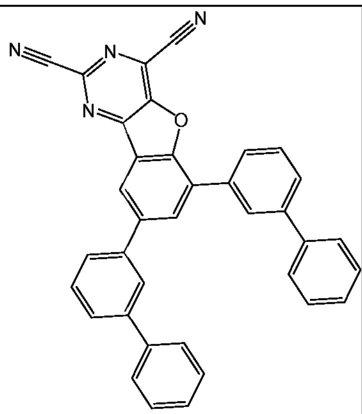 | 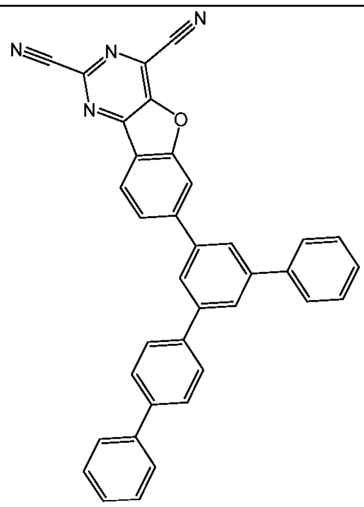 |

| | | |
|---|---|---|
| 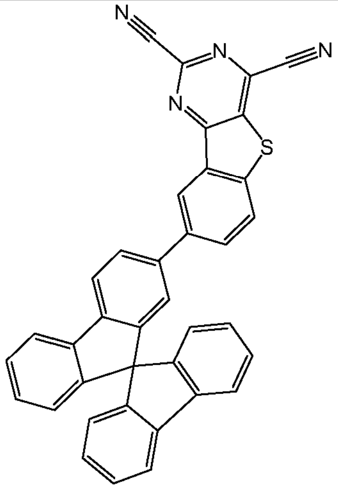 | 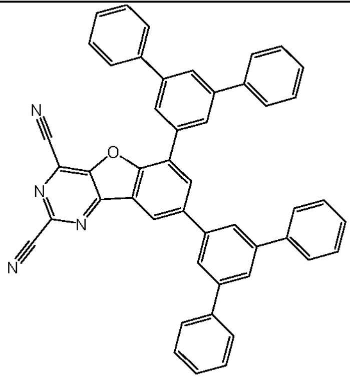 | 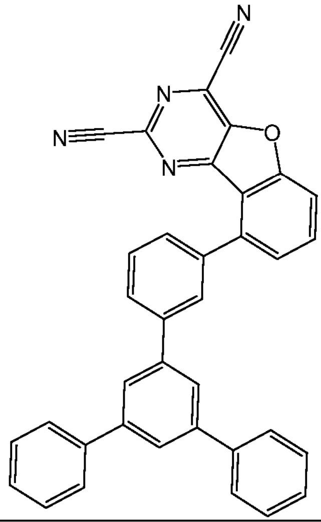 |
| 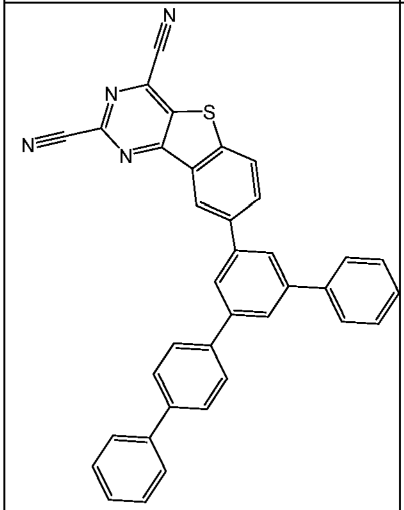 | 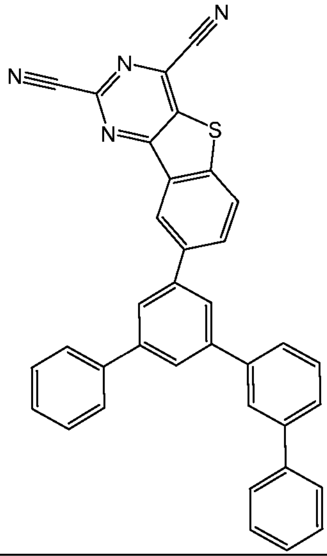 | 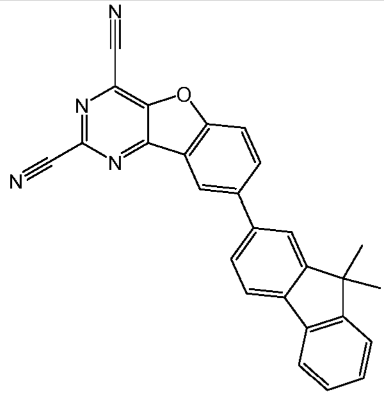 |
| 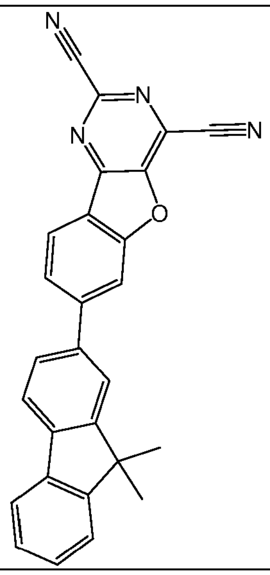 | 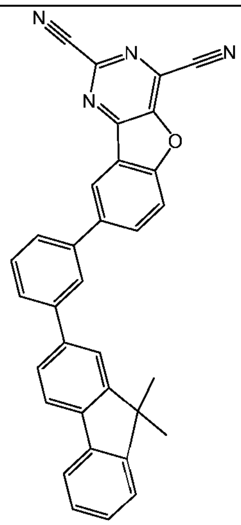 | 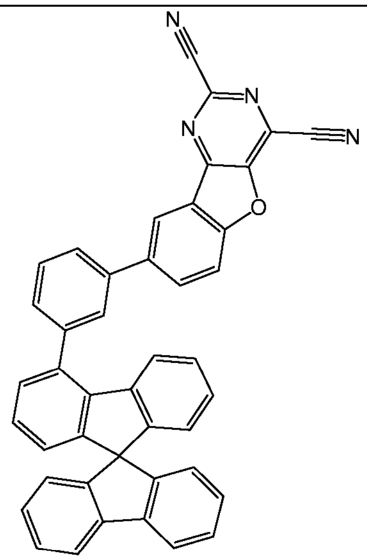 |

| | | |
|---|---|---|
| 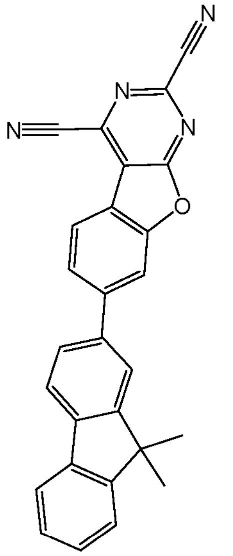 | 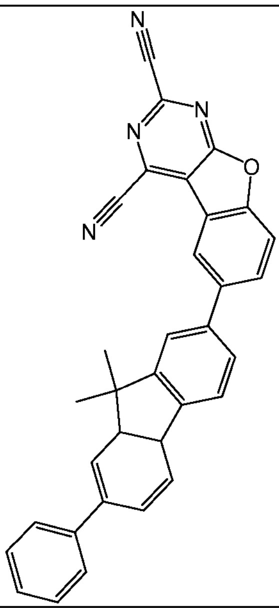 | 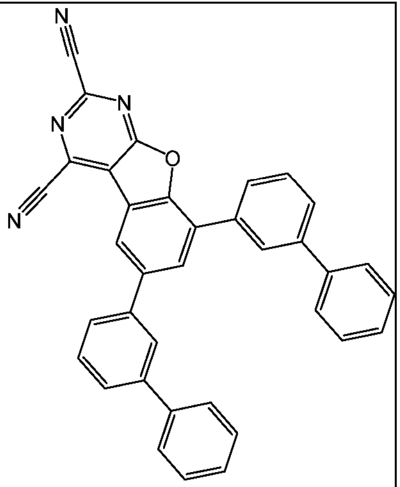 |
| 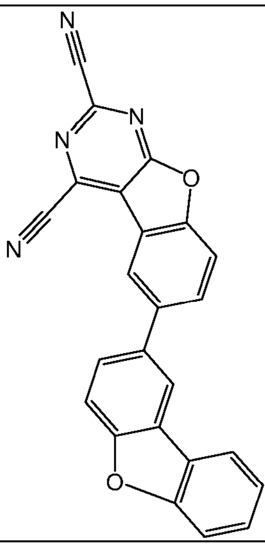 | 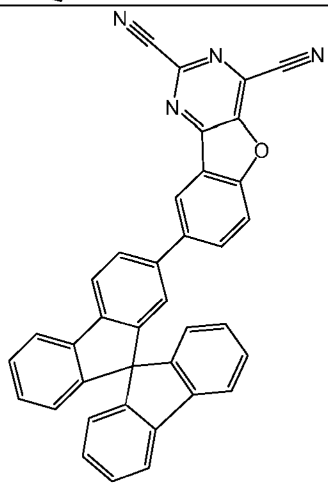 | 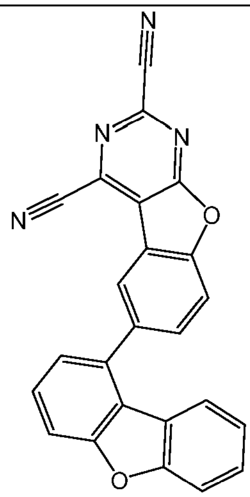 |
| 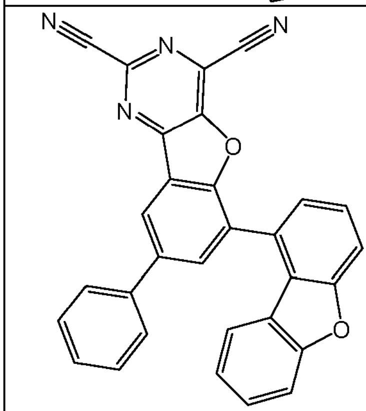 | 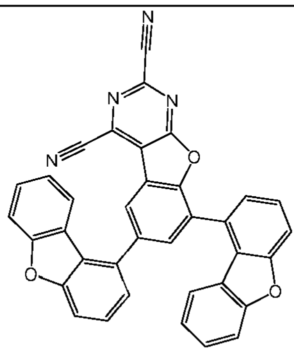 | 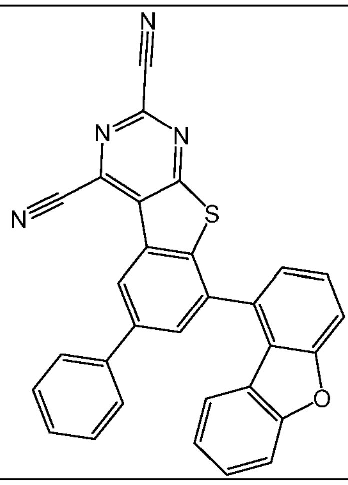 |

| | | |
|---|---|---|
| 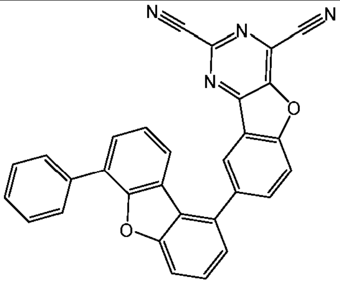 | 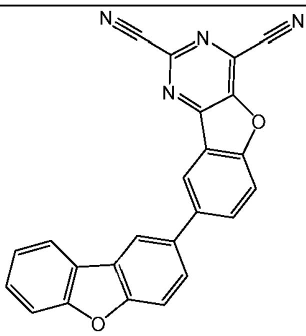 | 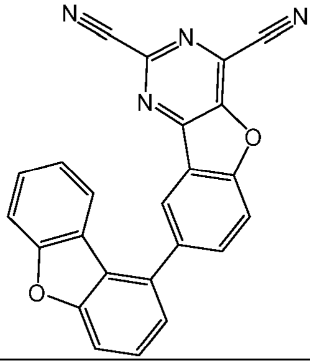 |
| 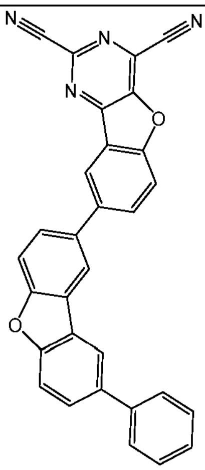 | 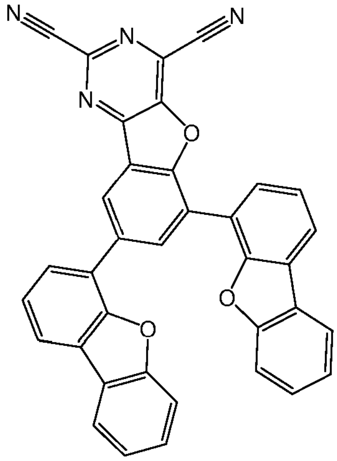 | 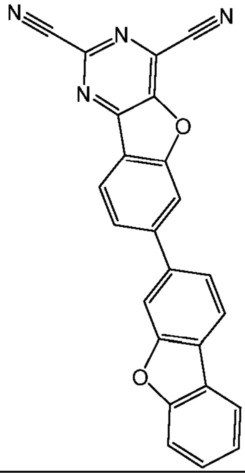 |
| 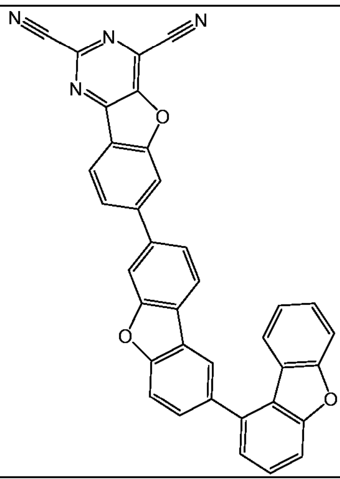 | 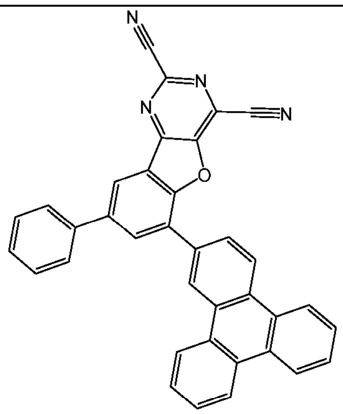 | 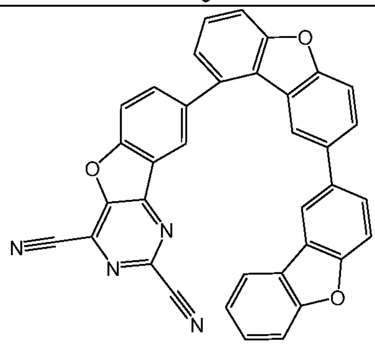 |
| 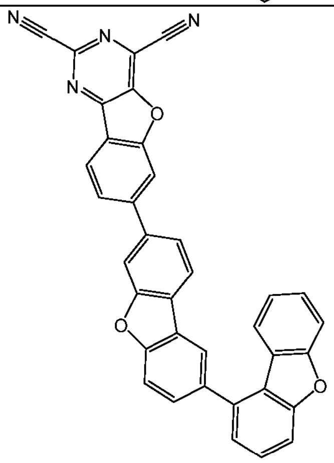 | 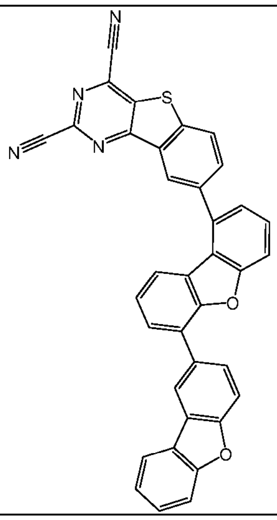 | 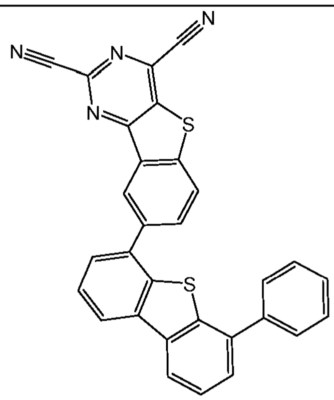 |

| | | |
|---|---|---|
| 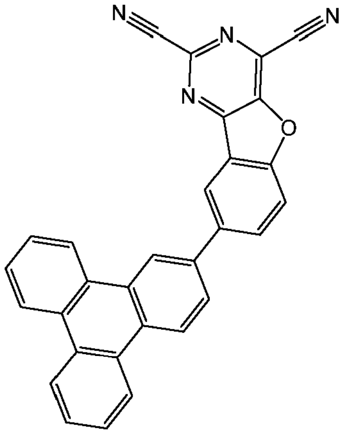 | 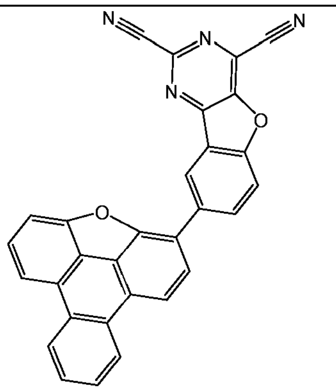 | 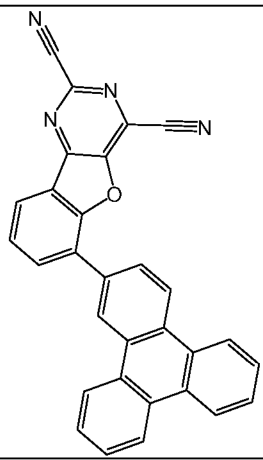 |
| 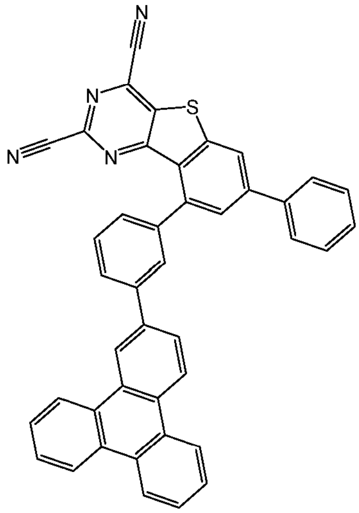 | 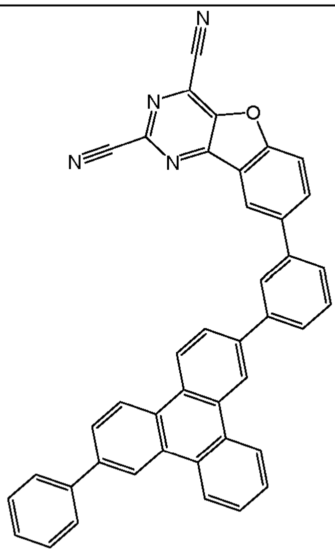 | 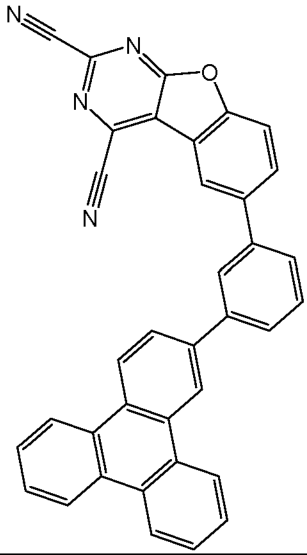 |
| 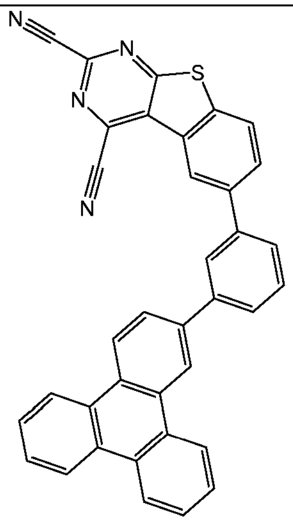 | 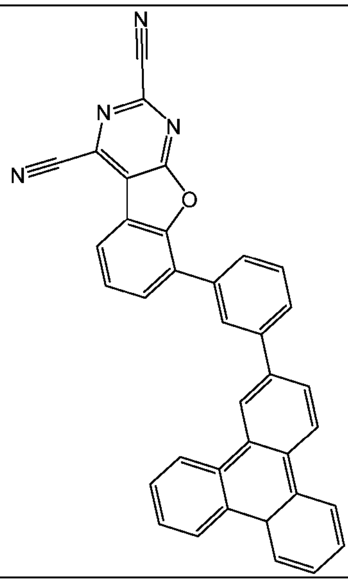 | 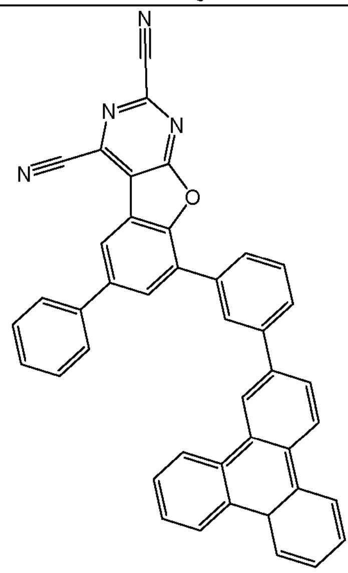 |

| | | |
|---|---|---|
| 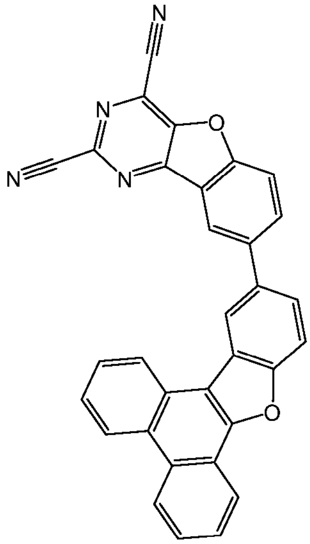 | 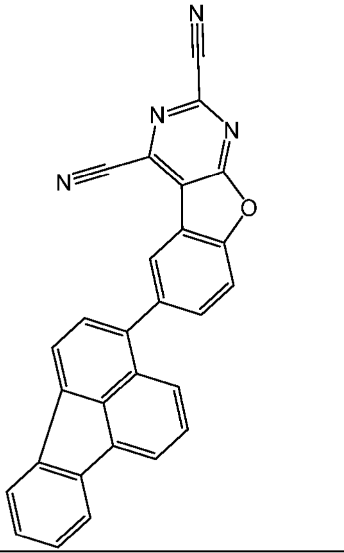 | 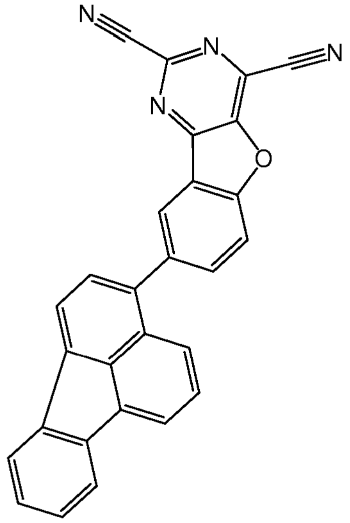 |
| 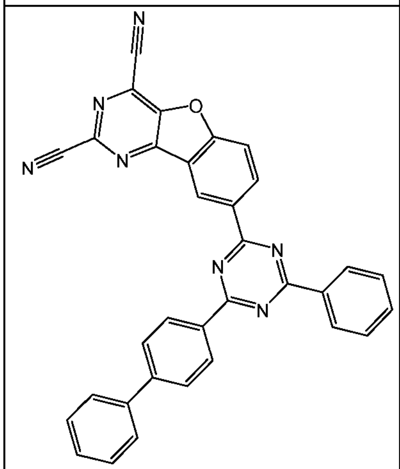 | 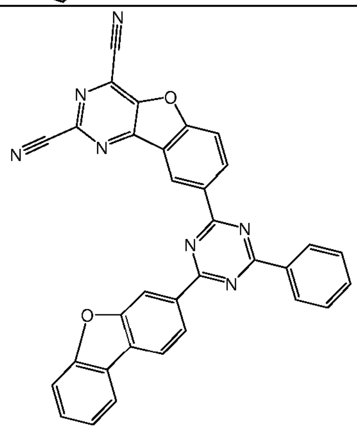 | 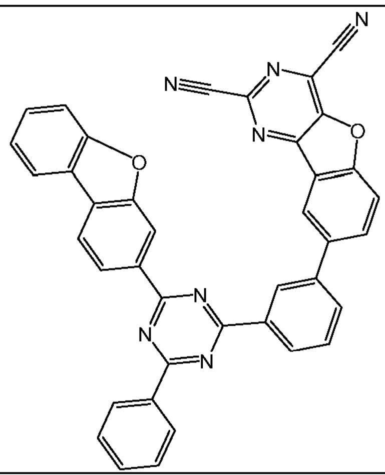 |
| 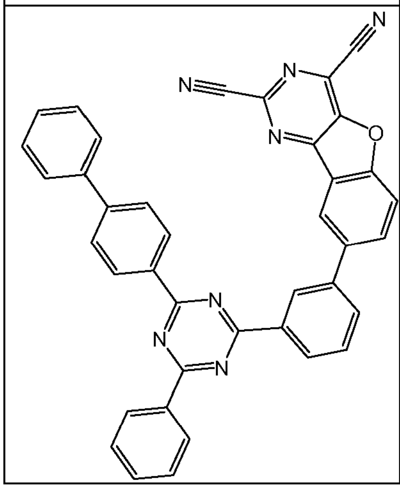 | 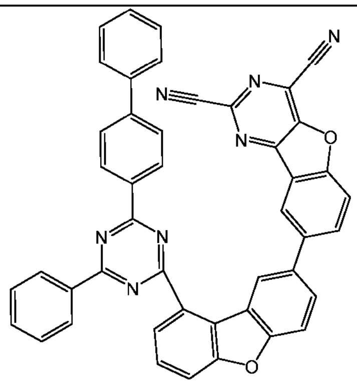 | 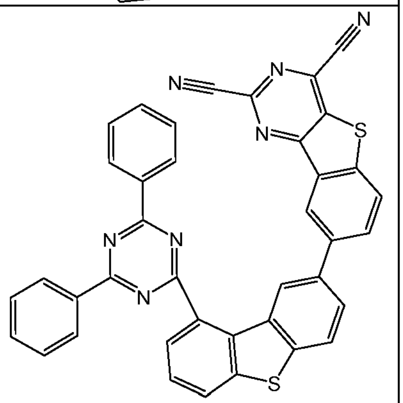 |

| | | |
|---|---|---|
| 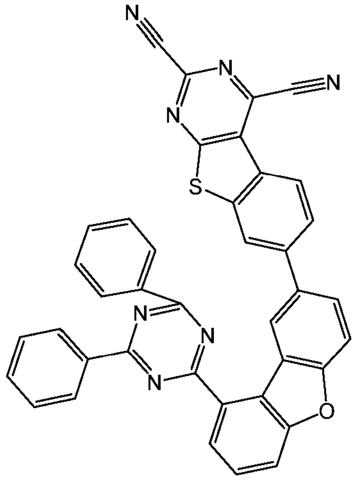 | 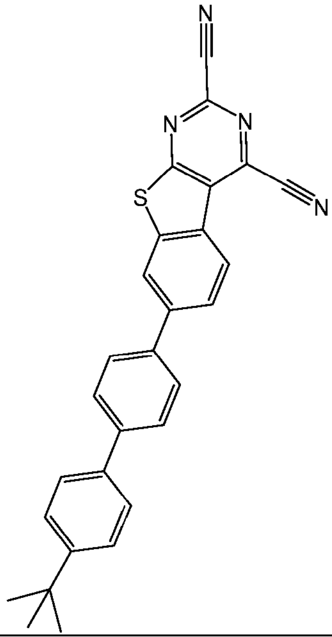 | 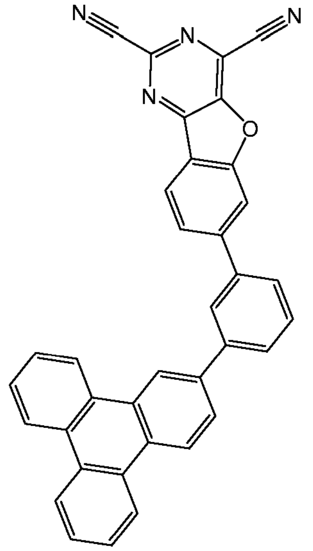 |
| 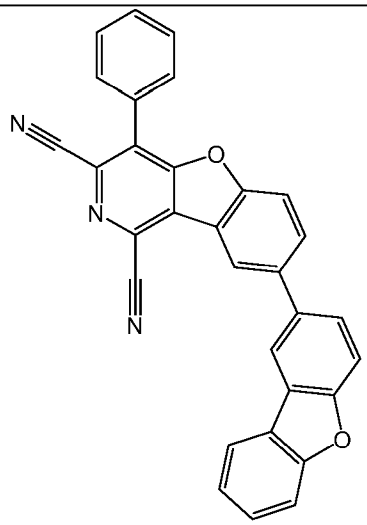 | 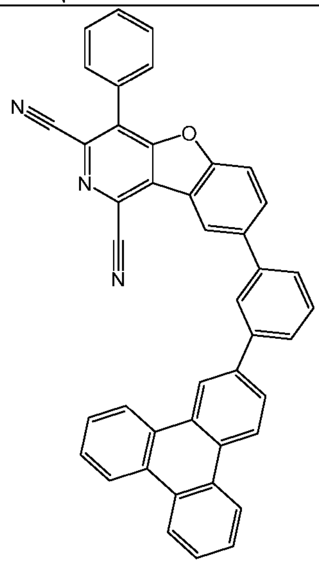 | 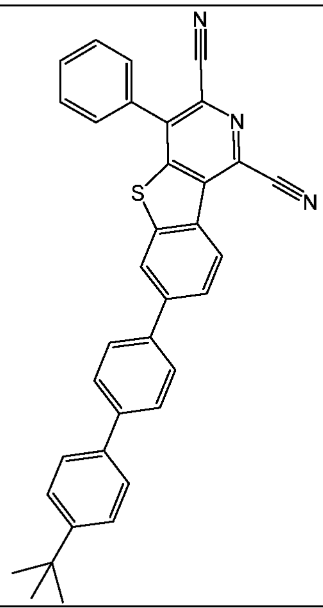 |
| 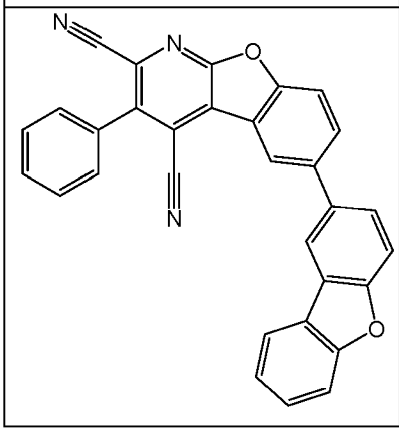 | 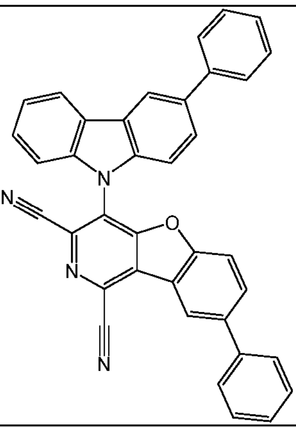 | 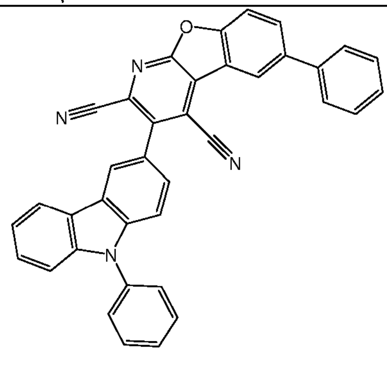 |

| | | |
|---|---|---|
| 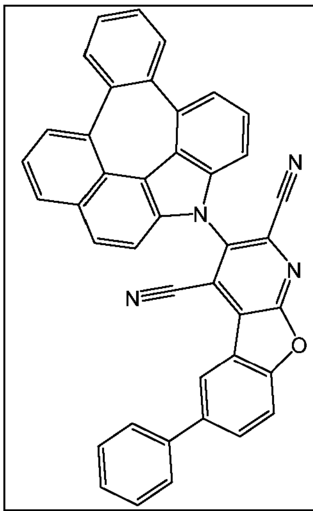 | 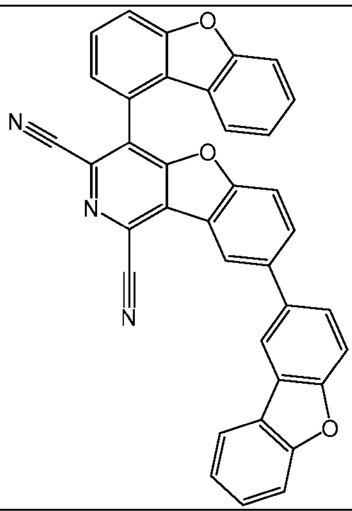 | 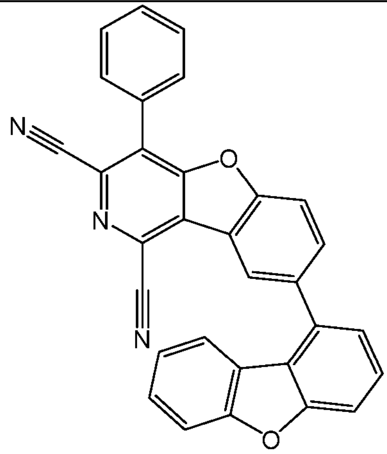 |
| 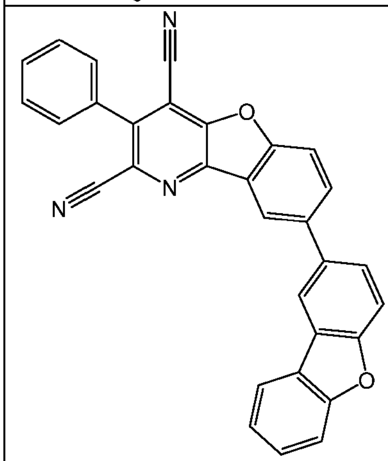 | 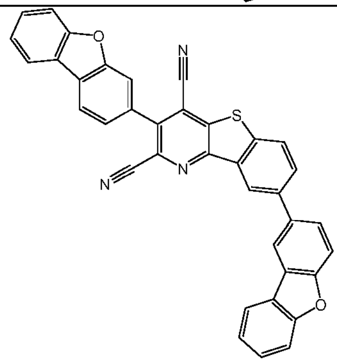 | 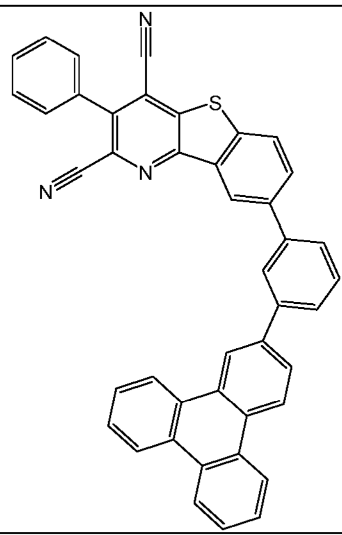 |
| 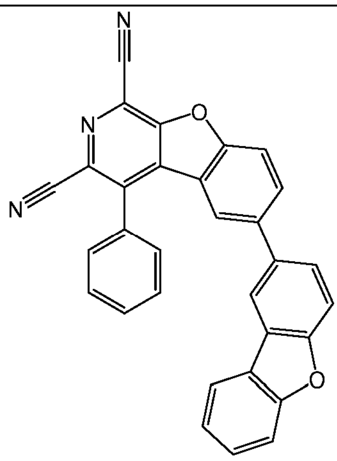 | 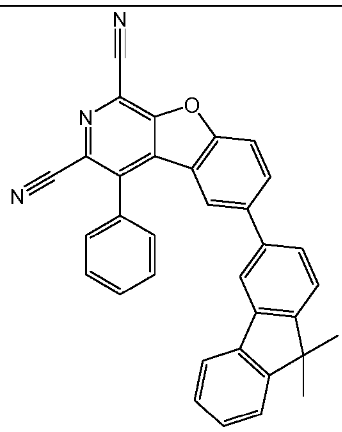 | 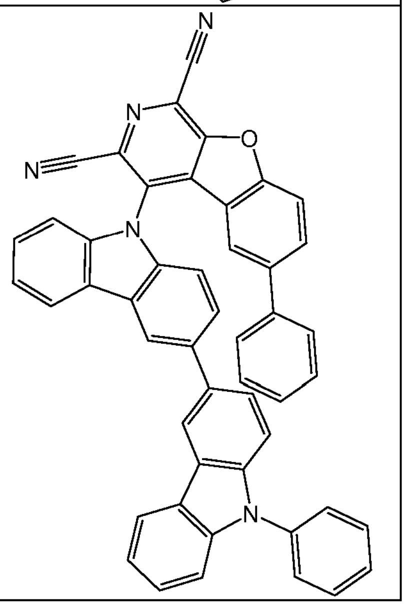 |

| | | |
|---|---|---|
| 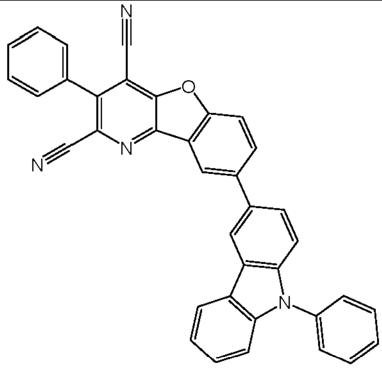 | 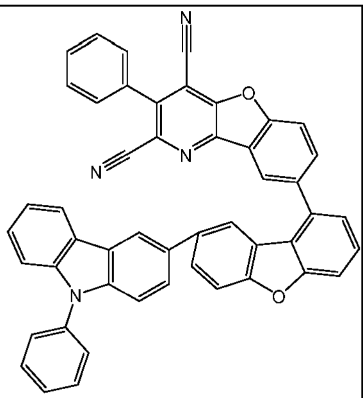 | 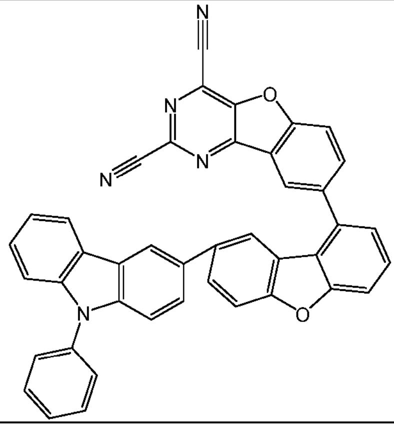 |
| 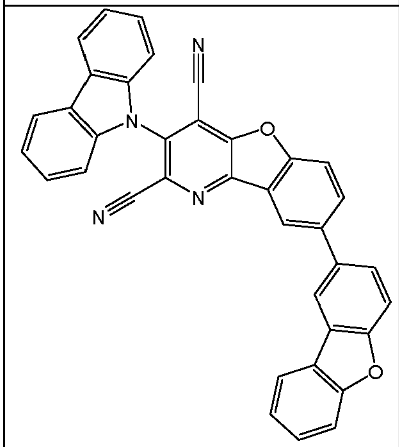 | 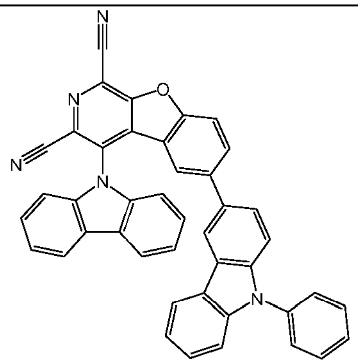 | 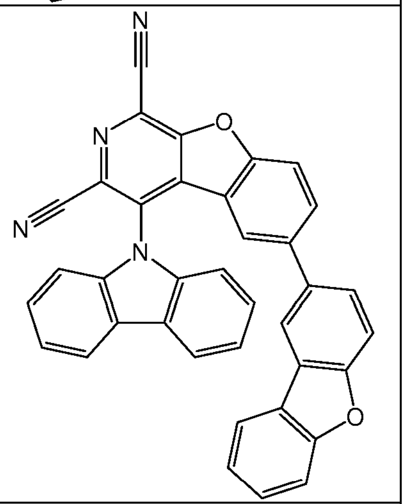 |
| 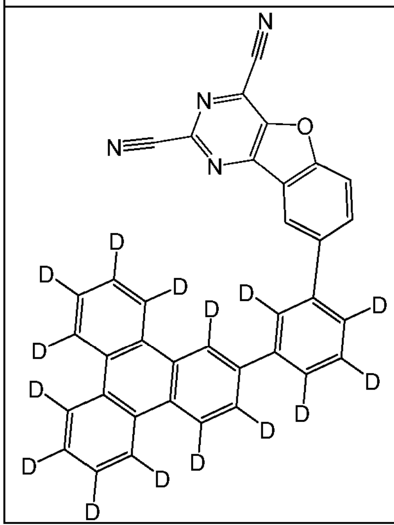 | 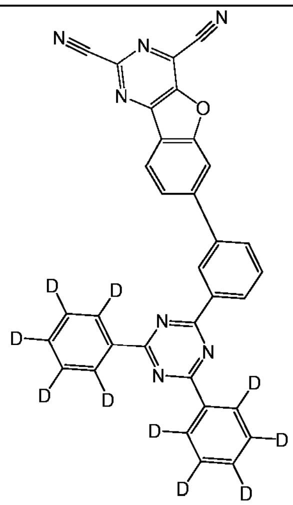 | 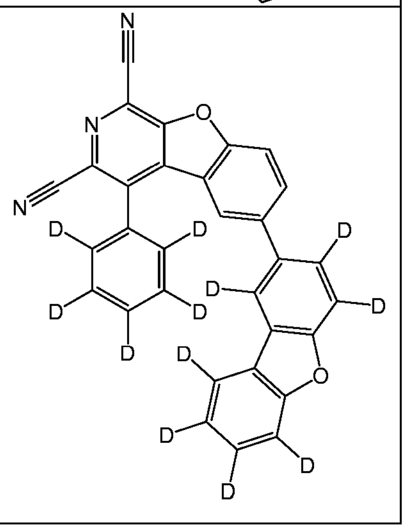 |

Besonders geeignete Verbindungen der Formeln (1), (1a), (1b), (1c), (1d), (1e) und (1f) sind die Verbindungen E1 bis E27 der Tabelle 2.

Tabelle 2:

| | | |
|---|---|---|
| 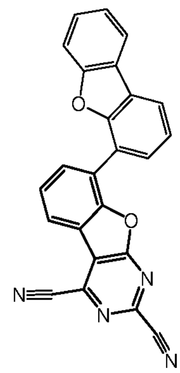 | 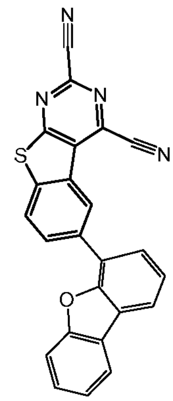 | 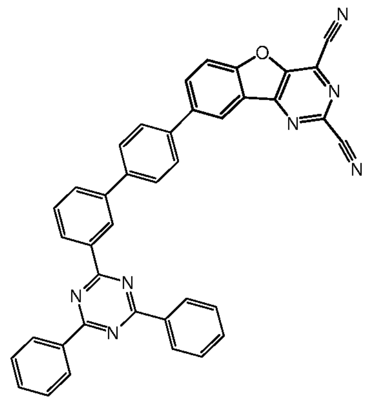 |
| E1 | E2 | E3 |
| 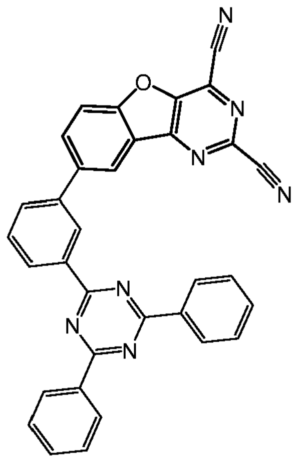 | 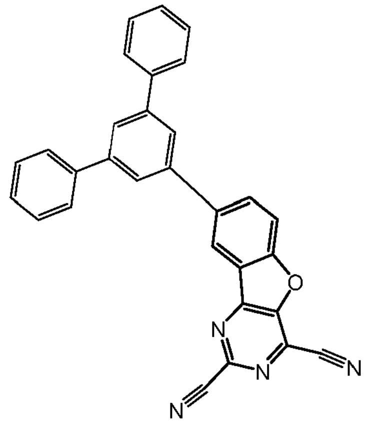 | 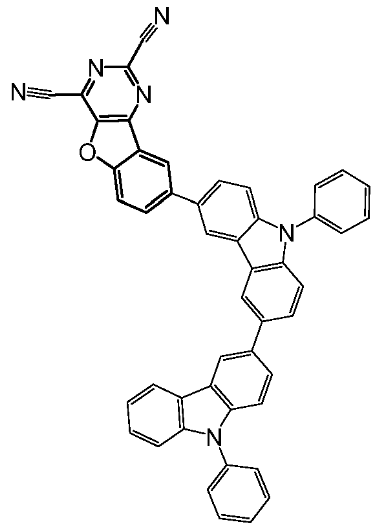 |
| E4 | E5 | E6 |
| 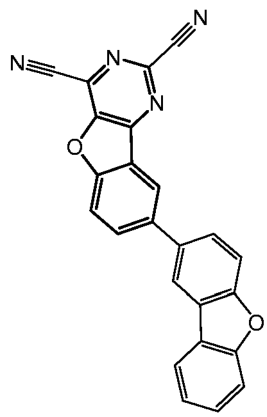 | 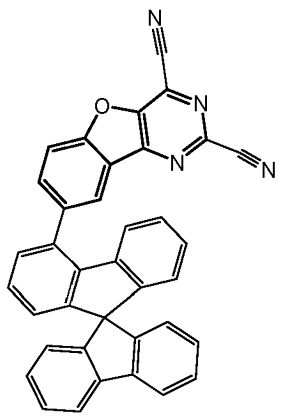 | 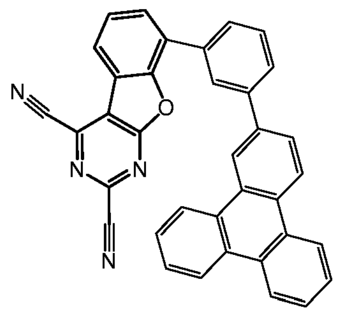 |
| E7 | E8 | E9 |

(fortgesetzt)

| | | |
|---|---|---|
| 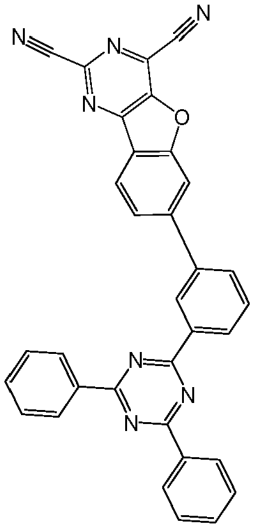 | 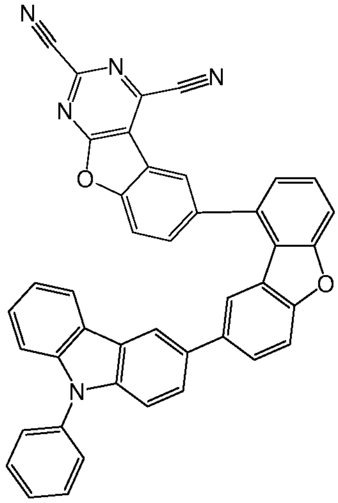 | 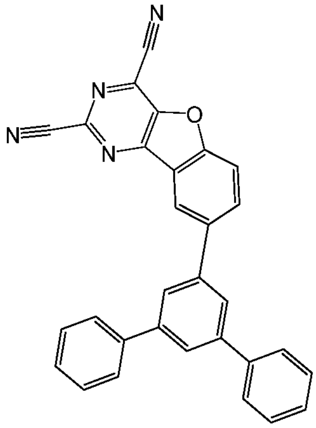 |
| E10 | E11 | E12 |
| 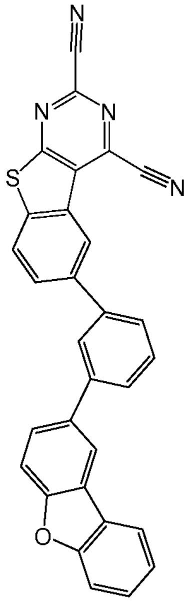 | 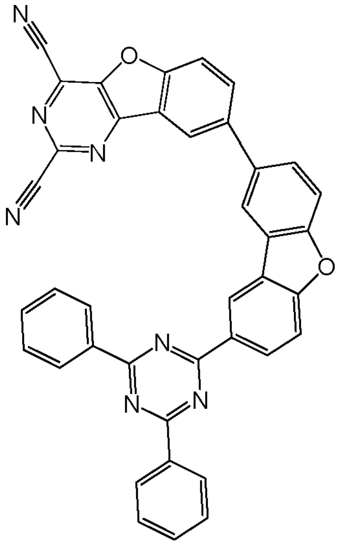 | 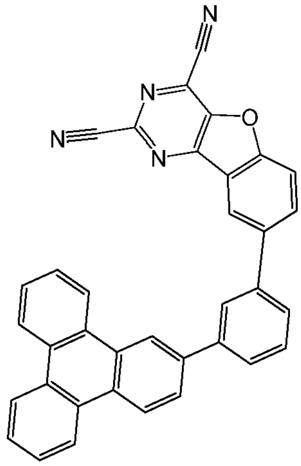 |
| E13 | E14 | E15 |
| 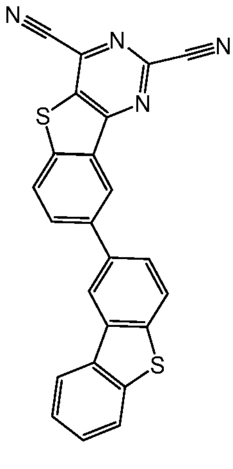 | 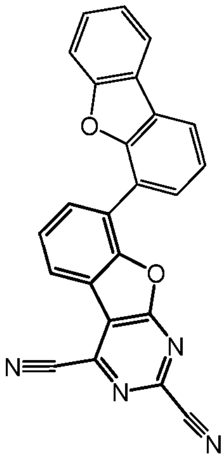 | 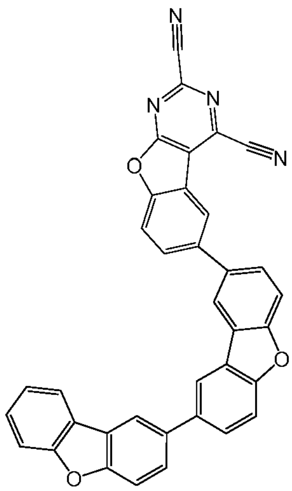 |

(fortgesetzt)

| E16 | E17 | E18 |
|---|---|---|
| 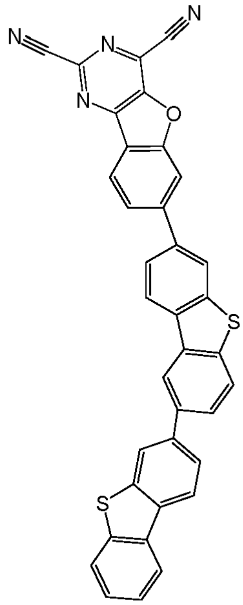 | 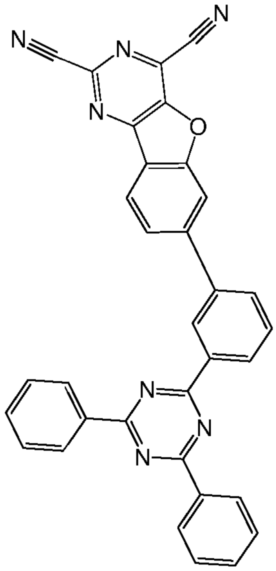 | 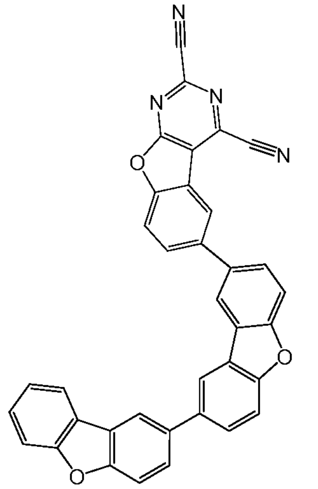 |
| E19 | E20 | E21 |
| 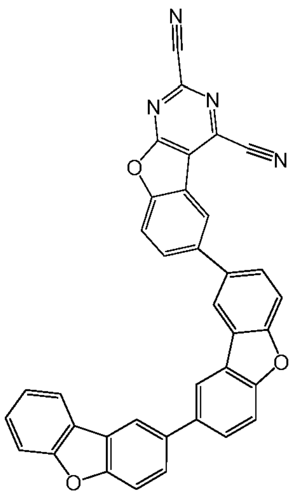 | 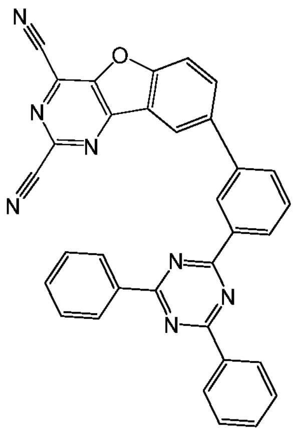 | 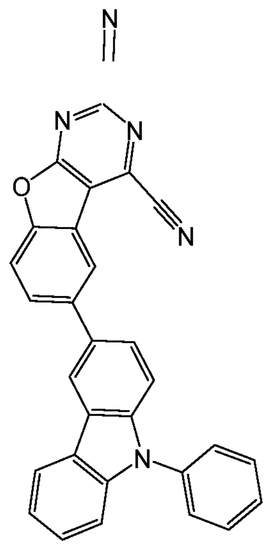 |
| E22 | E23 | E24 |

(fortgesetzt)

| 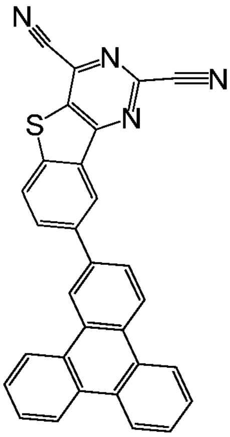 | 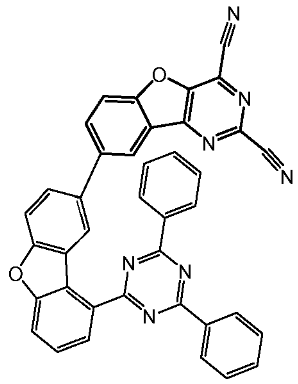 | 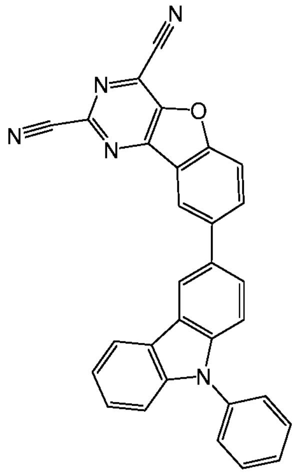 |
| --- | --- | --- |
| E25 | E26 | E27. |

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden.

Geeignete Verbindungen mit einer Azadibenzofuran- oder Azadibenzothiophen-Gruppe können vielfach kommerziell erhalten werden, wobei die in den Beispielen dargelegten Ausgangsverbindungen durch bekannte Verfahren erhältlich sind, so dass hierauf verwiesen wird.

Im den folgenden Syntheseschemata sind die Verbindungen zur Vereinfachung der Strukturen mit einer geringen Anzahl an Substituenten gezeigt. Dies schließt das Vorhandensein von beliebigen weiteren Substituenten in den Verfahren nicht aus. Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Die Herstellung von Vorstufen für Verbindungen der Formel (1) kann beispielsweise nach folgendem Schema 1 erfolgen, wobei V und L-Rx eine der zuvor angegebenen oder bevorzugt angegebenen Bedeutungen haben.

Schema 1:

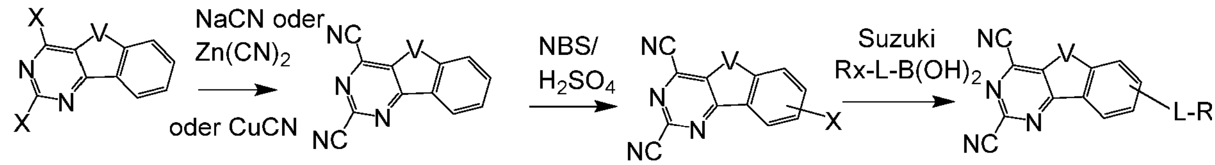


X = Cl Br, I

Kr2017068927
Organic Letters (2007), 9(9), 1711-1714

WO2018060307

WO2018060307

Schema 2:

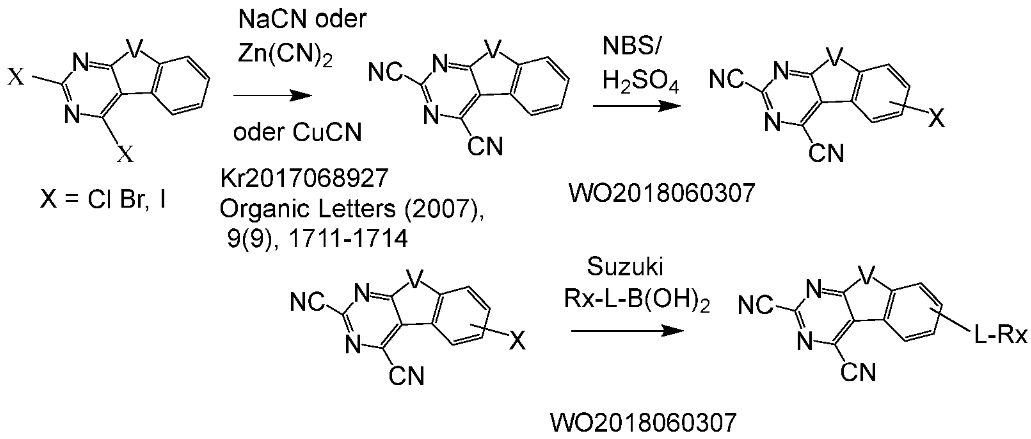


Schema 3:

A)

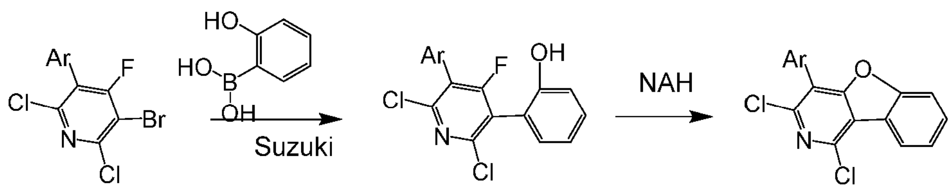


B)

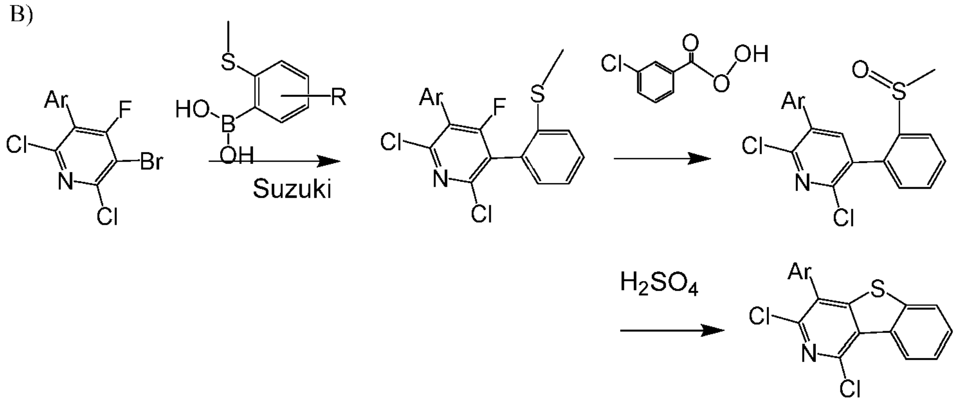


Schema 4:

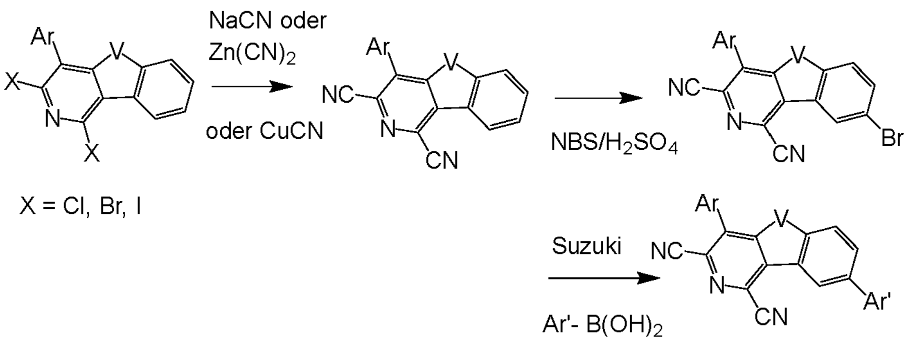

Schema 5:

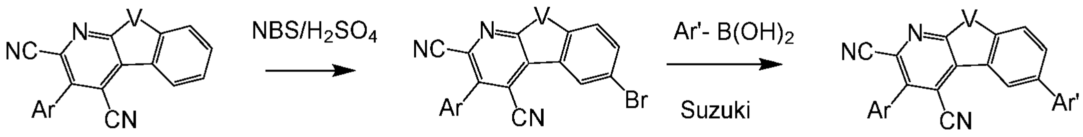


Durch diese Verfahren, gegebenenfalls gefolgt von Aufreinigung, wie z. B. Umkristallisation oder Sublimation, lassen sich die Verbindungen der Formel (1) in hoher Reinheit, bevorzugt mehr als 99 % (bestimmt mittels $^1$H-NMR und/oder HPLC) erhalten.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen oder von Mischungen von erfindungsgemäßen Verbindungen mit weiteren funktionalen Materialien, wie Matrixmaterialien, fluoreszierenden Emittern, phosphoreszierenden Emittern und/oder Emittern die TADF zeigen, erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Die erfindungsgemäßen Verbindungen der Formel (1), wie zuvor beschrieben oder bevorzugt beschrieben, eignen sich für die Verwendung in einer organischen elektrolumineszierenden Vorrichtung, insbesondere als Matrixmaterial.

Wenn die erfindungsgemäße Verbindung als Matrixmaterial oder synonym Hostmaterial in einer emittierenden Schicht eingesetzt wird, wird sie bevorzugt in Kombination mit einer weiteren Verbindung eingesetzt.

Ein weiterer Gegenstand der Erfindung ist daher eine Mischung, enthaltend mindestens eine Verbindung der Formel (1) oder mindestens eine bevorzugte Verbindung einer der Formeln (1), (1a), (1b), (1c), (1d), (1e) und (1f), oder eine Verbindung der Tabelle 1 oder eine der Verbindungen **E1** bis **E27** und mindestens eine weitere Verbindung, ausgewählt aus der Gruppe der Matrixmaterialien, der phosphoreszierenden Emitter, der fluoreszierenden Emitter und/oder der Emitter, die TADF (thermally activated delayed fluorescence) zeigen. Geeignete Matrixmaterialien und Emitter, die in dieser erfindungsgemäßen Mischung verwendet werden können, werden nachfolgend beschrieben.

Ein weiterer Gegenstand der vorliegenden Erfindung ist ebenfalls eine Formulierung, enthaltend mindestens eine erfindungsgemäße Verbindung, wie zuvor beschrieben, oder eine erfindungsgemäße Mischung, wie zuvor beschrieben, und mindestens ein Lösemittel. Das Lösemittel kann ein oben genanntes Lösemittel oder eine Mischung dieser Lösemittel sein.

Ein weiterer Gegenstand der vorliegenden Erfindung ist eine organische elektrolumineszierende Vorrichtung umfassend eine Anode, eine Kathode und mindestens eine organische Schicht, enthaltend mindestens eine Verbindung der Formel (1) oder mindestens eine bevorzugte Verbindung einer der Formeln (1), (1a), (1b), (1c), (1d), (1e) und (1f), oder eine Verbindung der Tabelle 1 oder eine der Verbindungen **E1** bis E27.

Die erfindungsgemäße organische elektrolumineszierende Vorrichtung (synonym dazu organische Elektrolumineszenzvorrichtung) ist beispielsweise ein organischer lichtemittierender Transistor (OLET), ein organisches Feld-Quench-Device (OFQD), eine organische lichtemittierende elektrochemische Zelle (OLEC, LEC, LEEC), eine organische Laserdiode (O-Laser) oder eine organische lichtemittierende Diode (OLED). Die erfindungsgemäße organische elektrolumineszierende Vorrichtung ist insbesondere eine organische lichtemittierende Diode oder eine organische lichtemittierende elektrochemische Zelle. Besonders bevorzugt ist die erfindungsgemäße Vorrichtung eine OLED.

Die organische Schicht der erfindungsgemäßen Vorrichtung enthält bevorzugt neben einer lichtemittierenden Schicht (EML), eine Lochinjektionsschicht (HIL), eine Lochtransportschicht (HTL), eine Lochblockierschicht (HBL), eine Elektronentransportschicht (ETL), eine Elektroneninjektionsschicht (EIL), eine Exzitonenblockierschicht, eine Elektronenblockierschicht und/oder Ladungserzeugungsschichten (Charge-Generation layers). Es können in der erfindungsgemäßen Vorrichtung auch mehrere Schichten dieser Gruppe, bevorzugt ausgewählt aus EML, HIL, HTL, ETL, EIL und HBL, enthalten sein. Ebenso können zwischen zwei emittierende Schichten Interlayer eingebracht sein, welche beispielsweise eine exzitonenblockierende Funktion aufweisen.

Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können.

Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-Elektrolumineszenzvorrichtung handeln, insbesondere für weiß emittierende OLEDs. Die Vorrichtung kann auch anorganische Materialien enthalten oder auch Schichten, welche vollständig aus anorganischen Materialien aufgebaut sind.

Es bereitet dem Fachmann keinerlei Schwierigkeiten auf eine Vielzahl im Stand der Technik bekannter Materialien zurückzugreifen, um geeignete Materialien zur Verwendung in den zuvor beschriebenen Schichten der organischen Elektrolumineszenzvorrichtung auszuwählen. Dabei stellt der Fachmann ihm gängige Überlegungen betreffend die chemischen und physikalischen Eigenschaften der Materialien an, da ihm bekannt ist, dass die Materialien in einer organischen Elektrolumineszenzvorrichtung miteinander in einer Wechselbeziehung stehen. Das betrifft beispielsweise die Energielagen der Orbitale (HOMO, LUMO) oder auch die Lage von Triplett- und Singulett-Energien, aber auch andere Materialeigenschaften. Die erfindungsgemäße Verbindung der Formel (1), wie zuvor beschrieben oder bevorzugt beschrieben, kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für fluoreszierende Emitter, phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial in einer emittierenden Schicht oder als Elektronentransport- bzw. Lochblockiermaterial in einer Elektronentransport- bzw. Lochblockierschicht eingesetzt.

Ein weiterer Gegenstand der vorliegenden Erfindung ist eine organische elektrolumineszierende Vorrichtung, wie zuvor beschrieben, wobei die organische Schicht mindestens eine lichtemittierende Schicht enthält, die die mindestens eine Verbindung der Formel (1) oder die mindestens eine bevorzugte Verbindung einer der Formeln (1), (1a), (1b), (1c), (1d), (1e) und (1f), oder eine Verbindung der Tabelle 1 oder eine der Verbindungen **E1** bis **E27** enthält.

In einer Ausführungsform der Erfindung wird für die erfindungsgemäße Vorrichtung in der lichtemittierenden Schicht ein weiteres Matrixmaterial ausgewählt, das mit Verbindungen der Formel (1), wie zuvor beschrieben oder bevorzugt beschrieben, oder mit den Verbindungen der Tabelle 1 oder den Verbindungen **E1** bis **E27** verwendet wird.

Ein weiterer Gegenstand der vorliegenden Erfindung ist demzufolge eine organische elektrolumineszierende Vorrichtung, wie zuvor beschrieben, wobei die organische Schicht mindestens eine lichtemittierende Schicht enthält, die die mindestens eine Verbindung der Formel (1) oder die mindestens eine bevorzugte Verbindung einer der Formeln (1), (1a), (1b), (1c), (1d), (1e) und (1f), oder eine Verbindung der Tabelle 1 oder eine der Verbindungen **E1** bis **E27** und ein weiteres Matrixmaterial enthält.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, Triarylamine, Carbazolderivate, Biscarbazole, Indolocarbazolderivate, Indenocarbazolderivate, Azacarbazolderivate, bipolare Matrixmaterialien, Azaborole oder Boronester, Triazinderivate, Zinkkomplexe, Diazasilol- bzw. Tetraazasilol-Derivate, Diazaphosphol-Derivate, verbrückte CarbazolDerivate, Triphenylenderivate oder Dibenzofuranderivate. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise eine *wide band-gap* Verbindung.

Unter *wide-band-gap*-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Besonders geeignete Matrixmaterialien, die vorteilhaft mit Verbindungen der Formel (1), wie zuvor beschrieben oder bevorzugt beschrieben, in einem Mixed-Matrix-System kombiniert werden, können aus den Verbindungen der Formeln (6), (7), (8), (9), (10) oder (11) ausgewählt werden, wie nachfolgend beschrieben.

Ein weiterer Gegenstand der Erfindung ist demzufolge eine organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Matrixmaterial 1, wie zuvor beschrieben oder als bevorzugt beschrieben, und mindestens eine Verbindung der Formeln (6), (7), (8), (9) oder (10) als Matrixmaterial 2 enthält,

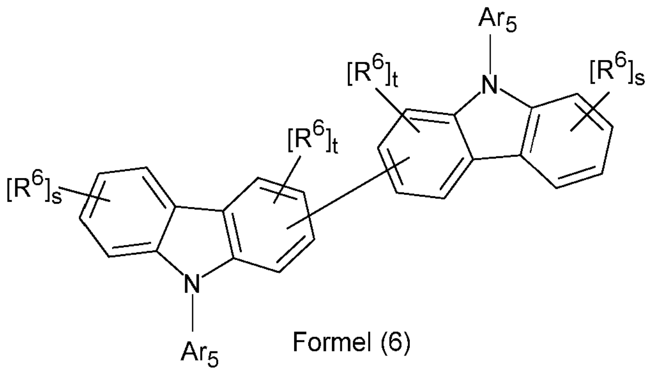

Formel (6)

,

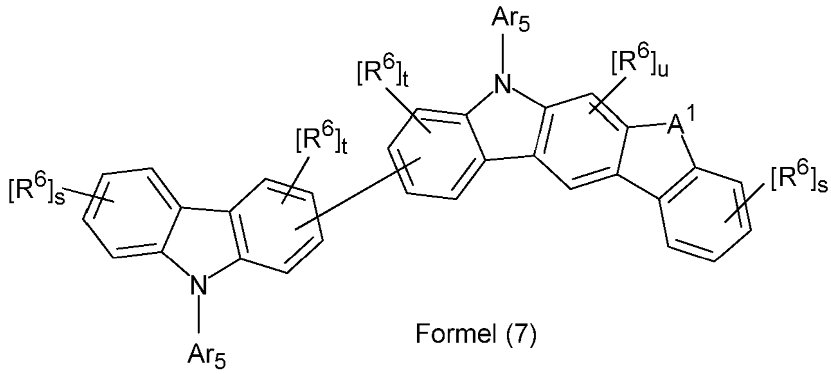

Formel (7)

,

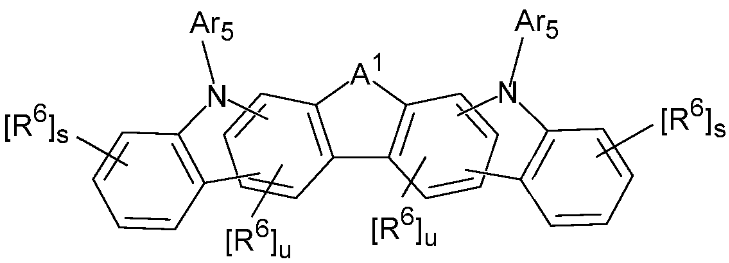

Formel (8)

,

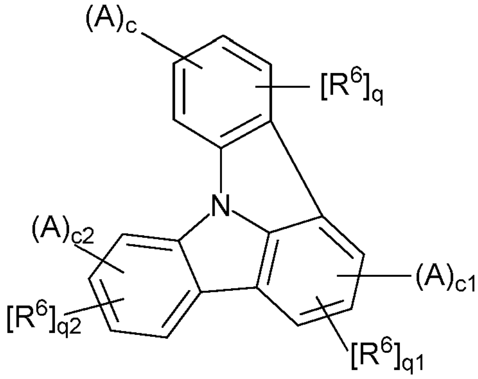

Formel (9)

,

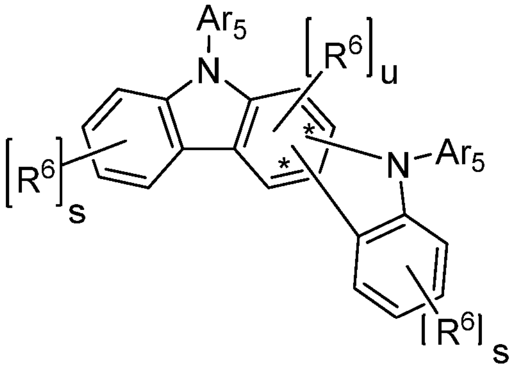

Formel (10),

wobei für die verwendeten Symbole und Indizes gilt:

$A^1$ ist $C(R^7)_2$, $NR^7$, O oder S;

A ist bei jedem Auftreten unabhängig voneinander eine Gruppe der Formel (3) oder (4),

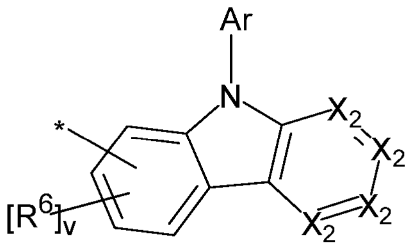

Formel (3) ,

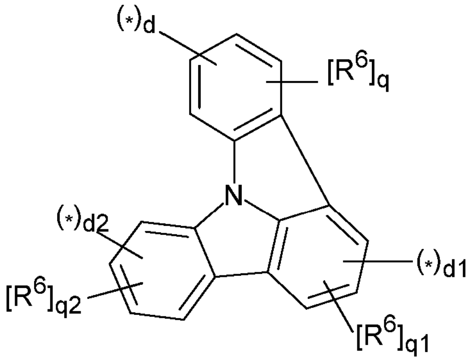

Formel (4) ;

$X_2$ ist bei jedem Auftreten gleich oder verschieden CH, $CR^6$ oder N, wobei maximal 2 Symbole $X_2$ N bedeuten können;

* kennzeichnet die Bindungsstelle an die Formel (9);

$R^6$ ist bei jedem Auftreten gleich oder verschieden D, CN, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^7$ substituiert sein kann und wobei eine oder mehrere nicht benachbarte $CH_2$-Gruppen durch $Si(R^7)_2$, C=O, $NR^7$, O, S oder $CONR^7$ ersetzt sein können, oder ein aromatisches oder heteroaromatisches Ringsystem mit 5 bis 60 Ringatomen, das jeweils durch einen oder mehrere Reste $R^7$ substituiert sein kann; dabei können zwei Reste $R^6$ auch miteinander ein aromatisches, heteroaromatisches, aliphatisches oder heteroaliphatisches Ringsystem bilden;

Ar ist bei jedem Auftreten gleich oder verschieden ein aromatisches oder heteroaromatisches Ringsystem mit 5 bis 40 Ringatomen, welches mit einem oder mehreren Resten $R^7$ substituiert sein kann;

$Ar^5$ steht gleich oder verschieden bei jedem Auftreten für ein aromatisches oder heteroaromatisches Ringsystem mit 5 bis 40 Ringatomen, welches mit einem oder mehreren Resten $R^7$ substituiert sein kann;

$R^7$ ist bei jedem Auftreten gleich oder verschieden D, F, Cl, Br, I, $N(R^8)_2$, CN, $NO_2$, $OR^8$, $SR^8$, $Si(R^8)_3$, $B(OR^8)_2$, $C(=O)R^8$, $P(=O)(R^8)_2$, $S(=O)R^8$, $S(=O)_2R^8$, $OSO_2R^8$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^8$ substituiert sein kann, wobei eine oder mehrere nicht benachbarte $CH_2$-Gruppen durch $Si(R^8)_2$, C=O, $NR^8$, O, S oder $CONR^8$ ersetzt sein können, oder ein aromatisches oder heteroaromatisches Ringsystem mit 5 bis 40 Ringatomen, das jeweils durch einen oder mehrere Reste $R^8$ substituiert sein kann; dabei können zwei oder mehrere Reste $R^7$ miteinander ein aromatisches, heteroaromatisches, aliphatisches oder heteroaliphatisches Ringsystem bilden, vorzugsweise bilden die Reste $R^7$ kein solches Ringsystem;

$R^8$ ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;

c, c1, c2 bedeuten jeweils unabhängig voneinander bei jedem Auftreten 0 oder 1, wobei die Summe der Indizes bei jedem Auftreten c+c1+c2 1 bedeutet;

d, d1, d2 bedeuten jeweils unabhängig voneinander bei jedem Auftreten 0 oder 1, wobei die Summe der Indizes bei jedem Auftreten d+d1+d2 1 bedeutet;

q, q1, q2 bedeuten jeweils unabhängig bei jedem Auftreten 0 oder 1;

s ist bei jedem Auftreten gleich oder verschieden 0, 1, 2, 3 oder 4;

t ist bei jedem Auftreten gleich oder verschieden 0, 1, 2, oder 3;

u ist bei jedem Auftreten gleich oder verschieden 0, 1 oder 2; und

v ist 0 oder 1.

In Verbindungen der Formeln (6), (7), (8) oder (10) ist s bevorzugt 0 oder 1, besonders bevorzugt 0.

In Verbindungen der Formeln (6), (7) oder (8) ist t bevorzugt 0 oder 1, besonders bevorzugt 0.

In Verbindungen der Formeln (6), (7), (8) oder (10) ist u bevorzugt 0 oder 1, besonders bevorzugt 0.

Die Summe der Indices s, t und u in Verbindungen der Formeln (6), (7), (8) oder (10) beträgt vorzugsweise höchstens 6, insbesondere bevorzugt höchstens 4 und besonders bevorzugt höchstens 2.

In Verbindungen der Formel (9) bedeuten c, c1, c2 jeweils unabhängig voneinander bei jedem Auftreten 0 oder 1, wobei die Summe der Indizes bei jedem Auftreten c+c1+c2 1 bedeutet. Bevorzugt hat c2 die Bedeutung 1.

In einer bevorzugten Ausführungsform der Verbindungen der Formeln (6), (7), (8), (9) oder (10), die erfindungsgemäß mit Verbindungen der Formel (1) kombiniert werden können, wie zuvor beschrieben, ist $R^6$ gleich oder verschieden bei jedem Auftreten ausgewählt aus der Gruppe bestehend aus D, F, CN, $NO_2$, $Si(R^7)_3$, $B(OR^7)_2$, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten $R^7$ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste $R^7$ substituiert sein kann.

In einer bevorzugten Ausführungsform der Verbindungen der Formeln (6), (7), (8), (9) oder (10), die erfindungsgemäß mit Verbindungen der Formel (1) kombiniert werden können, wie zuvor beschrieben, ist $R^6$ gleich oder verschieden bei jedem Auftreten ausgewählt aus der Gruppe bestehend aus D, einem aromatischen oder heteroaromatischen Ringsystem mit 6 bis 30 aromatischen Ringatomen, welches mit einem oder mehreren Resten $R^7$ substituiert sein kann. Ein bevorzugter Rest $R^7$ ist die Gruppe $N(Ar)_2$.

Bevorzugt wird $Ar_5$ in Verbindungen der Formeln (6), (7), (8) oder (10) ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluorenyl, welches über die 1-, 2-, 3- oder 4-Position verknüpft sein kann, Spirobifluorenyl, welches über die 1-, 2-, 3- oder 4-Position verknüpft sein kann, Naphthyl, insbesondere 1- oder 2-verknüpftes Naphthyl, oder Reste abgeleitet von Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten $R^7$ substituiert sein können. Bevorzugt ist $Ar_5$ nicht substituiert.

Wenn $A^1$ in Formel (7) oder (8) für $NR^7$ steht, steht der Substituent $R^7$, der an das Stickstoffatom gebunden ist, bevorzugt für ein aromatisches oder heteroaromatisches Ringsystem mit 5 bis 24 aromatischen Ringatomen, welches auch durch einen oder mehrere Reste $R^8$ substituiert sein kann. In einer besonders bevorzugten Ausführungsform steht dieser Substituent $R^7$ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen. Bevorzugte Ausführungsformen für $R^7$ sind Phenyl, Biphenyl, Terphenyl und Quaterphenyl, die bevorzugt unsubstituiert sind, sowie Reste abgeleitet von Triazin, Pyrimidin und Chinazolin, die durch einen oder mehrere Reste $R^8$ substituiert sein können.

Wenn $A^1$ in Formel (7) oder (8) für $C(R^7)_2$ steht, stehen die Substituenten $R^7$, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste $R^8$ substituiert sein kann. Ganz besonders bevorzugt steht $R^7$ für eine Methylgruppe oder für eine Phenylgruppe. Dabei können die Reste $R^7$ auch miteinander ein Ringsystem bilden, was zu einem Spirosystem führt.

In einer bevorzugten Ausführungsform der Verbindungen der Formeln (6), (7), (8), (9) und (10) sind diese Verbindungen teilweise oder vollständig deuteriert, besonders bevorzugt vollständig deuteriert.

Die Herstellung der Verbindungen der Formeln (6), (7), (8), (9) und (10) ist generell bekannt und einige der

Verbindungen sind kommerziell erhältlich.

Verbindungen der Formel (9) sind beispielsweise in WO2021180614, Seiten 110 bis 119, insbesondere als Beispiele auf den Seiten 120 bis 127. Deren Herstellung ist in WO2021180614 auf Seite 128 sowie in den Synthesebeispielen auf Seite 214 bis 218 offenbart.

Ein weiterer Gegenstand der Erfindung ist auch eine organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Matrixmaterial 1, wie zuvor beschrieben oder als bevorzugt beschrieben, und mindestens eine Verbindung der Formel (11) enthält,

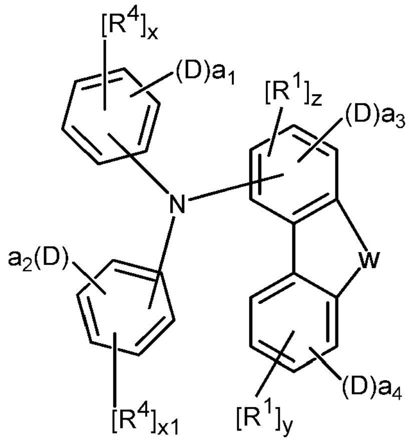

Formel (11),

wobei für die verwendeten Symbole und Indizes gilt:

W ist O, S, $C(R)_2$, N-$Ar_1$;

R ist jeweils unabhängig voneinander eine geradkettige oder verzweigte Alkylgruppe mit 1 bis 4 C-Atomen, die teilweise oder vollständig deuteriert sein kann, oder ein unsubstituiertes oder teilweise oder vollständig deuteriertes aromatisches Ringsystem mit 6 bis 18 C-Atomen, wobei zwei Substituenten R mit dem C-Atom, an das sie gebunden sind, ein mono- oder polycyclisches, aliphatisches oder aromatisches oder heteroaromatisches unsubstituiertes, teilweise deuteriertes oder vollständig deuteriertes Ringsystem bilden können, das durch ein oder mehrere Substituenten $R^5$ substituiert sein kann;

$Ar_1$ ist bei jedem Auftreten gleich oder verschieden ein aromatisches oder heteroaromatisches Ringsystem mit 5 bis 30 Ringatomen, das mit einem oder mehreren Resten $R^5$ substituiert sein kann; dabei können zwei Reste $Ar_1$, welche an dasselbe N-Atom, P-Atom oder B-Atom binden, auch durch eine Einfachbindung oder eine Brücke, ausgewählt aus $C(R^5)_2$, O oder S, miteinander verbrückt sein;

$R^1$ ist bei jedem Auftreten gleich oder verschieden ausgewählt aus der Gruppe bestehend aus F, Cl, Br, I, CN, $NO_2$, C(=O)R', $P(=O)(Ar_1)_2$, $P(Ar_1)_2$, $B(Ar_1)_2$, $Si(Ar_1)_3$, $Si(R')_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R' substituiert sein kann, wobei eine oder mehrere nicht-benachbarte $CH_2$-Gruppen durch R'C=CR', $Si(R')_2$, C=O, C=S, C=NR', P(=O)(R'), SO, $SO_2$, NR', O, S oder CONR' ersetzt sein können und wobei ein oder mehrere H-Atome durch D, F, Cl, Br, I, CN oder $NO_2$ ersetzt sein können;

R' ist bei jedem Auftreten gleich oder verschieden ein aliphatischer, aromatischer oder heteroaromatischer organischer Rest, insbesondere ein Kohlenwasserstoffrest, mit 1 bis 20 C-Atomen;

$R^4$ ist bei jedem Auftreten gleich oder verschieden ausgewählt aus der Gruppe bestehend aus F, Cl, Br, I, CN, $NO_2$, $N(Ar_1)_2$, $NH_2$, $N(R^5)_2$, $C(=O)Ar_1$, C(=O)H, $C(=O)R^5$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten $R^5$ substituiert sein kann, wobei eine oder mehrere nicht-benachbarte $CH_2$-Gruppen durch HC=CH, $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, NH, $NR^5$, O, S, CONH oder $CONR^5$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R^5$ substituiert sein kann, einer Aryloxy- oder Heteroaryloxygruppe mit 5 bis 60 Ringatomen, die mit einem oder mehreren

Resten $R^5$ substituiert sein kann, oder einer Kombination dieser Systeme, wobei optional zwei oder mehr benachbarte Substituenten $R^4$ ein monocyclisches oder polycyclisches, aliphatisches, aromatisches oder heteroaromatisches Ringsystem bilden können, das mit einem oder mehreren Resten $R^5$ substituiert sein kann;

$R^5$ ist bei jedem Auftreten gleich oder verschieden ausgewählt aus der Gruppe bestehend aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^5$ miteinander ein mono- oder polycyclisches, aliphatisches Ringsystem bilden;

x, x1 sind bei jedem Auftreten unabhängig 0, 1, 2, 3 oder 4;

y, z sind jeweils unabhängig voneinander 0, 1 oder 2;

a1, a2 sind jeweils unabhängig voneinander 0, 1, 2, 3, 4 oder 5;

a3 ist 0, 1, 2 oder 3;

a4 ist 0, 1, 2, 3 oder 4.

Die Herstellung der Triarylamine der Formel (11) ist dem Fachmann bekannt und einige der Verbindungen sind kommerziell erhältlich.

Die Verbindungen der Formeln (6), (7), (8), (9), (10) oder (11) sind bevorzugt teilweise deuteriert oder vollständig deuteriert.

In Verbindungen der Formel (11), wie zuvor beschrieben, ist die Summe der Indizes $a_1+a_2+a_3+a_4$ bevorzugt ausgewählt aus 3, 4, 5, 6, 7, 8, 9, 10, 11, 12, 13, 14, 15, 16 oder 17. Dieses weitere Matrixmaterial ist demzufolge an jedem N-gebundenen Substituenten mindestens teilweise deuteriert. In einer bevorzugten Ausführungsform sind zwei der N-gebundenen Substituenten teilweise deuteriert und der dritte N-gebundene Substituent ist vollständig deuteriert. In einer weiteren bevorzugten Ausführungsform sind zwei der N-gebundenen Substituenten vollständig deuteriert und der dritte N-gebundene Substituent ist teilweise deuteriert. In einer weiteren bevorzugten Ausführungsform ist jeder N-gebundenen Substituent vollständig deuteriert.

In einer bevorzugten Ausführungsform des weiteren Matrixmaterials ist dieses ein Gemisch aus deuterierten Verbindungen der Formel (11), wie zuvor beschrieben oder nachfolgend bevorzugt beschrieben, wobei der Deuterierungsgrad der Verbindungen der Formel (11) mindestens 50% bis 90% beträgt, bevorzugt 70% bis 100 % beträgt. Entsprechende Deuterierungsmethoden sind dem Fachmann bekannt und beispielsweise in KR2016041014, WO2017122988, KR202005282, KR101978651 und WO2018110887 oder in Bulletin oft he Chemical Society of Japan, 2021, 94(2), 600-605 oder Asian Journal of Organic Chemistry, 2017, 6(8), 1063-1071 beschrieben.

Ein geeignetes Verfahren zur Deuterierung eines Arylamins oder eines Heteroarylamins durch Austausch eines oder mehrerer H-Atome gegen D-Atome, ist eine Behandlung des zu deuterierenden Arylamins oder Heteroarylamins in Gegenwart eines Platinkatalysators oder Palladiumkatalysators und einer Deuteriumquelle. Der Begriff "Deuteriumquelle" bedeutet jede Verbindung, die ein oder mehrere D-Atome enthält und diese unter geeigneten Bedingungen freisetzen kann.

Der Platinkatalysator ist vorzugsweise trockenes Platin auf Kohle, vorzugsweise 5% trockenes Platin auf Kohle. Der Palladiumkatalysator ist vorzugsweise trockenes Palladium auf Kohle, vorzugsweise 5% trockenes Palladium auf Kohle. Eine geeignete Deuteriumquelle ist $D_2O$, Benzol-d6, Chloroform-d, Acetonitril-d3, Aceton-d6, Essigsäured4, Methanol-d4, Toluol-d8. Eine bevorzugte Deuteriumquelle ist $D_2O$ oder eine Kombination aus $D_2O$ und einem vollständig deuterierten organischen Lösungsmittel. Eine besonders bevorzugte Deuteriumquelle ist die Kombination aus $D_2O$ mit einem vollständig deuterierten organischen Lösungsmittel, wobei das vollständig deuterierte Lösungsmittel hier nicht eingeschränkt ist. Besonders geeignete vollständig deuterierte Lösungsmittel sind Benzen-d6 und Toluol-d8. Eine besonders bevorzugte Deuteriumquelle ist eine Kombination von $D_2O$ und Toluol-d8. Die Reaktion wird vorzugsweise unter Erhitzen durchgeführt, stärker bevorzugt unter Erhitzen auf Temperaturen zwischen 100 °C und 200 °C. Weiterhin wird die Reaktion vorzugsweise unter Druck durchgeführt.

Bevorzugte Verbindungen der Formel (11) werden durch die Formeln (11a), (11b), (11c), (11d), (11e), (11f), (11g), (11h), (11i), (11j), (11k), (11l), (11m), (11n), (11o) und (11p) dargestellt,

| | |
|---|---|
| 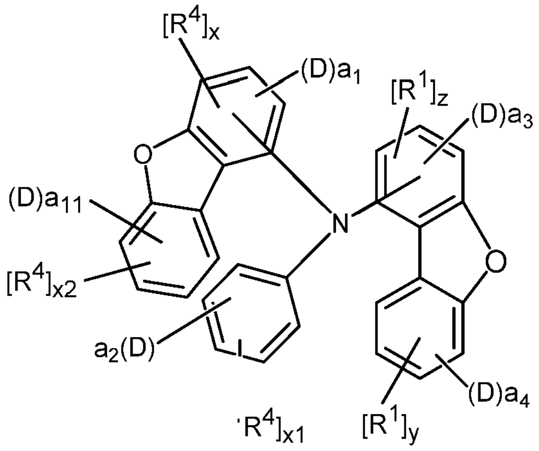 | 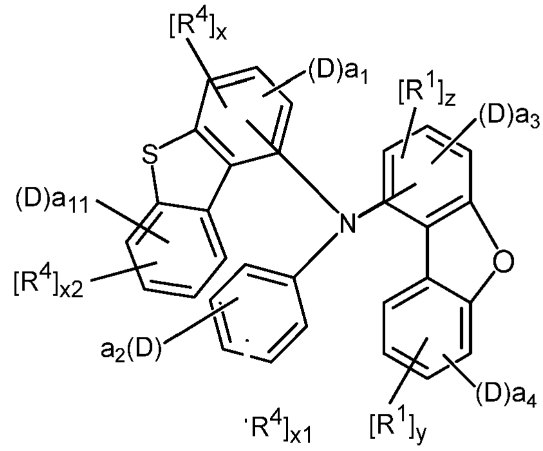 |
| Formel (11a) | Formel (11b) |
| 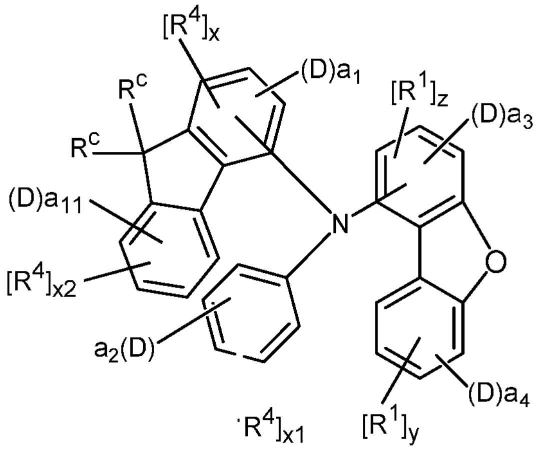 | 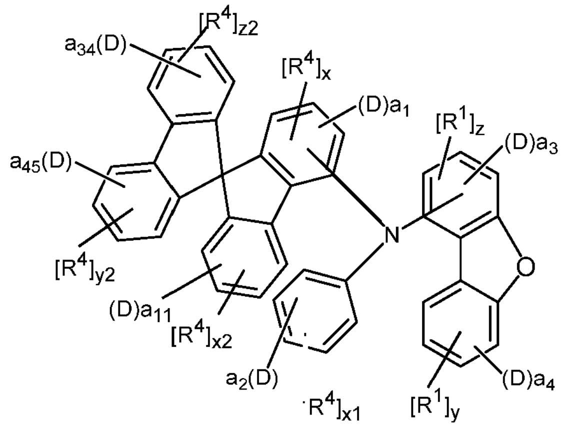 |
| Formel (11c) | Formel (11d) |
| 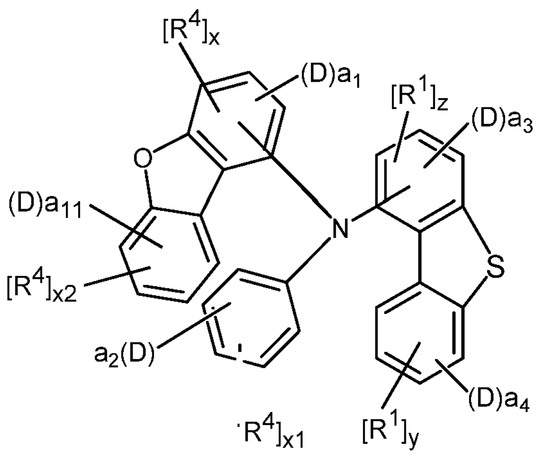 | 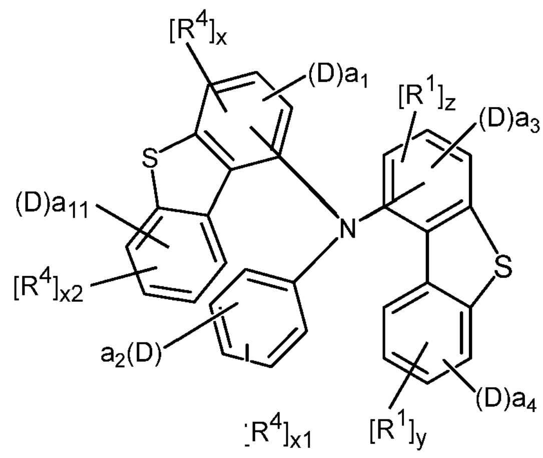 |
| Formel (11e) | Formel (11f) |

(fortgesetzt)

| | |
|---|---|
| 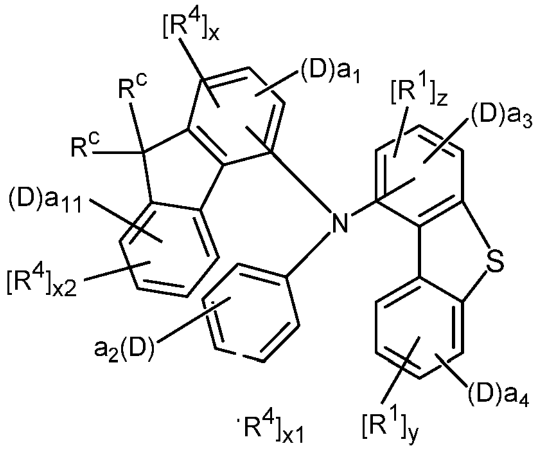 | 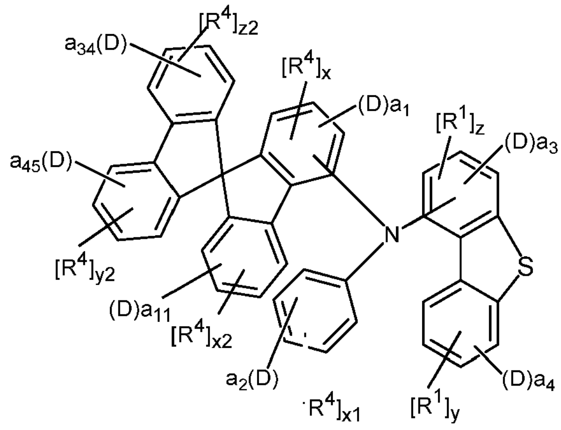 |
| Formel (11g) | Formel (11h) |
| 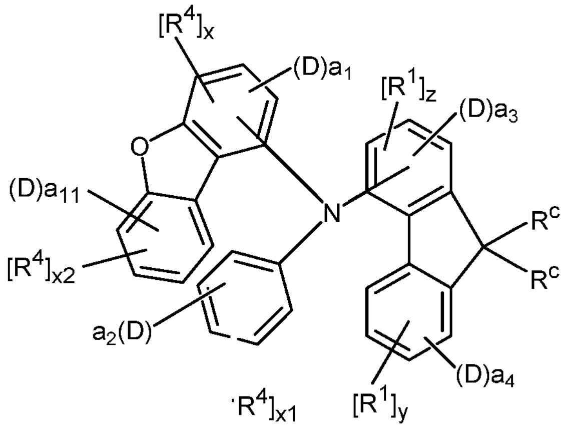 | 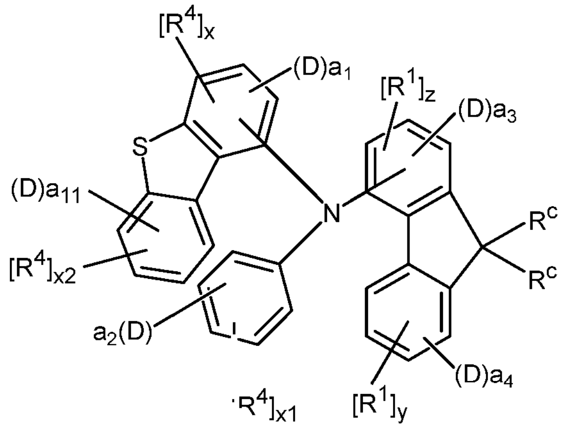 |
| Formel (11i) | Formel (11j) |
| 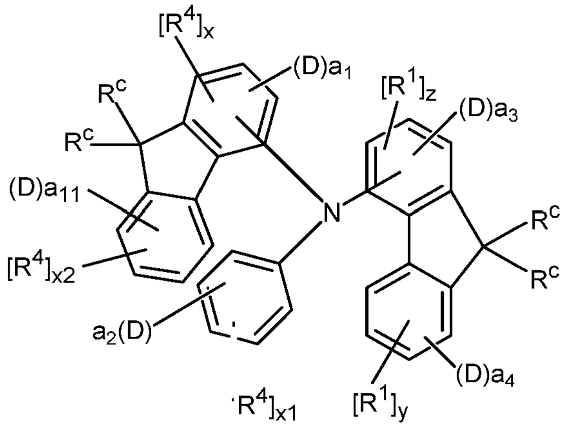 | 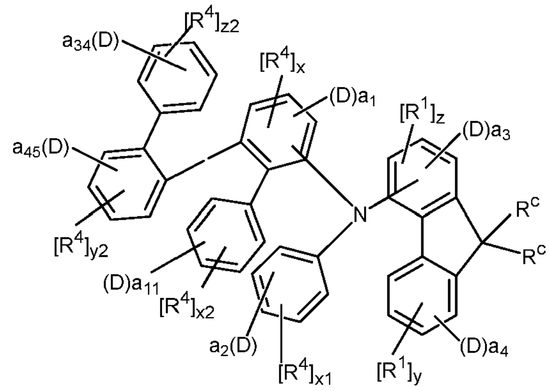 |
| Formel (11k) | Formel (11l) |

(fortgesetzt)

| | |
|---|---|
| 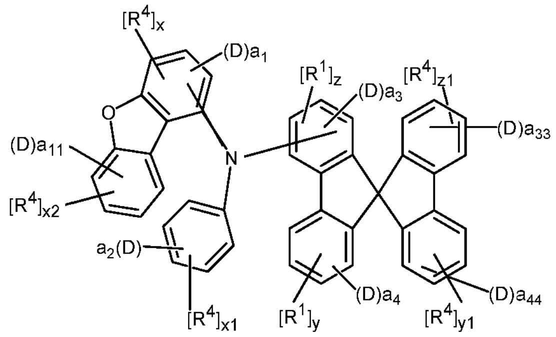 | 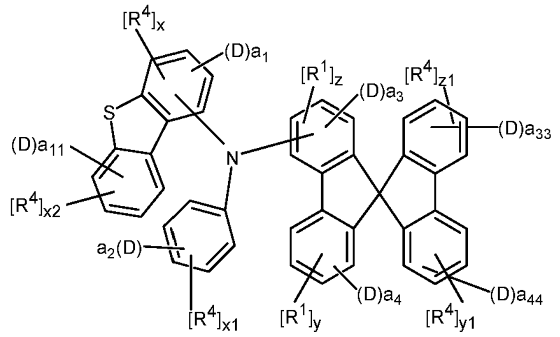 |
| Formel (11m) | Formel (11n) |
| 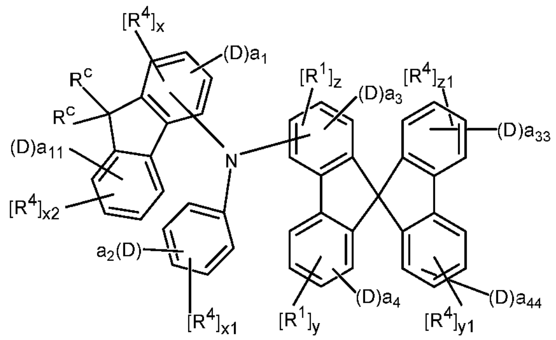 | 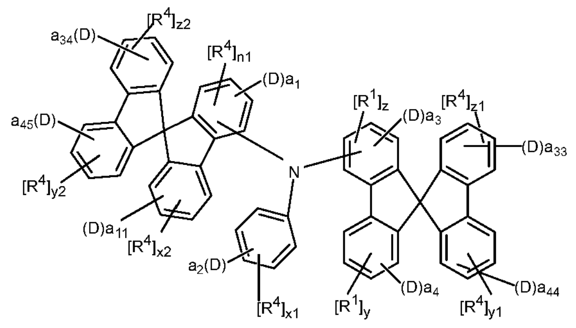 |
| Formel (11o) | Formel (11p), |

wobei $a_1$, $a_2$, $a_3$, $a_4$, x, x1, y, z, $R^1$ und $R^4$ eine zuvor genannte oder zuvor oder nachfolgend bevorzugt genannte Bedeutung haben und

| | |
|---|---|
| $R^c$ | jeweils unabhängig voneinander eine geradkettige oder verzweigte Alkylgruppe mit |
| 1 bis 4 | C-Atomen, die teilweise oder vollständig deuteriert sein kann, oder ein unsubstituiertes oder teilweise oder vollständig deuteriertes aromatisches Ringsystem mit |
| 6 bis 18 | C-Atomen ist; |
| x2 | ist 0, 1, 3 oder 3; |
| y1, z1 | sind jeweils unabhängig voneinander 0, 1 oder 2; |
| y1, z1, y2, z2 | sind jeweils unabhängig voneinander 0, 1 oder 2, bevorzugt 0; |
| $a_{11}$ | ist 0, 1, 2, 3 oder 4; |
| $a_{33}$, $a_{44}$ | sind jeweils unabhängig voneinander 0, 1, 2, 3 oder 4 und |
| $a_{34}$, $a_{45}$ | sind jeweils unabhängig voneinander 0, 1, 2, 3 oder 4. |

$R^c$ ist bevorzugt gleich und eine geradkettige oder verzweigte Alkylgruppe mit 1 bis 4 C-Atomen, die teilweise oder vollständig deuteriert sein kann, oder ein unsubstituiertes oder teilweise oder vollständig deuteriertes Phenyl.

In Verbindungen der Formeln (11), (11a), (11b), (11c), (11d), (11e), (11f), (11g), (11h), (11i), (11j), (11k), (11l), (11m), (11n), (11o) und (11p) ist y+z bevorzugt 0.

Bevorzugt ist das N-Atom in Verbindungen der Formeln (11), (11a), (11b), (11c), (11d), (11e), (11f), (11g), (11h), (11i), (11j), (11k), (11l), (11m), (11n), (11o) und (11p) in 1-Position zu Dibenzofuran oder Dibenzothiophengruppen gebunden oder in 4-Position zu Fluoren- oder Spirobifluorengruppen gebunden.

Bevorzugt wird $R^4$ in Verbindungen der Formeln (11), (11a), (11b), (11c), (11d), (11e), (11f), (11g), (11h), (11i), (11j), (11k), (11l), (11m), (11n), (11o) und (11p) ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluorenyl, welches über die 1-, 2-, 3- oder 4-Position verknüpft sein kann, Spirobifluorenyl, welches über die 1-, 2-, 3- oder 4-Position verknüpft sein kann, Naphthyl, insbesondere 1- oder 2-verknüpftes Naphthyl, oder Reste abgeleitet von Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren oder Triphenylen, welche jeweils mit einem oder mehreren Resten $R^5$ substituiert sein können. Bevorzugt ist

$R^4$ nicht substituiert.

Bevorzugt wird $R^1$ in Verbindungen der Formeln (11), (11a), (11b), (11c), (11d), (11e), (11f), (11g), (11h), (11i), (11j), (11k), (11l), (11m), (11n), (11o) und (11p) ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluorenyl, welches über die 1-, 2-, 3- oder 4-Position verknüpft sein kann, Spirobifluorenyl, welches über die 1-, 2-, 3- oder 4-Position verknüpft sein kann, Naphthyl, insbesondere 1- oder 2-verknüpftes Naphthyl, oder Reste abgeleitet von Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Phenanthren oder Triphenylen, welche jeweils mit einem oder mehreren Resten $R^5$ substituiert sein können. Bevorzugt ist $R^1$ nicht substituiert.

Bevorzugt sind x, x1, y, z, x2, y1 und z1 0.

Besonders bevorzugt werden die Verbindungen der Formeln (6), (9), (10) und (11) als weiteres Matrixmaterial verwendet.

Besonders geeignete Verbindungen der Formeln (6), (7), (8), (9), (10) oder (11), die erfindungsgemäß ausgewählt werden, und bevorzugt in Kombination mit mindestens einer Verbindung der Formel (1) in der erfindungsgemäßen elektrolumineszierenden Vorrichtung verwendet werden, sind die Verbindungen **H1** bis **H54** der Tabelle 3.

Tabelle 3:

| | | |
|---|---|---|
| 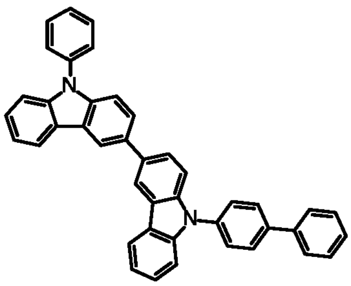 | 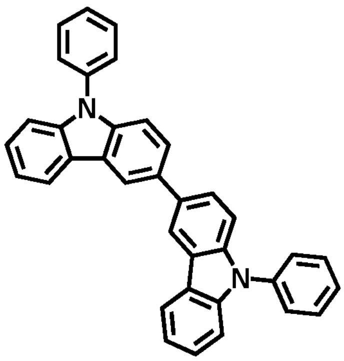 | 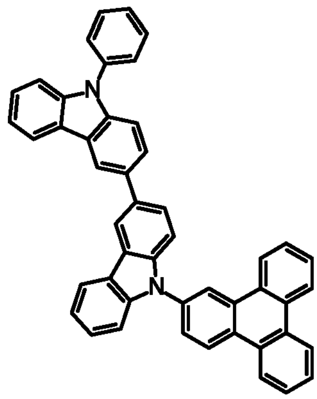 |
| H1 | H2 | H3 |
| 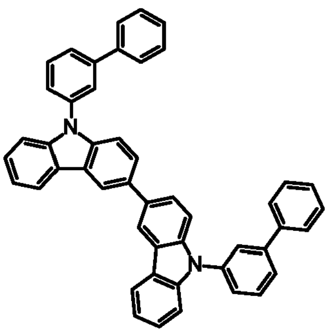 | 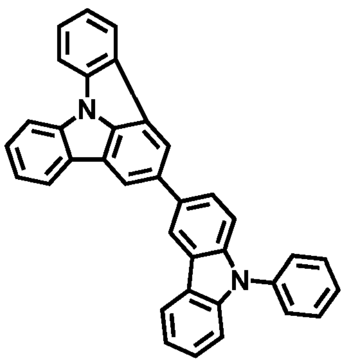 | 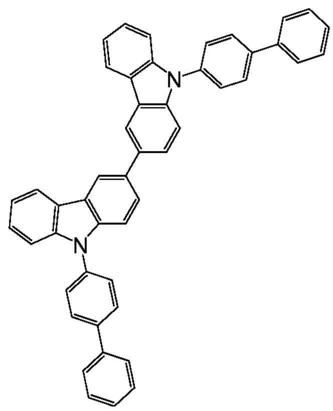 |
| H4 | H5 | H6 |

(fortgesetzt)

| | | |
|---|---|---|
| 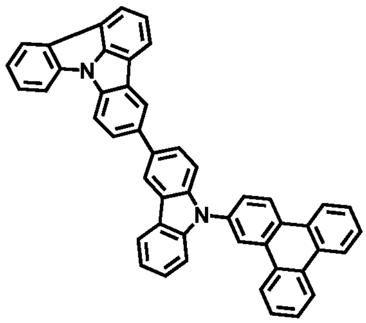 | 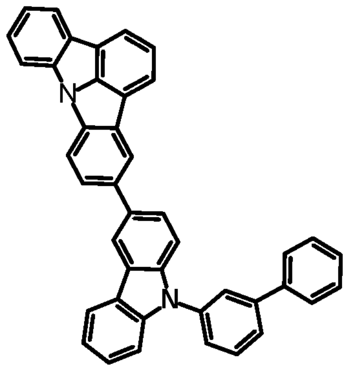 | 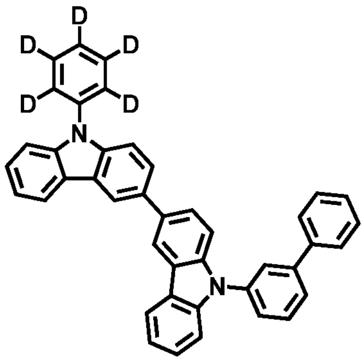 |
| H7 | H8 | H9 |
| 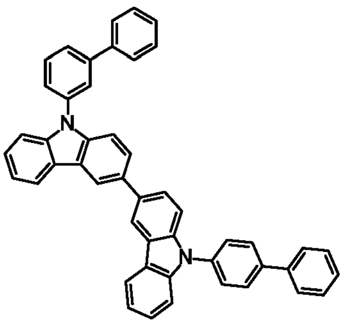 | 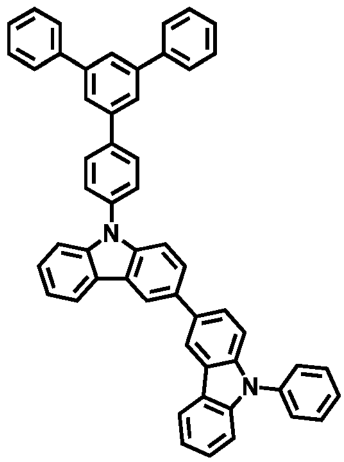 | 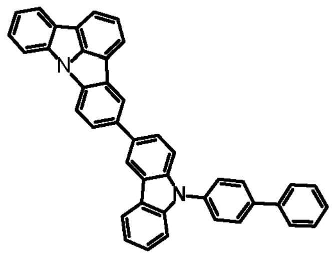 |
| H10 | H11 | H12 |
| 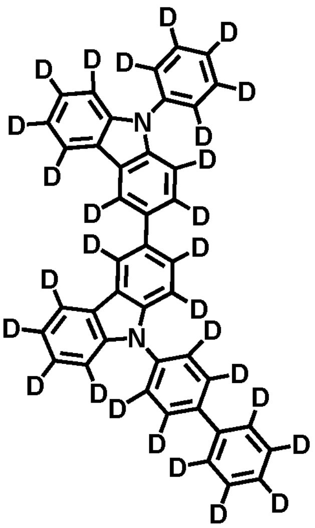 | 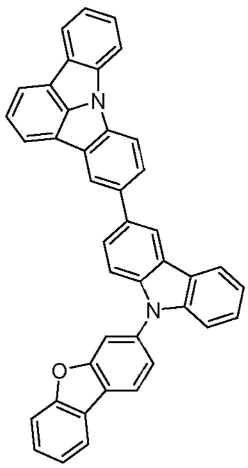 | 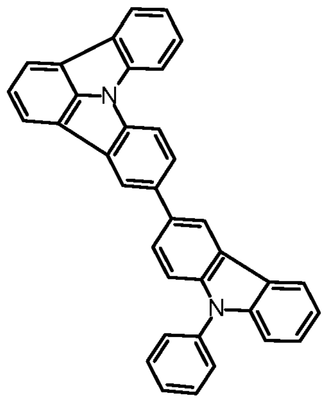 |
| H13 | H14 | H15 |

(fortgesetzt)

| | | |
|---|---|---|
| 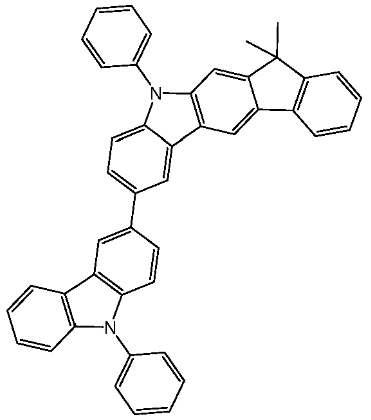 | 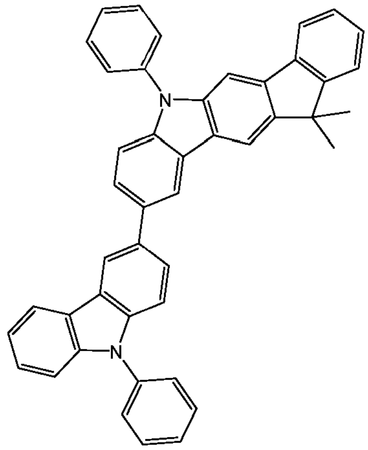 | 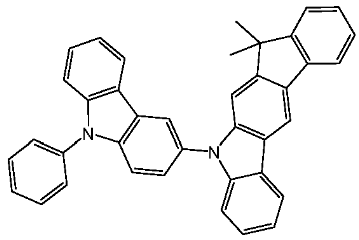 |
| H16 | H17 | H18 |
| 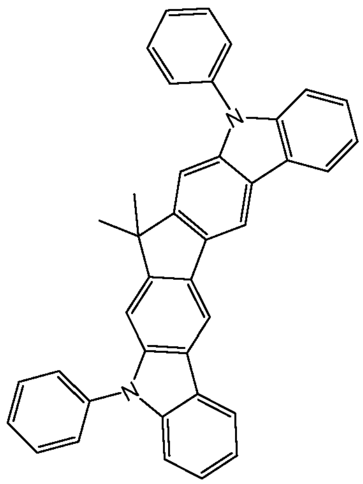 | 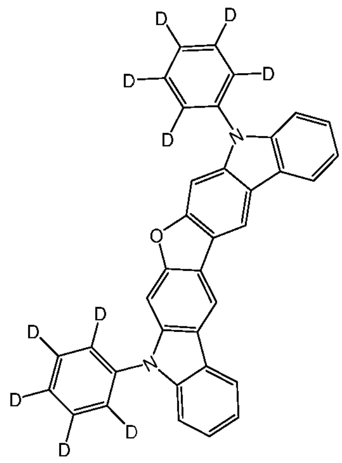 | 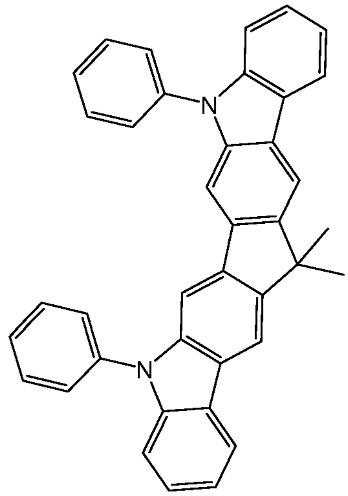 |
| H19 | H20 | H21 |
| 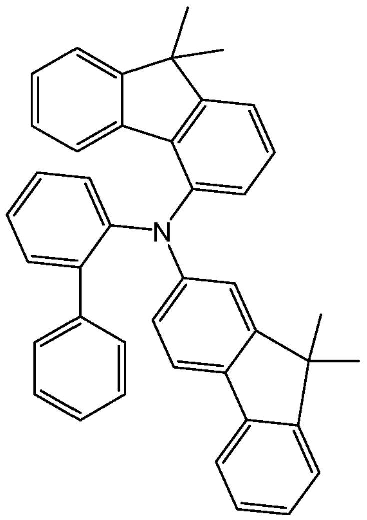 | 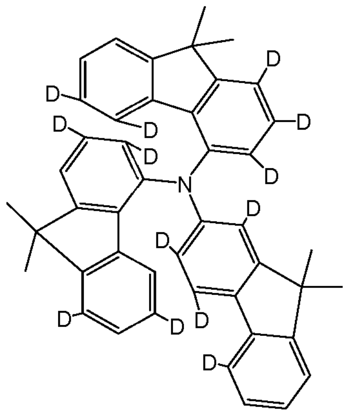 | 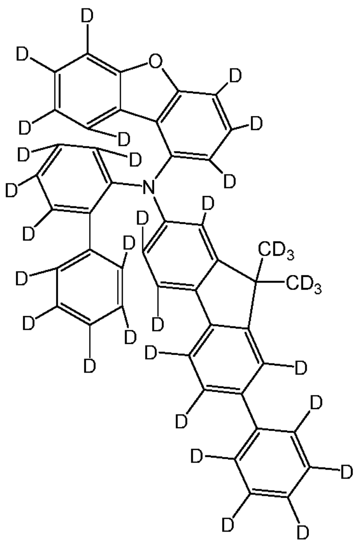 |
| H22 | H23 | H24 |

(fortgesetzt)

| | | |
|---|---|---|
| 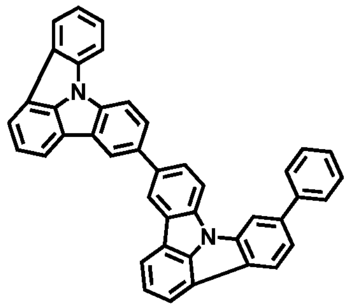 | 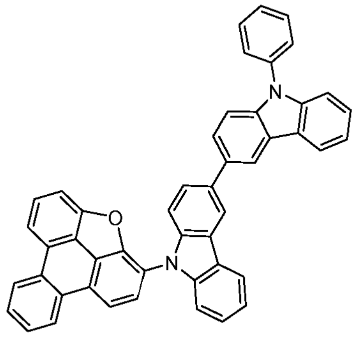 | 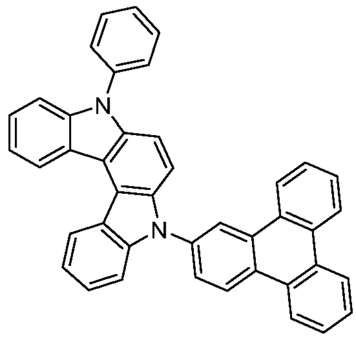 |
| H25 | H26 | H27 |
| 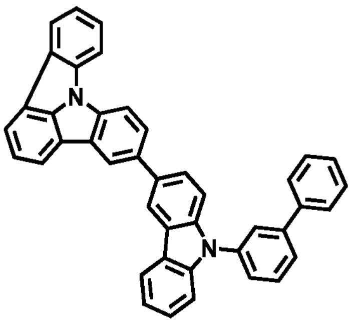 | 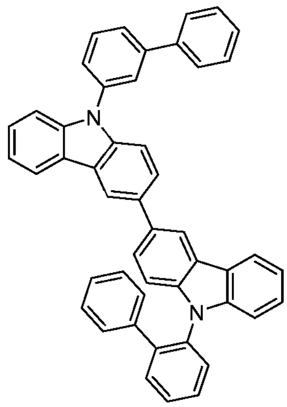 | 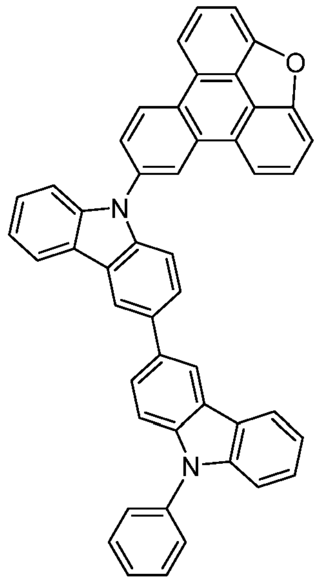 |
| H28 | H29 | H30 |
| 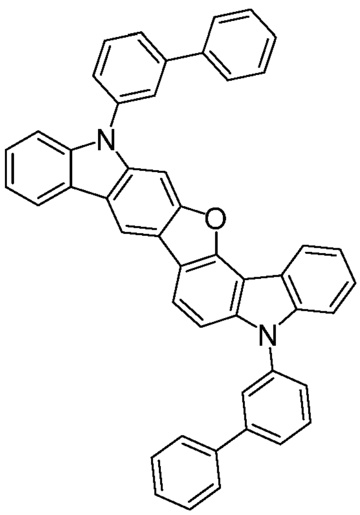 | 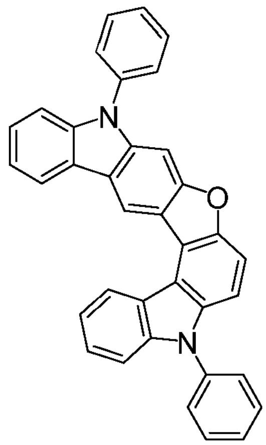 | 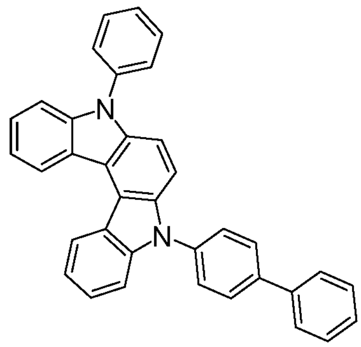 |
| H31 | H32 | H33 |

(fortgesetzt)

| | | |
|---|---|---|
| 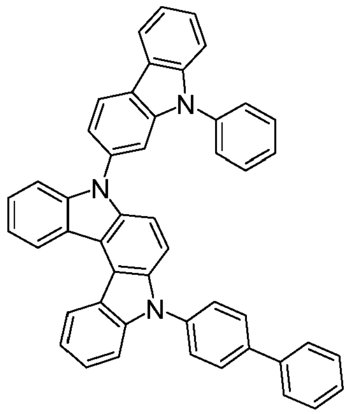 | 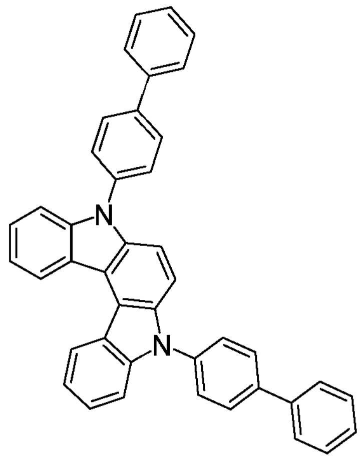 | 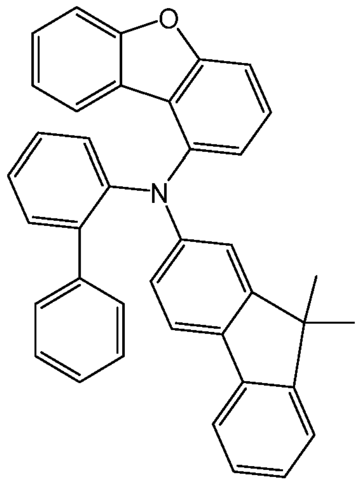 |
| H34 | H35 | H36 |
| 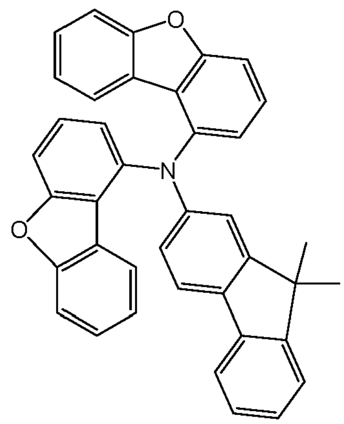 | 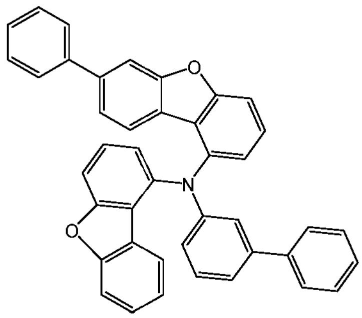 | 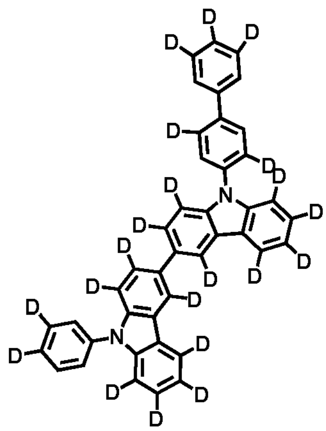 |
| H37 | H38 | H39 |
| 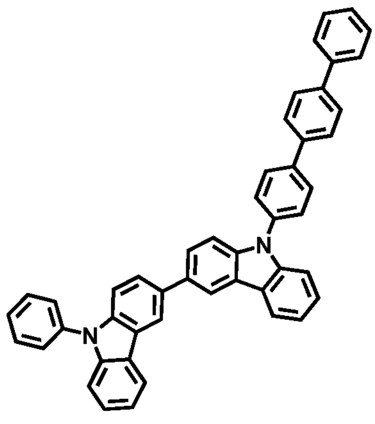 | 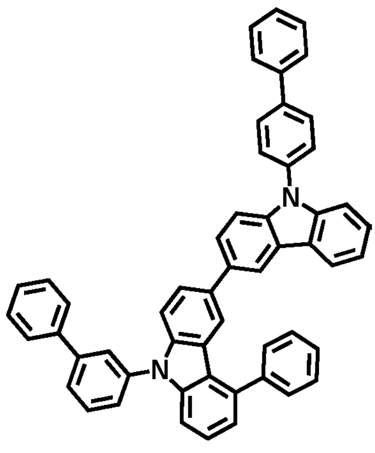 | 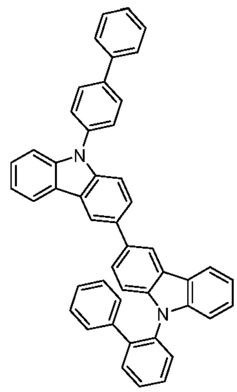 |
| H40 | H41 | H42 |

(fortgesetzt)

| | | |
|---|---|---|
| 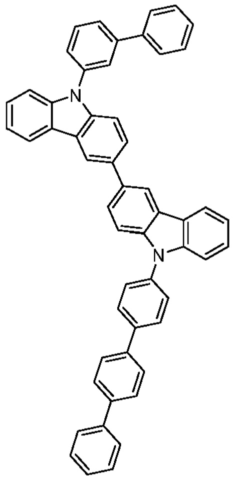 | 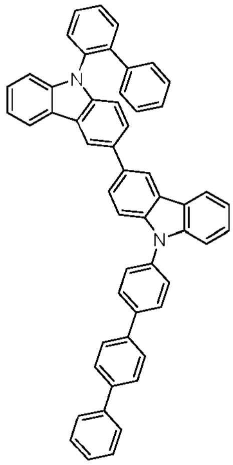 | 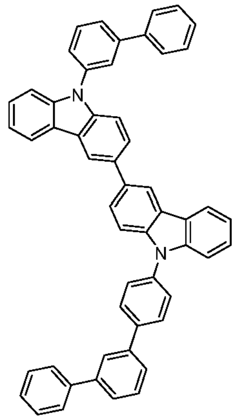 |
| H43 | H44 | H45 |
| 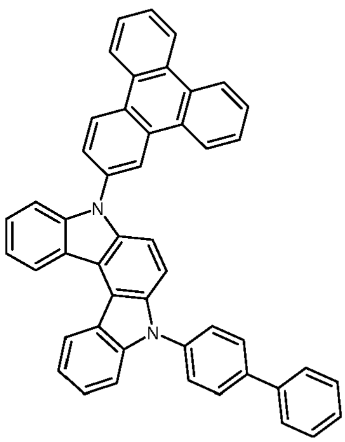 | 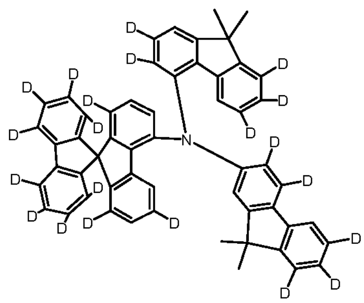 | 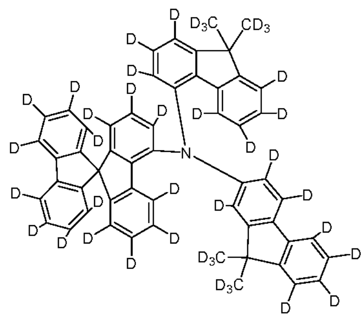 |
| H46 | H47 | H48 |
| 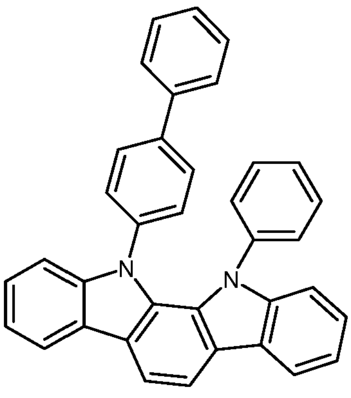 | 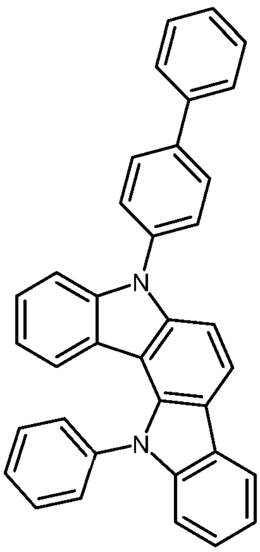 | 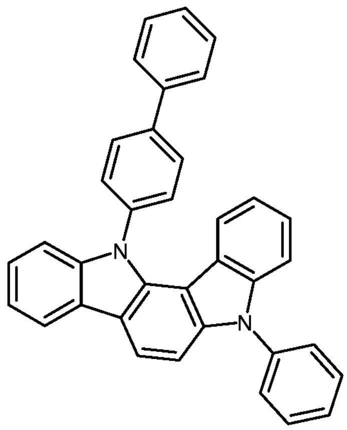 |
| H49 | H50 | H51 |

(fortgesetzt)

| | | |
|---|---|---|
| 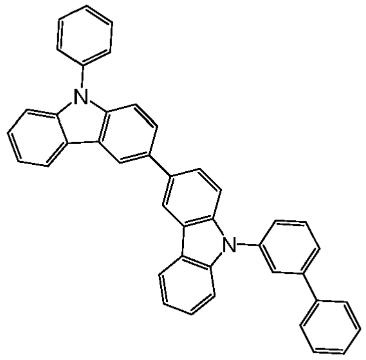 | 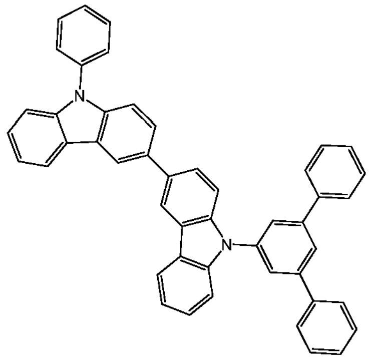 | 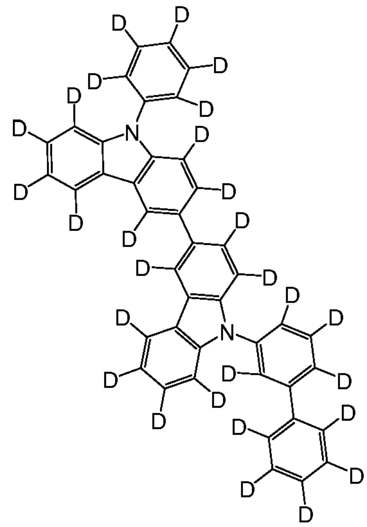 |
| H52 | H53 | H54 |

Die vorstehend genannten Hostmaterialien der Formel (1) sowie deren bevorzugt beschriebene Ausführungsformen oder die Verbindungen der Tabelle 1 und der Verbindungen **E1** bis **E27** können in der erfindungsgemäßen Vorrichtung beliebig mit den genannten Matrixmaterialien/Hostmaterialien der Formeln (6), (7), (8), (9), (10) oder (11) sowie deren bevorzugt beschriebene Ausführungsformen oder den Verbindungen **H1** bis **H54** kombiniert werden.

Ganz besonders bevorzugte Mischungen der Verbindungen der Formel (1) mit den Hostmaterialien der Formeln (6), (7), (8), (9), (10) oder (11) für die erfindungsgemäße Vorrichtung erhält man durch Kombination der Verbindungen **E1** bis **E27** mit den Verbindungen **H1** bis **H54** wie im Folgenden in Tabelle 4 gezeigt.

Tabelle 4:

| | | | | | | | | |
|---|---|---|---|---|---|---|---|---|
| M1 | **E1** | **H1** | M2 | **E2** | **H1** | M3 | **E3** | **H1** |
| M4 | **E4** | **H1** | M5 | **E5** | **H1** | M6 | **E6** | **H1** |
| M7 | **E7** | **H1** | M8 | **E8** | **H1** | M9 | **E9** | **H1** |
| M10 | **E10** | **H1** | M11 | **E11** | **H1** | M12 | **E12** | **H1** |
| M13 | **E13** | **H1** | M14 | **E14** | **H1** | M15 | **E15** | **H1** |
| M16 | **E16** | **H1** | M17 | **E17** | **H1** | M18 | **E18** | **H1** |
| M19 | **E19** | **H1** | M20 | **E20** | **H1** | M21 | **E21** | **H1** |
| M22 | **E22** | **H1** | M23 | **E23** | **H1** | M24 | **E24** | **H1** |
| M25 | **E25** | **H1** | M26 | **E26** | **H1** | M27 | **E27** | **H1** |
| M28 | **E1** | **H2** | M29 | **E2** | **H2** | M30 | **E3** | **H2** |
| M31 | **E4** | **H2** | M32 | **E5** | **H2** | M33 | **E6** | **H2** |
| M34 | **E7** | **H2** | M35 | **E8** | **H2** | M36 | **E9** | **H2** |
| M37 | **E10** | **H2** | M38 | **E11** | **H2** | M39 | **E12** | **H2** |
| M40 | **E13** | **H2** | M41 | **E14** | **H2** | M42 | **E15** | **H2** |
| M43 | **E16** | **H2** | M44 | **E17** | **H2** | M45 | **E18** | **H2** |
| M46 | **E19** | **H2** | M47 | **E20** | **H2** | M48 | **E21** | **H2** |
| M49 | **E22** | **H2** | M50 | **E23** | **H2** | M51 | **E24** | **H2** |
| M52 | **E25** | **H2** | M53 | **E26** | **H2** | M54 | **E27** | **H2** |
| M55 | **E1** | **H3** | M56 | **E2** | **H3** | M57 | **E3** | **H3** |

(fortgesetzt)

| | | | | | | | | |
|---|---|---|---|---|---|---|---|---|
| M58 | **E4** | **H3** | M59 | **E5** | **H3** | M60 | **E6** | **H3** |
| M61 | **E7** | **H3** | M62 | **E8** | **H3** | M63 | **E9** | **H3** |
| M64 | **E10** | **H3** | M65 | **E11** | **H3** | M66 | **E12** | **H3** |
| M67 | **E13** | **H3** | M68 | **E14** | **H3** | M69 | **E15** | **H3** |
| M70 | **E16** | **H3** | M71 | **E17** | **H3** | M72 | **E18** | **H3** |
| M73 | **E19** | **H3** | M74 | **E20** | **H3** | M75 | **E21** | **H3** |
| M76 | **E22** | **H3** | M77 | **E23** | **H3** | M78 | **E24** | **H3** |
| M79 | **E25** | **H3** | M80 | **E26** | **H3** | M81 | **E27** | **H3** |
| M82 | **E1** | **H4** | M83 | **E2** | **H4** | M84 | **E3** | **H4** |
| M85 | **E4** | **H4** | M86 | **E5** | **H4** | M87 | **E6** | **H4** |
| M88 | **E7** | **H4** | M89 | **E8** | **H4** | M90 | **E9** | **H4** |
| M91 | **E10** | **H4** | M92 | **E11** | **H4** | M93 | **E12** | **H4** |
| M94 | **E13** | **H4** | M95 | **E14** | **H4** | M96 | **E15** | **H4** |
| M97 | **E16** | **H4** | M98 | **E17** | **H4** | M99 | **E18** | **H4** |
| M100 | **E19** | **H4** | M101 | **E20** | **H4** | M102 | **E21** | **H4** |
| M103 | **E22** | **H4** | M104 | **E23** | **H4** | M105 | **E24** | **H4** |
| M106 | **E25** | **H4** | M107 | **E26** | **H4** | M108 | **E27** | **H4** |
| M109 | **E1** | **H5** | M110 | **E2** | **H5** | M111 | **E3** | **H5** |
| M112 | **E4** | **H5** | M113 | **E5** | **H5** | M114 | **E6** | **H5** |
| M115 | **E7** | **H5** | M116 | **E8** | **H5** | M117 | **E9** | **H5** |
| M118 | **E10** | **H5** | M119 | **E11** | **H5** | M120 | **E12** | **H5** |
| M121 | **E13** | **H5** | M122 | **E14** | **H5** | M123 | **E15** | **H5** |
| M124 | **E16** | **H5** | M125 | **E17** | **H5** | M126 | **E18** | **H5** |
| M127 | **E19** | **H5** | M128 | **E20** | **H5** | M129 | **E21** | **H5** |
| M130 | **E22** | **H5** | M131 | **E23** | **H5** | M132 | **E24** | **H5** |
| M133 | **E25** | **H5** | M134 | **E26** | **H5** | M135 | **E27** | **H5** |
| M136 | **E1** | **H6** | M137 | **E2** | **H6** | M138 | **E3** | **H6** |
| M139 | **E4** | **H6** | M140 | **E5** | **H6** | M141 | **E6** | **H6** |
| M142 | **E7** | **H6** | M143 | **E8** | **H6** | M144 | **E9** | **H6** |
| M145 | **E10** | **H6** | M146 | **E11** | **H6** | M147 | **E12** | **H6** |
| M148 | **E13** | **H6** | M149 | **E14** | **H6** | M150 | **E15** | **H6** |
| M151 | **E16** | **H6** | M152 | **E17** | **H6** | M153 | **E18** | **H6** |
| M154 | **E19** | **H6** | M155 | **E20** | **H6** | M156 | **E21** | **H6** |
| M157 | **E22** | **H6** | M158 | **E23** | **H6** | M159 | **E24** | **H6** |
| M160 | **E25** | **H6** | M161 | **E26** | **H6** | M162 | **E27** | **H6** |
| M163 | **E1** | **H7** | M164 | **E2** | **H7** | M165 | **E3** | **H7** |
| M166 | **E4** | **H7** | M167 | **E5** | **H7** | M168 | **E6** | **H7** |
| M169 | **E7** | **H7** | M170 | **E8** | **H7** | M171 | **E9** | **H7** |
| M172 | **E10** | **H7** | M173 | **E11** | **H7** | M174 | **E12** | **H7** |
| M175 | **E13** | **H7** | M176 | **E14** | **H7** | M177 | **E15** | **H7** |

(fortgesetzt)

| | | | | | | | | |
|---|---|---|---|---|---|---|---|---|
| M178 | **E16** | **H7** | M179 | **E17** | **H7** | M180 | **E18** | **H7** |
| M181 | **E19** | **H7** | M182 | **E20** | **H7** | M183 | **E21** | **H7** |
| M184 | **E22** | **H7** | M185 | **E23** | **H7** | M186 | **E24** | **H7** |
| M187 | **E25** | **H7** | M188 | **E26** | **H7** | M189 | **E27** | **H7** |
| M190 | **E1** | **H8** | M191 | **E2** | **H4** | M192 | **E3** | **H4** |
| M193 | **E4** | **H8** | M194 | **E5** | **H8** | M195 | **E6** | **H8** |
| M196 | **E7** | **H8** | M197 | **E8** | **H8** | M198 | **E9** | **H8** |
| M199 | **E10** | **H8** | M200 | **E11** | **H8** | M201 | **E12** | **H8** |
| M202 | **E13** | **H8** | M203 | **E14** | **H8** | M204 | **E15** | **H8** |
| M205 | **E16** | **H8** | M206 | **E17** | **H8** | M207 | **E18** | **H8** |
| M208 | **E19** | **H8** | M209 | **E20** | **H8** | M210 | **E21** | **H8** |
| M211 | **E22** | **H8** | M212 | **E23** | **H8** | M213 | **E24** | **H8** |
| M214 | **E25** | **H8** | M215 | **E26** | **H8** | M216 | **E27** | **H8** |
| M217 | **E1** | **H9** | M218 | **E2** | **H9** | M219 | **E3** | **H9** |
| M220 | **E4** | **H9** | M221 | **E5** | **H9** | M222 | **E6** | **H9** |
| M223 | **E7** | **H9** | M224 | **E8** | **H9** | M225 | **E9** | **H9** |
| M226 | **E10** | **H9** | M227 | **E11** | **H9** | M228 | **E12** | **H9** |
| M229 | **E13** | **H9** | M230 | **E14** | **H9** | M231 | **E15** | **H9** |
| M232 | **E16** | **H9** | M233 | **E17** | **H9** | M234 | **E18** | **H9** |
| M235 | **E19** | **H9** | M236 | **E20** | **H9** | M237 | **E21** | **H9** |
| M238 | **E22** | **H9** | M239 | **E23** | **H9** | M240 | **E24** | **H9** |
| M241 | **E25** | **H9** | M242 | **E26** | **H9** | M243 | **E27** | **H9** |
| M244 | **E1** | **H10** | M245 | **E2** | **H10** | M246 | **E3** | **H10** |
| M247 | **E4** | **H10** | M248 | **E5** | **H10** | M249 | **E6** | **H10** |
| M250 | **E7** | **H10** | M251 | **E8** | **H10** | M252 | **E9** | **H10** |
| M253 | **E10** | **H10** | M254 | **E11** | **H10** | M255 | **E12** | **H10** |
| M256 | **E13** | **H10** | M257 | **E14** | **H10** | M258 | **E15** | **H10** |
| M259 | **E16** | **H10** | M260 | **E17** | **H10** | M261 | **E18** | **H10** |
| M262 | **E19** | **H10** | M263 | **E20** | **H10** | M264 | **E21** | **H10** |
| M265 | **E22** | **H10** | M266 | **E23** | **H10** | M267 | **E24** | **H10** |
| M268 | **E25** | **H10** | M269 | **E26** | **H10** | M270 | **E27** | **H10** |
| M271 | **E1** | **H11** | M272 | **E2** | **H11** | M273 | **E3** | **H11** |
| M274 | **E4** | **H11** | M275 | **E5** | **H11** | M276 | **E6** | **H11** |
| M277 | **E7** | **H11** | M278 | **E8** | **H11** | M279 | **E9** | **H11** |
| M280 | **E10** | **H11** | M281 | **E11** | **H11** | M282 | **E12** | **H11** |
| M283 | **E13** | **H11** | M284 | **E14** | **H11** | M285 | **E15** | **H11** |
| M286 | **E16** | **H11** | M287 | **E17** | **H11** | M288 | **E18** | **H11** |
| M289 | **E19** | **H11** | M290 | **E20** | **H11** | M291 | **E21** | **H11** |
| M292 | **E22** | **H11** | M293 | **E23** | **H11** | M294 | **E24** | **H11** |
| M295 | **E25** | **H11** | M296 | **E26** | **H11** | M297 | **E27** | **H11** |

(fortgesetzt)

| | | | | | | | | |
|---|---|---|---|---|---|---|---|---|
| M298 | **E1** | **H12** | M299 | **E2** | **H12** | M300 | **E3** | **H12** |
| M301 | **E4** | **H12** | M302 | **E5** | **H12** | M303 | **E6** | **H12** |
| M304 | **E7** | **H12** | M305 | **E8** | **H12** | M306 | **E9** | **H12** |
| M307 | **E10** | **H12** | M308 | **E11** | **H12** | M309 | **E12** | **H12** |
| M310 | **E13** | **H12** | M311 | **E14** | **H12** | M312 | **E15** | **H12** |
| M313 | **E16** | **H12** | M314 | **E17** | **H12** | M315 | **E18** | **H12** |
| M316 | **E19** | **H12** | M317 | **E20** | **H12** | M318 | **E21** | **H12** |
| M319 | **E22** | **H12** | M320 | **E23** | **H12** | M321 | **E24** | **H12** |
| M322 | **E25** | **H12** | M323 | **E26** | **H12** | M324 | **E27** | **H12** |
| M325 | **E1** | **H13** | M326 | **E2** | **H13** | M327 | **E3** | **H13** |
| M328 | **E4** | **H13** | M329 | **E5** | **H13** | M330 | **E6** | **H13** |
| M331 | **E7** | **H13** | M332 | **E8** | **H13** | M333 | **E9** | **H13** |
| M334 | **E10** | **H13** | M335 | **E11** | **H13** | M336 | **E12** | **H13** |
| M337 | **E13** | **H13** | M338 | **E14** | **H13** | M339 | **E15** | **H13** |
| M340 | **E16** | **H13** | M341 | **E17** | **H13** | M342 | **E18** | **H13** |
| M343 | **E19** | **H13** | M344 | **E20** | **H13** | M345 | **E21** | **H13** |
| M346 | **E22** | **H13** | M347 | **E23** | **H13** | M348 | **E24** | **H13** |
| M349 | **E25** | **H13** | M350 | **E26** | **H13** | M351 | **E27** | **H13** |
| M352 | **E1** | **H14** | M353 | **E2** | **H14** | M354 | **E3** | **H14** |
| M355 | **E4** | **H14** | M356 | **E5** | **H14** | M357 | **E6** | **H14** |
| M358 | **E7** | **H14** | M359 | **E8** | **H14** | M360 | **E9** | **H14** |
| M361 | **E10** | **H14** | M362 | **E11** | **H14** | M363 | **E12** | **H14** |
| M364 | **E13** | **H14** | M365 | **E14** | **H14** | M366 | **E15** | **H14** |
| M367 | **E16** | **H14** | M368 | **E17** | **H14** | M369 | **E18** | **H14** |
| M370 | **E19** | **H14** | M371 | **E20** | **H14** | M372 | **E21** | **H14** |
| M373 | **E22** | **H14** | M374 | **E23** | **H14** | M375 | **E24** | **H14** |
| M376 | **E25** | **H14** | M377 | **E26** | **H14** | M378 | **E27** | **H14** |
| M379 | **E1** | **H15** | M380 | **E2** | **H15** | M381 | **E3** | **H15** |
| M382 | **E4** | **H15** | M383 | **E5** | **H15** | M384 | **E6** | **H15** |
| M385 | **E7** | **H15** | M386 | **E8** | **H15** | M387 | **E9** | **H15** |
| M388 | **E10** | **H15** | M389 | **E11** | **H15** | M390 | **E12** | **H15** |
| M391 | **E13** | **H15** | M392 | **E14** | **H15** | M393 | **E15** | **H15** |
| M394 | **E16** | **H15** | M395 | **E17** | **H15** | M396 | **E18** | **H15** |
| M397 | **E19** | **H15** | M398 | **E20** | **H15** | M399 | **E21** | **H15** |
| M400 | **E22** | **H15** | M401 | **E23** | **H15** | M402 | **E24** | **H15** |
| M403 | **E25** | **H15** | M404 | **E26** | **H15** | M405 | **E27** | **H15** |
| M406 | **E1** | **H16** | M407 | **E2** | **H16** | M408 | **E3** | **H16** |
| M409 | **E4** | **H16** | M410 | **E5** | **H16** | M411 | **E6** | **H16** |
| M412 | **E7** | **H16** | M413 | **E8** | **H16** | M414 | **E9** | **H16** |
| M415 | **E10** | **H16** | M416 | **E11** | **H16** | M417 | **E12** | **H16** |

(fortgesetzt)

| | | | | | | | | |
|---|---|---|---|---|---|---|---|---|
| M418 | **E13** | **H16** | M419 | **E14** | **H16** | M420 | **E15** | **H16** |
| M421 | **E16** | **H16** | M422 | **E17** | **H16** | M423 | **E18** | **H16** |
| M424 | **E19** | **H16** | M425 | **E20** | **H16** | M426 | **E21** | **H16** |
| M427 | **E22** | **H16** | M428 | **E23** | **H16** | M429 | **E24** | **H16** |
| M430 | **E25** | **H16** | M431 | **E26** | **H16** | M432 | **E27** | **H16** |
| M433 | **E1** | **H17** | M434 | **E2** | **H17** | M435 | **E3** | **H17** |
| M436 | **E4** | **H17** | M437 | **E5** | **H17** | M438 | **E6** | **H17** |
| M439 | **E7** | **H17** | M440 | **E8** | **H17** | M441 | **E9** | **H17** |
| M442 | **E10** | **H17** | M443 | **E11** | **H17** | M444 | **E12** | **H17** |
| M445 | **E13** | **H17** | M446 | **E14** | **H17** | M447 | **E15** | **H17** |
| M448 | **E16** | **H17** | M449 | **E17** | **H17** | M450 | **E18** | **H17** |
| M451 | **E19** | **H17** | M452 | **E20** | **H17** | M453 | **E21** | **H17** |
| M454 | **E22** | **H17** | M455 | **E23** | **H17** | M456 | **E24** | **H17** |
| M457 | **E25** | **H17** | M458 | **E26** | **H17** | M459 | **E27** | **H17** |
| M460 | **E1** | **H18** | M461 | **E2** | **H18** | M462 | **E3** | **H18** |
| M463 | **E4** | **H18** | M464 | **E5** | **H18** | M465 | **E6** | **H18** |
| M466 | **E7** | **H18** | M467 | **E8** | **H18** | M468 | **E9** | **H18** |
| M469 | **E10** | **H18** | M470 | **E11** | **H18** | M471 | **E12** | **H18** |
| M472 | **E13** | **H18** | M473 | **E14** | **H18** | M474 | **E15** | **H18** |
| M475 | **E16** | **H18** | M476 | **E17** | **H18** | M477 | **E18** | **H18** |
| M478 | **E19** | **H18** | M479 | **E20** | **H18** | M480 | **E21** | **H18** |
| M481 | **E22** | **H18** | M482 | **E23** | **H18** | M483 | **E24** | **H18** |
| M484 | **E25** | **H18** | M485 | **E26** | **H18** | M486 | **E27** | **H18** |
| M487 | **E1** | **H19** | M488 | **E2** | **H19** | M489 | **E3** | **H19** |
| M490 | **E4** | **H19** | M491 | **E5** | **H19** | M492 | **E6** | **H19** |
| M493 | **E7** | **H19** | M494 | **E8** | **H19** | M495 | **E9** | **H19** |
| M496 | **E10** | **H19** | M497 | **E11** | **H19** | M498 | **E12** | **H19** |
| M499 | **E13** | **H19** | M500 | **E14** | **H19** | M501 | **E15** | **H19** |
| M502 | **E16** | **H19** | M503 | **E17** | **H19** | M504 | **E18** | **H19** |
| M505 | **E19** | **H19** | M506 | **E20** | **H19** | M507 | **E21** | **H19** |
| M508 | **E22** | **H19** | M509 | **E23** | **H19** | M510 | **E24** | **H19** |
| M511 | **E25** | **H19** | M512 | **E26** | **H19** | M513 | **E27** | **H19** |
| M514 | **E1** | **H20** | M515 | **E2** | **H20** | M516 | **E3** | **H20** |
| M517 | **E4** | **H20** | M518 | **E5** | **H20** | M519 | **E6** | **H20** |
| M520 | **E7** | **H20** | M521 | **E8** | **H20** | M522 | **E9** | **H20** |
| M523 | **E10** | **H20** | M524 | **E11** | **H20** | M525 | **E12** | **H20** |
| M526 | **E13** | **H20** | M527 | **E14** | **H20** | M528 | **E15** | **H20** |
| M529 | **E16** | **H20** | M530 | **E17** | **H20** | M531 | **E18** | **H20** |
| M532 | **E19** | **H20** | M533 | **E20** | **H20** | M534 | **E21** | **H20** |
| M535 | **E22** | **H20** | M536 | **E23** | **H20** | M537 | **E24** | **H20** |

(fortgesetzt)

| | | | | | | | | |
|---|---|---|---|---|---|---|---|---|
| M538 | **E25** | **H20** | M539 | **E26** | **H20** | M540 | **E27** | **H20** |
| M541 | **E1** | **H21** | M542 | **E2** | **H21** | M543 | **E3** | **H21** |
| M544 | **E4** | **H21** | M545 | **E5** | **H21** | M546 | **E6** | **H21** |
| M547 | **E7** | **H21** | M548 | **E8** | **H21** | M549 | **E9** | **H21** |
| M550 | **E10** | **H21** | M551 | **E11** | **H21** | M552 | **E12** | **H21** |
| M553 | **E13** | **H21** | M554 | **E14** | **H21** | M555 | **E15** | **H21** |
| M556 | **E16** | **H21** | M557 | **E17** | **H21** | M558 | **E18** | **H21** |
| M559 | **E19** | **H21** | M560 | **E20** | **H21** | M561 | **E21** | **H21** |
| M562 | **E22** | **H21** | M563 | **E23** | **H21** | M564 | **E24** | **H21** |
| M565 | **E25** | **H21** | M566 | **E26** | **H21** | M567 | **E27** | **H21** |
| M568 | **E1** | **H22** | M569 | **E2** | **H22** | M570 | **E3** | **H22** |
| M571 | **E4** | **H22** | M572 | **E5** | **H22** | M573 | **E6** | **H22** |
| M574 | **E7** | **H22** | M575 | **E8** | **H22** | M576 | **E9** | **H22** |
| M577 | **E10** | **H22** | M578 | **E11** | **H22** | M579 | **E12** | **H22** |
| M580 | **E13** | **H22** | M581 | **E14** | **H22** | M582 | **E15** | **H22** |
| M583 | **E16** | **H22** | M584 | **E17** | **H22** | M585 | **E18** | **H22** |
| M586 | **E19** | **H22** | M587 | **E20** | **H22** | M588 | **E21** | **H22** |
| M589 | **E22** | **H22** | M590 | **E23** | **H22** | M591 | **E24** | **H22** |
| M592 | **E25** | **H22** | M593 | **E26** | **H22** | M594 | **E27** | **H22** |
| M595 | **E1** | **H23** | M596 | **E2** | **H23** | M597 | **E3** | **H23** |
| M598 | **E4** | **H23** | M599 | **E5** | **H23** | M600 | **E6** | **H23** |
| M601 | **E7** | **H23** | M602 | **E8** | **H23** | M603 | **E9** | **H23** |
| M604 | **E10** | **H23** | M605 | **E11** | **H23** | M606 | **E12** | **H23** |
| M607 | **E13** | **H23** | M608 | **E14** | **H23** | M609 | **E15** | **H23** |
| M610 | **E16** | **H23** | M611 | **E17** | **H23** | M612 | **E18** | **H23** |
| M613 | **E19** | **H23** | M614 | **E20** | **H23** | M615 | **E21** | **H23** |
| M616 | **E22** | **H23** | M617 | **E23** | **H23** | M618 | **E24** | **H23** |
| M619 | **E25** | **H23** | M620 | **E26** | **H23** | M621 | **E27** | **H23** |
| M622 | **E1** | **H24** | M623 | **E2** | **H24** | M624 | **E3** | **H24** |
| M625 | **E4** | **H24** | M626 | **E5** | **H24** | M627 | **E6** | **H24** |
| M628 | **E7** | **H24** | M629 | **E8** | **H24** | M630 | **E9** | **H24** |
| M631 | **E10** | **H24** | M632 | **E11** | **H24** | M633 | **E12** | **H24** |
| M634 | **E13** | **H24** | M635 | **E14** | **H24** | M636 | **E15** | **H24** |
| M637 | **E16** | **H24** | M638 | **E17** | **H24** | M639 | **E18** | **H24** |
| M640 | **E19** | **H24** | M641 | **E20** | **H24** | M642 | **E21** | **H24** |
| M643 | **E22** | **H24** | M644 | **E23** | **H24** | M645 | **E24** | **H24** |
| M646 | **E25** | **H24** | M647 | **E26** | **H24** | M648 | **E27** | **H24** |
| M649 | **E1** | **H25** | M650 | **E2** | **H25** | M651 | **E3** | **H25** |
| M652 | **E4** | **H25** | M653 | **E5** | **H25** | M654 | **E6** | **H25** |
| M655 | **E7** | **H25** | M656 | **E8** | **H25** | M657 | **E9** | **H25** |

(fortgesetzt)

| | | | | | | | | |
|---|---|---|---|---|---|---|---|---|
| M658 | **E10** | **H25** | M659 | **E11** | **H25** | M660 | **E12** | **H25** |
| M661 | **E13** | **H25** | M662 | **E14** | **H25** | M663 | **E15** | **H25** |
| M664 | **E16** | **H25** | M665 | **E17** | **H25** | M666 | **E18** | **H25** |
| M667 | **E19** | **H25** | M668 | **E20** | **H25** | M669 | **E21** | **H25** |
| M670 | **E22** | **H25** | M671 | **E23** | **H25** | M672 | **E24** | **H25** |
| M673 | **E25** | **H25** | M674 | **E26** | **H25** | M675 | **E27** | **H25** |
| M676 | **E1** | **H26** | M677 | **E2** | **H26** | M678 | **E3** | **H26** |
| M679 | **E4** | **H26** | M680 | **E5** | **H26** | M681 | **E6** | **H26** |
| M682 | **E7** | **H26** | M683 | **E8** | **H26** | M684 | **E9** | **H26** |
| M685 | **E10** | **H26** | M686 | **E11** | **H26** | M687 | **E12** | **H26** |
| M688 | **E13** | **H26** | M689 | **E14** | **H26** | M690 | **E15** | **H26** |
| M691 | **E16** | **H26** | M692 | **E17** | **H26** | M693 | **E18** | **H26** |
| M694 | **E19** | **H26** | M695 | **E20** | **H26** | M696 | **E21** | **H26** |
| M697 | **E22** | **H26** | M698 | **E23** | **H26** | M699 | **E24** | **H26** |
| M700 | **E25** | **H26** | M701 | **E26** | **H26** | M702 | **E27** | **H26** |
| M703 | **E1** | **H27** | M704 | **E2** | **H27** | M705 | **E3** | **H27** |
| M706 | **E4** | **H27** | M707 | **E5** | **H27** | M708 | **E6** | **H27** |
| M709 | **E7** | **H27** | M710 | **E8** | **H27** | M711 | **E9** | **H27** |
| M712 | **E10** | **H27** | M713 | **E11** | **H27** | M714 | **E12** | **H27** |
| M715 | **E13** | **H27** | M716 | **E14** | **H27** | M717 | **E15** | **H27** |
| M718 | **E16** | **H27** | M719 | **E17** | **H27** | M720 | **E18** | **H27** |
| M721 | **E19** | **H27** | M722 | **E20** | **H27** | M723 | **E21** | **H27** |
| M724 | **E22** | **H27** | M725 | **E23** | **H27** | M726 | **E24** | **H27** |
| M727 | **E25** | **H27** | M728 | **E26** | **H27** | M729 | **E27** | **H27** |
| M730 | **E1** | **H28** | M731 | **E2** | **H28** | M732 | **E3** | **H28** |
| M733 | **E4** | **H28** | M734 | **E5** | **H28** | M735 | **E6** | **H28** |
| M736 | **E7** | **H28** | M737 | **E8** | **H28** | M738 | **E9** | **H28** |
| M739 | **E10** | **H28** | M740 | **E11** | **H28** | M741 | **E12** | **H28** |
| M742 | **E13** | **H28** | M743 | **E14** | **H28** | M744 | **E15** | **H28** |
| M745 | **E16** | **H28** | M746 | **E17** | **H28** | M747 | **E18** | **H28** |
| M748 | **E19** | **H28** | M749 | **E20** | **H28** | M750 | **E21** | **H28** |
| M751 | **E22** | **H28** | M752 | **E23** | **H28** | M753 | **E24** | **H28** |
| M754 | **E25** | **H28** | M755 | **E26** | **H28** | M756 | **E27** | **H28** |
| M757 | **E1** | **H29** | M758 | **E2** | **H29** | M759 | **E3** | **H29** |
| M760 | **E4** | **H29** | M761 | **E5** | **H29** | M762 | **E6** | **H29** |
| M763 | **E7** | **H29** | M764 | **E8** | **H29** | M765 | **E9** | **H29** |
| M766 | **E10** | **H29** | M767 | **E11** | **H29** | M768 | **E12** | **H29** |
| M769 | **E13** | **H29** | M770 | **E14** | **H29** | M771 | **E15** | **H29** |
| M772 | **E16** | **H29** | M773 | **E17** | **H29** | M774 | **E18** | **H29** |
| M775 | **E19** | **H29** | M776 | **E20** | **H29** | M777 | **E21** | **H29** |

(fortgesetzt)

| | | | | | | | | |
|---|---|---|---|---|---|---|---|---|
| M778 | **E22** | **H29** | M779 | **E23** | **H29** | M780 | **E24** | **H29** |
| M781 | **E25** | **H29** | M782 | **E26** | **H29** | M783 | **E27** | **H29** |
| M784 | **E1** | **H30** | M785 | **E2** | **H30** | M786 | **E3** | **H30** |
| M787 | **E4** | **H30** | M788 | **E5** | **H30** | M789 | **E6** | **H30** |
| M790 | **E7** | **H30** | M791 | **E8** | **H30** | M792 | **E9** | **H30** |
| M793 | **E10** | **H30** | M794 | **E11** | **H30** | M795 | **E12** | **H30** |
| M796 | **E13** | **H30** | M797 | **E14** | **H30** | M798 | **E15** | **H30** |
| M799 | **E16** | **H30** | M800 | **E17** | **H30** | M801 | **E18** | **H30** |
| M802 | **E19** | **H30** | M803 | **E20** | **H30** | M804 | **E21** | **H30** |
| M805 | **E22** | **H30** | M806 | **E23** | **H30** | M807 | **E24** | **H30** |
| M808 | **E25** | **H30** | M809 | **E26** | **H30** | M810 | **E27** | **H30** |
| M811 | **E1** | **H31** | M812 | **E2** | **H31** | M813 | **E3** | **H31** |
| M814 | **E4** | **H31** | M815 | **E5** | **H31** | M816 | **E6** | **H31** |
| M817 | **E7** | **H31** | M818 | **E8** | **H31** | M819 | **E9** | **H31** |
| M820 | **E10** | **H31** | M821 | **E11** | **H31** | M822 | **E12** | **H31** |
| M823 | **E13** | **H31** | M824 | **E14** | **H31** | M825 | **E15** | **H31** |
| M826 | **E16** | **H31** | M827 | **E17** | **H31** | M828 | **E18** | **H31** |
| M829 | **E19** | **H31** | M830 | **E20** | **H31** | M831 | **E21** | **H31** |
| M832 | **E22** | **H31** | M833 | **E23** | **H31** | M834 | **E24** | **H31** |
| M835 | **E25** | **H31** | M836 | **E26** | **H31** | M837 | **E27** | **H31** |
| M838 | **E1** | **H32** | M839 | **E2** | **H32** | M840 | **E3** | **H32** |
| M841 | **E4** | **H32** | M842 | **E5** | **H32** | M843 | **E6** | **H32** |
| M844 | **E7** | **H32** | M845 | **E8** | **H32** | M846 | **E9** | **H32** |
| M847 | **E10** | **H32** | M848 | **E11** | **H32** | M849 | **E12** | **H32** |
| M850 | **E13** | **H32** | M851 | **E14** | **H32** | M852 | **E15** | **H32** |
| M853 | **E16** | **H32** | M854 | **E17** | **H32** | M855 | **E18** | **H32** |
| M856 | **E19** | **H32** | M857 | **E20** | **H32** | M858 | **E21** | **H32** |
| M859 | **E22** | **H32** | M860 | **E23** | **H32** | M861 | **E24** | **H32** |
| M862 | **E25** | **H32** | M863 | **E26** | **H32** | M864 | **E27** | **H32** |
| M865 | **E1** | **H33** | M866 | **E2** | **H33** | M867 | **E3** | **H33** |
| M868 | **E4** | **H33** | M869 | **E5** | **H33** | M870 | **E6** | **H33** |
| M871 | **E7** | **H33** | M872 | **E8** | **H33** | M873 | **E9** | **H33** |
| M874 | **E10** | **H33** | M875 | **E11** | **H33** | M876 | **E12** | **H33** |
| M877 | **E13** | **H33** | M878 | **E14** | **H33** | M879 | **E15** | **H33** |
| M880 | **E16** | **H33** | M881 | **E17** | **H33** | M882 | **E18** | **H33** |
| M883 | **E19** | **H33** | M884 | **E20** | **H33** | M885 | **E21** | **H33** |
| M886 | **E22** | **H33** | M887 | **E23** | **H33** | M888 | **E24** | **H33** |
| M889 | **E25** | **H33** | M890 | **E26** | **H33** | M891 | **E27** | **H33** |
| M892 | **E1** | **H34** | M893 | **E2** | **H34** | M894 | **E3** | **H34** |
| M895 | **E4** | **H34** | M896 | **E5** | **H34** | M897 | **E6** | **H34** |

(fortgesetzt)

| | | | | | | | | |
|---|---|---|---|---|---|---|---|---|
| M898 | **E7** | **H34** | M899 | **E8** | **H34** | M900 | **E9** | **H34** |
| M901 | **E10** | **H34** | M902 | **E11** | **H34** | M903 | **E12** | **H34** |
| M904 | **E13** | **H34** | M905 | **E14** | **H34** | M906 | **E15** | **H34** |
| M907 | **E16** | **H34** | M908 | **E17** | **H34** | M909 | **E18** | **H34** |
| M910 | **E19** | **H34** | M911 | **E20** | **H34** | M912 | **E21** | **H34** |
| M913 | **E22** | **H34** | M914 | **E23** | **H34** | M915 | **E24** | **H34** |
| M916 | **E25** | **H34** | M917 | **E26** | **H34** | M918 | **E27** | **H34** |
| M919 | **E1** | **H35** | M920 | **E2** | **H35** | M921 | **E3** | **H35** |
| M922 | **E4** | **H35** | M923 | **E5** | **H35** | M924 | **E6** | **H35** |
| M925 | **E7** | **H35** | M926 | **E8** | **H35** | M927 | **E9** | **H35** |
| M928 | **E10** | **H35** | M929 | **E11** | **H35** | M930 | **E12** | **H35** |
| M931 | **E13** | **H35** | M932 | **E14** | **H35** | M933 | **E15** | **H35** |
| M934 | **E16** | **H35** | M935 | **E17** | **H35** | M936 | **E18** | **H35** |
| M937 | **E19** | **H35** | M938 | **E20** | **H35** | M939 | **E21** | **H35** |
| M940 | **E22** | **H35** | M941 | **E23** | **H35** | M942 | **E24** | **H35** |
| M943 | **E25** | **H35** | M944 | **E26** | **H35** | M945 | **E27** | **H35** |
| M946 | **E1** | **H36** | M947 | **E2** | **H36** | M948 | **E3** | **H36** |
| M949 | **E4** | **H36** | M950 | **E5** | **H36** | M951 | **E6** | **H36** |
| M952 | **E7** | **H36** | M953 | **E8** | **H36** | M954 | **E9** | **H36** |
| M955 | **E10** | **H36** | M956 | **E11** | **H36** | M957 | **E12** | **H36** |
| M958 | **E13** | **H36** | M959 | **E14** | **H36** | M960 | **E15** | **H36** |
| M961 | **E16** | **H36** | M962 | **E17** | **H36** | M963 | **E18** | **H36** |
| M964 | **E19** | **H36** | M965 | **E20** | **H36** | M966 | **E21** | **H36** |
| M967 | **E22** | **H36** | M968 | **E23** | **H36** | M969 | **E24** | **H36** |
| M970 | **E25** | **H36** | M971 | **E26** | **H36** | M972 | **E27** | **H36** |
| M973 | **E1** | **H37** | M974 | **E2** | **H37** | M975 | **E3** | **H37** |
| M976 | **E4** | **H37** | M977 | **E5** | **H37** | M978 | **E6** | **H37** |
| M979 | **E7** | **H37** | M980 | **E8** | **H37** | M981 | **E9** | **H37** |
| M982 | **E10** | **H37** | M983 | **E11** | **H37** | M984 | **E12** | **H37** |
| M985 | **E13** | **H37** | M986 | **E14** | **H37** | M987 | **E15** | **H37** |
| M988 | **E16** | **H37** | M989 | **E17** | **H37** | M990 | **E18** | **H37** |
| M991 | **E19** | **H37** | M992 | **E20** | **H37** | M993 | **E21** | **H37** |
| M994 | **E22** | **H37** | M995 | **E23** | **H37** | M996 | **E24** | **H37** |
| M997 | **E25** | **H37** | M998 | **E26** | **H37** | M999 | **E27** | **H37** |
| M1000 | **E1** | **H38** | M1001 | **E2** | **H38** | M1002 | **E3** | **H38** |
| M1003 | **E4** | **H38** | M1004 | **E5** | **H38** | M1005 | **E6** | **H38** |
| M1006 | **E7** | **H38** | M1007 | **E8** | **H38** | M1008 | **E9** | **H38** |
| M1009 | **E10** | **H38** | M1010 | **E11** | **H38** | M1011 | **E12** | **H38** |
| M1012 | **E13** | **H38** | M1013 | **E14** | **H38** | M1014 | **E15** | **H38** |
| M1015 | **E16** | **H38** | M1016 | **E17** | **H38** | M1017 | **E18** | **H38** |

(fortgesetzt)

| | | | | | | | | |
|---|---|---|---|---|---|---|---|---|
| M1018 | **E19** | **H38** | M1019 | **E20** | **H38** | M1020 | **E21** | **H38** |
| M1021 | **E22** | **H38** | M1022 | **E23** | **H38** | M1023 | **E24** | **H38** |
| M1024 | **E25** | **H38** | M1025 | **E26** | **H38** | M1026 | **E27** | **H38** |
| M1027 | **E1** | **H39** | M1028 | **E2** | **H39** | M1029 | **E3** | **H39** |
| M1030 | **E4** | **H39** | M1031 | **E5** | **H39** | M1032 | **E6** | **H39** |
| M1033 | **E7** | **H39** | M1034 | **E8** | **H39** | M1035 | **E9** | **H39** |
| M1036 | **E10** | **H39** | M1037 | **E11** | **H39** | M1038 | **E12** | **H39** |
| M1039 | **E13** | **H39** | M1040 | **E14** | **H39** | M1041 | **E15** | **H39** |
| M1042 | **E16** | **H39** | M1043 | **E17** | **H39** | M1044 | **E18** | **H39** |
| M1045 | **E19** | **H39** | M1046 | **E20** | **H39** | M1047 | **E21** | **H39** |
| M1048 | **E22** | **H39** | M1049 | **E23** | **H39** | M1050 | **E24** | **H39** |
| M1051 | **E25** | **H39** | M1052 | **E26** | **H39** | M1053 | **E27** | **H39** |
| M1054 | **E1** | **H40** | M1055 | **E2** | **H40** | M1056 | **E3** | **H40** |
| M1057 | **E4** | **H40** | M1058 | **E5** | **H40** | M1059 | **E6** | **H40** |
| M1060 | **E7** | **H40** | M1061 | **E8** | **H40** | M1062 | **E9** | **H40** |
| M1063 | **E10** | **H40** | M1064 | **E11** | **H40** | M1065 | **E12** | **H40** |
| M1066 | **E13** | **H40** | M1067 | **E14** | **H40** | M1068 | **E15** | **H40** |
| M1069 | **E16** | **H40** | M1070 | **E17** | **H40** | M1071 | **E18** | **H40** |
| M1072 | **E19** | **H40** | M1073 | **E20** | **H40** | M1074 | **E21** | **H40** |
| M1075 | **E22** | **H40** | M1076 | **E23** | **H40** | M1077 | **E24** | **H40** |
| M1078 | **E25** | **H40** | M1079 | **E26** | **H40** | M1080 | **E27** | **H40** |
| M1081 | **E1** | **H41** | M1082 | **E2** | **H41** | M1083 | **E3** | **H41** |
| M1084 | **E4** | **H41** | M1085 | **E5** | **H41** | M1086 | **E6** | **H41** |
| M1087 | **E7** | **H41** | M1088 | **E8** | **H41** | M1089 | **E9** | **H41** |
| M1090 | **E10** | **H41** | M1091 | **E11** | **H41** | M1092 | **E12** | **H41** |
| M1093 | **E13** | **H41** | M1094 | **E14** | **H41** | M1095 | **E15** | **H41** |
| M1096 | **E16** | **H41** | M1097 | **E17** | **H41** | M1098 | **E18** | **H41** |
| M1099 | **E19** | **H41** | M1100 | **E20** | **H41** | M1101 | **E21** | **H41** |
| M1102 | **E22** | **H41** | M1103 | **E23** | **H41** | M1104 | **E24** | **H41** |
| M1105 | **E25** | **H41** | M1106 | **E26** | **H41** | M1107 | **E27** | **H41** |
| M1108 | **E1** | **H42** | M1109 | **E2** | **H42** | M1110 | **E3** | **H42** |
| M1111 | **E4** | **H42** | M1112 | **E5** | **H42** | M1113 | **E6** | **H42** |
| M1114 | **E7** | **H42** | M1115 | **E8** | **H42** | M1116 | **E9** | **H42** |
| M1117 | **E10** | **H42** | M1118 | **E11** | **H42** | M1119 | **E12** | **H42** |
| M1120 | **E13** | **H42** | M1121 | **E14** | **H42** | M1122 | **E15** | **H42** |
| M1123 | **E16** | **H42** | M1124 | **E17** | **H42** | M1125 | **E18** | **H42** |
| M1126 | **E19** | **H42** | M1127 | **E20** | **H42** | M1128 | **E21** | **H42** |
| M1129 | **E22** | **H42** | M1130 | **E23** | **H42** | M1131 | **E24** | **H42** |
| M1132 | **E25** | **H42** | M1133 | **E26** | **H42** | M1134 | **E27** | **H42** |
| M1135 | **E1** | **H43** | M1136 | **E2** | **H43** | M1137 | **E3** | **H43** |

(fortgesetzt)

| | | | | | | | | |
|---|---|---|---|---|---|---|---|---|
| M1138 | **E4** | **H43** | M1139 | **E5** | **H43** | M1140 | **E6** | **H43** |
| M1141 | **E7** | **H43** | M1142 | **E8** | **H43** | M1143 | **E9** | **H43** |
| M1144 | **E10** | **H43** | M1145 | **E11** | **H43** | M1146 | **E12** | **H43** |
| M1147 | **E13** | **H43** | M1148 | **E14** | **H43** | M1149 | **E15** | **H43** |
| M1150 | **E16** | **H43** | M1151 | **E17** | **H43** | M1152 | **E18** | **H43** |
| M1153 | **E19** | **H43** | M1154 | **E20** | **H43** | M1155 | **E21** | **H43** |
| M1156 | **E22** | **H43** | M1157 | **E23** | **H43** | M1158 | **E24** | **H43** |
| M1159 | **E25** | **H43** | M1160 | **E26** | **H43** | M1161 | **E27** | **H43** |
| M1162 | **E1** | **H44** | M1163 | **E2** | **H44** | M1164 | **E3** | **H44** |
| M1165 | **E4** | **H44** | M1166 | **E5** | **H44** | M1167 | **E6** | **H44** |
| M1168 | **E7** | **H44** | M1169 | **E8** | **H44** | M1170 | **E9** | **H44** |
| M1171 | **E10** | **H44** | M1172 | **E11** | **H44** | M1173 | **E12** | **H44** |
| M1174 | **E13** | **H44** | M1175 | **E14** | **H44** | M1176 | **E15** | **H44** |
| M1177 | **E16** | **H44** | M1178 | **E17** | **H44** | M1179 | **E18** | **H44** |
| M1180 | **E19** | **H44** | M1181 | **E20** | **H44** | M1182 | **E21** | **H44** |
| M1183 | **E22** | **H44** | M1184 | **E23** | **H44** | M1185 | **E24** | **H44** |
| M1186 | **E25** | **H44** | M1187 | **E26** | **H44** | M1188 | **E27** | **H44** |
| M1189 | **E1** | **H45** | M1190 | **E2** | **H45** | M1191 | **E3** | **H45** |
| M1192 | **E4** | **H45** | M1193 | **E5** | **H45** | M1194 | **E6** | **H45** |
| M1195 | **E7** | **H45** | M1196 | **E8** | **H45** | M1197 | **E9** | **H45** |
| M1198 | **E10** | **H45** | M1199 | **E11** | **H45** | M1200 | **E12** | **H45** |
| M1201 | **E13** | **H45** | M1202 | **E14** | **H45** | M1203 | **E15** | **H45** |
| M1204 | **E16** | **H45** | M1205 | **E17** | **H45** | M1206 | **E18** | **H45** |
| M1207 | **E19** | **H45** | M1208 | **E20** | **H45** | M1209 | **E21** | **H45** |
| M1210 | **E22** | **H45** | M1211 | **E23** | **H45** | M1212 | **E24** | **H45** |
| M1213 | **E25** | **H45** | M1214 | **E26** | **H45** | M1215 | **E27** | **H45** |
| M1216 | **E1** | **H46** | M1217 | **E2** | **H46** | M1218 | **E3** | **H46** |
| M1219 | **E4** | **H46** | M1220 | **E5** | **H46** | M1221 | **E6** | **H46** |
| M1222 | **E7** | **H46** | M1223 | **E8** | **H46** | M1224 | **E9** | **H46** |
| M1225 | **E10** | **H46** | M1226 | **E11** | **H46** | M1227 | **E12** | **H46** |
| M1228 | **E13** | **H46** | M1229 | **E14** | **H46** | M1230 | **E15** | **H46** |
| M1231 | **E16** | **H46** | M1232 | **E17** | **H46** | M1233 | **E18** | **H46** |
| M1234 | **E19** | **H46** | M1235 | **E20** | **H46** | M1236 | **E21** | **H46** |
| M1237 | **E22** | **H46** | M1238 | **E23** | **H46** | M1239 | **E24** | **H46** |
| M1240 | **E25** | **H46** | M1241 | **E26** | **H46** | M1242 | **E27** | **H46** |
| M1243 | **E1** | **H47** | M1244 | **E2** | **H47** | M1245 | **E3** | **H47** |
| M1246 | **E4** | **H47** | M1247 | **E5** | **H47** | M1248 | **E6** | **H47** |
| M1249 | **E7** | **H47** | M1250 | **E8** | **H47** | M1251 | **E9** | **H47** |
| M1252 | **E10** | **H47** | M1253 | **E11** | **H47** | M1254 | **E12** | **H47** |
| M1255 | **E13** | **H47** | M1256 | **E14** | **H47** | M1257 | **E15** | **H47** |

(fortgesetzt)

| | | | | | | | | |
|---|---|---|---|---|---|---|---|---|
| M1258 | **E16** | **H47** | M1259 | **E17** | **H47** | M1260 | **E18** | **H47** |
| M1261 | **E19** | **H47** | M1262 | **E20** | **H47** | M1263 | **E21** | **H47** |
| M1264 | **E22** | **H47** | M1265 | **E23** | **H47** | M1266 | **E24** | **H47** |
| M1267 | **E25** | **H47** | M1268 | **E26** | **H47** | M1269 | **E27** | **H47** |
| M1270 | **E1** | **H48** | M1271 | **E2** | **H48** | M1272 | **E3** | **H48** |
| M1273 | **E4** | **H48** | M1274 | **E5** | **H48** | M1275 | **E6** | **H48** |
| M1276 | **E7** | **H48** | M1277 | **E8** | **H48** | M1278 | **E9** | **H48** |
| M1279 | **E10** | **H48** | M1280 | **E11** | **H48** | M1281 | **E12** | **H48** |
| M1282 | **E13** | **H48** | M1283 | **E14** | **H48** | M1284 | **E15** | **H48** |
| M1285 | **E16** | **H48** | M1286 | **E17** | **H48** | M1287 | **E18** | **H48** |
| M1288 | **E19** | **H48** | M1289 | **E20** | **H48** | M1290 | **E21** | **H48** |
| M1291 | **E22** | **H48** | M1292 | **E23** | **H48** | M1293 | **E24** | **H48** |
| M1294 | **E25** | **H48** | M1295 | **E26** | **H48** | M1296 | **E27** | **H48** |
| M1297 | **E1** | **H49** | M1298 | **E2** | **H49** | M1299 | **E3** | **H49** |
| M1300 | **E4** | **H49** | M1301 | **E5** | **H49** | M1302 | **E6** | **H49** |
| M1303 | **E7** | **H49** | M1304 | **E8** | **H49** | M1305 | **E9** | **H49** |
| M1306 | **E10** | **H49** | M1307 | **E11** | **H49** | M1308 | **E12** | **H49** |
| M1309 | **E13** | **H49** | M1310 | **E14** | **H49** | M1311 | **E15** | **H49** |
| M1312 | **E16** | **H49** | M1313 | **E17** | **H49** | M1314 | **E18** | **H49** |
| M1315 | **E19** | **H49** | M1316 | **E20** | **H49** | M1317 | **E21** | **H49** |
| M1318 | **E22** | **H49** | M1319 | **E23** | **H49** | M1320 | **E24** | **H49** |
| M1321 | **E25** | **H49** | M1322 | **E26** | **H49** | M1323 | **E27** | **H49** |
| M1324 | **E1** | **H50** | M1325 | **E2** | **H50** | M1326 | **E3** | **H50** |
| M1327 | **E4** | **H50** | M1328 | **E5** | **H50** | M1329 | **E6** | **H50** |
| M1330 | **E7** | **H50** | M1331 | **E8** | **H50** | M1332 | **E9** | **H50** |
| M1333 | **E10** | **H50** | M1334 | **E11** | **H50** | M1335 | **E12** | **H50** |
| M1336 | **E13** | **H50** | M1337 | **E14** | **H50** | M1338 | **E15** | **H50** |
| M1339 | **E16** | **H50** | M1340 | **E17** | **H50** | M1341 | **E18** | **H50** |
| M1342 | **E19** | **H50** | M1343 | **E20** | **H50** | M1344 | **E21** | **H50** |
| M1345 | **E22** | **H50** | M1346 | **E23** | **H50** | M1347 | **E24** | **H50** |
| M1348 | **E25** | **H50** | M1349 | **E26** | **H50** | M1350 | **E27** | **H50** |
| M1351 | **E1** | **H51** | M1352 | **E2** | **H51** | M1353 | **E3** | **H51** |
| M1354 | **E4** | **H51** | M1355 | **E5** | **H51** | M1356 | **E6** | **H51** |
| M1357 | **E7** | **H51** | M1358 | **E8** | **H51** | M1359 | **E9** | **H51** |
| M1360 | **E10** | **H51** | M1361 | **E11** | **H51** | M1362 | **E12** | **H51** |
| M1363 | **E13** | **H51** | M1364 | **E14** | **H51** | M1365 | **E15** | **H51** |
| M1366 | **E16** | **H51** | M1367 | **E17** | **H51** | M1368 | **E18** | **H51** |
| M1369 | **E19** | **H51** | M1370 | **E20** | **H51** | M1371 | **E21** | **H51** |
| M1372 | **E22** | **H51** | M1373 | **E23** | **H51** | M1374 | **E24** | **H51** |
| M1375 | **E25** | **H51** | M1376 | **E26** | **H51** | M1377 | **E27** | **H51** |

(fortgesetzt)

| | | | | | | | | |
|---|---|---|---|---|---|---|---|---|
| M1378 | **E1** | **H52** | M1379 | **E2** | **H52** | M1380 | **E3** | **H52** |
| M1381 | **E4** | **H52** | M1382 | **E5** | **H52** | M1383 | **E6** | **H52** |
| M1384 | **E7** | **H52** | M1385 | **E8** | **H52** | M1386 | **E9** | **H52** |
| M1387 | **E10** | **H52** | M1388 | **E11** | **H52** | M1389 | **E12** | **H52** |
| M1390 | **E13** | **H52** | M1391 | **E14** | **H52** | M1392 | **E15** | **H52** |
| M1393 | **E16** | **H52** | M1394 | **E17** | **H52** | M1395 | **E18** | **H52** |
| M1396 | **E19** | **H52** | M1397 | **E20** | **H52** | M1398 | **E21** | **H52** |
| M1399 | **E22** | **H52** | M1400 | **E23** | **H52** | M1401 | **E24** | **H52** |
| M1402 | **E25** | **H52** | M1403 | **E26** | **H52** | M1404 | **E27** | **H52** |
| M1405 | **E1** | **H53** | M1406 | **E2** | **H53** | M1407 | **E3** | **H53** |
| M1408 | **E4** | **H53** | M1409 | **E5** | **H53** | M1410 | **E6** | **H53** |
| M1411 | **E7** | **H53** | M1412 | **E8** | **H53** | M1413 | **E9** | **H53** |
| M1414 | **E10** | **H53** | M1415 | **E11** | **H53** | M1416 | **E12** | **H53** |
| M1417 | **E13** | **H53** | M1418 | **E14** | **H53** | M1419 | **E15** | **H53** |
| M1420 | **E16** | **H53** | M1421 | **E17** | **H53** | M1422 | **E18** | **H53** |
| M1423 | **E19** | **H53** | M1424 | **E20** | **H53** | M1425 | **E21** | **H53** |
| M1426 | **E22** | **H53** | M1427 | **E23** | **H53** | M1428 | **E24** | **H53** |
| M1429 | **E25** | **H53** | M1430 | **E26** | **H53** | M1431 | **E27** | **H53** |
| M1432 | **E1** | **H54** | M1433 | **E2** | **H54** | M1434 | **E3** | **H54** |
| M1435 | **E4** | **H54** | M1436 | **E5** | **H54** | M1437 | **E6** | **H54** |
| M1438 | **E7** | **H54** | M1439 | **E8** | **H54** | M1440 | **E9** | **H54** |
| M1441 | **E10** | **H54** | M1442 | **E11** | **H54** | M1443 | **E12** | **H54** |
| M1444 | **E13** | **H54** | M1445 | **E14** | **H54** | M 1446 | **E15** | **H54** |
| M1447 | **E16** | **H54** | M1448 | **E17** | **H54** | M1449 | **E18** | **H54** |
| M1450 | **E19** | **H54** | M1451 | **E20** | **H54** | M1452 | **E21** | **H54** |
| M1453 | **E22** | **H54** | M1454 | **E23** | **H54** | M1455 | **E24** | **H54** |
| M 1456 | **E25** | **H54** | M1457 | **E26** | **H54** | M1458 | **E27** | **H54** |

Die Konzentration des Hostmaterials der Formel (1), wie zuvor beschrieben oder bevorzugt beschrieben, in der erfindungsgemäßen Mischung oder in der lichtemittierenden Schicht der erfindungsgemäßen Vorrichtung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Mischung oder bezogen auf die gesamte Zusammensetzung der lichtemittierenden Schicht.

Die Konzentration des Hostmaterials einer der Formeln (6), (7), (8), (9), (10) oder (11), wie zuvor beschrieben oder als bevorzugt beschrieben, in der erfindungsgemäßen Mischung oder in der lichtemittierenden Schicht der erfindungsgemäßen Vorrichtung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Mischung oder bezogen auf die gesamte Zusammensetzung der lichtemittierenden Schicht.

Die vorliegende Erfindung betrifft auch eine Mischung, die neben den vorstehend genannten Hostmaterialien der Formel (1), zukünftig Hostmaterial 1 genannt, und des Hostmaterials einer der Formeln (6), (7), (8), (9), (10) oder (11), zukünftig Hostmaterial 2 genannt, wie zuvor beschrieben oder bevorzugt beschrieben, insbesondere Mischungen M1 bis M1458, mindestens noch einen phosphoreszierenden Emitter enthält.

Die vorliegende Erfindung betrifft auch eine organische elektrolumineszierende Vorrichtung, wie zuvor beschrieben oder bevorzugt beschrieben, wobei die lichtemittierende Schicht neben den vorstehend genannten Hostmaterialien der Formeln (1) und einer der Formeln (6), (7), (8), (9), (10) oder (11), wie zuvor beschrieben oder bevorzugt beschrieben, insbesondere den Materialkombinationen M1 bis M1458, mindestens noch einen phosphoreszierenden Emitter enthält.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Bevorzugte phosphoreszierende Emitter gemäß der vorliegenden Erfindung entsprechen der Formel (IIIa),

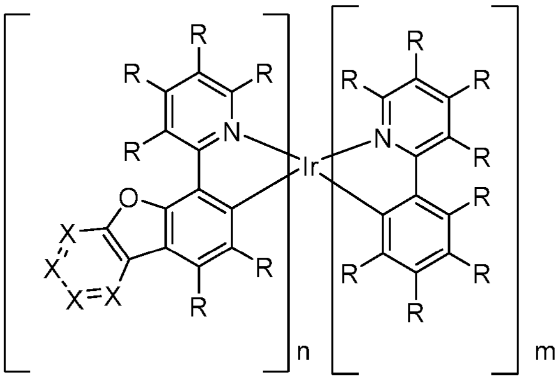

Formel (IIIa),

wobei die Symbole und Indizes für diese Formel (IIIa) die Bedeutung haben:

n+m ist 3, n ist 1 oder 2, m ist 2 oder 1,
X ist N oder CR,
R ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 7 C-Atomen, die teilweise oder vollständig mit Deuterium substituiert sein kann.

Ein weiterer Gegenstand der Erfindung ist demzufolge eine organische elektrolumineszierende Vorrichtung, wie zuvor beschrieben oder bevorzugt beschrieben, dadurch gekennzeichnet, dass die lichtemittierende Schicht neben den Hostmaterialien 1 und 2 mindestens einen phosphoreszierenden Emitter enthält, der der Formel (IIIa) entspricht, wie zuvor beschrieben.

In Emittern der Formel (IIIa) ist n bevorzugt 1 und m ist bevorzugt 2.

In Emittern der Formel (IIIa) ist bevorzugt ein X ausgewählt aus N und die anderen X bedeuten CR.

In Emittern der Formel (IIIa) ist mindestens ein R bevorzugt unterschiedlich von H. In Emittern der Formel (IIIa) sind bevorzugt zwei R unterschiedlich von H und haben eine der sonst zuvor für die Emitter der Formel (IIIa) angegebenen Bedeutungen.

Bevorzugte phosphoreszierende Emitter gemäß der vorliegenden Erfindung entsprechen den Formeln (I), (II), (III), (IV) oder (V),

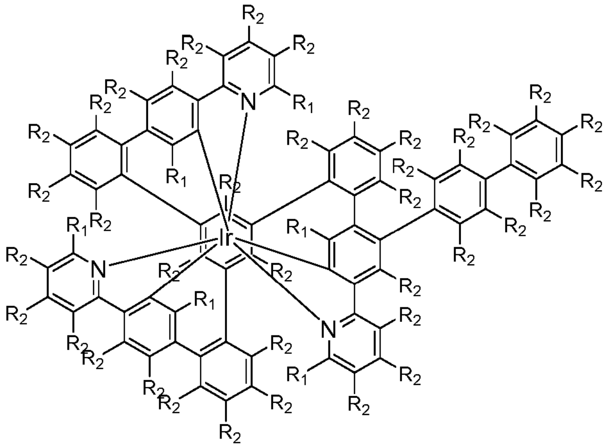

Formel (I) ,

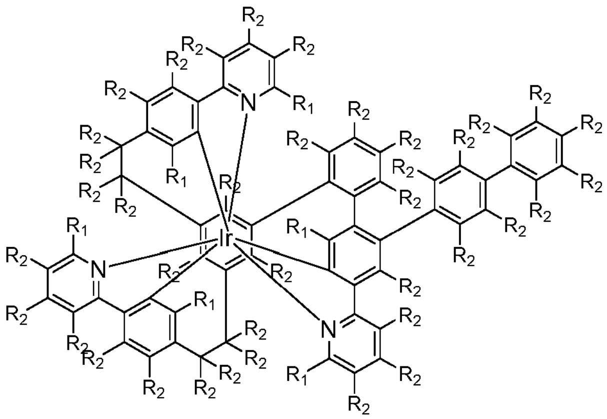

Formel (II) ,

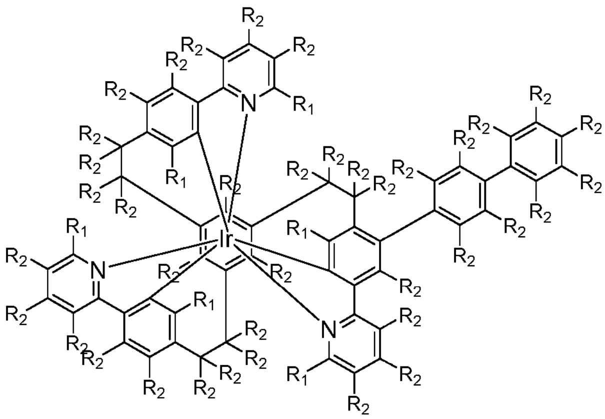

Formel (III) ,

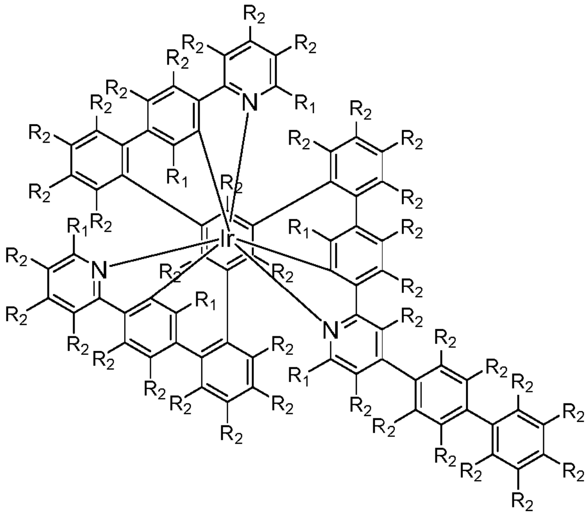

Formel (IV) ,

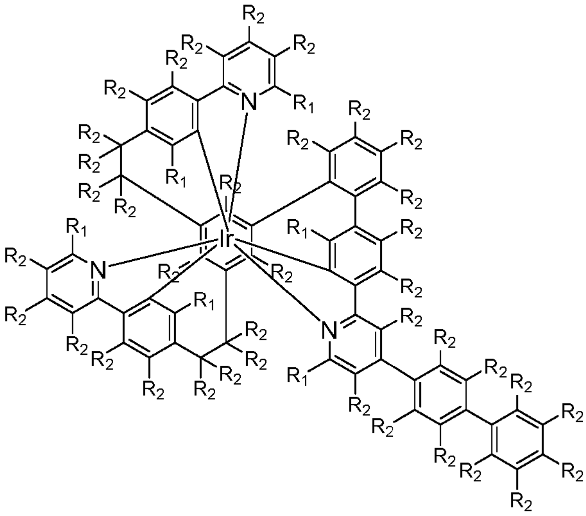

Formel (V) ,

wobei die Symbole und Indizes für diese Formeln (I), (II), (III), (IV) und (V) die Bedeutung haben:
$R_1$ ist H oder D, $R_2$ ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte phosphoreszierende Emitter gemäß der vorliegenden Erfindung entsprechen den Formeln (VI), (VII) oder (VIII),

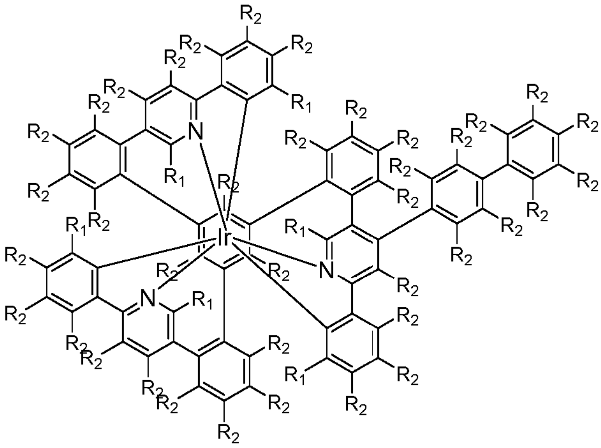

Formel (VI) ,

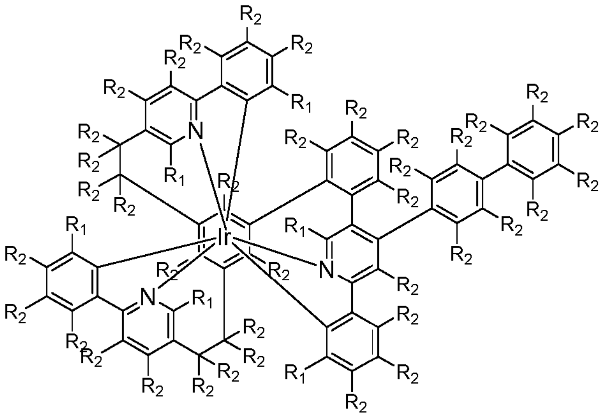

Formel (VII) ,

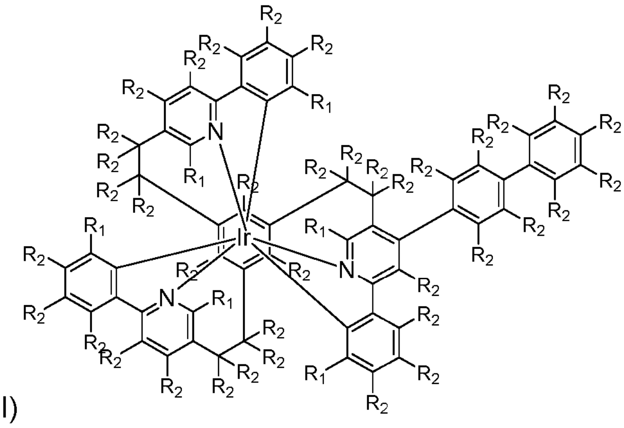

Formel (VIII) ,

wobei die Symbole und Indizes für diese Formeln (VI), (VII) und (VIII) die Bedeutung haben:
$R_1$ ist H oder D, $R_2$ ist H, D, F oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte Beispiele von phosphoreszierenden Emittern sind in WO2019007867 auf den Seiten 120 bis 126 in Tabelle 5 und auf den Seiten 127 bis 129 in Tabelle 6 beschrieben. Die Emitter sind durch diese Referenz in die Beschreibung aufgenommen.

Besonders bevorzugte Beispiele von phosphoreszierenden Emittern sind in der folgenden Tabelle 5 aufgeführt.

Tabelle 5:

| | | |
|---|---|---|
| 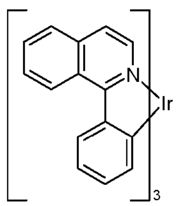 | 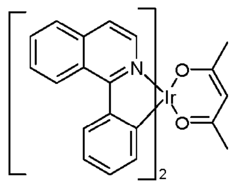 | 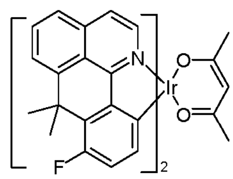 |
| 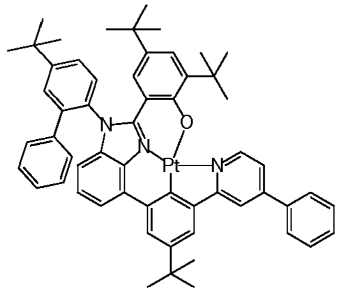 | 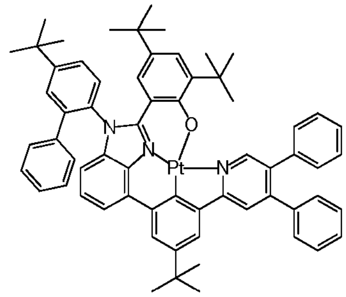 | 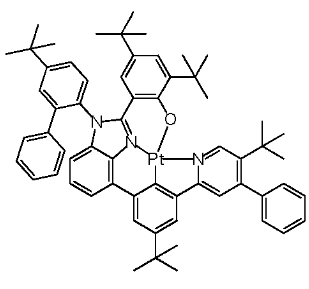 |

| | | |
|---|---|---|
| 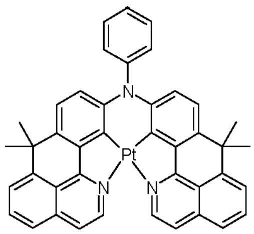 | 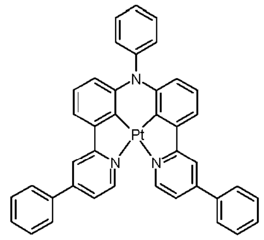 | 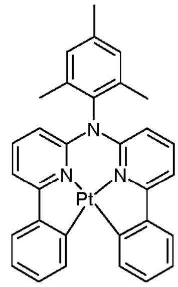 |
| 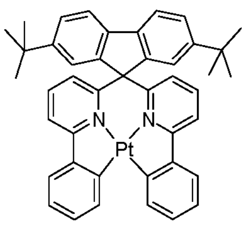 | 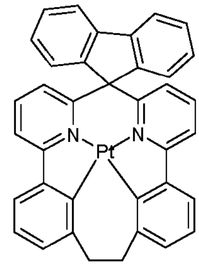 | 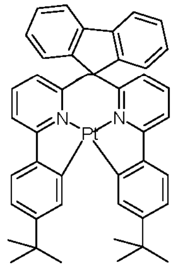 |
| 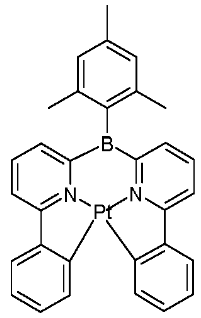 | 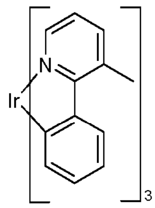 | 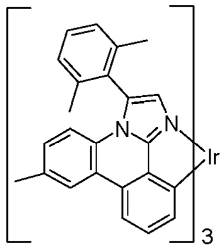 |
| 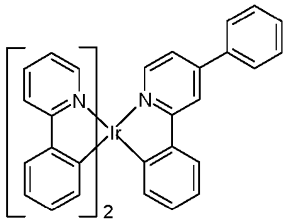 | 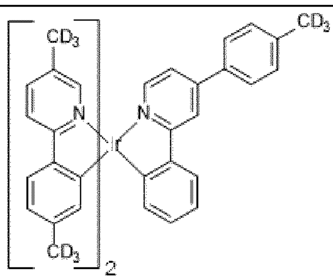 | 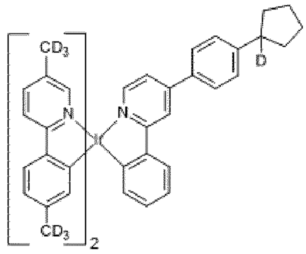 |
| 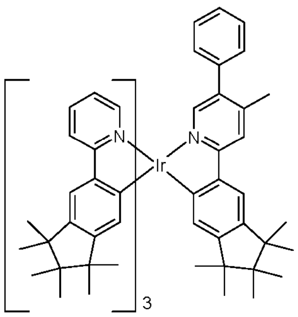 | 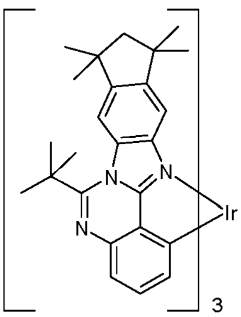 | 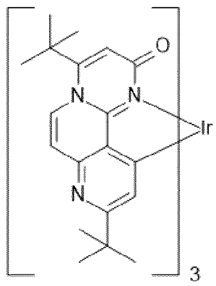 |
| 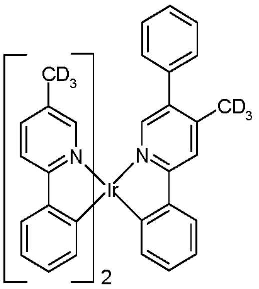 | 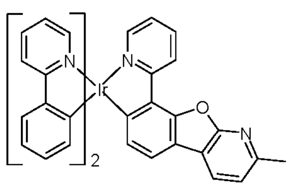 | 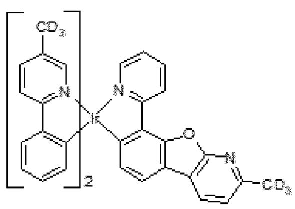 |

| | | |
|---|---|---|
| 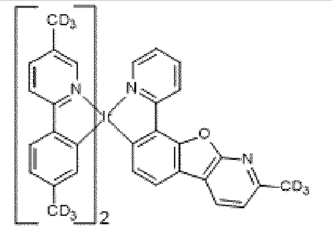 | 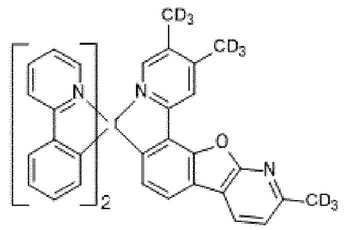 | 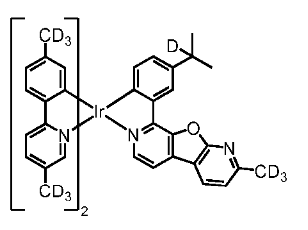 |
| 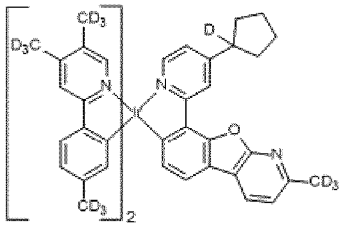 | 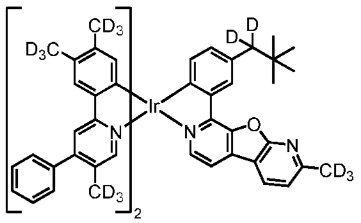 | 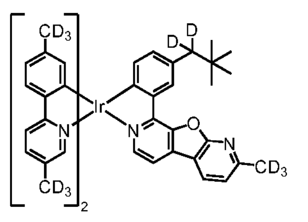 |
| 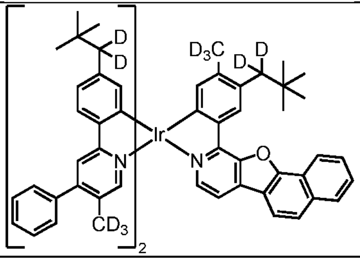 | 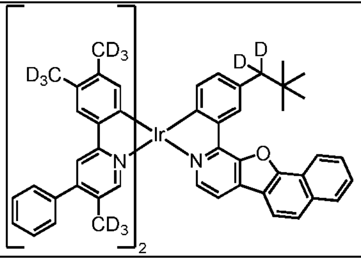 | 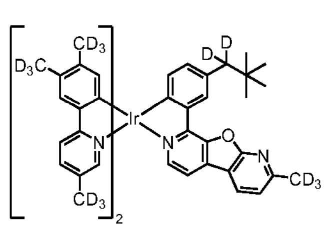 |
| 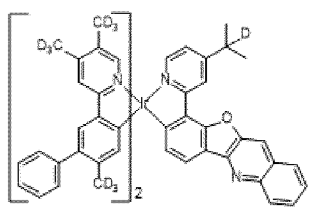 | 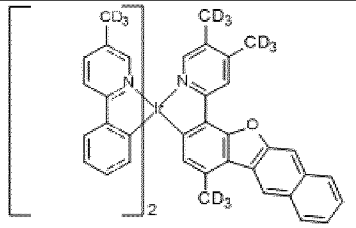 | 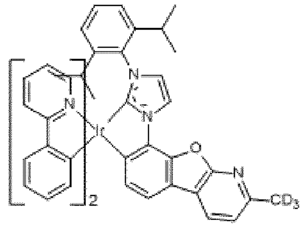 |
| 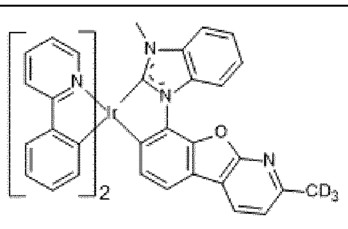 | 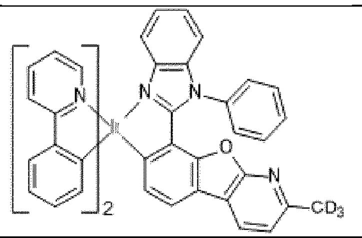 | 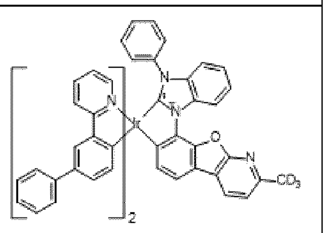 |
| 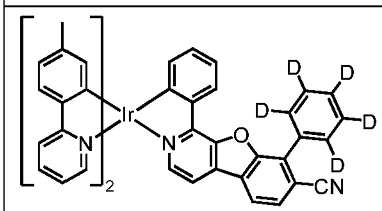 | 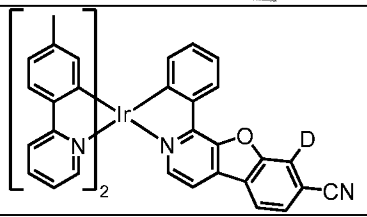 | 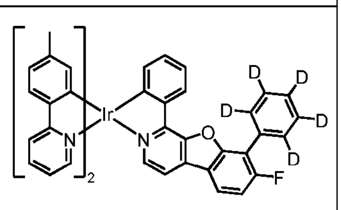 |
| 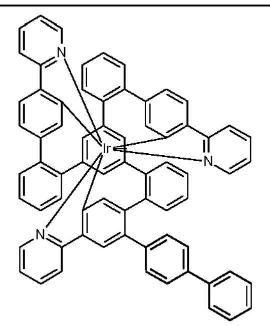 | 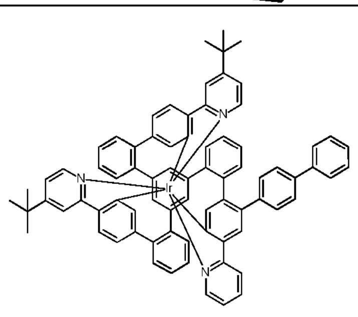 | 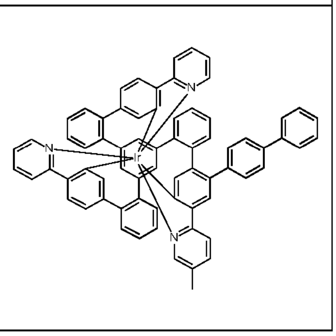 |

| | | |
|---|---|---|
| 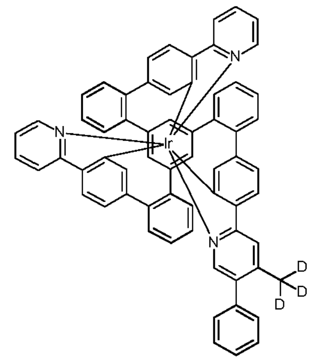 | 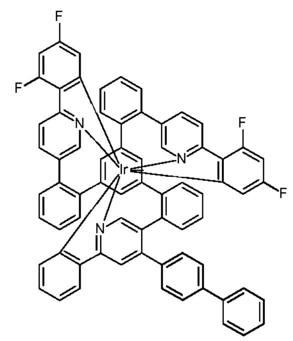 | 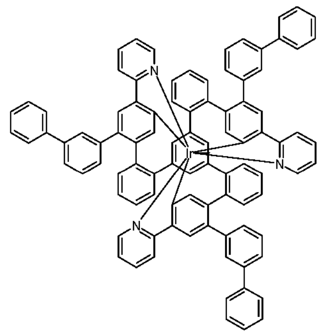 |
| 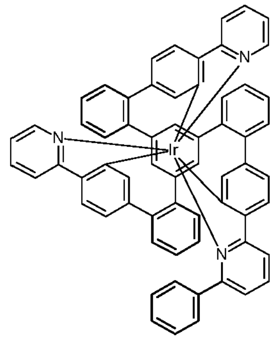 | 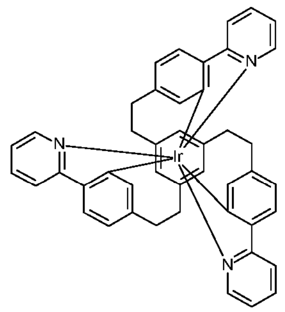 | 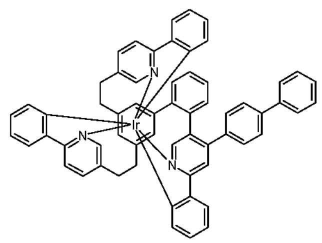 |
| 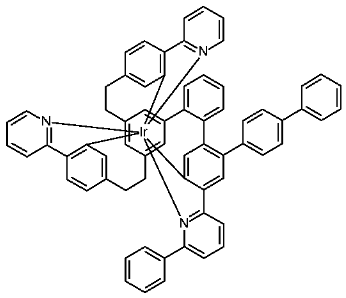 | 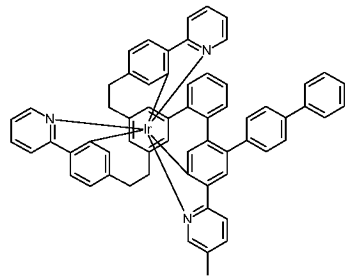 | 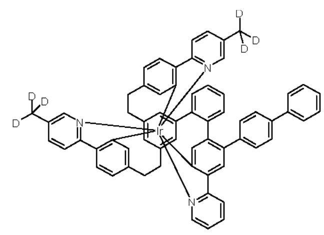 |
| 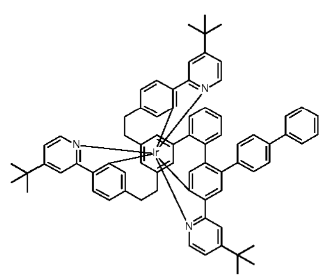 | 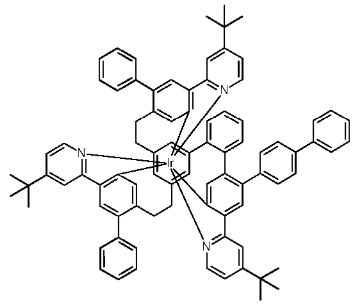 | 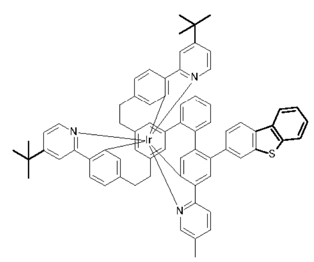 |
| 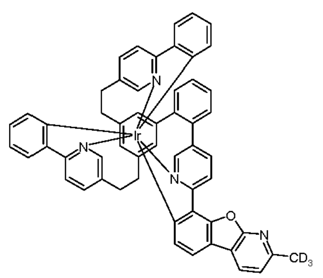 | 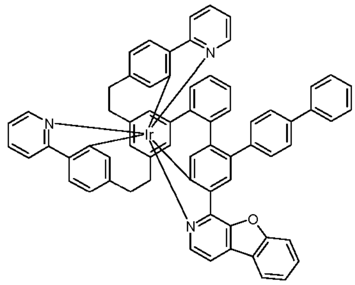 | 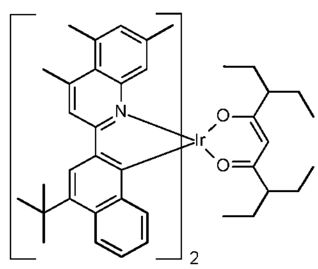 |

| | | |
|---|---|---|
| 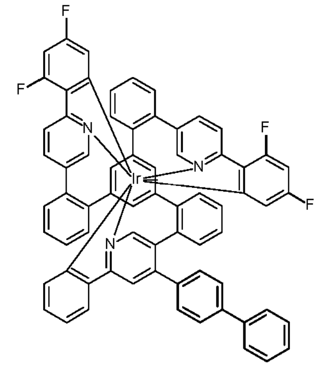 | 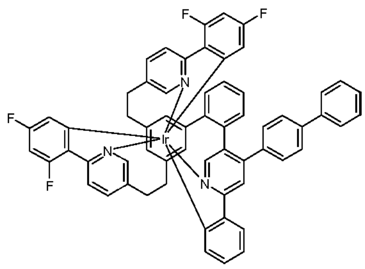 | 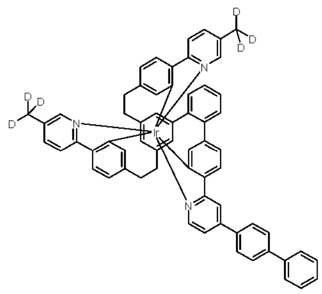 |
| 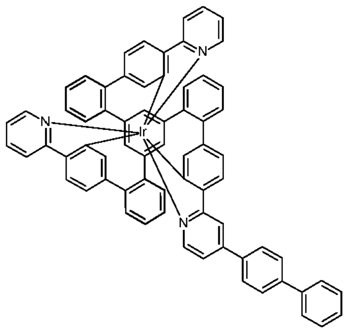 | 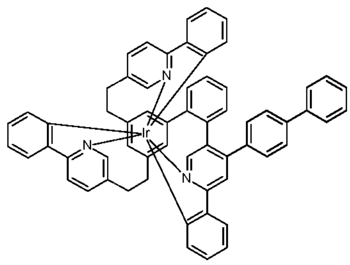 | 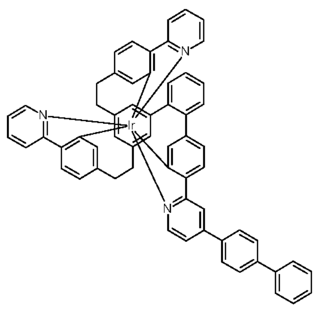 |
| 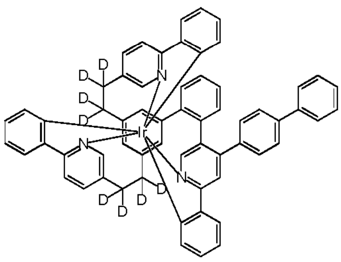 | 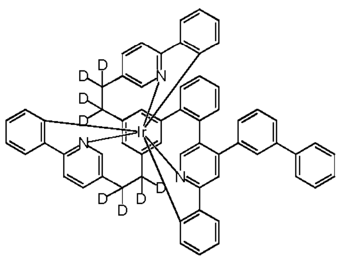 | 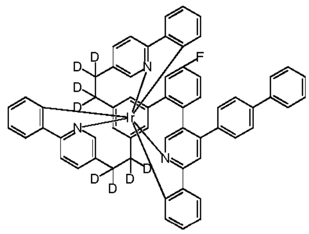 |

In den erfindungsgemäßen Mischungen oder in der lichtemittierenden Schicht der erfindungsgemäßen Vorrichtung wird bevorzugt jede Mischung ausgewählt aus der Summe der Mischungen M1 bis M1458 mit einer Verbindung der Formel (IIIa) oder einer Verbindung der Formeln (I) bis (VIII) oder einer Verbindung aus Tabelle 5 kombiniert.

Die lichtemittierende Schicht in der erfindungsgemäßen organischen elektrolumineszierenden Vorrichtung enthaltend mindestens einen phosphoreszierenden Emitter ist bevorzugt eine infra-rot emittierende, gelb, orange, rot, grün, blau oder ultraviolett emittierende Schicht, besonders bevorzugt eine gelb oder grün emittierende Schicht und ganz besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt.

Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Kombination der Hostmaterialien der Formeln (1) und einer der Formeln (6), (7), (8), (9), (10) oder (11) und den entsprechenden Emitter enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstofffreier Lösung, 10-5 molar, gemessen, wobei die Messung bei Raumtemperatur erfolgt und jedes Lösemittel geeignet ist, in dem sich der gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV

umgerechnet gemäß: E(T1 in eV) = 1240/E(T1 in nm) = 1240/PLmax. (in nm).

Bevorzugte phosphoreszierende Emitter sind demzufolge gelbe Emitter, vorzugsweise der Formel (IIIa), der Formeln (I) bis (VIII) oder aus Tabelle 5, deren Triplettenergie $T_1$ bevorzugt bei -2.3 eV bis ~2.1 eV liegt.

Bevorzugte phosphoreszierende Emitter sind demzufolge grüne Emitter, vorzugsweise der Formel (IIIa), der Formeln (I) bis (VIII) oder aus Tabelle 5, deren Triplettenergie $T_1$ bevorzugt bei -2.5 eV bis -2.3 eV liegt.

Besonders bevorzugte phosphoreszierende Emitter sind demzufolge grüne Emitter, vorzugsweise der Formel (IIIa), der Formeln (I) bis (VIII) oder aus Tabelle 5, wie zuvor beschrieben, deren Triplettenergie $T_1$ bevorzugt bei ~2.5 eV bis -2.3 eV liegt.

Ganz besonders bevorzugt werden grüne Emitter, vorzugsweise der Formel (IIIa), der Formeln (I) bis (VIII) oder aus Tabelle 5, wie zuvor beschrieben, für die erfindungsgemäße Mischung bzw. erfindungsgemäße emittierende Schicht ausgewählt.

In der lichtemittierenden Schicht der erfindungsgemäßen Vorrichtung oder in der erfindungsgemäßen Mischung können auch fluoreszierende Emitter enthalten sein. Bevorzugte fluoreszierende emittierende Verbindungen sind ausgewählt aus der Klasse der Arylamine, wobei bevorzugt mindestens eines der aromatischen oder heteroaromatischen Ringsysteme des Arylamins ein kondensiertes Ringsystem, besonders bevorzugt mit mindestens 14 Ringatomen ist. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine. Ebenfalls bevorzugt sind Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

In einer weiteren bevorzugten Ausführungsform der Erfindung kann die mindestens eine lichtemittierende Schicht der organischen elektrolumineszierenden Vorrichtung neben den Hostmaterialien 1 und 2, wie zuvor beschrieben oder als bevorzugt beschrieben, weitere Hostmaterialien oder Matrixmaterialien umfassen, sogenannte Mixed-Matrix-Systeme. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu den Hostmaterialien 1 und 2, wie zuvor beschrieben). Besonders geeignete Matrixmaterialien, welche in Kombination als Matrixkomponente eines Mixed-Matrix-Systems verwendet werden können, sind ausgewählt aus *wide-band*gap-Materialien, bipolaren Hostmaterialien, Elektronentransportmaterialien (ETM) und Lochtransportmaterialien (HTM).

Bevorzugt wird das Mixed-Matrix-System auf einen Emitter der Formel (IIIa), der Formeln (I) bis (VIII) oder aus Tabelle 5 optimiert.

Gemäß einer Ausführungsform der vorliegenden Erfindung enthält die Mischung neben den Bestandteilen des Hostmaterials der Formel (1) und des Hostmaterials 2, wie zuvor beschrieben, keine weiteren Bestandteile, das heißt, funktionelle Materialien. Es handelt sich um Materialmischungen, die als solche zur Herstellung der lichtemittierenden Schicht, verwendet werden. Man bezeichnet diese Mischungen auch als Premix-Systeme, die als einzige Materialquelle bei der Aufdampfung der Hostmaterialien für die lichtemittierende Schicht verwendet wird und die ein konstantes Mischungsverhältnis bei der Aufdampfung haben. Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Gemäß einer alternativen Ausführungsform der vorliegenden Erfindung enthält die Mischung neben den Bestandteilen des Hostmaterials der Formel (1) und des Hostmaterials 2, wie zuvor beschrieben, noch einen phosphoreszierenden Emitter, wie zuvor beschrieben. Bei geeignetem Mischungsverhältnis bei der Aufdampfung kann auch diese Mischung als einzige Materialquelle verwendet werden, wie zuvor beschrieben.

Die Komponenten bzw. Bestandteile der lichtemittierenden Schicht der erfindungsgemäßen Vorrichtung können somit durch Aufdampfen oder aus Lösung prozessiert werden. Die Materialkombination der Hostmaterialien 1 und 2, wie zuvor beschrieben oder bevorzugt beschrieben, gegebenenfalls mit dem phosphoreszierenden Emitter, wie zuvor beschrieben oder bevorzugt beschrieben, werden dazu in einer Formulierung bereitgestellt, die mindestens ein Lösemittel enthält. Geeignete Formulierungen wurden zuvor beschrieben

Die lichtemittierende Schicht in der erfindungsgemäßen Vorrichtung gemäß den bevorzugten Ausführungsformen und der emittierenden Verbindung enthält vorzugsweise zwischen 99,9 und 1 Vol.-%, weiter vorzugsweise zwischen 99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1) und mindestens einer Verbindung einer der Formeln (6), (7), (8), (9), (10) oder (11) gemäß den bevorzugten Ausführungsformen, bezogen auf die gesamte

Zusammensetzung aus Emitter und Matrixmaterial. Entsprechend enthält die lichtemittierende Schicht in der erfindungsgemäßen Vorrichtung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung der aus Emitter und Matrixmaterial bestehenden lichtemittierenden Schicht. Werden die Verbindungen aus Lösung verarbeitet, so werden statt der oben angegebenen Mengen in Vol.-% bevorzugt die entsprechenden Mengen in Gew.-% verwendet.

Die lichtemittierende Schicht in der erfindungsgemäßen Vorrichtung gemäß den bevorzugten Ausführungsformen und der emittierenden Verbindung enthält vorzugsweise das Hostmaterial 1 und das Hostmaterial 2 in einem Volumenprozentverhältnis zwischen 3:1 und 1:3, bevorzugt zwischen 1:2.5 und 1:1, besonders bevorzugt zwischen 1:2 und 1:1. Werden die Verbindungen aus Lösung verarbeitet, so werden statt des oben angegebenen Verhältnisses in Vol.-% bevorzugt das entsprechende Verhältnis in Gew.-% verwendet.

Die vorliegende Erfindung betrifft auch eine organische elektrolumineszierende Vorrichtung, wie zuvor beschrieben oder bevorzugt beschrieben, wobei die organische Schicht eine Lochinjektionsschicht (HIL) und/oder eine Lochtransportschicht (HTL) enthält, deren lochinjizierendes Material und lochtransportierendes Material zur Klasse der Arylamine gehört. Bevorzugte Verbindungen mit Lochtransportfunktion, die nicht einer der Formeln für das Hostmaterial 2 entsprechen, vorzugsweise zur Verwendung in einer Lochinjektionsschicht, einer Lochtransportschicht, einer Elektronenblockierschicht und/oder als zusätzliches Matrixmaterial in der erfindungsgemäßen emittierenden Schicht, sind in der folgenden Tabelle 6 gezeigt. Die Verbindungen der Tabelle 6 sind, wie die Strukturen zeigen, nicht deuterierte Verbindungen.

Tabelle 6:

| | |
|---|---|
| 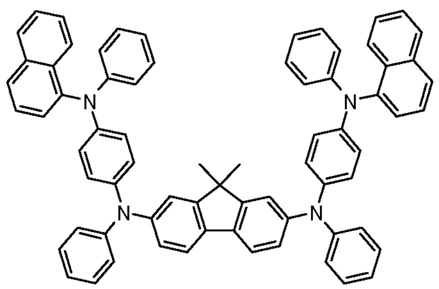 | 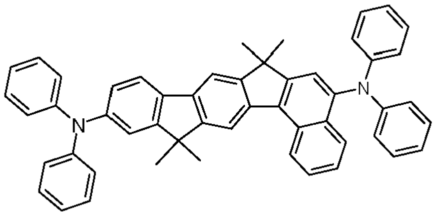 |
| HT-1 | HT-2 |
| 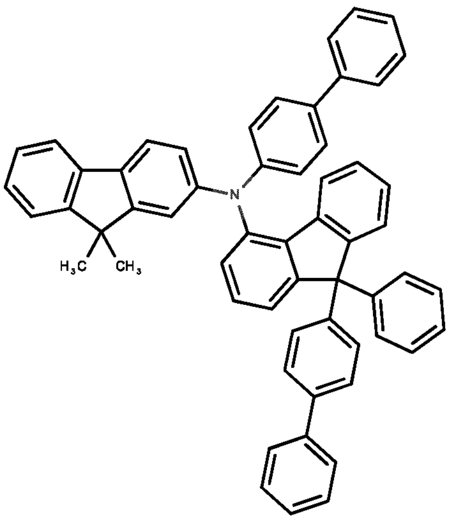 | 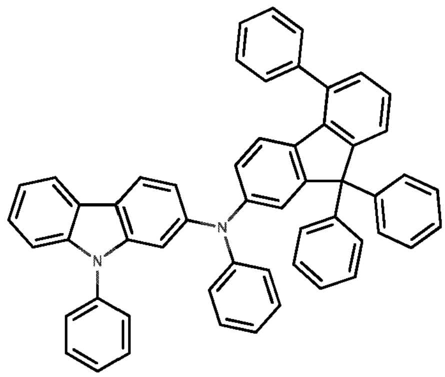 |
| HT-3 | HT-4 |

(fortgesetzt)

| | |
|---|---|
| 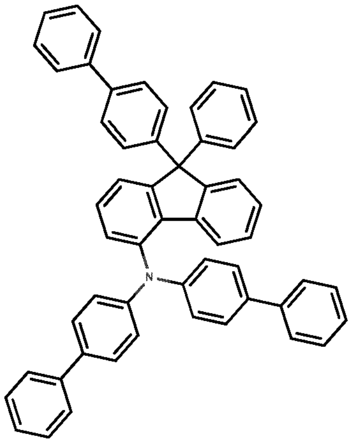 | 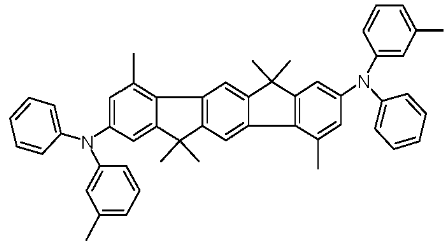 |
| HT-5 | HT-6 |
| 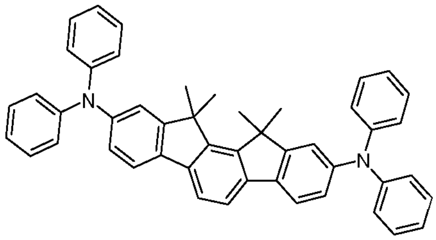 | 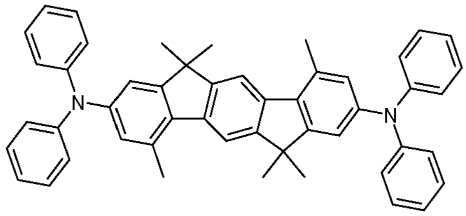 |
| HT-7 | HT-8 |
| 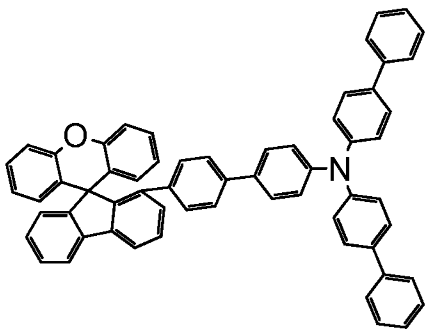 | 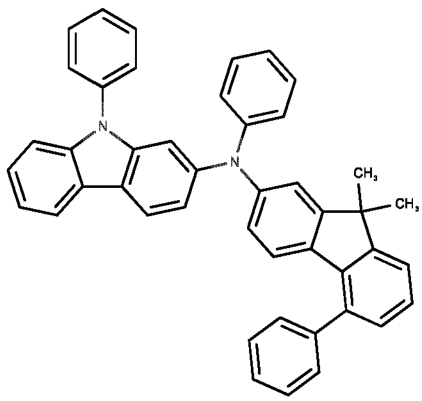 |
| HT-9 | HT-10 |
| 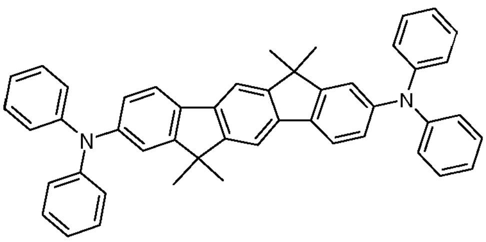 | 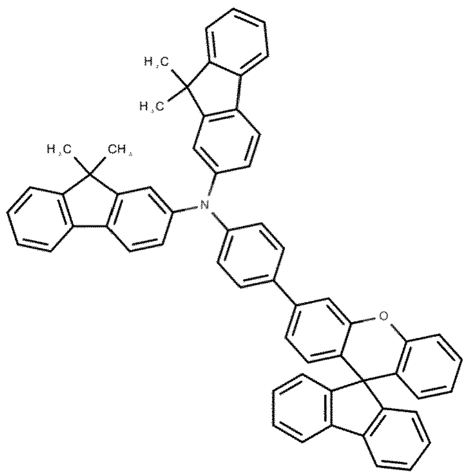 |

(fortgesetzt)

| HT-11 | HT-12 |
|---|---|
| 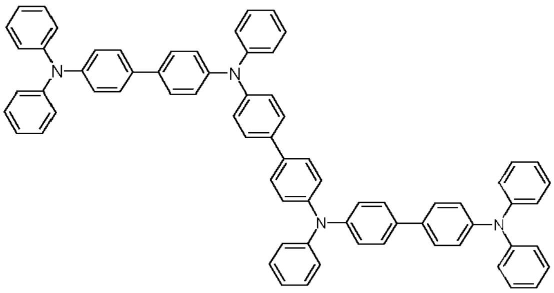 | 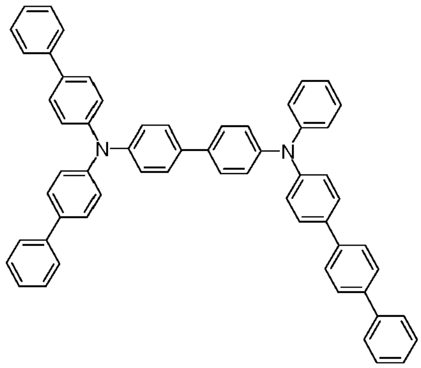 |
| HT-13 | HT-14 |
| 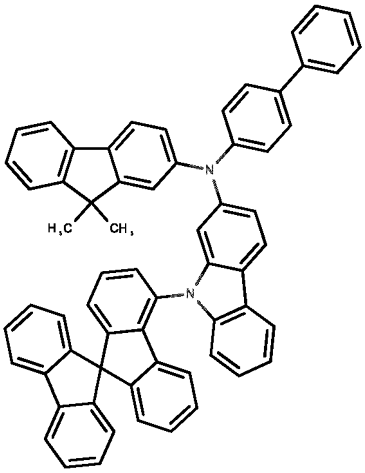 | 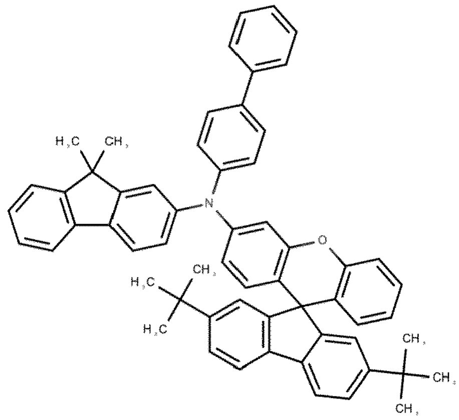 |
| HT-15 | HT-16 |
| 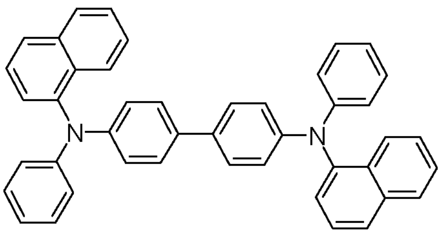 | 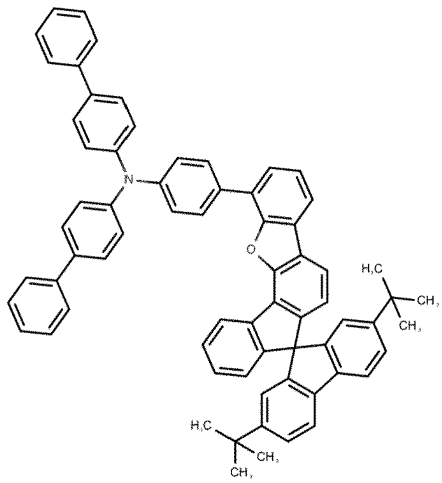 |
| HT-17 | HT-18 |

(fortgesetzt)

| | |
|---|---|
| 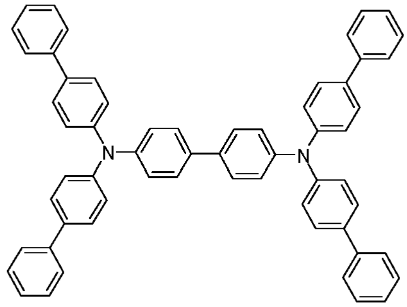 | 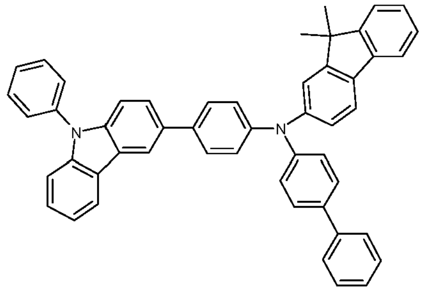 |
| HT-19 | HT-20 |
| 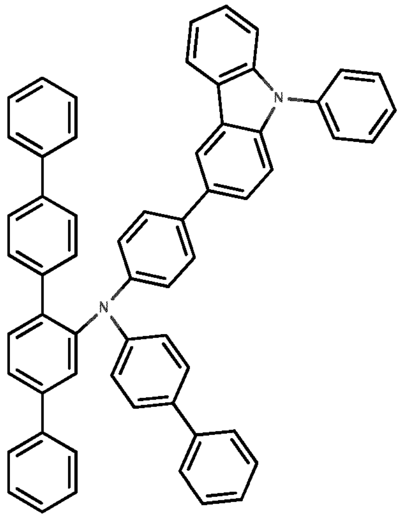 | 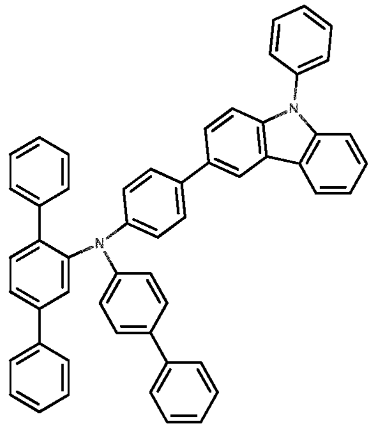 |
| HT-21 | HT-22 |
| 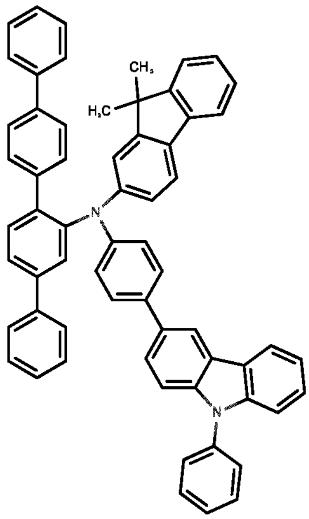 | 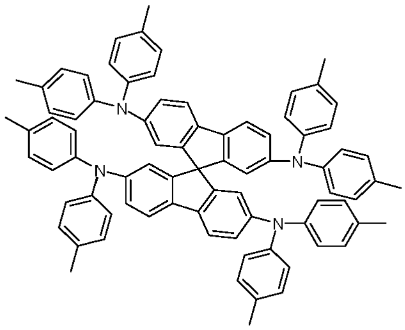 |
| HT-23 | HT-24 |

(fortgesetzt)

| | |
|---|---|
| 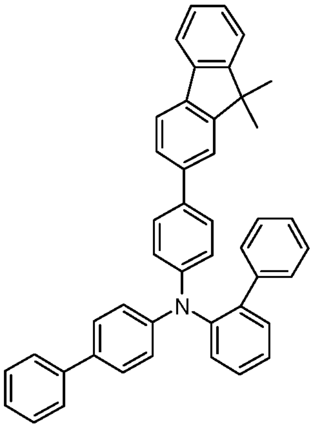 | 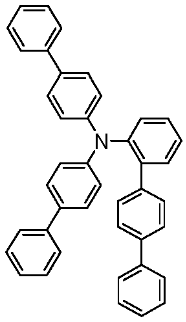 |
| HT-25 | HT-26 |
| 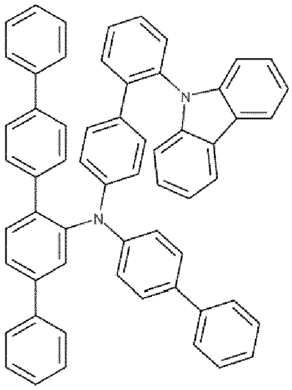 | 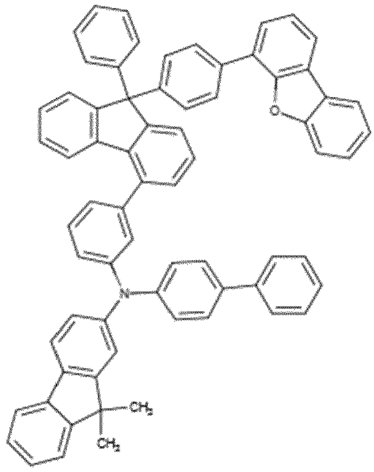 |
| HT-27 | HT-28 |
| 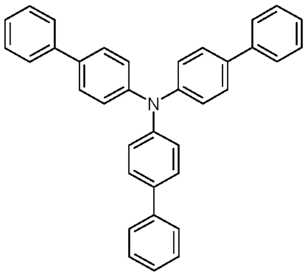 | 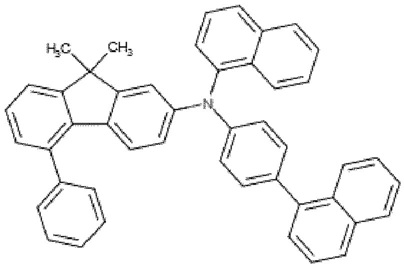 |
| HT-29 | HT-30 |
| 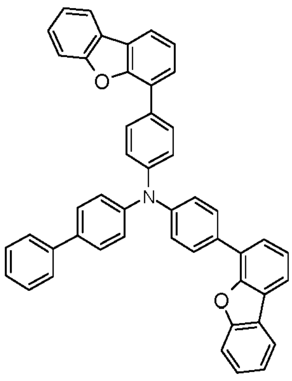 | 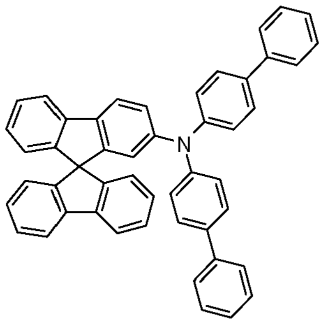 |

(fortgesetzt)

| | |
|---|---|
| HT-31 | HT-32 |
| 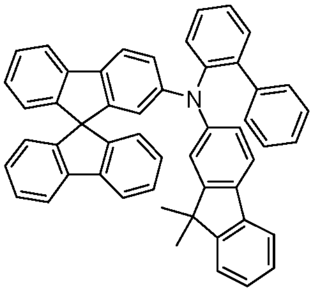 | 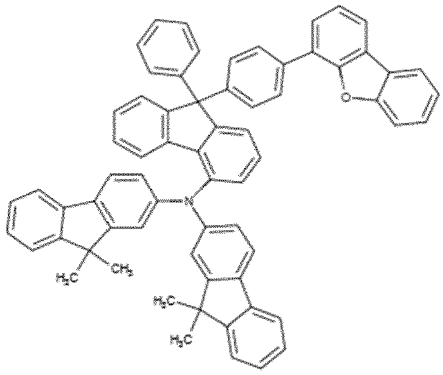 |
| HT-33 | HT-34 |
| 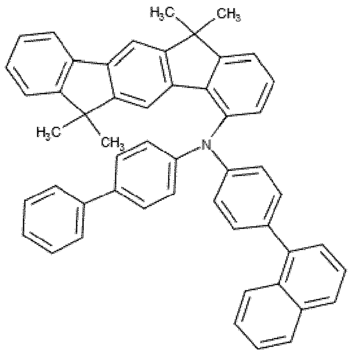 | 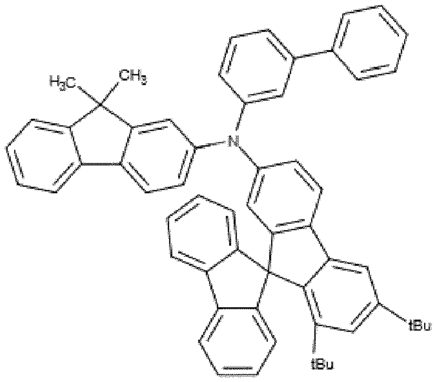 |
| HT-35 | HT-36 |
| 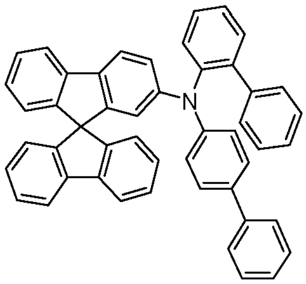 | 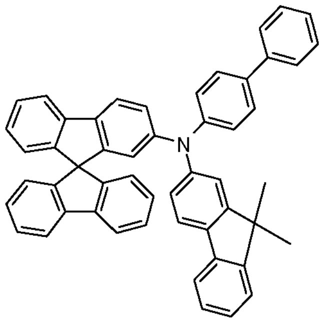 |
| HT-37 | HT-38 |
| 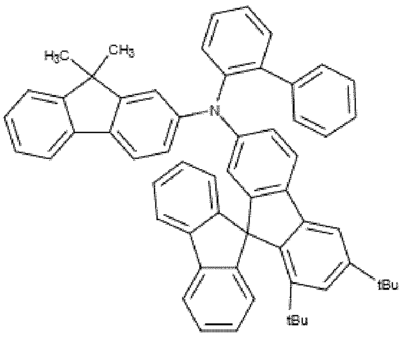 | 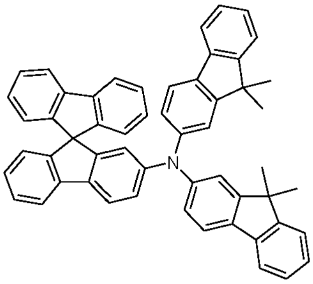 |
| HT-39 | HT-40 |

(fortgesetzt)

| | |
|---|---|
| 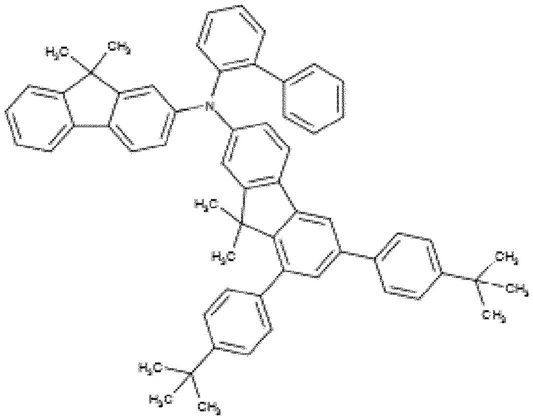 | 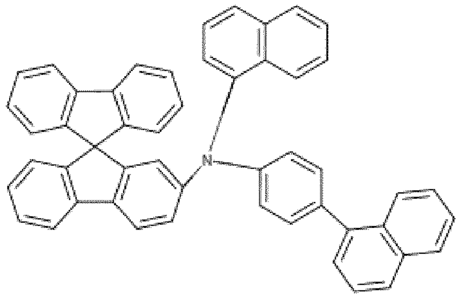 |
| HT-41 | HT-42 |
| 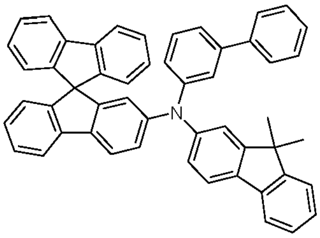 | 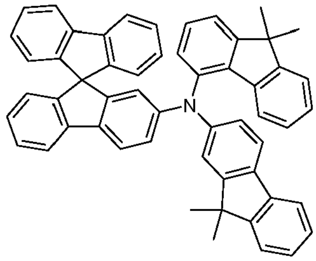 |
| HT-43 | HT-44 |
| 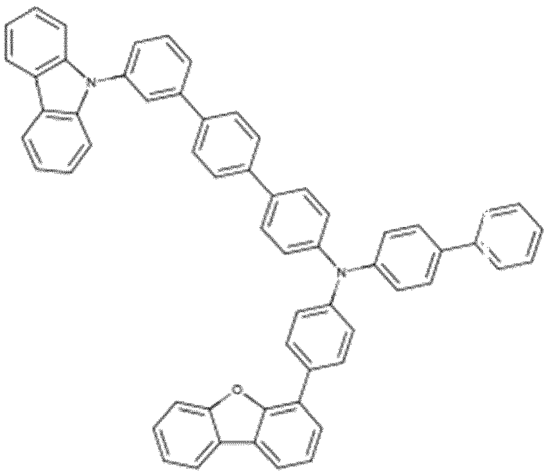 | 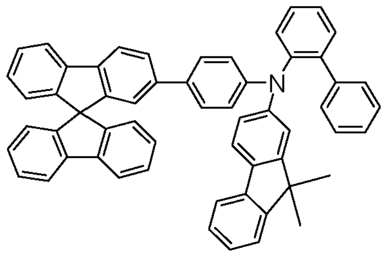 |
| HT-45 | HT-46 |

(fortgesetzt)

| | |
|---|---|
| 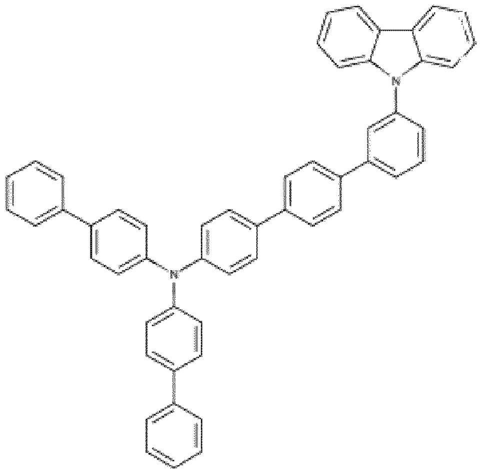 | 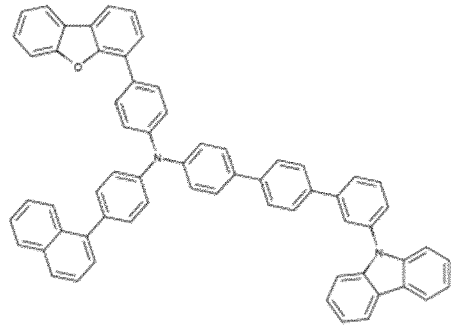 |
| HT-47 | HT-48 |
| 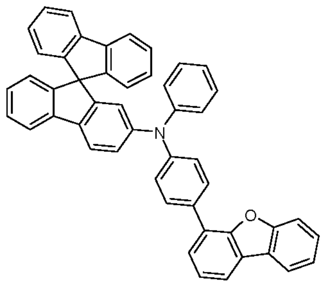 | 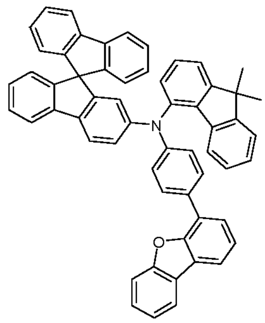 |
| HT-49 | HT-50 |
| 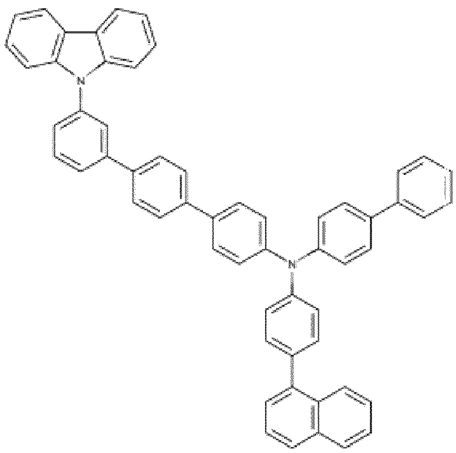 | 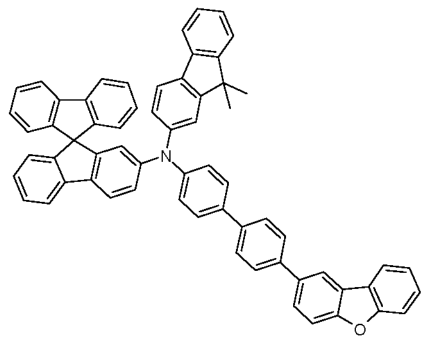 |
| HT-51 | HT-52 |

(fortgesetzt)

| | |
|---|---|
| 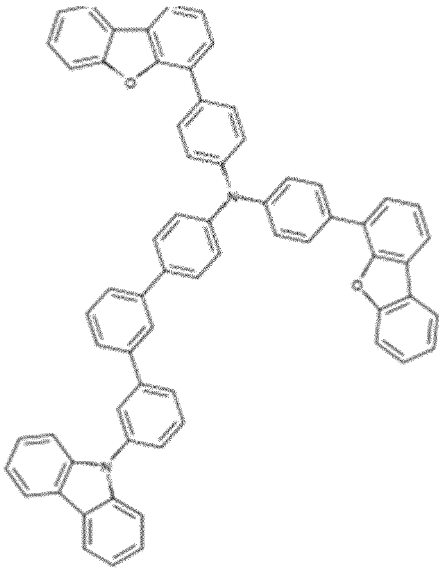 | 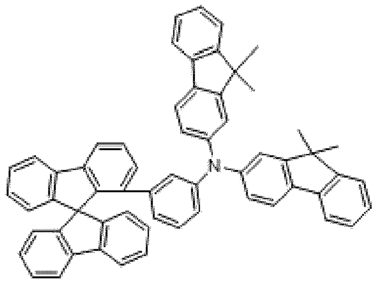 |
| HT-53 | HT-54 |
| 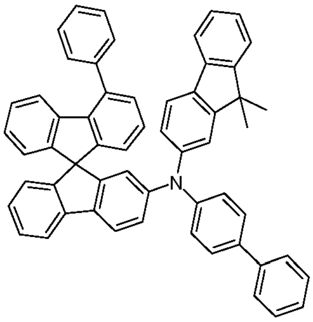 | 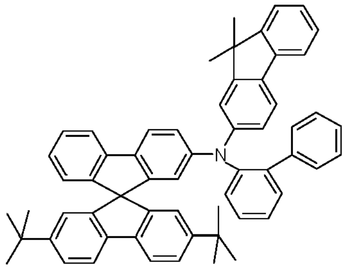 |
| HT-55 | HT-56 |
| 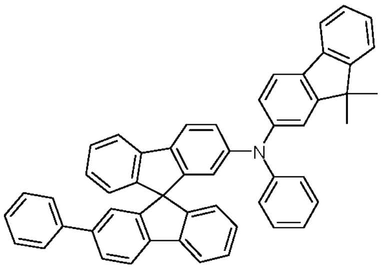 | 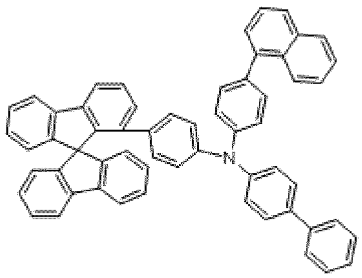 |
| HT-57 | HT-58 |
| 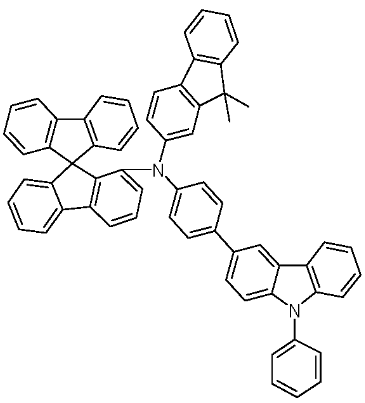 | 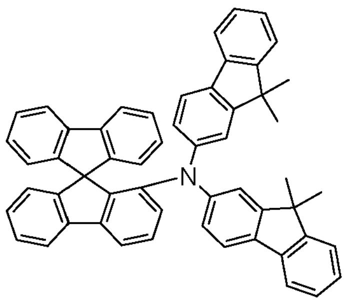 |

(fortgesetzt)

| | |
|---|---|
| HT-59 | HT-60 |
| 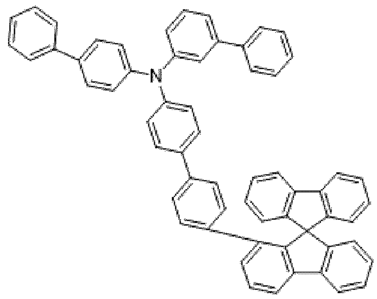 | 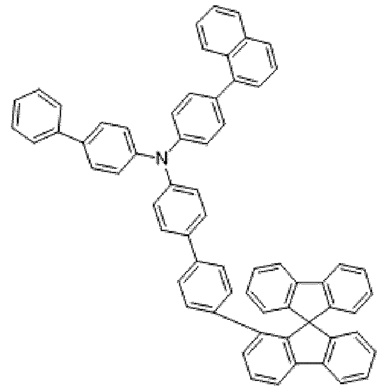 |
| HT-61 | HT-62 |
| 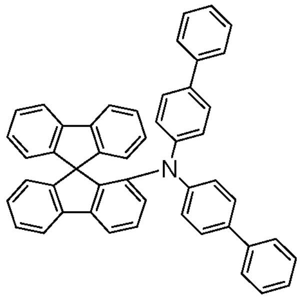 | 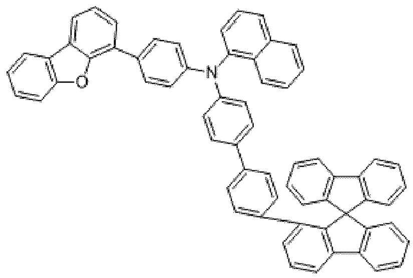 |
| HT-63 | HT-64 |
| 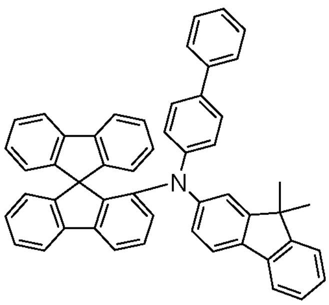 | 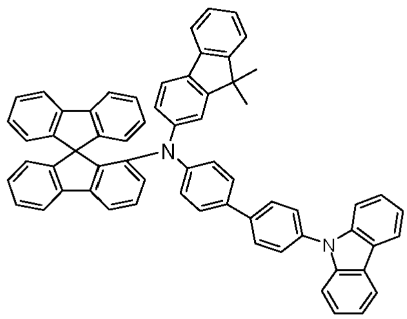 |
| HT-65 | HT-66 |
| 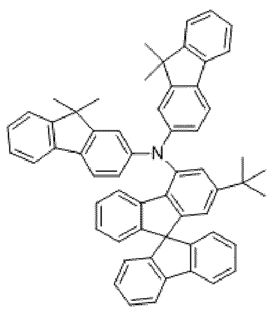 | 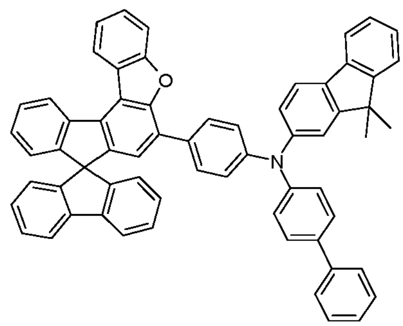 |
| HT-67 | HT-68 |

(fortgesetzt)

| | |
|---|---|
| 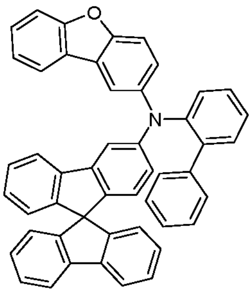 | 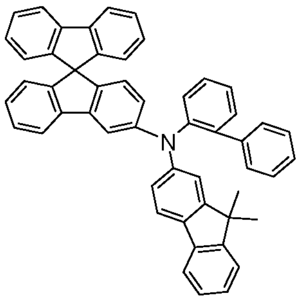 |
| HT-69 | HT-70 |
| 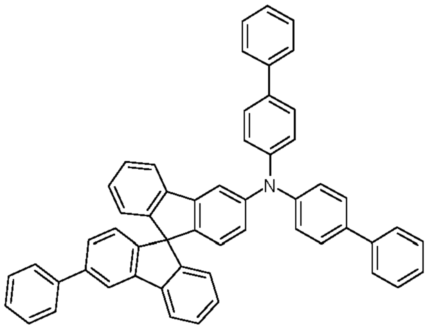 | 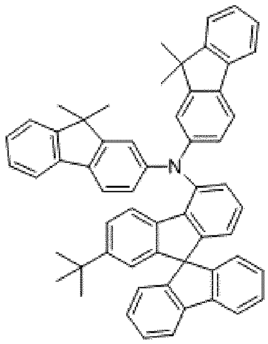 |
| HT-71 | HT-72 |
| 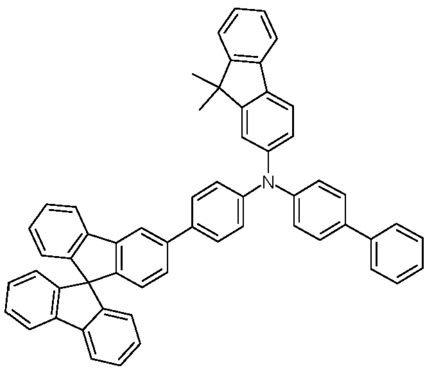 | 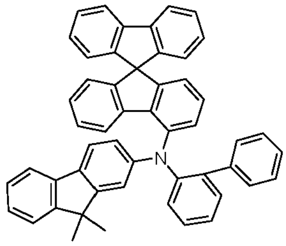 |
| HT-73 | HT-74 |
| 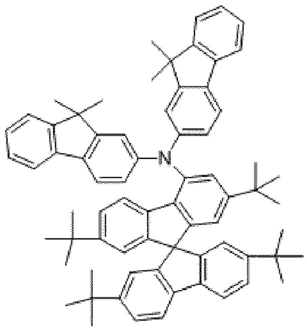 | 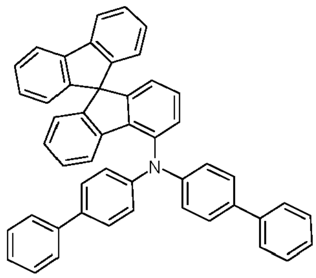 |
| HT-75 | HT-76 |

(fortgesetzt)

| | |
|---|---|
| 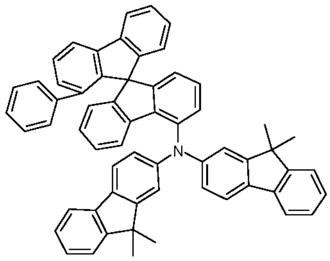 | 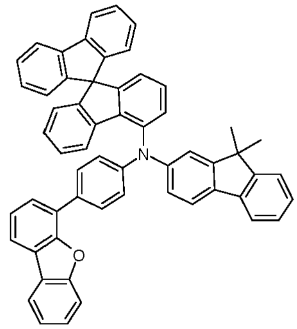 |
| HT-77 | HT-78 |
| 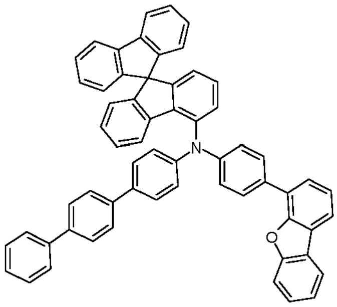 | 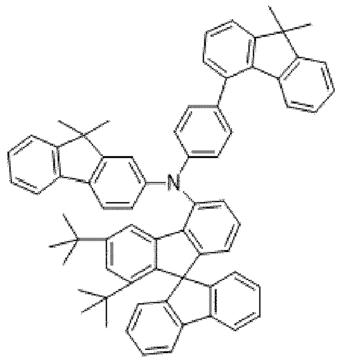 |
| HT-79 | HT-80 |
| 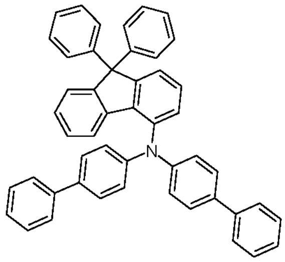 | 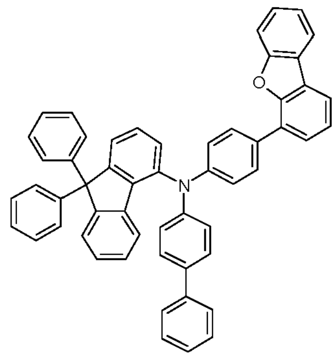 |
| HT-81 | HT-82 |
| 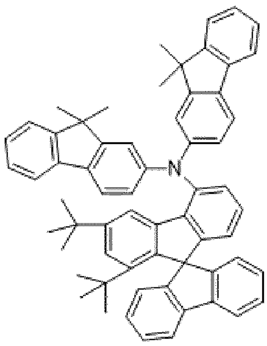 | 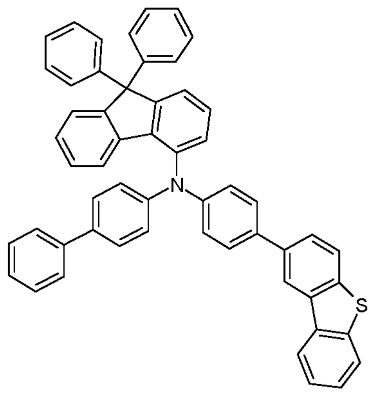 |
| HT-83 | HT-84 |

(fortgesetzt)

| | |
|---|---|
| 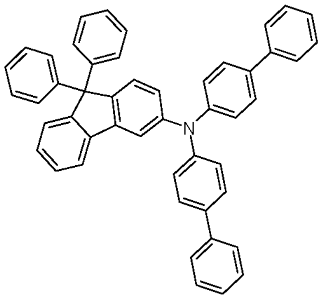 | 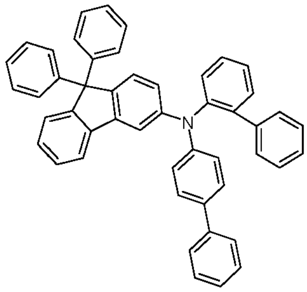 |
| HT-85 | HT-86 |
| 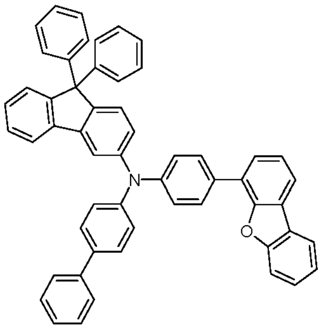 | 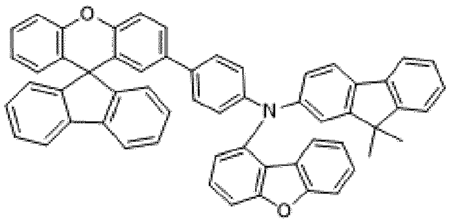 |
| HT-87 | HT-88 |
| 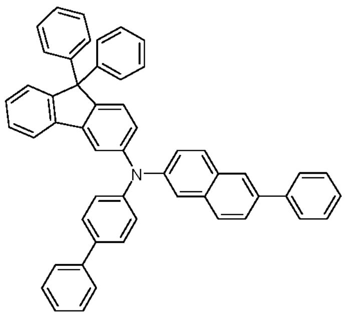 | 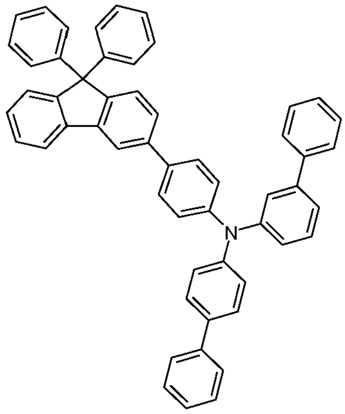 |
| HT-89 | HT-90 |
| 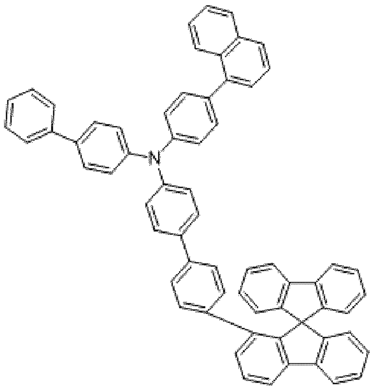 | 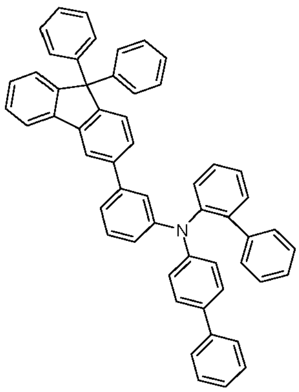 |
| HT-91 | HT-92 |

(fortgesetzt)

| | |
|---|---|
| 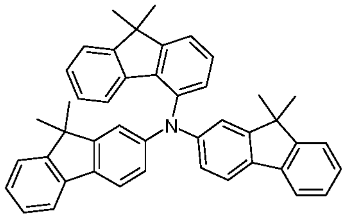 | 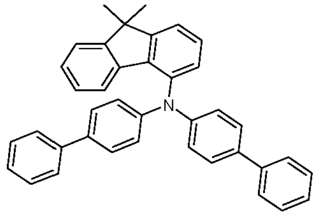 |
| HT-93 | HT-94 |
| 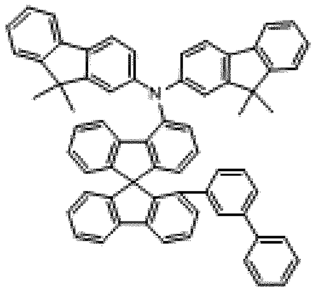 | 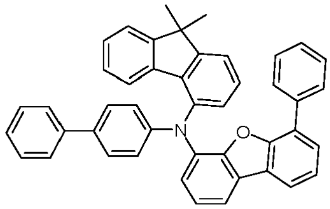 |
| HT-95 | HT-96 |
| 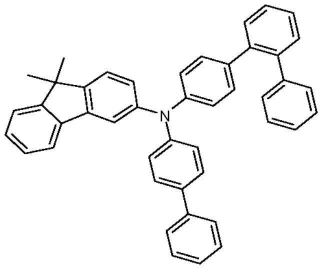 | 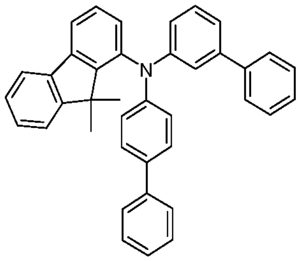 |
| HT-97 | HT-98 |
| 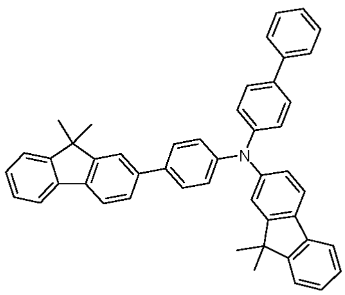 | 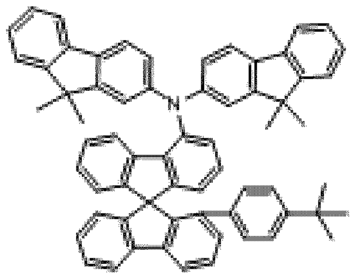 |
| HT-99 | HT-100 |
| 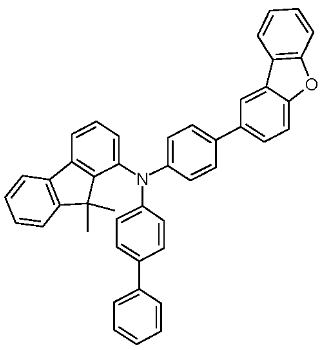 | 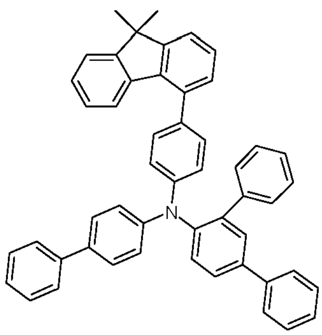 |
| HT-101 | HT-102 |

(fortgesetzt)

| | |
|---|---|
| 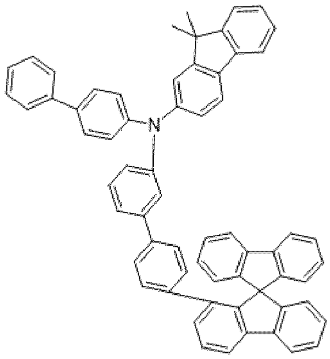 | 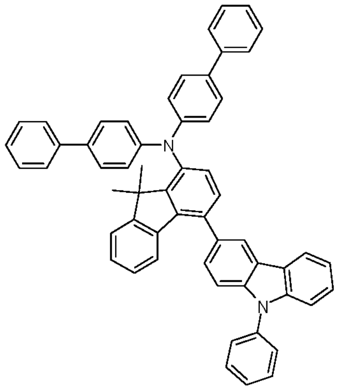 |
| HT-103 | HT-104 |
| 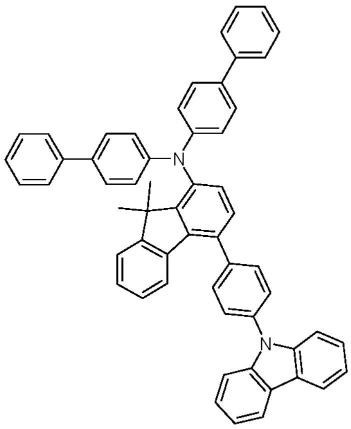 | 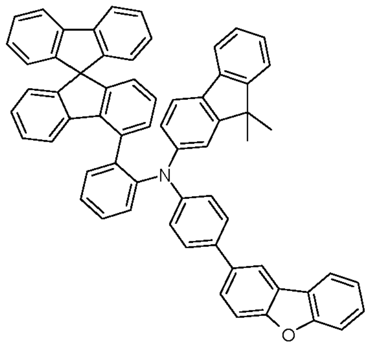 |
| HT-105 | HT-106 |
| 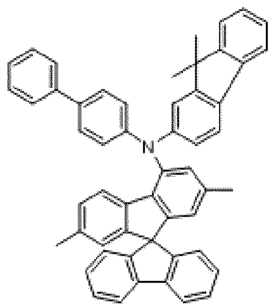 | 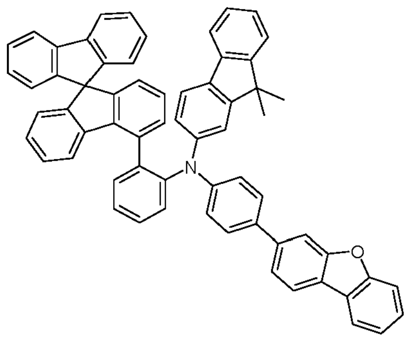 |
| HT-107 | HT-108 |
| 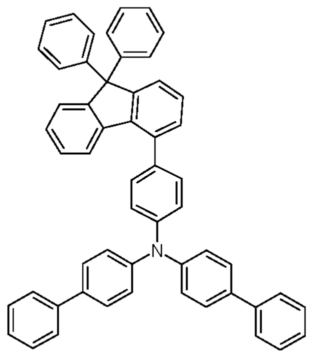 | 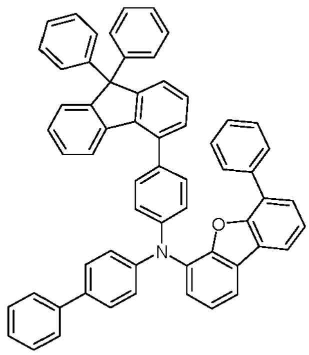 |
| HT-109 | HT-110. |

Die Abfolge der Schichten in der erfindungsgemäßen organischen Elektrolumineszenzvorrichtung ist bevorzugt die folgende:
Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge.

Dabei soll erneut darauf hingewiesen werden, dass nicht alle der genannten Schichten vorhanden sein müssen, und/oder dass zusätzlich weitere Schichten vorhanden sein können.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate.

Als Kathode der erfindungsgemäßen Vorrichtung eignen sich Metalle mit geringer Austrittsarbeit, Metalllegierungen oder mehrlagige Strukturen aus verschiedenen Metallen, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_X$, $Al/PtO_X$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die erfindungsgemäße organische elektrolumineszierende Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

Die Herstellung der erfindungsgemäßen Vorrichtung ist hierbei nicht eingeschränkt. Es ist möglich, dass eine oder mehrere organische Schichten, auch die lichtemittierende Schicht, mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist die erfindungsgemäße organische Elektrolumineszenzvorrichtung dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist die erfindungsgemäße organische Elektrolumineszenzvorrichtung dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Hostmaterialien 1 und 2 und phosphoreszierende Emitter nötig. Das Verarbeiten aus Lösung hat den Vorteil, dass beispielsweise die lichtemittierende Schicht sehr einfach und kostengünstig aufgebracht werden kann. Diese Technik eignet sich insbesondere für die Massenproduktion organischer elektrolumineszierender Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung der erfindungsgemäßen organischen elektrolumineszierenden Vorrichtung, wie zuvor beschrieben oder bevorzugt beschrieben, dadurch gekennzeichnet, dass die organische Schicht, vorzugsweise die lichtemittierende Schicht, die Lochinjektionsschicht und/oder Lochtransportschicht, durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung mittels Gasphasenabscheidung bestehen grundsätzlich zwei Möglichkeiten, wie die erfindungsgemäße organische Schicht, bevorzugt die lichtemittierende Schicht, auf ein beliebiges Substrat bzw. die vorherige Schicht aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed", Premix-Systeme) und das Gemisch in einer einzigen Materialquelle vorgelegt werden, aus der es schließlich verdampft wird ("premix-evaporation"). Dadurch lässt sich auf einfache und schnelle Art und Weise das Aufdampfen der lichtemittierenden Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge ein Verfahren zur Herstellung der erfindungsgemäßen Vorrichtung, dadurch gekennzeichnet, dass die lichtemittierende Schicht der organischen Schicht durch Gasphasenabscheidung aufgebracht wird, wobei die mindestens eine Verbindung der Formel (1) zusammen mit den weiteren Materialien, die die lichtemittierende Schicht bilden, nacheinander oder gleichzeitig aus mindestens zwei Materialquellen aus der Gasphase abgeschieden werden.

In einer bevorzugten Ausführungsform der vorliegenden Erfindung wird die lichtemittierende Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren zur Herstellung der erfindungsgemäßen Vorrichtung, dadurch gekennzeichnet, dass die lichtemittierende Schicht der organischen Schicht durch Gasphasenabscheidung aufgebracht wird, wobei die mindestens eine Verbindung der Formel (1) zusammen mit mindestens einem weiteren Matrixmaterial als Vormischung (premix), nacheinander oder gleichzeitig mit den lichtemittierenden Materialien, ausgewählt aus der Gruppe der phosphoreszierenden Emitter, der fluoreszierenden Emitter und/oder der Emitter, die TADF (thermally activated delayed fluorescence) zeigen, aus der Gasphase abgeschieden werden.

Die erfindungsgemäßen Vorrichtungen zeichnen sich durch folgende überraschende Vorteile gegenüber dem Stand der Technik aus:

Die Verwendung der beschriebenen Materialkombination der Hostmaterialien 1 und 2, wie zuvor beschrieben, führt insbesondere aufgrund der Substitution mit zwei Cyanogruppen und einem bestimmten Substitutionsmuster im Hostmaterial 1 zu einer Steigerung der Lebensdauer der Vorrichtungen und/oder zu einer höheren Stabilität der Vorrichtungen.

Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer und/oder höhere Stabilität aus. Dies gilt vor allem gegenüber ähnlichen Verbindungen enthaltend zwei Cyanogruppen, welche am Azadibenzofuran- oder Azadibenzothienl-Grundgerüst ein anderes Substitutionsmuster aufweisen.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen gemäß Formel (1) bzw. die zuvor und nachfolgend ausgeführten bevorzugten Ausführungsformen, insbesondere als Matrixmaterial oder als elektronenleitende Materialien, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.

2. Elektronische Vorrichtungen, insbesondere organische Elektrolumineszenzvorrichtungen enthaltend Verbindungen gemäß Formel (1) bzw. die zuvor und nachfolgend ausgeführten bevorzugten Ausführungsformen als elektronenleitende Materialien, und/oder Matrixmaterialien weisen eine hervorragende Effizienz auf. Hierbei bewirken erfindungsgemäße Verbindungen gemäß Formel (1) bzw. die zuvor und nachfolgend ausgeführten bevorzugten Ausführungsformen eine geringe Betriebsspannung bei Verwendung in elektronischen Vorrichtungen.

3. Die erfindungsgemäßen Verbindungen gemäß Formel (1) bzw. die zuvor und nachfolgend ausgeführten bevorzugten Ausführungsformen zeigen eine sehr hohe Stabilität und Lebensdauer.

4. Mit Verbindungen gemäß Formel (1) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanälen vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen gemäß Formel (1) bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektron-Leiterstrukturen.

6. Verbindungen gemäß Formel (1) bzw. die zuvor und nachfolgend ausgeführten bevorzugten Ausführungsformen weisen eine ausgezeichnete Glasfilmbildung auf.

7. Verbindungen gemäß Formel (1) bzw. die zuvor und nachfolgend ausgeführten bevorzugten Ausführungsformen bilden aus Lösungen sehr gute Filme.

8. Die Verbindungen gemäß Formel (1) bzw. die zuvor und nachfolgend ausgeführten bevorzugten Ausführungsformen weisen ein tiefes Triplett-Niveau $T_1$ auf, welches beispielsweise im Bereich von 2,40 eV - 2,90 eV liegen kann.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbarte Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

## Beispiele

Allgemeine Methoden:

In allen quantenchemischen Berechnungen wird das Programmpaket Gaussian16 (Rev. B.01) verwendet. Der neutrale Singulettgrundzustand wird auf dem B3LYP/6-31G(d)-Niveau optimiert. HOMO- und LUMO-Werte werden auf dem B3LYP/6-31G(d)-Niveau für die mit B3LYP/6-31G(d) optimierte Grundzustandsenergie bestimmt. Daraufhin werden TD-DFT-Singulett- und Triplettanregungen (vertikale Anregungen) mit der gleichen Methode (B3LYP/6-31G(d)) und der optimierten Grundzustandsgeometrie berechnet. Die Standardeinstellungen für SCF- und Gradientenkonvergenz werden verwendet.

Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMOcorr} = 0.90603 * \text{HOMO} - 0.84836$$

$$\text{LUMOcorr} = 0.99687 * \text{LUMO} - 0.72445$$

Das Triplett-Niveau T1 eines Materials ist definiert als die relative Anregungsenergie (in eV) des Triplettzustands mit der niedrigsten Energie, der sich aus der quantenchemischen Energierechnung ergibt.

Das Singulett-Niveau S1 eines Materials ist definiert als die relative Anregungsenergie (in eV) des Singulettzustands mit der zweitniedrigsten Energie, der sich aus der quantenchemischen Energierechnung ergibt.

Der energetisch niedrigste Singulettzustand wird als S0 bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian16 (Rev. B.01)" verwendet.

Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

Synthesebeispiel 1:

**a) Benzofuro[3,2-d]pyrimidin-2,4-dicarbonitril**

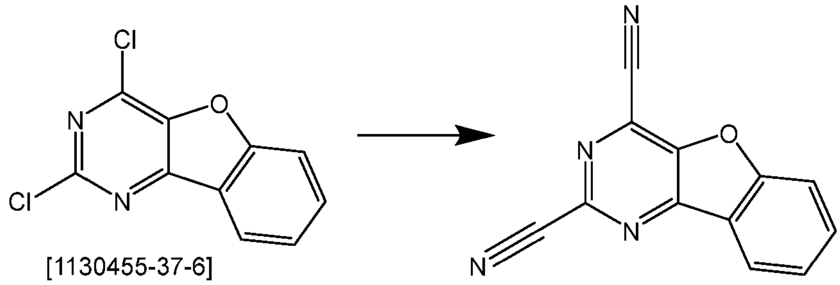

[1130455-37-6]

22,4 g (94.5 mmol) 2,4-Dichlorobenzofuro[3,2-d]pyrimidin, 16,9 g (189 mmol) Kupfer(I)cyanid und 13,5 g (208 mmol) Natriumcarbonat werden in 20 mL N,N-Dimethylformaldehyd suspendiert und die Reaktionsmischung wird 6 h unter Rückfluss auf 170 °C erhitzt. Nach Erkalten wird die Mischung mit Dichlormethan versetzt und über Kieselgel filtriert und anschließend zur Trockne eingeengt. Der Rückstand wird aus Toluol und aus Dichlormethan /Heptan umkristallisiert. Ausbeute: 13,5 g (61 mmol), 65% der Theorie

Analog dazu werden die folgenden Verbindungen hergestellt:

| | Edukt 1 | Edukt 2 | Ausbeute |
|---|---|---|---|
| 1a | 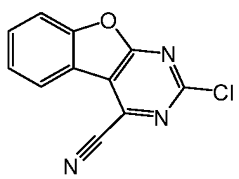 [ 2363033-70-7 ] | 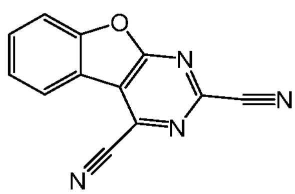 | 81% |
| 2a | 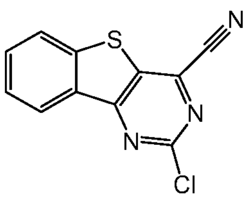 [2102496-35-3 ] | 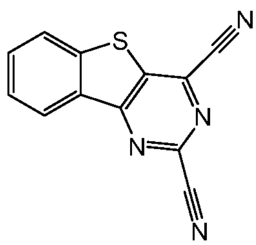 | 85% |

(fortgesetzt)

| | Edukt 1 | Edukt 2 | Ausbeute |
|---|---|---|---|
| 3a | 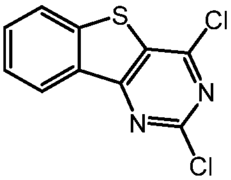 [160199-05-3] | 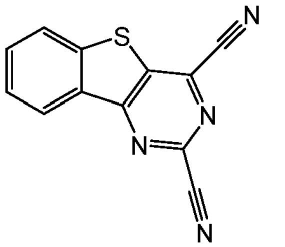 | 79% |
| 4a | 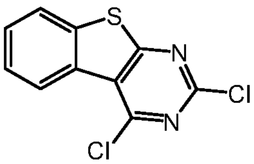 [76872-40-7] | 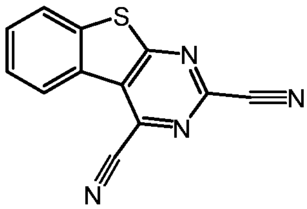 | 78% |
| 5a | 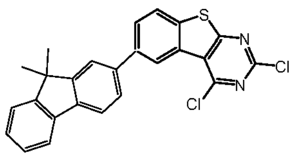 [2219361-27-8] | 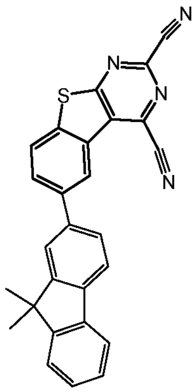 | 70% |
| 6a | 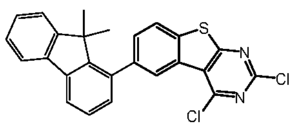 [2219361-24-5] | 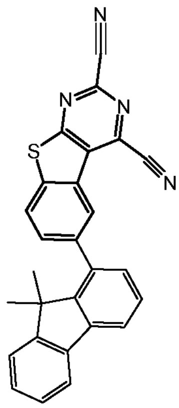 | 76% |
| 7a | 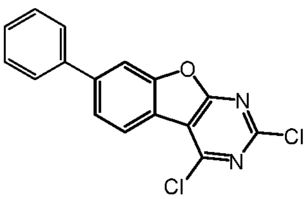 [2201128-28-9] | 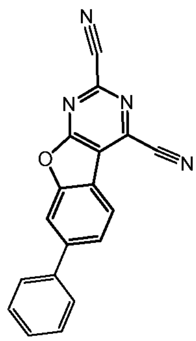 | 80% |

(fortgesetzt)

| | Edukt 1 | Edukt 2 | Ausbeute |
|---|---|---|---|
| 8a | 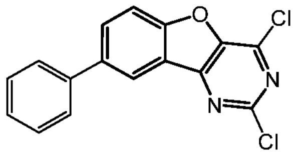 [1835207-37-8] | 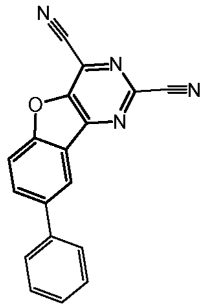 | 77% |
| 9a | 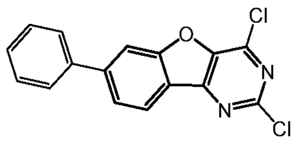 [2201128-14-3] | 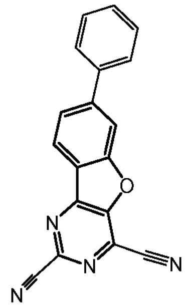 | 64% |
| 10a | 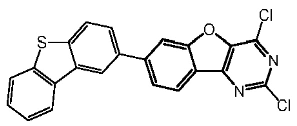 [2201128-2-2] | 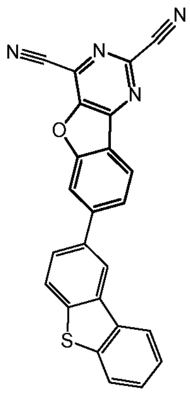 | 66% |
| 11a | 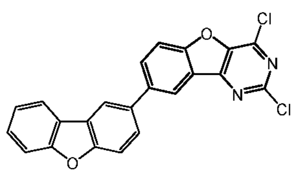 [2219361-31-4] | 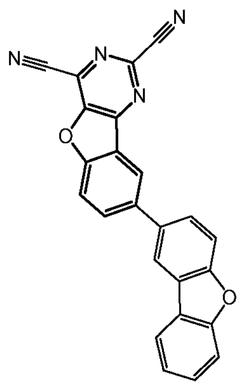 E7 | 815 |

(fortgesetzt)

| | Edukt 1 | Edukt 2 | Ausbeute |
|---|---|---|---|
| 12a | 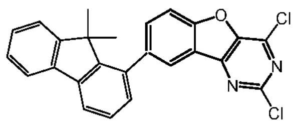 [2219361-42-7] | 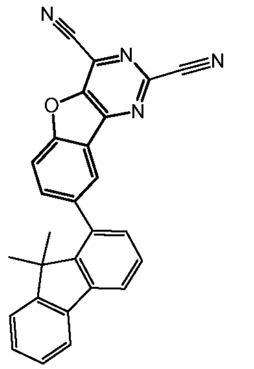 | 66% |
| 13a | 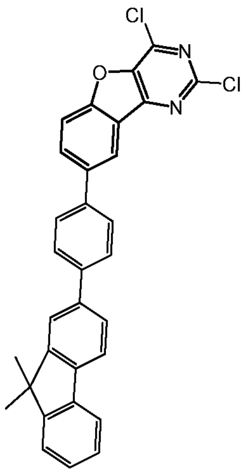 [2219361-33-6] | 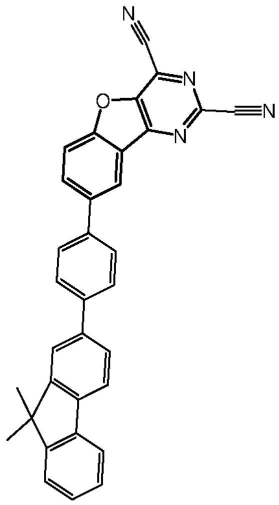 | 73% |
| 14a | 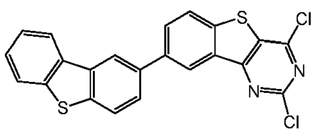 [2219361-62-1] | 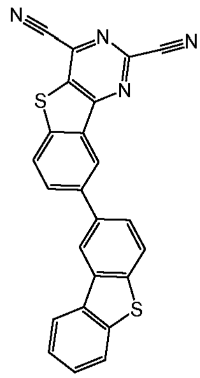 | 70% |

(fortgesetzt)

| | Edukt 1 | Edukt 2 | Ausbeute |
|---|---|---|---|
| 15a | 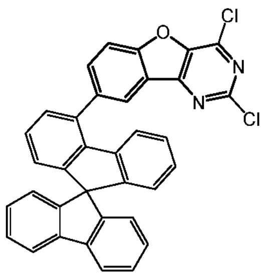 [2219361-32-5] | 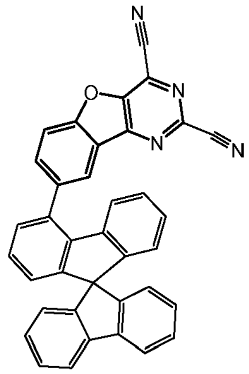 E8 | 61% |
| 16a | 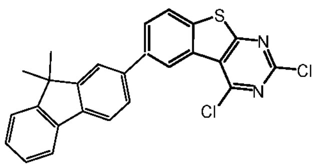 [2219361-27-8] | 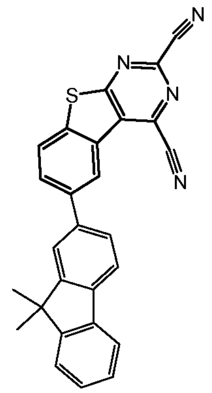 | 70% |
| 17a | 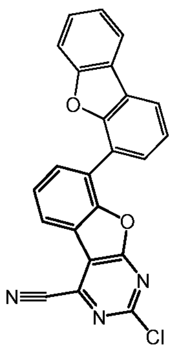 | 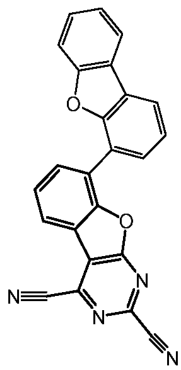 | 78% |

**b) 2-Chlorobenzofuro[3,2-d]pyrimidin-4-carbonitril**

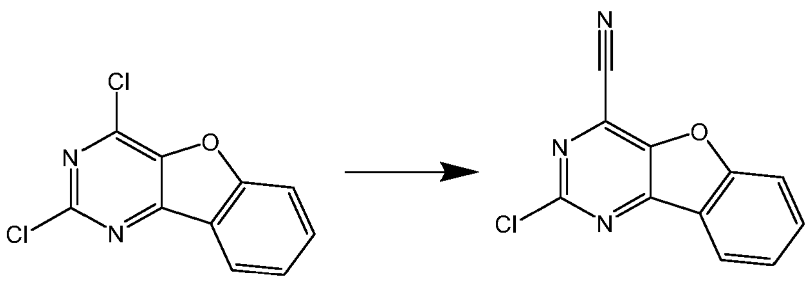

[1130455-37-6]

22,4 g (94.5 mmol) 2,4-Dichlorobenzofuro[3,2-d]pyrimidin, 16,9 g (189 mmol) Kupfer(I)cyanid und 6,3 g (95mmol) Natriumcarbonat werden in 20 mL N,N-Dimethylformaldehyd suspendiert und die Reaktionsmischung wird 6 h unter Rückfluss auf 170 °C erhitzt. Nach Erkalten wird die Mischung mit Dichlormethan versetzt und über Kieselgel filtriert und anschließend zur Trockne eingeengt. Der Rückstand wird aus Toluol und aus Dichlormethan /Heptan umkristallisiert. Ausbeute: 14 g (75 mmol), 65% der Theorie.

Analog dazu werden folgende Verbindungen hergestellt:

| | Edukt 1 | Edukt 2 | Ausbeute |
|---|---|---|---|
| 1b | 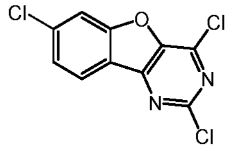 [2303611-55-2] | 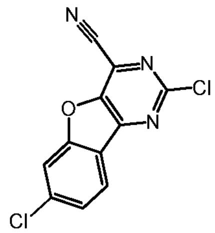 | 77% |
| 2b | 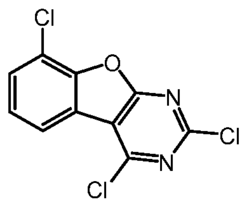 [2303611-57-4 | 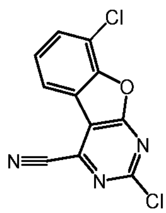 | 57% |
| 3b | 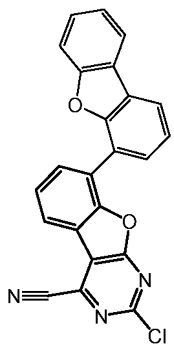 | 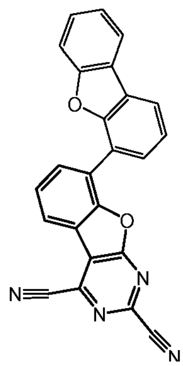 | 73% |
| 4b | 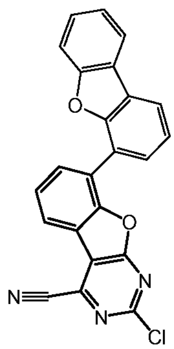 | 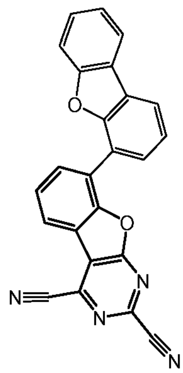 | 73% |

(fortgesetzt)

| | Edukt 1 | Edukt 2 | Ausbeute |
|---|---|---|---|
| 5b | 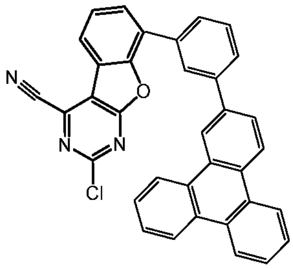 | 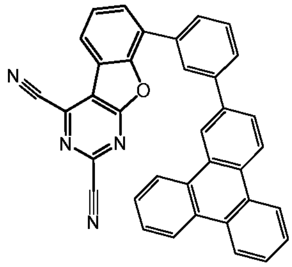 E9 | 76% |
| 6b | 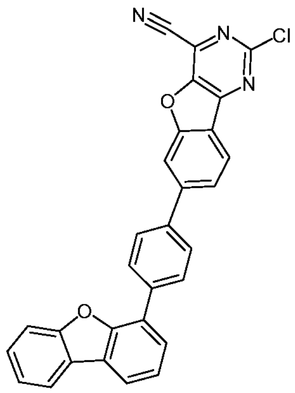 | 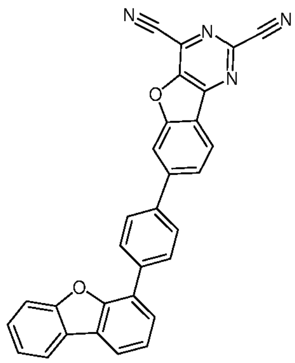 | 79% |
| 7b | 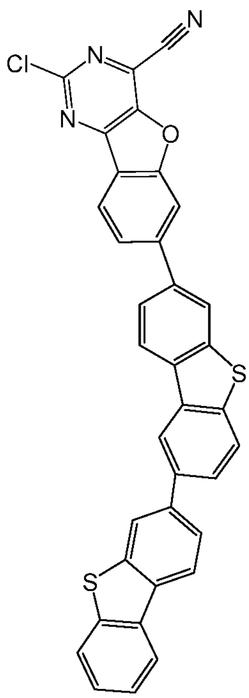 | 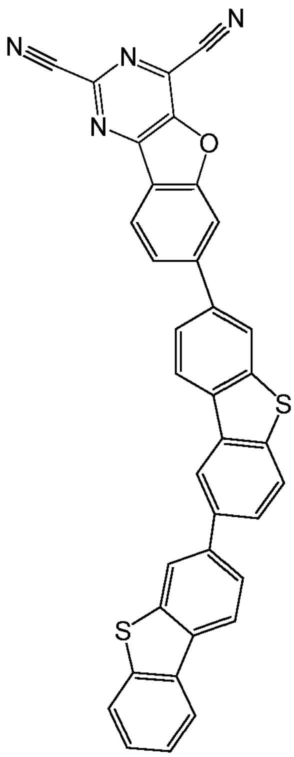 | 70% |

(fortgesetzt)

| | Edukt 1 | Edukt 2 | Ausbeute |
|---|---|---|---|
| 8b | 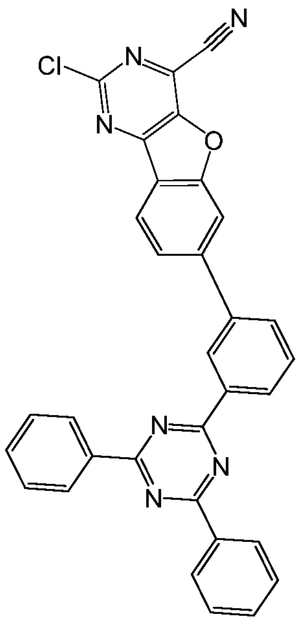 | 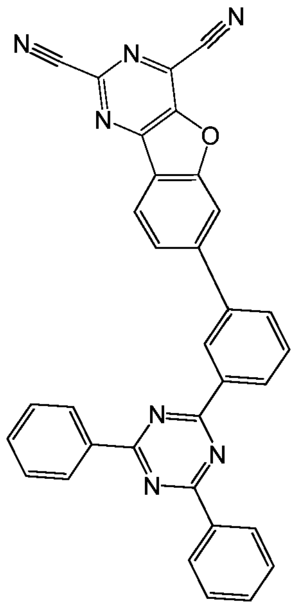 E10 | 65% |
| 9b | 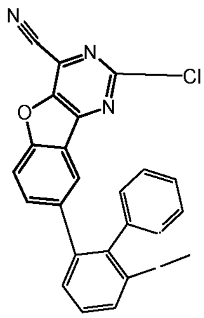 | 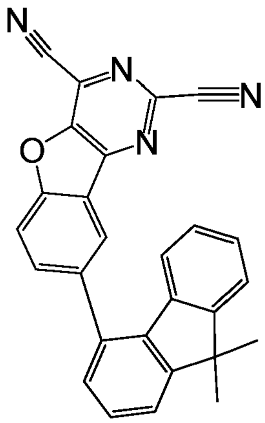 | 64% |
| 10b | 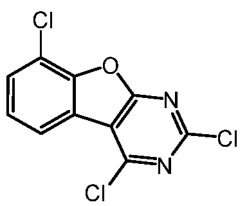 [2303611-57-4 | 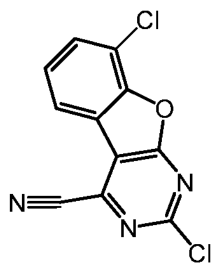 | 73% |

(fortgesetzt)

| | Edukt 1 | Edukt 2 | Ausbeute |
|---|---|---|---|
| 11b | 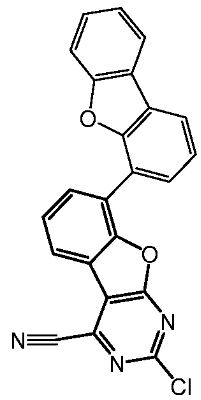 | 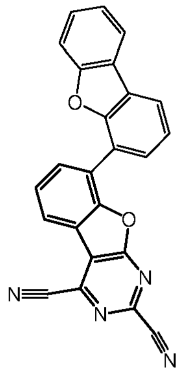 E1 | 70% |
| 12b | 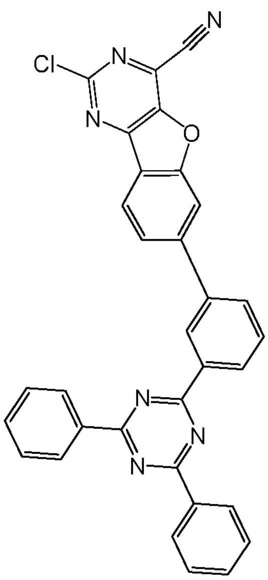 | 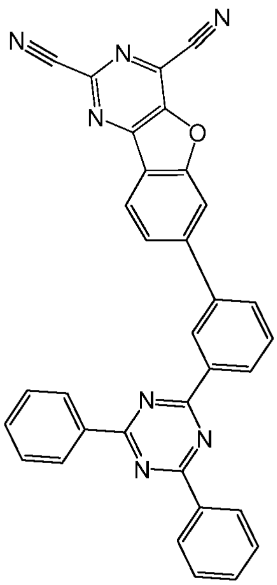 | 65% |

**c) 8-Bromobenzofuro[3,2-d]pyrimidin-2,4-dicarbonitril**

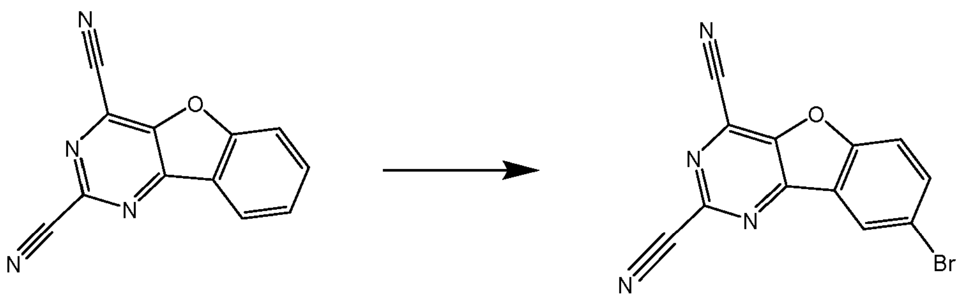

51,5 g (190.0 mmol) Benzofuro[3,2-d]pyrimidin-2,4-dicarbonitril werden in 2000 mL Essigsäure (100%) und 2000 mL Schwefelsäure (95-98%) suspendiert. Zu dieser Suspension werden portionsweise 34 g (190 mmol) NBS (N-Bromsuccinimid) zugegeben und 2 Stunden in Dunkelheit gerührt. Danach mit Wasser/Eis versetzt und der Feststoff wird abgetrennt und mit Ethanol nachgewaschen. Der Rückstand wird in Toluol umkristallisiert. Die Ausbeute beträgt 34,2 g (115 mmol), entsprechend 82% der Theorie.

Analog dazu werden die folgenden Verbindungen hergestellt:

| | Edukt 1 | Produkt | Ausbeute |
|---|---|---|---|
| 1c | 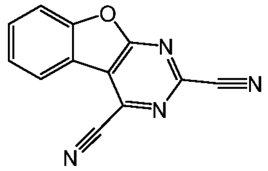 | 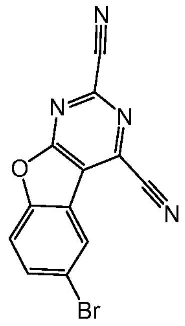 | 86% |
| 2c | 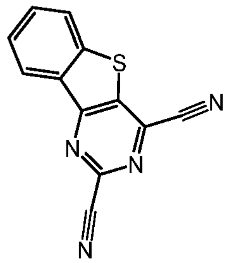 | 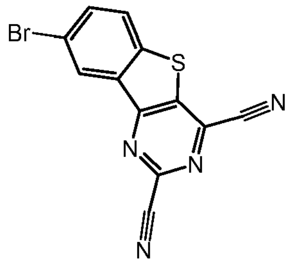 | 71% |
| 3c | 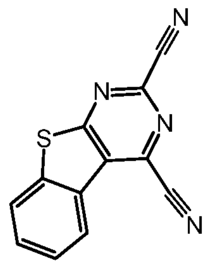 | 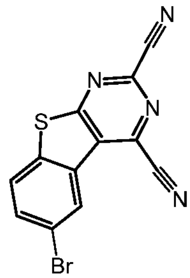 | 69% |

d) 8-[(4-Phenyl)phenyl]benzofuro[3,2-d]pyrimidin-2,4-dicarbonitril

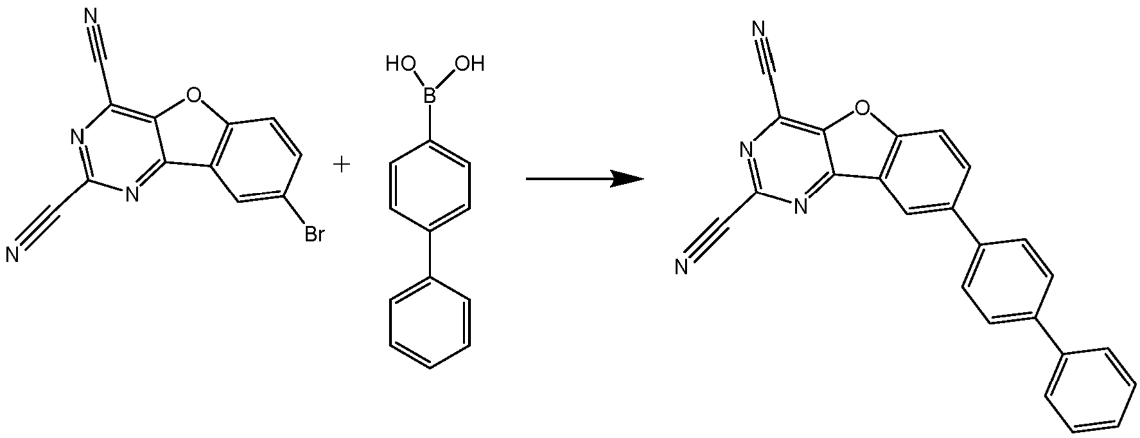

21,7 g (110.0 mmol) Biphenyl-boronsäure, 29,8 g (100 mmol) 8-Bromobenzofuro[3,2-d]pyrimidin-2,4-dicarbonitril und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II) acetat gegeben, und die Reaktionsmischung wird 16 h unter Rückfluss erhitzt. Nach Erkalten wird die organische Phase abgetrennt, über Kieselgel filtriert und anschließend zur Trockne eingeengt. Der Rückstand wird aus Toluol und aus Dichlormethan /Heptan umkristallisiert. Ausbeute: 30 g (81 mmol), 81 % der Theorie.

Analog dazu werden die folgenden Verbindungen hergestellt:

| | Edukt 1 | Edukt 2 | Produkt | Ausbeute |
|---|---|---|---|---|
| 1d | 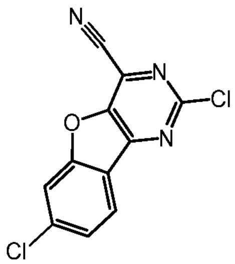 | 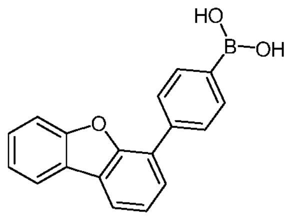 [796071-96-0 ] | 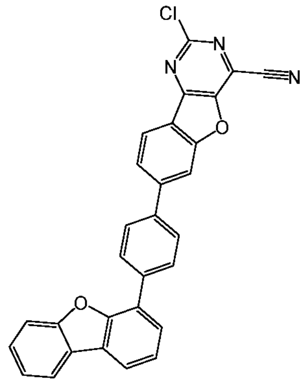 | 66% |
| 2d | 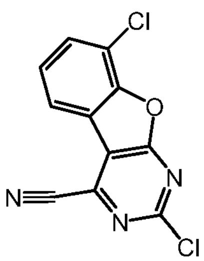 | 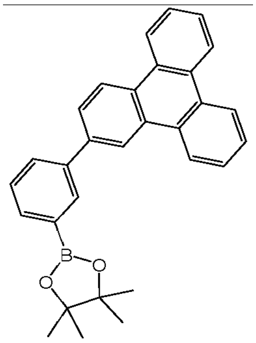 [1115639-92-3] | 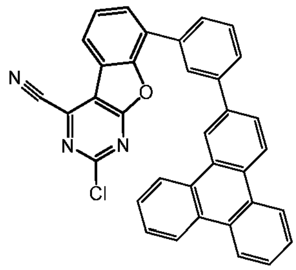 | 72% |
| 3d | 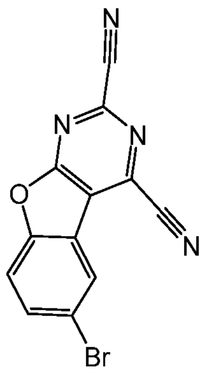 | 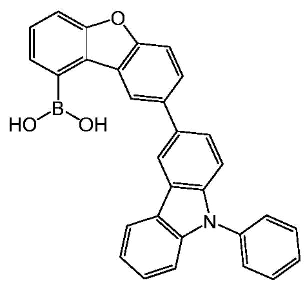 [1822311-30-7] | 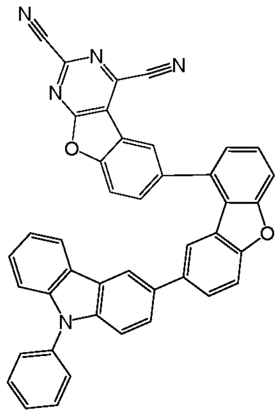 E11 | 79% |

(fortgesetzt)

| | Edukt 1 | Edukt 2 | Produkt | Ausbeute |
|---|---|---|---|---|
| 4d | 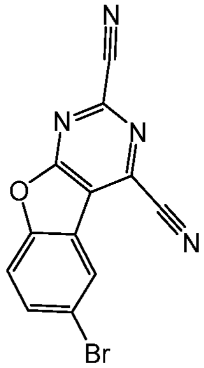 | 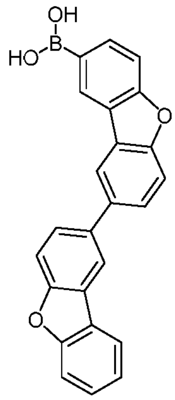 [1427560-52-8] | 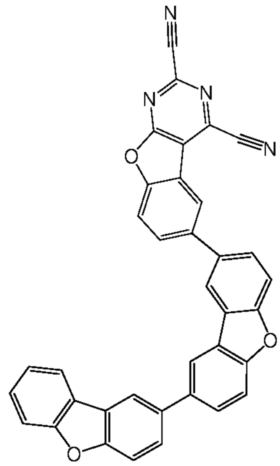 | 63% |
| 5d | 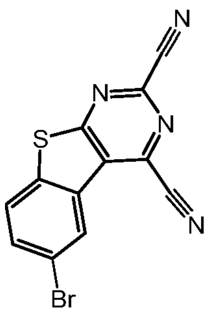 | 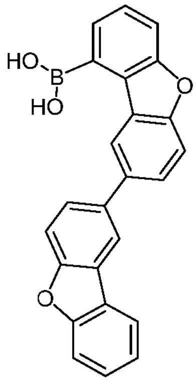 [1822320-55-7] | 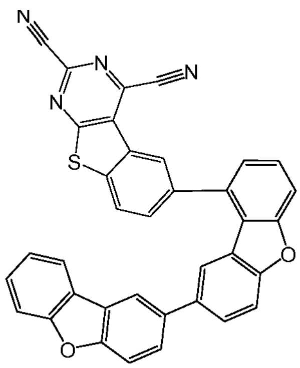 | 65% |
| 6d | 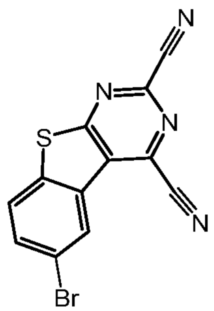 | 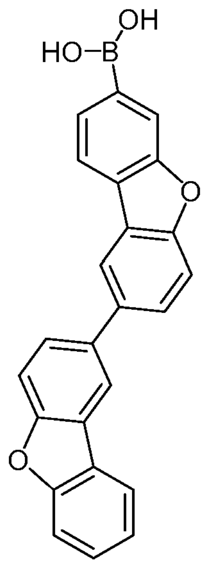 [2235385-15-4 | 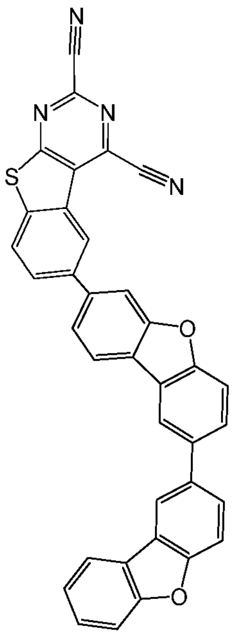 | 73% |

(fortgesetzt)

| | Edukt 1 | Edukt 2 | Produkt | Ausbeute |
|---|---|---|---|---|
| 7d | 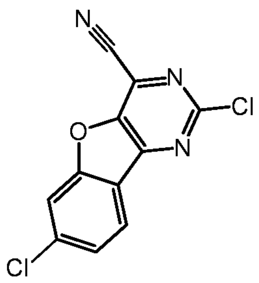 | 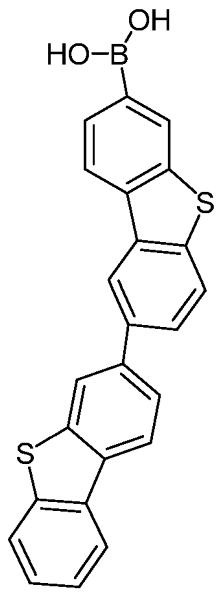 [412401-01-3] | 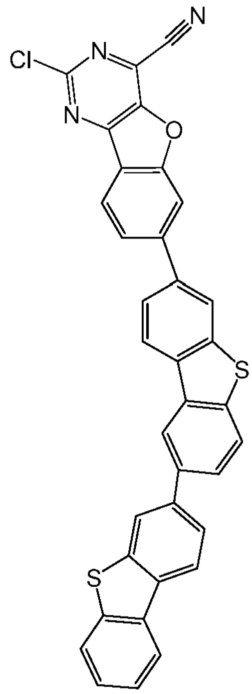 | 71% |
| 8d | 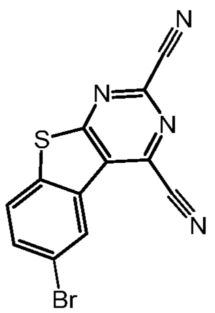 | 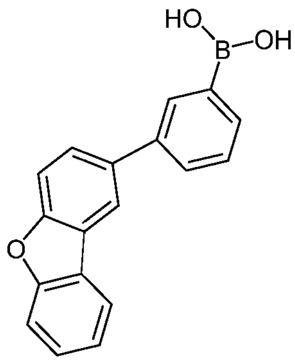 [1100752-08-6] | 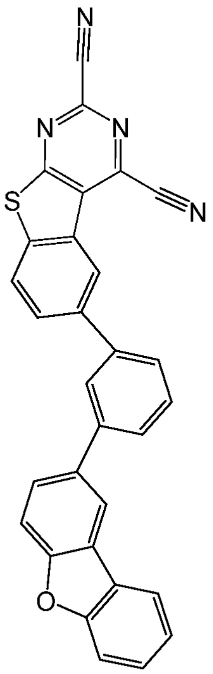 E13 | 67% |

(fortgesetzt)

| | Edukt 1 | Edukt 2 | Produkt | Ausbeute |
|---|---|---|---|---|
| 9d | 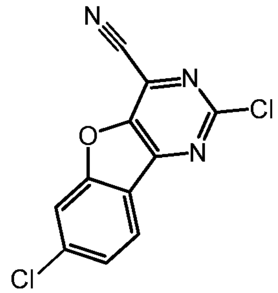 | 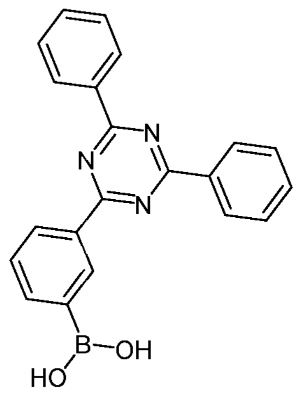<br>[1612243-82-9] | 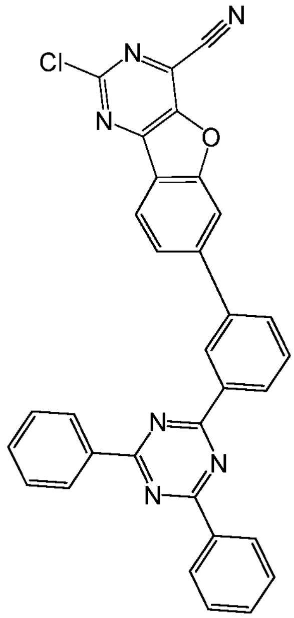 | 67% |
| 10d | 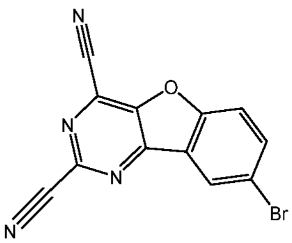 | 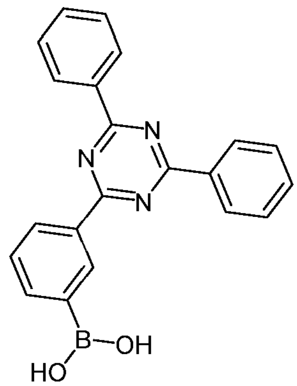<br>[1612243-82-9] | 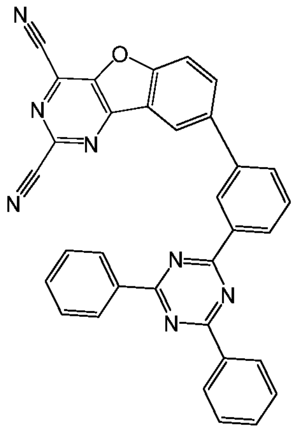 | 59% |
| 11d | 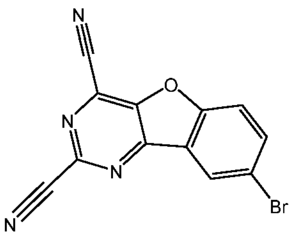 | 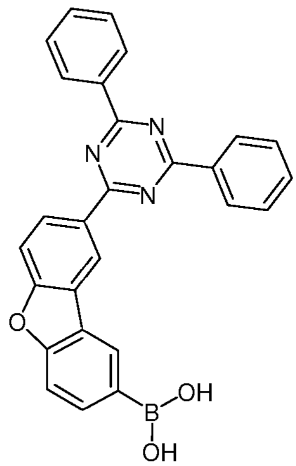<br>[2244910-30-1] | 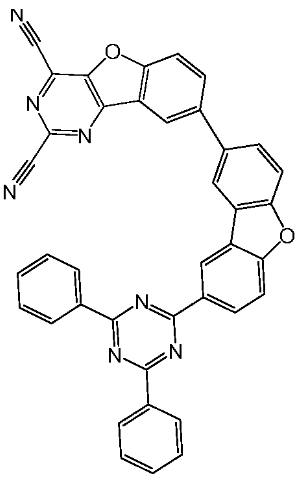<br>E14 | 63% |

(fortgesetzt)

| | Edukt 1 | Edukt 2 | Produkt | Ausbeute |
|---|---|---|---|---|
| 12d | 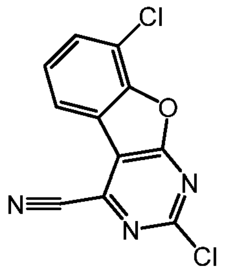 | 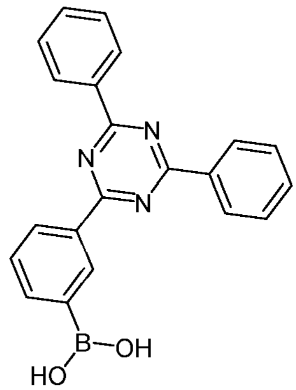 [1612243-82-9] | 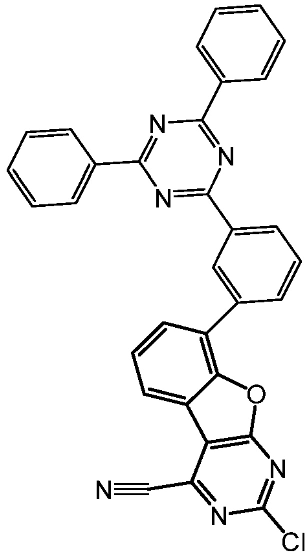 | 60% |
| 13d | 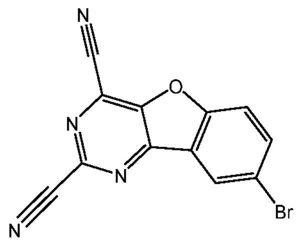 | 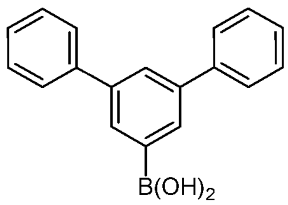 [128388-54-5 ] | 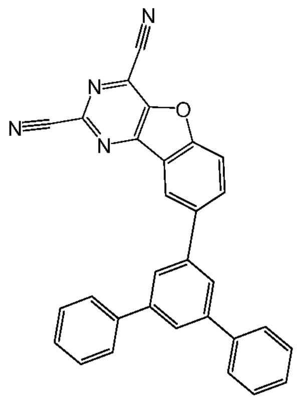 E12 | 71% |
| 14d | 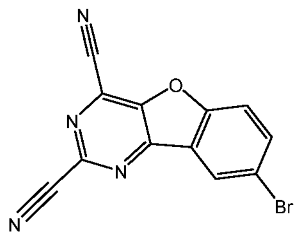 | 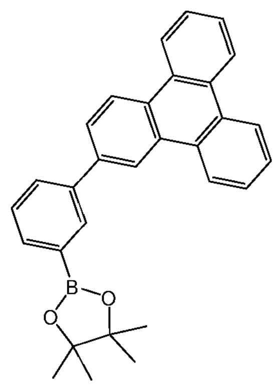 [1115639-92-3] | 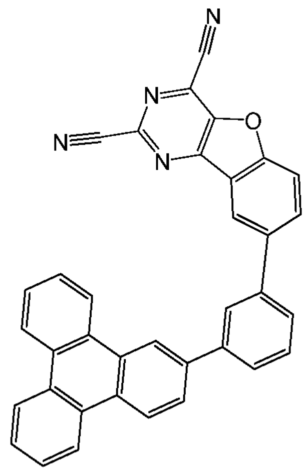 E15 | 75% |

(fortgesetzt)

| | Edukt 1 | Edukt 2 | Produkt | Ausbeute |
|---|---|---|---|---|
| 15d | 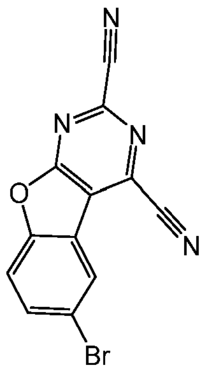 | 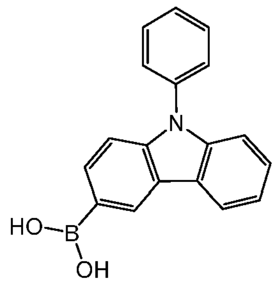 [854952-58-2] | 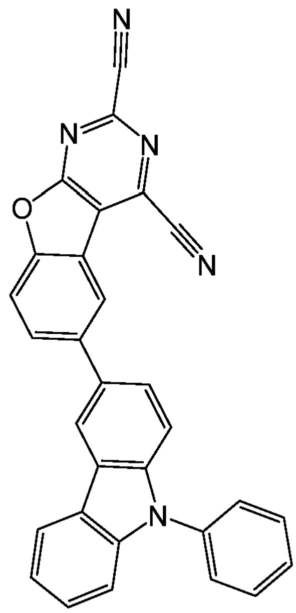 | 68% |
| 16d | 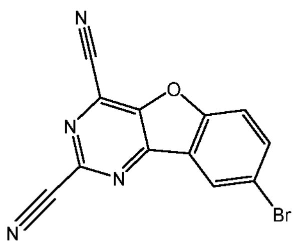 | 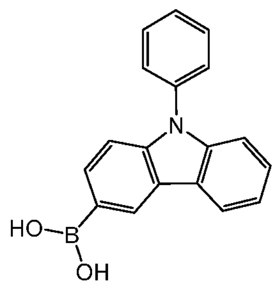 [854952-58-2] | 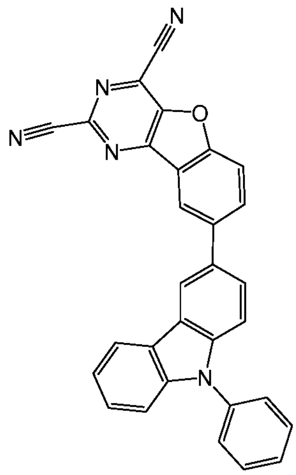 E27 | 65% |
| 17d | 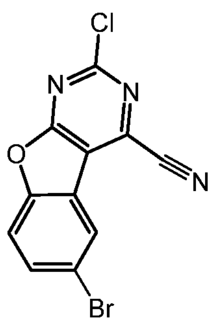 | 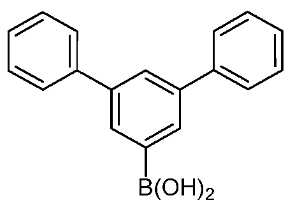 [128388-54-5 ] | 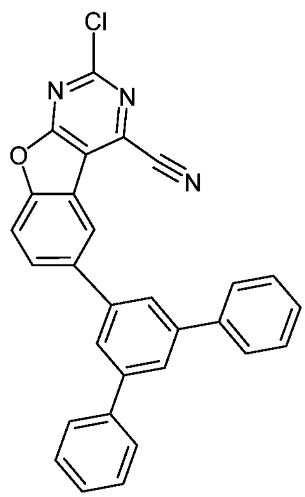 | 78% |

(fortgesetzt)

| | Edukt 1 | Edukt 2 | Produkt | Ausbeute |
|---|---|---|---|---|
| 18d | 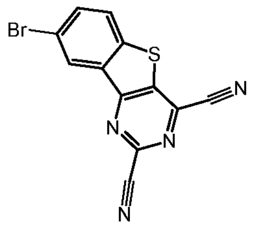 | 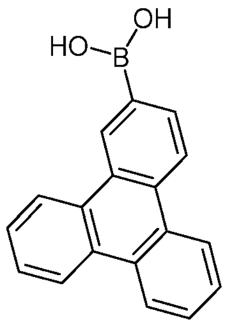 [654664-63-3] | 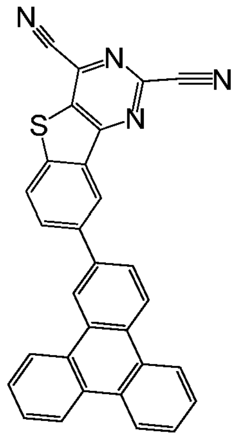 | 60% |
| 19d | 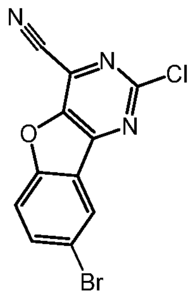 | 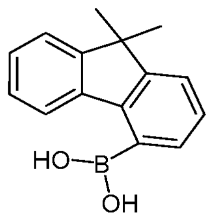 [1246022-50-3] | 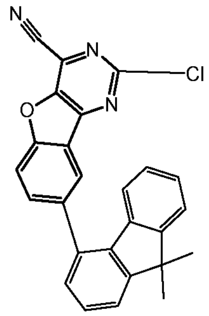 | 65% |
| 20d | 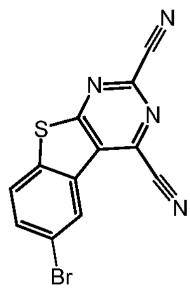 | 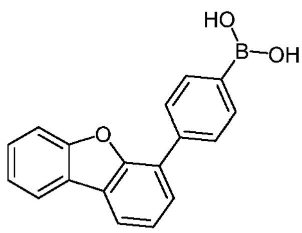 [796071-96-0 ] | 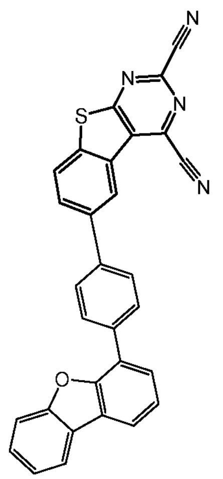 | 69% |

(fortgesetzt)

| | Edukt 1 | Edukt 2 | Produkt | Ausbeute |
|---|---|---|---|---|
| 21d | 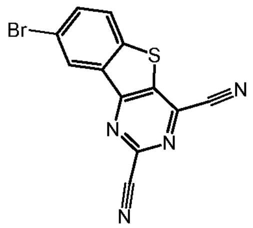 | 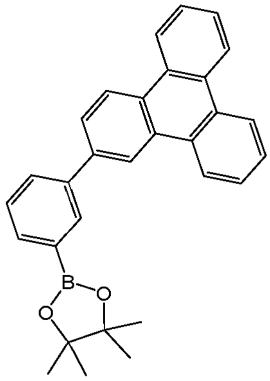 [1115639-92-3] | 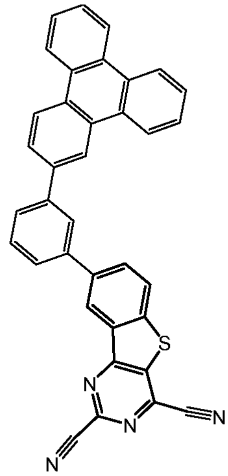 | 78% |
| 22d | 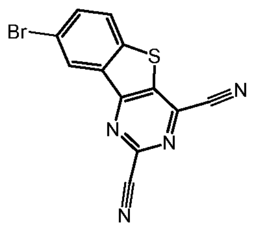 | 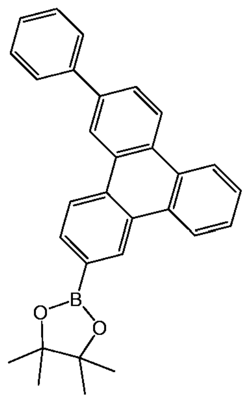 [2020404-50-4]] | 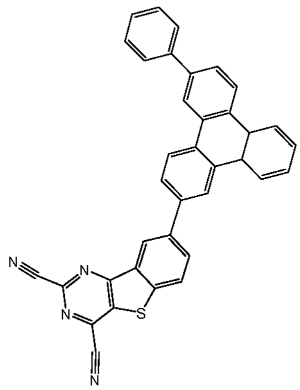 | 74% |
| 23d | 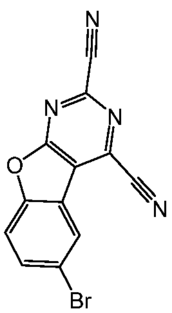 | 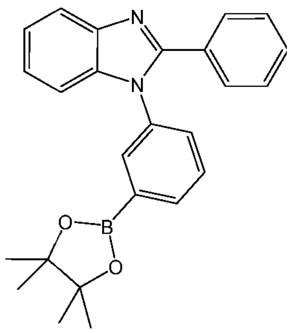 [1549686-33-0] | 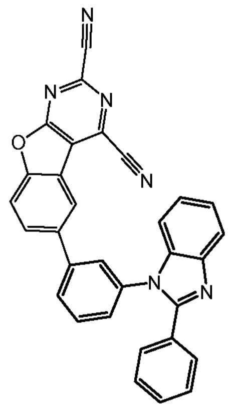 | 62% |

(fortgesetzt)

| | Edukt 1 | Edukt 2 | Produkt | Ausbeute |
|---|---|---|---|---|
| 24d | 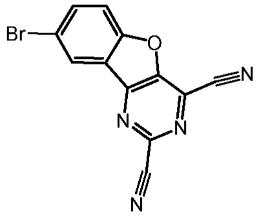 | 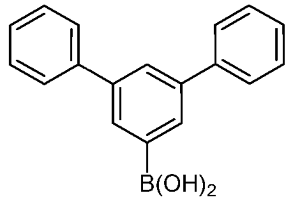 [128388-54-5 ] | 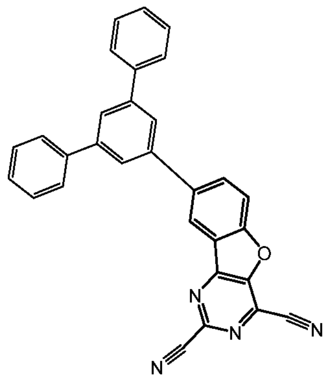 E5 | 80% |
| 25d | 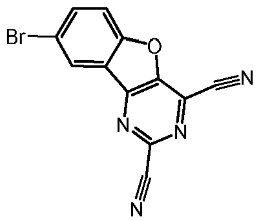 | 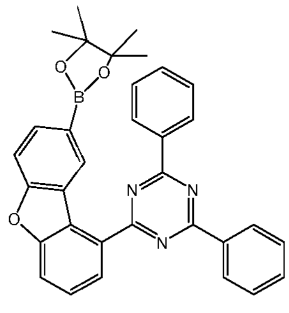 [2138490-96-5 ] | 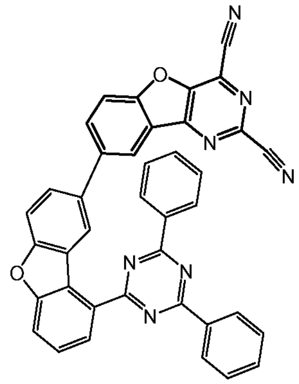 | 77% |
| 26d | 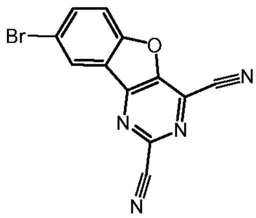 | 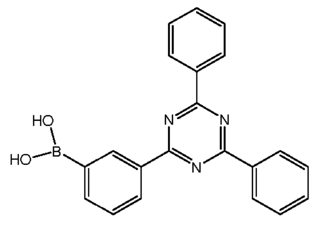 [1612243-82-9 ] | 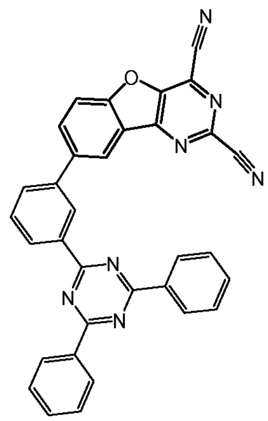 E4 | 74% |
| 27d | 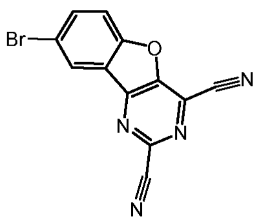 | 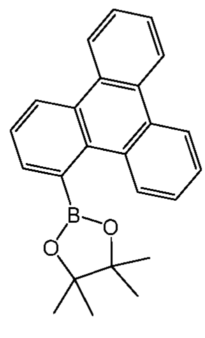 [1795376-65-6] | 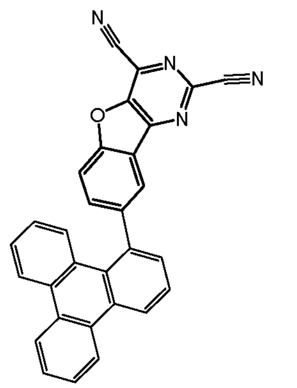 | 64% |

(fortgesetzt)

| | Edukt 1 | Edukt 2 | Produkt | Ausbeute |
|---|---|---|---|---|
| 28d | 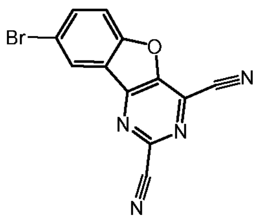 | 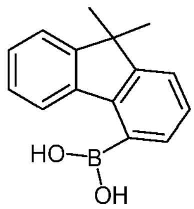 [1246022-50-3] | 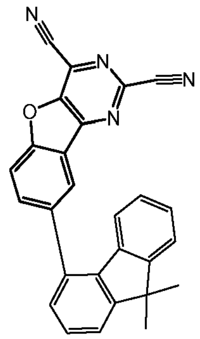 | 61% |
| 29d | 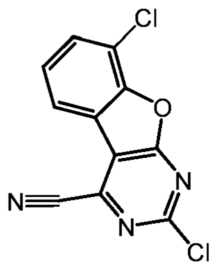 | 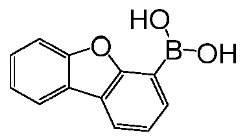 [100124-06-9] | 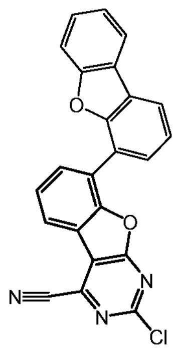 | 64% |
| 30d | 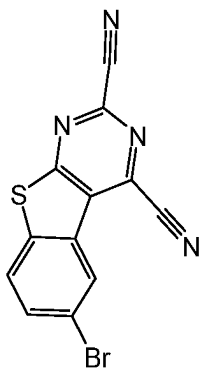 | 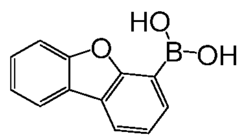 [100124-06-9] | 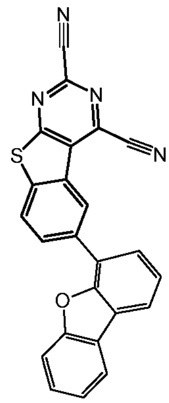 E2 | 63% |
| 31d | 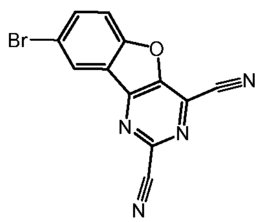 | 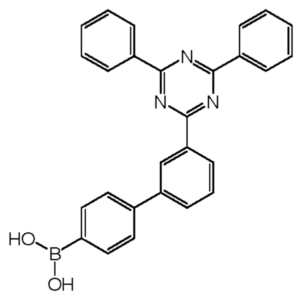 [2697710-14-6] | 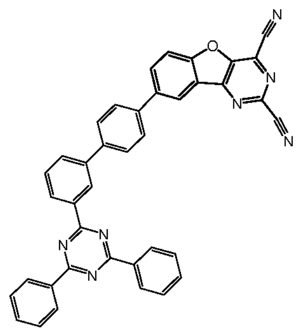 E3 | 62% |

(fortgesetzt)

| | Edukt 1 | Edukt 2 | Produkt | Ausbeute |
|---|---|---|---|---|
| 32d | 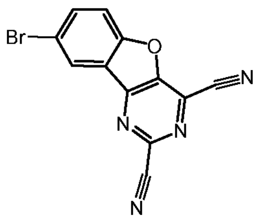 | 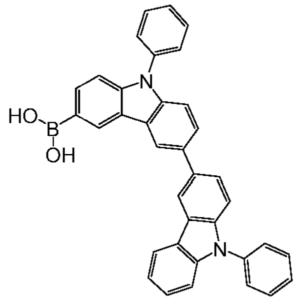 [918137-86-7] | 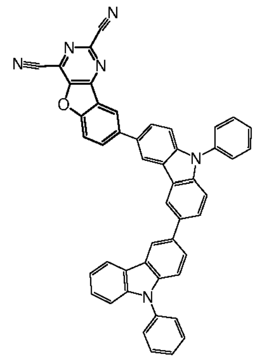 E6 | 64% |

Synthesebeispiel 2:

Allgemeine Deuterierung:

Die Ausgangsverbindung wird in einer Mischung aus deuteriertem Wasser (99% Deuteriumatom) und Toluol-d8 (99% Deuteriumatom) gelöst und 96 Stunden lang unter Druck in Gegenwart von trockenem Platin auf Kohle (5%) als Katalysator auf 160°C erhitzt. Nach dem Abkühlen des Reaktionsgemisches werden die Phasen getrennt und die wässrige Phase zweimal mit dem Tetrahydrofuran-Toluol-Gemisch extrahiert. Die rekombinierten organischen Phasen werden mit einer Natriumchloridlösung gewaschen, über Natriumsulfat getrocknet und filtriert. Das Lösungsmittel wird im Vakuum entfernt, um die rohe deuterierte Verbindung als Feststoff zu liefern. Die Verbindung wird durch Extraktion, Kristallisation und Sublimation weiter gereinigt.

Beispiel A: **1,1',2',3',4',5',6,6',7',8,8'-Undecadeuterio-N-(2,3,6,7,8-pentadeuterio-9,9-**dimethyl-fluoren-4-yl)-N-(3,4,6,7,8-pentadeuterio-9,9-dimethyl-fluoren-2-yl)-9,9'-spirobi[fluoren]-4-amin

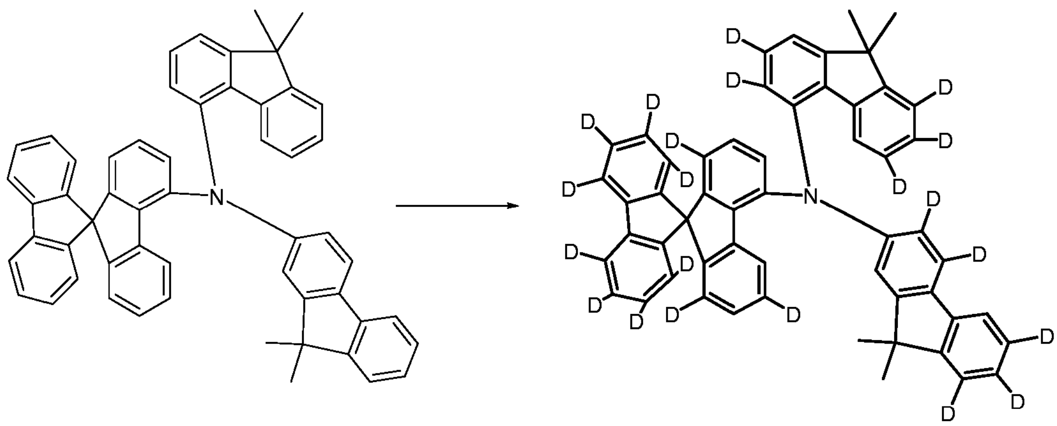

H47

Es wird N-(9,9-Dimethylfluoren-2-yl)-N-(9,9-dimethylfluoren-4-yl)-9,9'-spirobi[fluoren]-4'-amin (22,8 g, 32 mmol), Toluol-d8 (231 g, 2,31 mol), deuteriertes Wasser (1300 g, 64,9 mol) und trockenes Platin auf Kohle (5%) (30 g) werden 24 h bei 130°C gerührt. Das Rohprodukt wird durch zweimaliges Extrahieren mit einer Mischung aus Heptan und Toluol (4:1) und zweimaliges Sublimieren weiter gereinigt.

Ausbeute: 21,2g (28 mmol, 90 %) mit einer Reinheit von > 99,9 %. Die Identität wird durch HPLC-MS und 1H-NMR bestätigt.

Beispiel B: **1,2,3,5,6,7,8-Heptadeuterio-N-[1,2,3,5,6,7,8-heptadeuterio-9,9-bis(trideuteriomethyl)fluoren-4-yl]-9,9-bis(trideuteriomethyl)-N-[2,3,5-trideuterio-4-(2,3,4,5,6-pentadeuteriophenyl)phenyl]fluoren-4-amin**

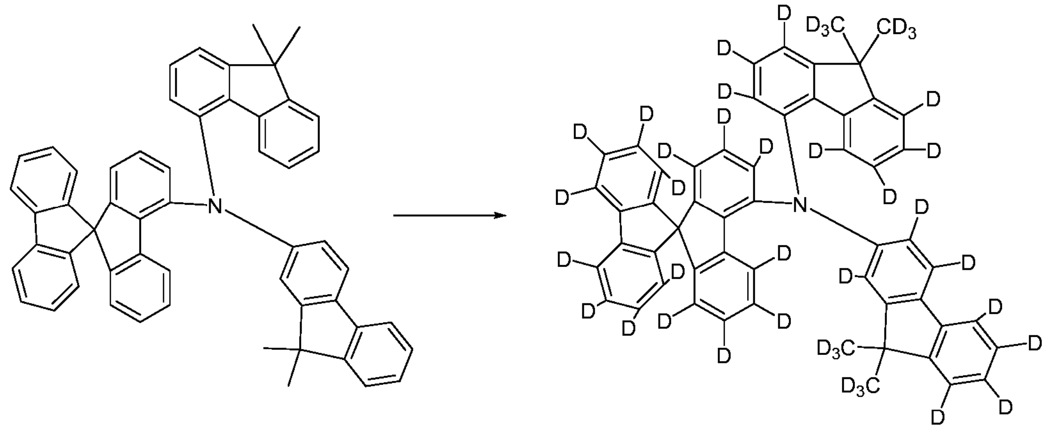

H48

Es wird N-(9,9-Dimethylfluoren-2-yl)-N-(9,9-dimethylfluoren-4-yl)-9,9'-spirobi[fluoren]-4'-amin (22.8 g, 31,8 mmol), Toluol-d8 (231 g, 2,31 mol), deuteriertes Wasser (1300 g, 64,9 mol) und trockenes Platin auf Kohle (5%) (30 g) 96 h bei 160°C gerührt. Das Rohprodukt wird durch zweimaliges Extrahieren mit einer Mischung aus Heptan und Toluol (4:1) und zweimaliges Sublimieren weiter gereinigt.

Ausbeute: 21,9 g (28,9 mmol, 95 %) mit einer Reinheit von > 99,9 %. Die Identität wird durch HPLC-MS bestätigt.

**Herstellung der OLEDs**

In den folgenden Beispielen V1 bis V7 und B1 bis B24 (siehe Tabellen 7 und 8) werden die Daten verschiedener OLEDs vorgestellt.

**Vorbehandlung für die Beispiele V1-V7 und B1-B24:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 8 zu entnehmen. Die zur Herstellung der OLEDs benötigten Materialien sind in Tabelle 9 gezeigt, sofern nicht zuvor beschrieben.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie VG1:H2:TEG1 (33%:60%:7%) bedeutet hierbei, dass das Material VG1 in einem Volumenanteil von 33%, das Material H2 in einem Anteil von 60% und der Emitter TEG1 in einem Anteil von 7% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Spannung und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 8 bezeichnet hierbei die Spannung, die für eine Leuchtdichte von 1000 cd/$m^2$ benötigt wird. SE1000 bezeichnet die Stromeffizienz, die bei 1000 cd/$m^2$ erreicht wird. EQE1000 schließlich bezeichnet die externe Quanteneffizienz bei einer Betriebsleuchtdichte von 1000 cd/$m^2$. Als Lebensdauer LT wird die Zeit definiert, nach der die Leuchtdichte bei Betrieb mit konstanter Stromdichte $j_0$ von der Startleuchtdichte auf einen gewissen Anteil L1 absinkt. Eine Angabe L1=80% in Tabelle 9 bedeutet, dass die in Spalte LT angegebene Lebensdauer der Zeit entspricht, nach der die Leuchtdichte auf 80% ihres Anfangswertes absinkt.

Die Daten der verschiedenen OLEDs sind in Tabelle 8 zusammengefasst. Die Beispiele V1 bis V7 sind Vergleichsbeispiele gemäß Stand der Technik, die Beispiele B1 bis B24 zeigen Daten von erfindungsgemäßen OLEDs.

Tabelle 7:

| Bsp. | HIL Dicke | HTL Dicke | EBL Dicke | EML Dicke | HBL Dicke | ETL Dicke | EIL Dicke |
|---|---|---|---|---|---|---|---|
| V1 | SpMA1:PD1 (95%:5%) 20nm | SpMA1 215nm | SpMA2 20nm | **VG1:H2:**TEG1 (33%:60%:7%) 30nm | ST2 10nm | ST2:LiQ (50%:50%) 30nm | LiQ 1nm |
| B1 | SpMA1:PD1 (95%:5%) 20nm | SpMA1 215nm | SpMA2 20nm | **E1:H2:**TEG1 (33%:60%:7%) 30nm | ST2 10nm | ST2:LiQ (50%:50%) 30nm | LiQ 1nm |
| V2 | SpMA1:PD1 (95%:5%) 20nm | SpMA1 215nm | SpMA2 20nm | **VG2:H2:**TEG1 (33%:60%:7%) 30nm | ST2 10nm | ST2:LiQ (50%:50%) 30nm | LiQ 1nm |
| B2 | SpMA1:PD1 (95%:5%) 20nm | SpMA1 215nm | SpMA2 20nm | **E2:H2:**TEG1 (33%:60%:7%) 30nm | ST2 10nm | ST2:LiQ (50%:50%) 30nm | LiQ 1nm |
| V3 | SpMA1:PD1 (95%:5%) 20nm | SpMA1 215nm | SpMA2 20nm | **VG3:H2:**TEG1 (33%:60%:7%) 30nm | ST2 10nm | ST2:LiQ (50%:50%) 30nm | LiQ 1nm |
| B3 | SpMA1:PD1 (95%:5%) 20nm | SpMA1 215nm | SpMA2 20nm | **E3:H2:**TEG1 (33%:60%:7%) 30nm | ST2 10nm | ST2:LiQ (50%:50%) 30nm | LiQ 1nm |
| V4 | SpMA1:PD1 (95%:5%) 20nm | SpMA1 215nm | SpMA2 20nm | **VG4:H2:**TEG1 (33%:60%:7%) 30nm | ST2 10nm | ST2:LiQ (50%:50%) 30nm | LiQ 1nm |
| B4 | SpMA1:PD1 (95%:5%) 20nm | SpMA1 215nm | SpMA2 20nm | **E4:H2:**TEG1 (33%:60%:7%) 30nm | ST2 10nm | ST2:LiQ (50%:50%) 30nm | LiQ 1nm |
| V5 | SpMA1:PD1 (95%:5%) 20nm | SpMA1 215nm | SpMA2 20nm | **VG5:H2:**TEG1 (33%:60%:7%) 30nm | ST2 10nm | ST2:LiQ (50%:50%) 30nm | LiQ 1nm |
| B5 | SpMA1:PD1 (95%:5%) 20nm | SpMA1 215nm | SpMA2 20nm | **E5:H2:**TEG1 (33%:60%:7%) 30nm | ST2 10nm | ST2:LiQ (50%:50%) 30nm | LiQ 1nm |
| V6 | SpMA1:PD1 (95%:5%) 20nm | SpMA1 215nm | SpMA2 20nm | **VG6:H1**:TEG1 (33%:60%:7%) 30nm | ST2 10nm | ST2:LiQ (50%:50%) 30nm | LiQ 1nm |

(fortgesetzt)

| Bsp. | HIL Dicke | HTL Dicke | EBL Dicke | EML Dicke | HBL Dicke | ETL Dicke | EIL Dicke |
|---|---|---|---|---|---|---|---|
| B6 | SpMA1:PD1 (95%:5%) 20nm | SpMA1 215nm | SpMA2 20nm | **E6:H1:**TEG1 (33%:60%:7%) 30nm | ST2 10nm | ST2:LiQ (50%:50%) 30nm | LiQ 1nm |
| V7 | SpMA1:PD1 (95%:5%) 20nm | SpMA1 215nm | SpMA2 20nm | **VG7:H2:**TEG1 (33%:60%:7%) 30nm | ST2 10nm | ST2:LiQ (50%:50%) 30nm | LiQ 1nm |
| B7 | SpMA1:PD1 (95%:5%) 20nm | SpMA1 215nm | SpMA2 20nm | **E7:H2:**TEG1 (33%:60%:7%) 30nm | ST2 10nm | ST2:LiQ (50%:50%) 30nm | LiQ 1nm |
| B8 | SpMA1:PD1 (95%:5%) 20nm | SpMA1 215nm | SpMA2 20nm | **E8:H2:**TEG1 (33%:60%:7%) 30nm | ST2 10nm | ST2:LiQ (50%:50%) 30nm | LiQ 1nm |
| B9 | SpMA1:PD1 (95%:5%) 20nm | SpMA1 215nm | SpMA2 20nm | **E9:H2:**TEG1 (33%:60%:7%) 30nm | ST2 10nm | ST2:LiQ (50%:50%) 30nm | LiQ 1nm |
| B10 | SpMA1:PD1 (95%:5%) 20nm | SpMA1 215nm | SpMA2 20nm | **E10:H2:**TEG1 (33%:60%:7%) 30nm | ST2 10nm | ST2:LiQ (50%:50%) 30nm | LiQ 1nm |
| B11 | SpMA1:PD1 (95%:5%) 20nm | SpMA1 215nm | SpMA2 20nm | **E11:H2:**TEG1 (33%:60%:7%) 30nm | ST2 10nm | ST2:LiQ (50%:50%) 30nm | LiQ 1nm |
| B12 | SpMA1:PD1 (95%:5%) 20nm | SpMA1 215nm | SpMA2 20nm | **E12:H2:**TEG1 (33%:60%:7%) 30nm | ST2 10nm | ST2:LiQ (50%:50%) 30nm | LiQ 1nm |
| B13 | SpMA1:PD1 (95%:5%) 20nm | SpMA1 215nm | SpMA2 20nm | **E14:H2:**TEG1 (33%:60%:7%) 30nm | ST2 10nm | ST2:LiQ (50%:50%) 30nm | LiQ 1nm |
| B14 | SpMA1:PD1 (95%:5%) 20nm | SpMA1 215nm | SpMA2 20nm | **E13:H16:**TEG1 (33%:60%:7%) 30nm | ST2 10nm | ST2:LiQ (50%:50%) 30nm | LiQ 1nm |
| B15 | SpMA1:PD1 (95%:5%) 20nm | SpMA1 215nm | SpMA2 20nm | **E13:H1:**TEG1 (33%:60%:7%) 30nm | ST2 10nm | ST2:LiQ (50%:50%) 30nm | LiQ 1nm |
| B16 | SpMA1:PD1 (95%:5%) 20nm | SpMA1 215nm | SpMA2 20nm | **E13:H3:**TEG1 (33%:60%:7%) 30nm | ST2 10nm | ST2:LiQ (50%:50%) 30nm | LiQ 1nm |

(fortgesetzt)

| Bsp. | HIL Dicke | HTL Dicke | EBL Dicke | EML Dicke | HBL Dicke | ETL Dicke | EIL Dicke |
|---|---|---|---|---|---|---|---|
| B17 | SpMA1:PD1 (95%:5%) 20nm | SpMA1 215nm | SpMA2 20nm | **E13:H47:**TEG1 (33%:60%:7%) 30nm | ST2 10nm | ST2:LiQ (50%:50%) 30nm | LiQ 1nm |
| B18 | SpMA1:PD1 (95%:5%) 20nm | SpMA1 215nm | SpMA2 20nm | **E13:H8:**TEG1 (33%:60%:7%) 30nm | ST2 10nm | ST2:LiQ (50%:50%) 30nm | LiQ 1nm |
| B19 | SpMA1:PD1 (95%:5%) 20nm | SpMA1 215nm | SpMA2 20nm | **E13:H9:**TEG2 (33%:60%:7%) 30nm | ST2 10nm | ST2:LiQ (50%:50%) 30nm | LiQ 1nm |
| B20 | SpMA1:PD1 (95%:5%) 20nm | SpMA1 215nm | SpMA2 20nm | **E15:H6:**TEG2 (33%:60%:7%) 30nm | ST2 10nm | ST2:LiQ (50%:50%) 30nm | LiQ 1nm |
| B21 | SpMA1:PD1 (95%:5%) 20nm | SpMA1 215nm | SpMA2 20nm | **E13:H5:**TEG3 (33%:60%:7%) 30nm | ST2 10nm | ST2:LiQ (50%:50%) 30nm | LiQ 1nm |
| B22 | SpMA1:PD1 (95%:5%) 20nm | SpMA1 215nm | SpMA2 20nm | **E15:H8:**TEG3 (33%:60%:7%) 30nm | ST2 10nm | ST2:LiQ (50%:50%) 30nm | LiQ 1nm |
| B23 | SpMA1:PD1 (95%:5%) 20nm | SpMA1 215nm | SpMA2 20nm | **E13:H3:**TEG1 (33%:60%:7%) 30nm | ST2 10nm | E4:LiQ (50%:50%) 30nm | LiQ 1nm |
| B24 | SpMA1:PD1 (95%:5%) 20nm | SpMA1 215nm | SpMA2 20nm | **E27:H8:**TEG3 (33%:60%:7%) 30nm | ST2 10nm | ST2:LiQ (50%:50%) 30nm | LiQ 1nm |

Tabelle 8:

| Ex. | U1000 (V) | SE1000 (cd/A) | EQE1000 (%) | CIE x/y at 1000 cd/m2 | $j_0$ (mA/cm$^2$) | L1 (%) | LT (h) |
|---|---|---|---|---|---|---|---|
| V1 | 4.2 | 71 | 16 | 0.34/0.64 | 20 | 80 | 120 |
| B1 | 3.8 | 76 | 17 | 0.35/0.62 | 20 | 80 | 165 |
| V2 | 3.6 | 71 | 16 | 0.34/0.61 | 20 | 80 | 75 |
| B2 | 3.4 | 72 | 17.5 | 0.32/0.62 | 20 | 80 | 175 |
| V3 | 3.8 | 69 | 17 | 0.35/0.62 | 20 | 80 | 130 |
| B3 | 3.4 | 72 | 18.5 | 0.33/0.63 | 20 | 80 | 170 |
| V4 | 3.6 | 68 | 18 | 0.32/0.62 | 20 | 80 | 125 |
| B4 | 3.2 | 70 | 19 | 0.34/0.61 | 20 | 80 | 180 |
| V5 | 4.2 | 71 | 17 | 0.34/0.61 | 20 | 80 | 95 |
| B5 | 3.4 | 75 | 18 | 0.33/0.63 | 20 | 80 | 175 |
| V6 | 3.5 | 70 | 17 | 0.35/0.62 | 20 | 80 | 130 |

(fortgesetzt)

| Ex. | U1000 (V) | SE1000 (cd/A) | EQE1000 (%) | CIE x/y at 1000 cd/m2 | $j_0$ (mA/cm$^2$) | L1 (%) | LT (h) |
|---|---|---|---|---|---|---|---|
| B6 | 3.3 | 75 | 19 | 0.33/0.63 | 20 | 80 | 155 |
| V7 | 3.5 | 68 | 17.5 | 0.33/0.63 | 20 | 80 | 135 |
| B7 | 3.2 | 75 | 18.5 | 0.33/0.63 | 20 | 80 | 185 |
| B8 | 3.4 | 71 | 18 | 0.33/0.62 | 20 | 80 | 200 |
| B9 | 3.4 | 76 | 18 | 0.35/0.62 | 20 | 80 | 215 |
| B10 | 3.5 | 69 | 16.5 | 0.35/0.62 | 20 | 80 | 220 |
| B11 | 3.5 | 75 | 18.5 | 0.33/0.63 | 20 | 80 | 210 |
| B12 | 3.4 | 67 | 18.5 | 0.33/0.63 | 20 | 80 | 235 |
| B13 | 3.6 | 75 | 18 | 0.32/0.63 | 20 | 80 | 145 |
| B14 | 4.1 | 68 | 16 | 0.35/0.62 | 20 | 80 | 170 |
| B15 | 3.3 | 76 | 18.5 | 0.35/0.62 | 20 | 80 | 235 |
| B16 | 3.4 | 65 | 18 | 0.35/0.62 | 20 | 80 | 240 |
| B17 | 3.3 | 76 | 19 | 0.35/0.62 | 20 | 80 | 245 |
| B18 | 3.2 | 70 | 17 | 0.34/0.61 | 20 | 80 | 280 |
| B19 | 3.4 | 76 | 19 | 0.35/0.62 | 20 | 80 | 295 |
| B20 | 3.3 | 69 | 18.5 | 0.35/0.62 | 20 | 80 | 280 |
| B21 | 3.2 | 76 | 18.5 | 0.34/0.61 | 20 | 80 | 275 |
| B22 | 3.1 | 76 | 19 | 0.35/0.62 | 20 | 80 | 260 |
| B23 | 3.7 | 70 | 18.5 | 0.34/0.61 | 20 | 80 | 240 |
| B24 | 3.6 | 76 | 17.9 | 0.35/0.63 | 20 | 80 | 158 |

Tabelle 9: Verwendete Materialien, sofern nicht zuvor beschrieben

| | |
|---|---|
| 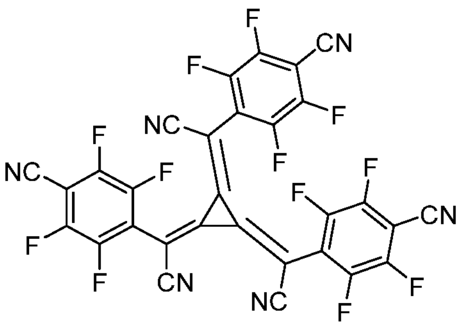 | 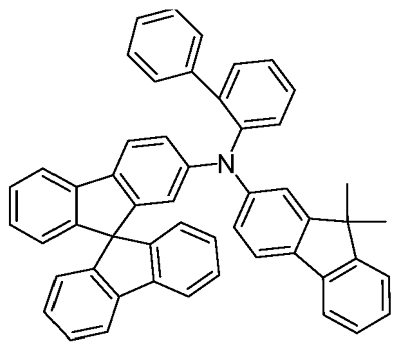 |
| PD1 (CAS Reg. No. 1224447-88-4) | SpMA1 |
| 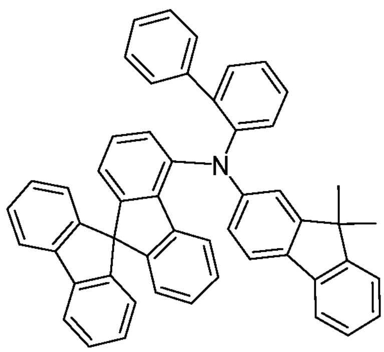 | 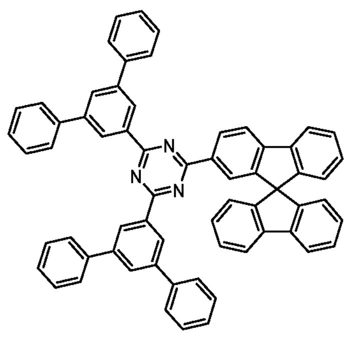 |

(fortgesetzt)

| SpMA2 | ST2 |
|---|---|
| 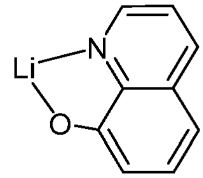 | 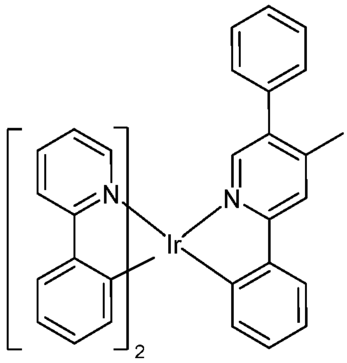 |
| LiQ | TEG1 |
| 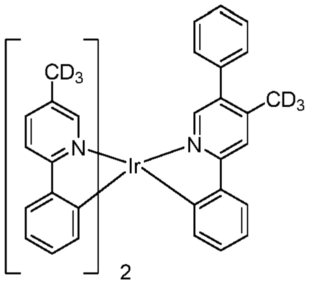 | 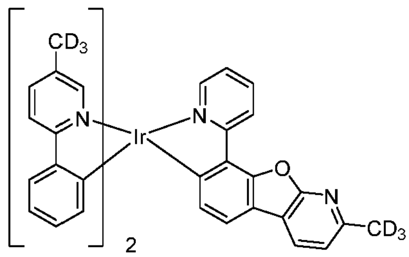 |
| TEG2 | TEG3 |
| 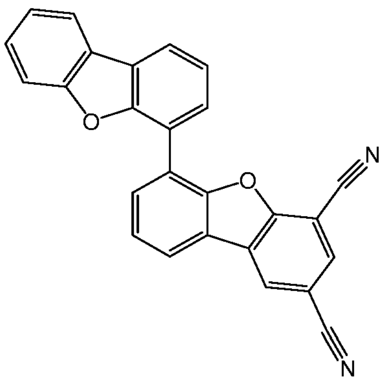 | 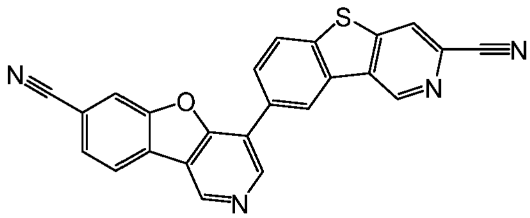 |
| **VG1** WO2019160315 | **VG2** WO2014097866 |
| 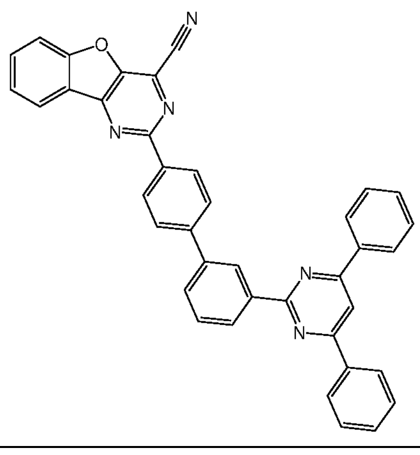 | 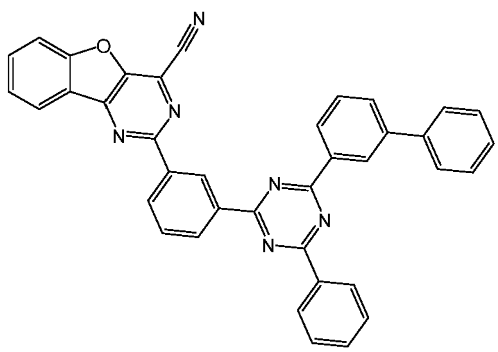 |
| **VG3** KR20170068927 | **VG4** KR20170068927 |

(fortgesetzt)

| | |
|---|---|
| 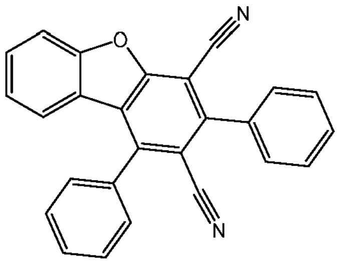 | 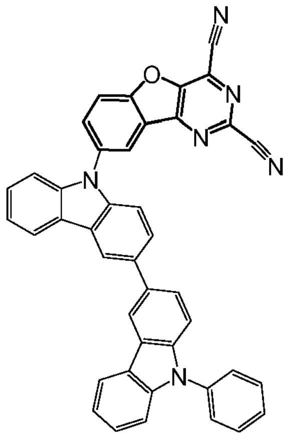 |
| **VG5** WO2019160315 | **VG6** WO2018060218 |

## Patentansprüche

1. Verbindung gemäß Formel (1),

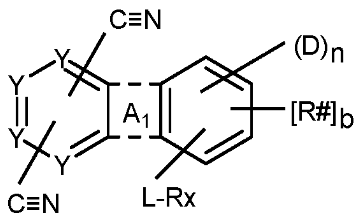

Formel (1),

wobei für die verwendeten Symbole und Indizes gilt:
Ring $A_1$ in Formel (1) entspricht der Formel (1A)

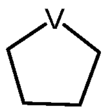

Formel (1A) ;

Y ist bei jedem Auftreten unabhängig voneinander N, C oder CR+, wobei mindestens ein Y für N steht und mindestens zwei Y für C stehen, an denen die CN-Gruppe gebunden ist; stehen zwei Y für N, so sind diese durch mindestens eine Gruppe C-CN voneinander getrennt;
V ist O oder S;
L ist eine Einfachbindung oder ein aromatisches oder heteroaromatisches Ringsystem mit 5 bis 30 Ringatomen, das unsubstituiert oder teilweise oder vollständig mit D substituiert sein kann, mit der Bedingung, dass das heteroaromatische Ringsystem eine über das N-Atom gebundene Carbazolgruppe ausschließt;
Rx ist ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann, mit der Bedingung, dass das Ringsystem und daran gebundene Substituenten keine N-gebundene Carbazolgruppe enthält;
R+ ist ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann;
R# ist bei jedem Auftreten gleich oder verschieden ein aromatisches Ringsystem mit 6 bis 30 Ringatomen, das mit einem oder mehreren Resten $R^2$ substituiert sein kann oder ein heteroaromatisches Ringsystem mit 5 bis 30 Ringatomen, das mit einem oder mehreren Resten $R^2$ substituiert sein kann, mit der Bedingung, dass das

Ringsystem und daran gebundene Substituenten keine N-gebundene Carbazolgruppe enthält;
$R^2$ ist bei jedem Auftreten gleich oder verschieden ausgewählt aus der Gruppe bestehend aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^2$ miteinander ein mono- oder polycyclisches, aliphatisches Ringsystem bilden;
b ist 0 oder 1;
n ist 0, 1, 2 oder 3.

2. Verbindung nach Anspruch 1, ausgewählt aus den Verbindungen der Formeln (1a), (1b), (1c), (1d), (1e) oder (1f),

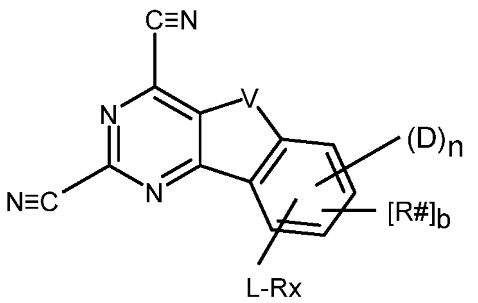

Formel (1a),

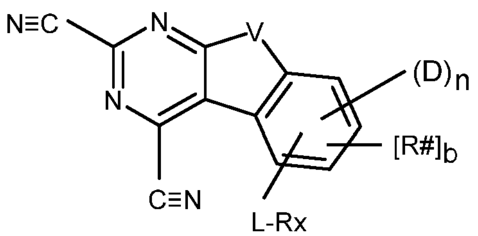

Formel (1b),

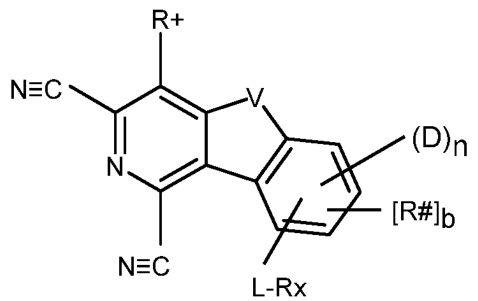

Formel (1c),

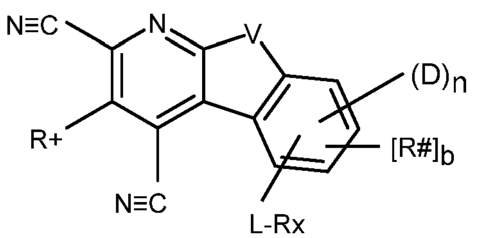

Formel (1d),

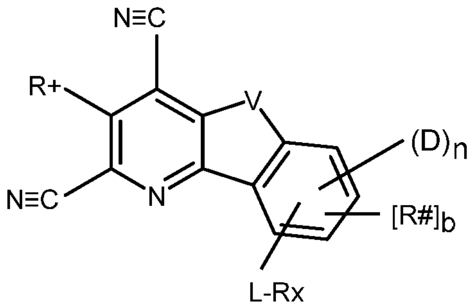

Formel (1e),

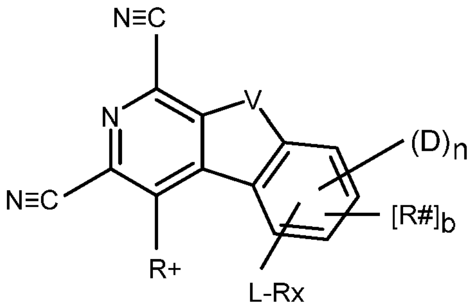

Formel (1f),

wobei die verwendeten Symbole V, R+, L, b, Rx, R# und n eine Bedeutung wie in Anspruch 1 haben.

**3.** Verbindung nach Anspruch 1 oder 2, wobei V O bedeutet.

**4.** Mischung, enthaltend mindestens eine Verbindung nach einem oder mehreren der Ansprüche 1 bis 3 und mindestens eine weitere Verbindung, ausgewählt aus der Gruppe der Matrixmaterialien, der phosphoreszierenden Emitter, der fluoreszierenden Emitter und/oder der Emitter, die TADF (thermally activated delayed fluorescence) zeigen.

**5.** Formulierung, enthaltend mindestens eine Verbindung nach einem oder mehreren der Ansprüche 1 bis 3 oder eine Mischung nach Anspruch 4 und mindestens ein Lösemittel.

**6.** Organische elektrolumineszierende Vorrichtung umfassend eine Anode, eine Kathode und mindestens eine organische Schicht, enthaltend mindestens eine Verbindung nach einem oder mehreren der Ansprüche 1 bis 3.

**7.** Organische elektrolumineszierende Vorrichtung nach Anspruch 6, wobei die organische Schicht mindestens eine lichtemittierende Schicht enthält, die die Verbindungen nach einem der Ansprüche 1 bis 3 enthält.

**8.** Organische elektrolumineszierende Vorrichtung nach Anspruch 6 oder 7, **dadurch gekennzeichnet, dass** die lichtemittierende Schicht ein weiteres Matrixmaterial enthält.

**9.** Organische elektrolumineszierende Vorrichtung nach Anspruch 8, **dadurch gekennzeichnet, dass** das zweite Matrixmaterial einer Verbindung der Formeln (6), (7), (8), (9) oder (10), entspricht,

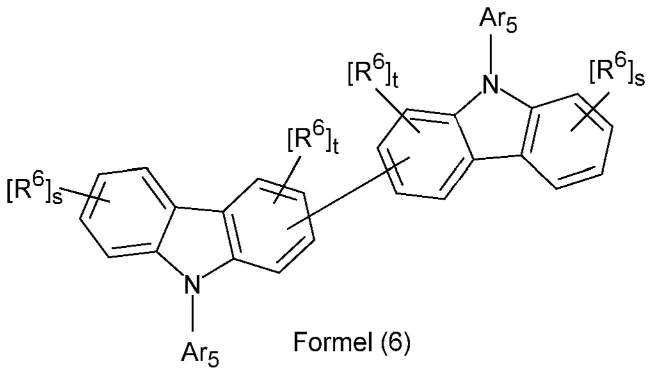

Formel (6)

,

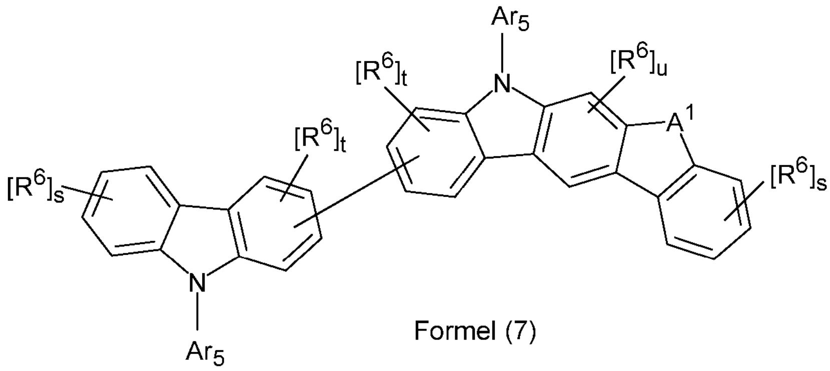

Formel (7)

,

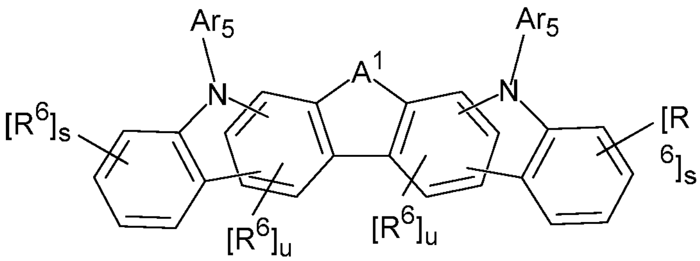

Formel (8)

,

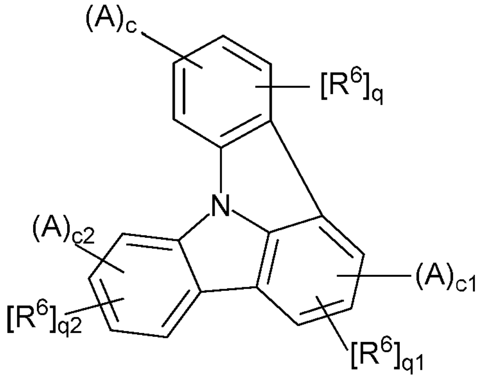

Formel (9)

,

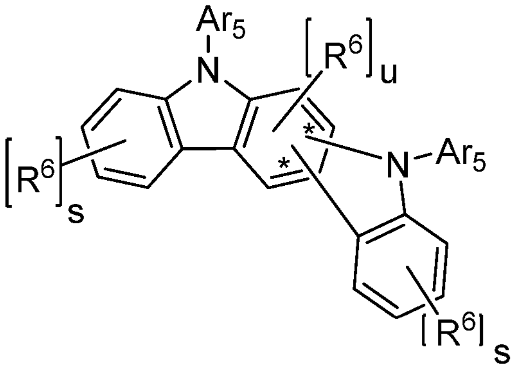

Formel (10),

wobei für die verwendeten Symbole und Indizes gilt:

$A^1$ ist $C(R^7)_2$, $NR^7$, O oder S;

A ist bei jedem Auftreten unabhängig voneinander eine Gruppe der Formel (3) oder (4),

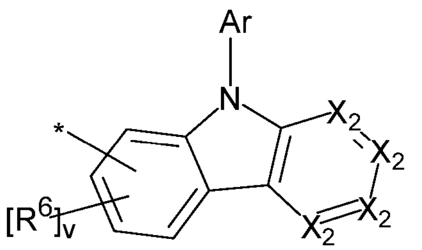

Formel (3) ,

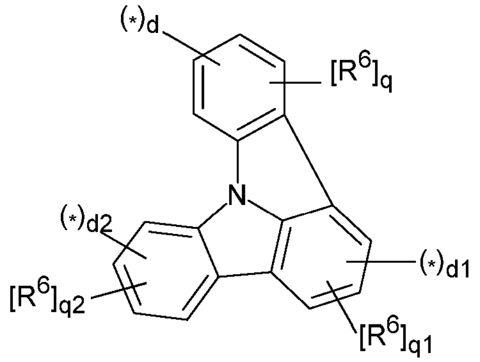

Formel (4) ;

$X_2$ ist bei jedem Auftreten gleich oder verschieden CH, $CR^6$ oder N, wobei maximal 2 Symbole $X_2$ N bedeuten können;

* kennzeichnet die Bindungsstelle an die Formel (9);

$R^6$ ist bei jedem Auftreten gleich oder verschieden D, CN, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^7$ substituiert sein kann und wobei eine oder mehrere nicht benachbarte $CH_2$-Gruppen durch $Si(R^7)_2$, C=O, $NR^7$, O, S oder $CONR^7$ ersetzt sein können, oder ein aromatisches oder heteroaromatisches Ringsystem mit 5 bis 60 Ringatomen, das jeweils durch einen oder mehrere Reste $R^7$ substituiert sein kann; dabei können zwei Reste $R^6$ auch miteinander ein aromatisches, heteroaromatisches, aliphatisches oder heteroaliphatisches Ringsystem bilden;

Ar ist bei jedem Auftreten gleich oder verschieden ein aromatisches oder heteroaromatisches Ringsystem mit 5 bis 40 Ringatomen, welches mit einem oder mehreren Resten $R^7$ substituiert sein kann;

$Ar^5$ steht gleich oder verschieden bei jedem Auftreten für ein aromatisches oder heteroaromatisches Ringsystem mit 5 bis 40 Ringatomen, welches mit einem oder mehreren Resten $R^7$ substituiert sein kann;

$R^7$ ist bei jedem Auftreten gleich oder verschieden D, F, Cl, Br, I, $N(R^8)_2$, CN, $NO_2$, $OR^8$, $SR^8$, $Si(R^8)_3$, $B(OR^8)_2$, $C(=O)R^8$, $P(=O)(R^8)_2$, $S(=O)R^8$, $S(=O)_2R^8$, $OSO_2R^8$, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten $R^8$ substituiert sein kann, wobei eine oder mehrere nicht benachbarte $CH_2$-Gruppen durch $Si(R^8)_2$, C=O, $NR^8$, O, S oder $CONR^8$ ersetzt sein können, oder ein aromatisches oder heteroaromatisches Ringsystem mit 5 bis 40 Ringatomen, das jeweils durch einen oder mehrere Reste $R^8$ substituiert sein kann; dabei können zwei oder mehrere Reste $R^7$ miteinander ein aromatisches, heteroaromatisches, aliphatisches oder heteroaliphatisches Ringsystem bilden, vorzugsweise bilden die Reste $R^7$ kein solches Ringsystem;

$R^8$ ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
c, c1, c2 bedeuten jeweils unabhängig voneinander bei jedem Auftreten 0 oder 1, wobei die Summe der Indizes bei jedem Auftreten c+c1+c2 1 bedeutet;
d, d1, d2 bedeuten jeweils unabhängig voneinander bei jedem Auftreten 0 oder 1, wobei die Summe der Indizes bei jedem Auftreten d+d1+d2 1 bedeutet;
q, q1, q2 bedeuten jeweils unabhängig bei jedem Auftreten 0 oder 1;
s ist bei jedem Auftreten gleich oder verschieden 0, 1, 2, 3 oder 4;
t ist bei jedem Auftreten gleich oder verschieden 0, 1, 2, oder 3;
u ist bei jedem Auftreten gleich oder verschieden 0, 1 oder 2; und
v ist 0 oder 1.

**10.** Organische elektrolumineszierende Vorrichtung nach Anspruch 8, **dadurch gekennzeichnet, dass** das zweite Matrixmaterial einer Verbindung der Formel (11) entspricht,

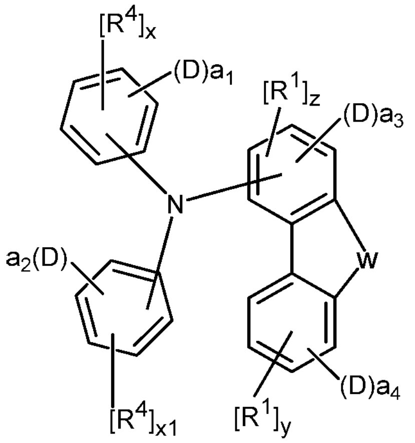

Formel (11),

wobei für die verwendeten Symbole und Indizes gilt:

W ist O, S, $C(R)_2$, N-$Ar_1$;
R ist jeweils unabhängig voneinander eine geradkettige oder verzweigte Alkylgruppe mit 1 bis 4 C-Atomen, die teilweise oder vollständig deuteriert sein kann, oder ein unsubstituiertes oder teilweise oder vollständig deuteriertes aromatisches Ringsystem mit 6 bis 18 C-Atomen, wobei zwei Substituenten R mit dem C-Atom, an das sie gebunden sind, ein mono- oder polycyclisches, aliphatisches oder aromatisches oder heteroaromatisches unsubstituiertes, teilweise deuteriertes oder vollständig deuteriertes Ringsystem bilden können, das durch ein oder mehrere Substituenten $R^5$ substituiert sein kann;
$Ar_1$ ist bei jedem Auftreten gleich oder verschieden ein aromatisches oder heteroaromatisches Ringsystem mit 5 bis 30 Ringatomen, das mit einem oder mehreren Resten $R^5$ substituiert sein kann; dabei können zwei Reste $Ar_1$, welche an dasselbe N-Atom, P-Atom oder B-Atom binden, auch durch eine Einfachbindung oder eine Brücke, ausgewählt aus $C(R^5)_2$, O oder S, miteinander verbrückt sein;
$R^1$ ist bei jedem Auftreten gleich oder verschieden ausgewählt aus der Gruppe bestehend aus F, Cl, Br, I, CN, $NO_2$, C(=O)R', $P(=O)(Ar_1)_2$, $P(Ar_1)_2$, $B(Ar_1)_2$, $Si(Ar_1)_3$, $Si(R')_3$, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R' substituiert sein kann, wobei eine oder mehrere nicht-benachbarte $CH_2$-Gruppen durch R'C=CR', $Si(R')_2$, C=O, C=S, C=NR', P(=O)(R'), SO, $SO_2$, NR', O, S oder CONR' ersetzt sein können und wobei ein oder mehrere H-Atome durch D, F, Cl, Br, I, CN oder $NO_2$ ersetzt sein können;
R' ist bei jedem Auftreten gleich oder verschieden ein aliphatischer, aromatischer oder heteroaromatischer organischer Rest;
$R^4$ ist bei jedem Auftreten gleich oder verschieden ausgewählt aus der Gruppe bestehend aus F, Cl, Br, I, CN, $NO_2$, $N(Ar_1)_2$, $NH_2$, $N(R^5)_2$, $C(=O)Ar_1$, C(=O)H, $C(=O)R^5$, $P(=O)(Ar_1)_2$, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem

oder mehreren Resten $R^5$ substituiert sein kann, wobei eine oder mehrere nicht-benachbarte $CH_2$-Gruppen durch HC=CH, $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, NH, $NR^5$, O, S, CONH oder $CONR^5$ ersetzt sein können und wobei ein oder mehrere H-Atome durch D, F, Cl, Br, I, CN oder $NO_2$ ersetzt sein können, einem aromatischen oder heteroaromatischen Ringsystem mit 5 bis 60 Ringatomen, das jeweils mit einem oder mehreren Resten $R^5$ substituiert sein kann, einer Aryloxy- oder Heteroaryloxygruppe mit 5 bis 60 Ringatomen, die mit einem oder mehreren Resten $R^5$ substituiert sein kann, oder einer Kombination dieser Systeme, wobei optional zwei oder mehr benachbarte Substituenten $R^4$ ein monocyclisches oder polycyclisches, aliphatisches, aromatisches oder heteroaromatisches Ringsystem bilden können, das mit einem oder mehreren Resten $R^5$ substituiert sein kann;

$R^5$ ist bei jedem Auftreten gleich oder verschieden ausgewählt aus der Gruppe bestehend aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können oder einem aromatischen oder heteroaromatischen Ringsystem mit 5 bis 30 Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten $R^5$ miteinander ein mono- oder polycyclisches, aliphatisches Ringsystem bilden;

x, x1 sind bei jedem Auftreten unabhängig 0, 1, 2, 3 oder 4;

y, z sind jeweils unabhängig voneinander 0, 1 oder 2;

a1, a2 sind jeweils unabhängig voneinander 0, 1, 2, 3, 4 oder 5;

a3 ist 0, 1, 2 oder 3;

a4 ist 0, 1, 2, 3 oder 4.

**11.** Organische elektrolumineszierende Vorrichtung nach einem oder mehreren der Ansprüche 6 bis 9, **dadurch gekennzeichnet, dass** die lichtemittierende Schicht einen phosphoreszierenden Emitter enthält.

**12.** Organische elektrolumineszierende Vorrichtung nach einem oder mehreren der Ansprüche 6 bis 10, **dadurch gekennzeichnet, dass** es sich um eine Elektrolumineszenzvorrichtung handelt, die ausgewählt ist aus den organischen lichtemittierenden Transistoren (OLETs), organischen Feld-Quench-Devices (OFQDs), organischen lichtemittierenden elektrochemischen Zellen (OLECs), organischen Laserdioden (O-Laser) und den organischen lichtemittierenden Dioden (OLEDs).

**13.** Verfahren zur Herstellung einer Vorrichtung nach einem oder mehreren der Ansprüche 6 bis 11, **dadurch gekennzeichnet, dass** die organische Schicht durch Gasphasenabscheidung oder aus Lösung aufgebracht wird.

**14.** Verfahren nach Anspruch 13, **dadurch gekennzeichnet, dass** die lichtemittierende Schicht der organischen Schicht durch Gasphasenabscheidung aufgebracht wird, wobei die mindestens eine Verbindung der Formel (1) zusammen mit den weiteren Materialien, die die lichtemittierende Schicht bilden, nacheinander oder gleichzeitig aus mindestens zwei Materialquellen aus der Gasphase abgeschieden werden.

**15.** Verfahren nach Anspruch 13 oder 14, **dadurch gekennzeichnet, dass** die lichtemittierende Schicht der organischen Schicht durch Gasphasenabscheidung aufgebracht wird, wobei die mindestens eine Verbindung der Formel (1) zusammen mit mindestens einem weiteren Matrixmaterial als Vormischung (premix), nacheinander oder gleichzeitig mit den lichtemittierenden Materialien, ausgewählt aus der Gruppe der phosphoreszierenden Emitter, der fluoreszierenden Emitter und/oder der Emitter, die TADF (thermally activated delayed fluorescence) zeigen, aus der Gasphase abgeschieden werden.

## Claims

**1.** Compound according to formula (1),

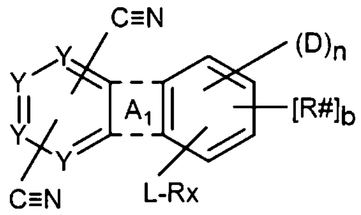

formula (1),

wherein applies to the symbols and indices used:
Ring A1 in formula (1) corresponds to formula (1A)

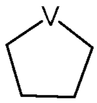

formula (1A) ;

Y in each occurrence is independently N, C or CR+, wherein at least one Y is N and at least two Ys are C to which the CN group is attached; if two Ys are N, they are separated from each other by at least one C-CN group;
V is O or S;
L is a single bond or an aromatic or heteroaromatic ring system with 5 to 30 ring atoms, which may be unsubstituted or partially or completely substituted with D, with the condition that the heteroaromatic ring system excludes a carbazole group bonded via the N atom;
Rx is an aromatic ring system having 6 to 30 ring atoms, which may be substituted by one or more radicals $R^2$, or a heteroaromatic ring system having 5 to 30 ring atoms, which may be substituted by one or more radicals $R^2$, with the proviso that the ring system and substituents bonded thereto do not contain an N-bonded carbazole group;
R+ is an aromatic ring system with 6 to 30 ring atoms, which may be substituted with one or more radicals $R^2$ or a heteroaromatic ring system with 5 to 30 ring atoms, which may be substituted with one or more radicals $R^2$;
R# in each occurrence is, identically or differently, an aromatic ring system having 6 to 30 ring atoms, which may be substituted by one or more radicals $R^2$, or a heteroaromatic ring system having 5 to 30 ring atoms, which may be substituted by one or more radicals $R^2$, with the proviso that the ring system and substituents attached thereto do not contain an N-linked carbazole group;
$R^2$ in each occurrence is the same or different and is selected from the group consisting of D, F, CN, a straight-chain alkyl group having 1 to 20 C atoms or a branched or cyclic alkyl group having 3 to 20 C atoms, wherein one or more non-adjacent $CH_2$ groups may be replaced by O or S and wherein one or more H atoms may be replaced by D, F, or CN or an aromatic or heteroaromatic ring system with 5 to 30 ring atoms, in which one or more H atoms can be replaced by D, F, Cl, Br, I or CN and which can be substituted by one or more alkyl groups with 1 to 4 carbon atoms each; two or more neighbouring substituents $R^2$ may together form a mono- or polycyclic aliphatic ring system;
b is 0 or 1;
n is 0, 1, 2 or 3.

**2.** The compound according to claim 1, selected from the compounds of formulae (1a), (1b), (1c), (1d), (1e) or (1f),

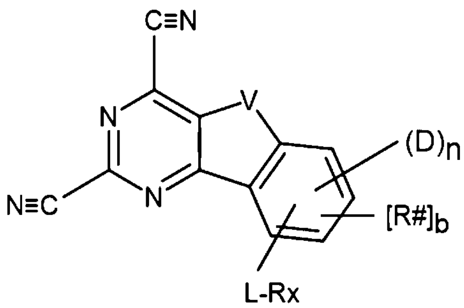

formula (1a),

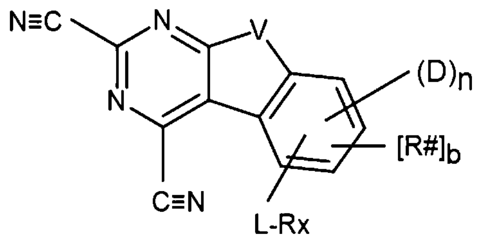

formula (1b),

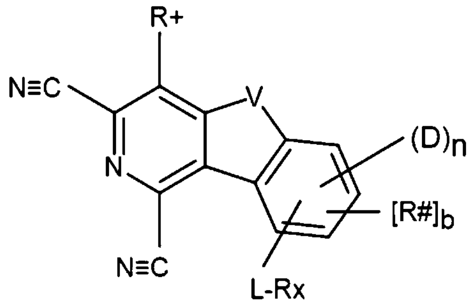

formula (1c),

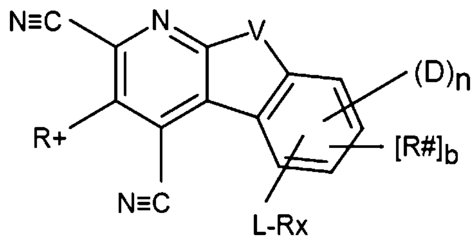

formula (1d),

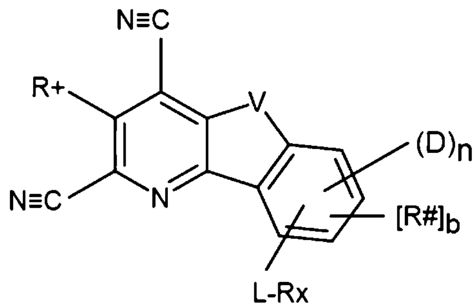

formula (1e),

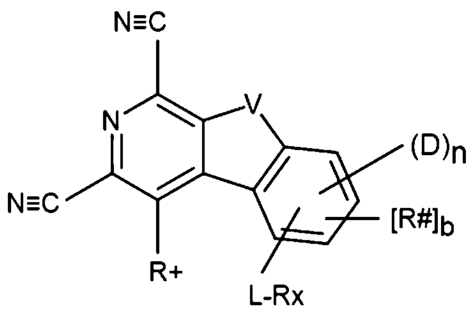

formula (1f),

wherein the symbols used V, R+, L, b, Rx, R# and n have a meaning as in claim 1.

**3.** The compound according to claim 1 or 2, wherein V is O.

4. A mixture containing at least one compound according to one or more of claims 1 to 3 and at least one further compound selected from the group consisting of the matrix materials, the phosphorescent emitters, the fluorescent emitters and/or the emitters which exhibit TADF (thermally activated delayed fluorescence).

5. A formulation containing at least one compound according to one or more of claims 1 to 3 or a mixture according to claim 4 and at least one solvent.

6. An organic electroluminescent device comprising an anode, a cathode and at least one organic layer containing at least one compound according to one or more of claims 1 to 3.

7. The organic electroluminescent device according to claim 6, wherein the organic layer contains at least one light-emitting layer containing the compounds according to any one of claims 1 to 3.

8. Organic electroluminescent device according to claim 6 or 7, **characterised in that** the light-emitting layer contains a further matrix material.

9. The organic electroluminescent device according to claim 8, **characterised in that** the second matrix material corresponds to a compound of the formulae (6), (7), (8), (9) or (10),

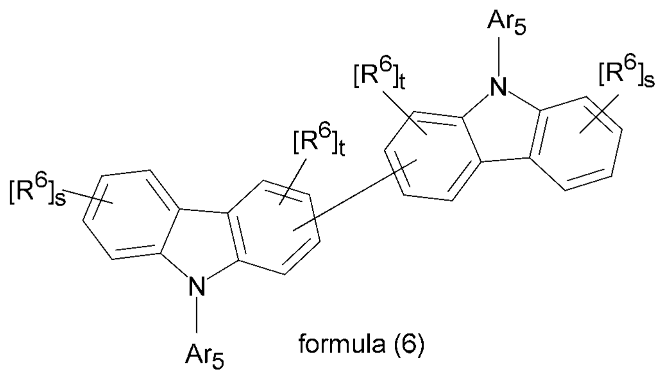

formula (6)

,

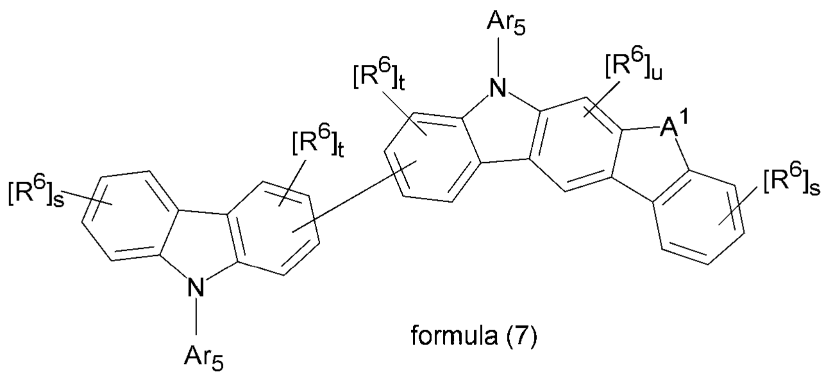

formula (7)

,

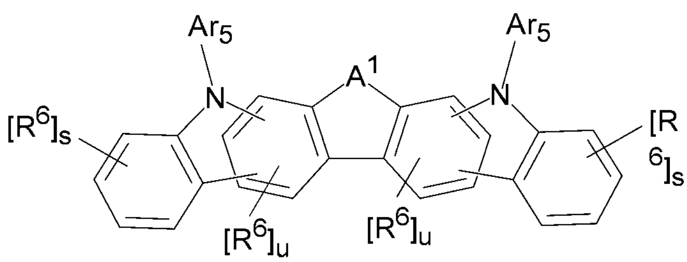

formula (8)

,

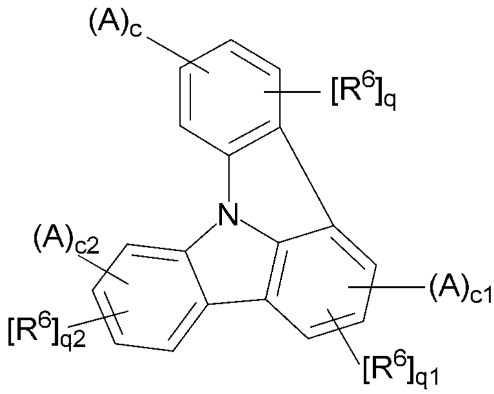

formula (9) ,

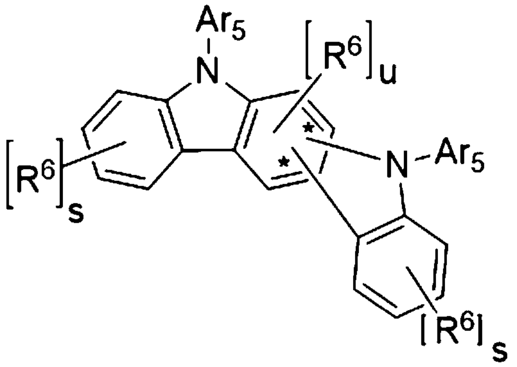

formula (10),

wherein the following applies to the symbols and indices used

$A^1$ is $C(R^7)_2$, $NR^7$, O or S;

A is a group of formula (3) or (4) for each occurrence independently of each other,

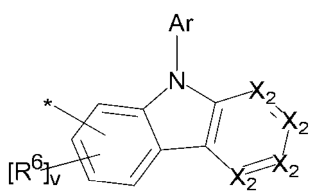

formula (3) ,

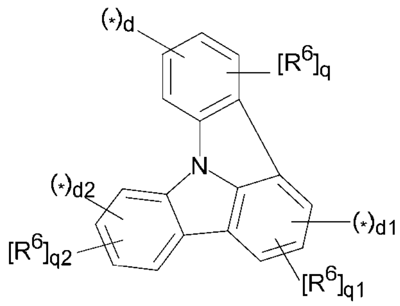

formula (4) ;

$X_2$ is the same or different CH, $CR^6$ or N for each occurrence, wherein a maximum of 2 symbols $X_2$ can mean N;

* indicates the binding site to the formula (9);

$R^6$ in each occurrence is the same or different D, CN, a straight-chain alkyl group having 1 to 20 carbon atoms or an alkenyl or alkynyl group having 2 to 20 carbon atoms or a branched or cyclic alkyl group having 3 to 20 carbon atoms, wherein the alkyl, alkenyl or alkynyl group is identical or different, alkenyl or alkynyl group may in each case be substituted by one or more radicals $R^7$ and wherein one or more non-adjacent $CH_2$ groups may be replaced by $Si(R^7)_2$, C=O, $NR^7$, O, S or $CONR^7$, or an aromatic or heteroaromatic ring system having 5 to 60 ring atoms, which may in each case be substituted by one or more radicals $R^7$; two radicals $R^6$ can also form an aromatic, heteroaromatic, aliphatic or heteroaliphatic ring system together;

Ar is an aromatic or heteroaromatic ring system with 5 to 40 ring atoms, which may be substituted with one or more

radicals $R^7$;
$Ar^5$ is identical or different in each occurrence for an aromatic or heteroaromatic ring system with 5 to 40 ring atoms, which can be substituted with one or more radicals $R^7$;
$R^7$ is the same or different in each occurrence D, F, Cl, Br, I, $N(R^8)_2$, CN, $NO_2$, $OR^8$, $SR^8$, $Si(R^8)_3$, $B(OR^8)_2$, $C(=O)R^8$, $P(=O)(R^8)_2$, $S(=O)R^8$, $S(=O)_2R^8$, $OSO_2R^8$, a straight-chain alkyl group having 1 to 20 carbon atoms or an alkenyl or alkynyl group having 2 to 20 carbon atoms or a branched or cyclic alkyl group having 3 to 20 carbon atoms, wherein the alkyl, alkenyl or alkynyl group may each be substituted by one or more radicals $R^8$, wherein one or more non-adjacent $CH_2$ groups are substituted by $Si(R^8)_2$, C=O, $NR^8$, O, S or $CONR^8$, or an aromatic or heteroaromatic ring system with 5 to 40 ring atoms, each of which may be substituted by one or more radicals $R^8$; two or more radicals $R^7$ can together form an aromatic, heteroaromatic, aliphatic or heteroaliphatic ring system, preferably the radicals $R^7$ do not form such a ring system;
$R^8$ in each occurrence is identical or different H, D, F or an aliphatic, aromatic or heteroaromatic organic radical, in particular a hydrocarbon radical, having 1 to 20 carbon atoms, in which one or more H atoms may also be replaced by F;
c, c1, c2 are each independently 0 or 1 at each occurrence, wherein the sum of the indices at each occurrence c+c1+c2 is 1;
d, d1, d2 each mean 0 or 1 independently of each other at each occurrence, wherein the sum of the indices at each occurrence d+d1+d2 means 1;
q, q1, q2 each mean 0 or 1 independently for each occurrence;
s is the same or different for each occurrence 0, 1, 2, 3 or 4;
t is the same or different 0, 1, 2 or 3 for each occurrence;
u is the same or different 0, 1 or 2 for each occurrence; and
v is 0 or 1.

**10.** The organic electroluminescent device according to claim 8, **characterised in that** the second matrix material corresponds to a compound of the formula (11)

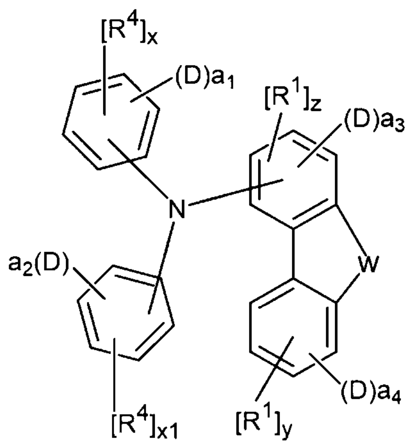

formula (11),

wherein the symbols and indices used are

W is O, S, $C(R)_2$, $N-Ar_1$;
R is, in each case independently of one another, a straight-chain or branched alkyl group having 1 to 4 carbon atoms, which may be partially or completely deuterated, or an unsubstituted or partially or completely deuterated aromatic ring system having 6 to 18 carbon atoms, wherein two substituents R with the carbon atom to which they are bonded form a mono- or di-substituted ring system, to which they are bonded may form a mono- or polycyclic, aliphatic or aromatic or heteroaromatic unsubstituted, partially deuterated or completely deuterated ring system which may be substituted by one or more substituents $R^5$;
$Ar_1$ is in each occurrence the same or different an aromatic or heteroaromatic ring system with 5 to 30 ring atoms, which can be substituted with one or more radicals $R^5$; two radicals $Ar_1$, which bind to the same N atom, P atom or B atom, can also be bridged together by a single bond or a bridge selected from $C(R^5)_2$, O or S;
$R^1$ in each occurrence is the same or different selected from the group consisting of F, Cl, Br, I, CN, $NO_2$, C(=O)R', $P(=O)(Ar_1)_2$, $P(Ar_1)_2$, $B(Ar_1)_2$, $Si(Ar_1)_3$, $Si(R')_3$, a straight-chain alkyl, alkoxy or thioalkyl group with 1 to 20 C atoms or a branched or cyclic alkyl, alkoxy or thioalkyl group with 1 to 20 C atoms, alkoxy or thioalkyl group with 3 to

20 C atoms or an alkenyl group with 2 to 20 C atoms, each of which may be substituted with one or more radicals R', wherein one or more non-adjacent $CH_2$ groups are replaced by R'C=CR', $Si(R')_2$, C=O, C=S, C=NR', P(=O)(R'), SO, $SO_2$, NR', O, S or CONR' and wherein one or more H atoms may be replaced by D, F, Cl, Br, I, CN or $NO_2$;
R' in each occurrence is, identically or differently, an aliphatic, aromatic or heteroaromatic organic radical;
$R^4$ is, in each occurrence, identically or differently selected from the group consistingof F, Cl, Br, I, CN, $NO_2$, $N(Ar_1)_2$, $NH_2$, $N(R^5)_2$, $C(=O)Ar_1$, C(=O)H, $C(=O)R^5$, $P(=O)(Ar_1)_2$, a straight-chain alkyl, alkoxy or thioalkyl group with 1 to 40 C atoms or a branched or cyclic alkyl, alkoxy or thioalkyl group with 1 to 40 C atoms, alkoxy or thioalkyl group having 3 to 40 carbon atoms or an alkenyl or alkynyl group having 2 to 40 carbon atoms, each of which may be substituted by one or more radicals $R^5$, wherein one or more non-adjacent $CH_2$ groups are substituted by HC=CH, $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, NH, $NR^5$, O, S, CONH or $CONR^5$ and wherein one or more H atoms may be replaced by D, F, Cl, Br, I, CN or $NO_2$, an aromatic or heteroaromatic ring system having from 5 to 60 ring atoms, each of which may be substituted by one or more radicals $R^5$, an aryloxy or heteroaryloxy group having from 5 to 60 ring atoms, which may be substituted with one or more radicals $R^5$, or a combination of these systems, wherein optionally two or more neighbouring substituents $R^4$ may form a monocyclic or polycyclic, aliphatic, aromatic or heteroaromatic ring system which may be substituted with one or more radicals $R^5$;
$R^5$ in each occurrence is the same or different and is selected from the group consisting of D, F, CN, a straight-chain alkyl group having 1 to 20 carbon atoms or a branched or cyclic alkyl group having 3 to 20 carbon atoms, wherein one or more non-adjacent CH2 groups may be replaced by O or S and wherein one or more H atoms may be replaced by D, F, or CN or an aromatic or heteroaromatic ring system with 5 to 30 ring atoms, in which one or more H atoms can be replaced by D, F, Cl, Br, I or CN and which can be substituted by one or more alkyl groups with 1 to 4 carbon atoms each; two or more neighbouring substituents $R^5$ may together form a mono- or polycyclic aliphatic ring system;
x, x1 are independently 0, 1, 2, 3 or 4 at each occurrence;
y, z are each independently 0, 1 or 2;
a1, a2 are each independently 0, 1, 2, 3, 4 or 5;
a3 is 0, 1, 2 or 3;
a4 is 0, 1, 2, 3 or 4.

**11.** An organic electroluminescent device according to one or more of claims 6 to 9, **characterised in that** the light-emitting layer contains a phosphorescent emitter.

**12.** The organic electroluminescent device according to one or more of claims 6 to 10, **characterised in that** it is an electroluminescent device selected from organic light-emitting transistors (OLETs), organic field quench devices (OFQDs), organic light-emitting electrochemical cells (OLECs), organic laser diodes (O-lasers) and organic light-emitting diodes (OLEDs).

**13.** A method of manufacturing a device according to one or more of claims 6 to 11, **characterised in that** the organic layer is applied by vapour deposition or from solution.

**14.** Process according to claim 13, **characterised in that** the light-emitting layer of the organic layer is applied by vapour phase deposition, wherein the at least one compound of formula (1) together with the further materials forming the light-emitting layer are deposited from the vapour phase successively or simultaneously from at least two material sources.

**15.** Process according to claim 13 or 14, **characterised in that** the light-emitting layer of the organic layer is applied by gas phase deposition, wherein the at least one compound of formula (1) together with at least one further matrix material as premix is deposited from the gas phase successively or simultaneously with the light-emitting materials selected from the group consisting of the phosphorescent emitters, the fluorescent emitters and/or the emitters which exhibit TADF (thermally activated delayed fluorescence).

## Revendications

**1.** Composé selon la formule (1),

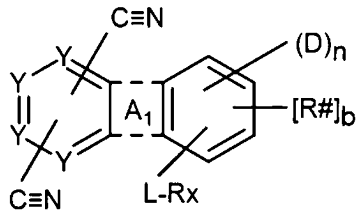

formule (1),

dans laquelle les symboles et les indices utilisés sont les suivants: le cycle A1 dans la formule (1) correspond à la formule (1A)

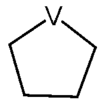

formule (1A) ;

Y est, à chaque occurrence, indépendamment N, C ou CR+, dans lequel au moins un Y est N et au moins deux Y sont C, auxquels le groupe CN est lié; si deux Y sont N, ils sont séparés par au moins un groupe C-CN;
V est O ou S;
L est une liaison simple ou un système cyclique aromatique ou hétéroaromatique ayant de 5 à 30 atomes dans le cycle, qui peut être non substitué ou partiellement ou totalement substitué par D, à condition que le système cyclique hétéroaromatique exclue un groupe carbazole lié par l'atome N;
Rx est un système cyclique aromatique ayant de 6 à 30 atomes dans le cycle, qui peut être substitué par un ou plusieurs radicaux $R^2$, ou un système cyclique hétéroaromatique ayant de 5 à 30 atomes dans le cycle, qui peut être substitué par un ou plusieurs radicaux $R^2$, à condition que le système cyclique et les substituants qui lui sont liés ne contiennent pas de groupe carbazole lié à l'azote;
R+ est un système cyclique aromatique ayant de 6 à 30 atomes dans le cycle, qui peut être substitué par un ou plusieurs radicaux $R^2$, ou un système cyclique hétéroaromatique ayant de 5 à 30 atomes dans le cycle, qui peut être substitué par un ou plusieurs radicaux $R^2$;
R# est, à chaque occurrence, identique ou différent, un système cyclique aromatique ayant de 6 à 30 atomes dans le cycle, qui peut être substitué par un ou plusieurs radicaux $R^2$, ou un système cyclique hétéroaromatique ayant de 5 à 30 atomes dans le cycle, qui peut être substitué par un ou plusieurs radicaux $R^2$, à condition que le système cyclique et les substituants qui lui sont liés ne contiennent pas de groupe carbazole lié à l'azote;
$R^2$ est choisi à chaque occurrence, de manière identique ou différente, dans le groupe constitué par D, F, CN, un groupe alkyle à chaîne linéaire ayant de 1 à 20 atomes de carbone ou un groupe alkyle ramifié ou cyclique ayant de 3 à 20 atomes de carbone, dans lequel un ou plusieurs groupes $CH_2$ non adjacents peuvent être remplacés par O ou S et dans lequel un ou plusieurs atomes de H sont remplacés par D, F, ou CN, ou un système cyclique aromatique ou hétéroaromatique ayant de 5 à 30 atomes dans le cycle, dans lequel un ou plusieurs atomes de H peuvent être remplacés par D, F, Cl, Br, I ou CN et qui peut être substitué par un ou plusieurs groupes alkyle ayant chacun de 1 à 4 atomes de carbone ; où deux ou plusieurs substituants $R^2$ adjacents peuvent former ensemble un système cyclique aliphatique mono- ou polycyclique;
b est égal à 0 ou 1;
n est égal à 0, 1, 2 ou 3.

**2.** Composé selon la revendication 1, choisi parmi les composés de formules (1a), (1b), (1c), (1d), (1e) ou (1f),

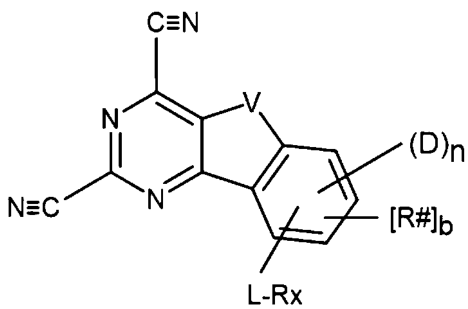

formule (1a),

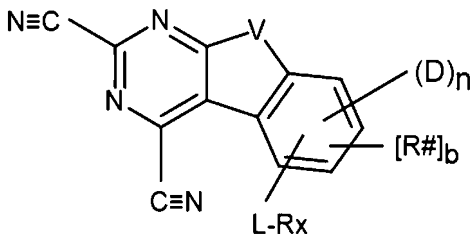

formule (1b),

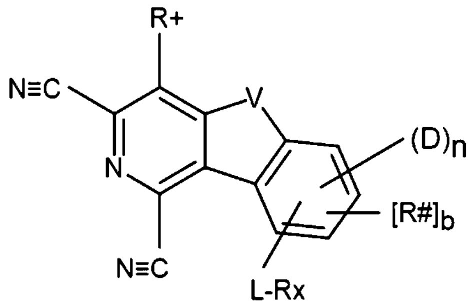

formule (1c),

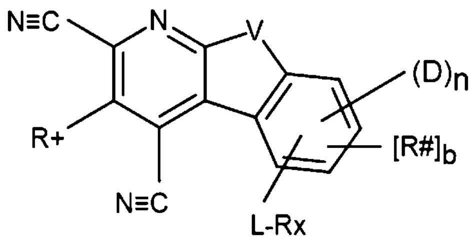

formule (1d),

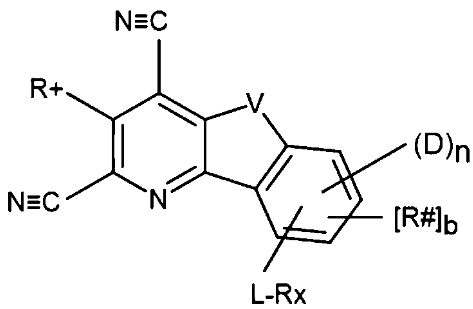

formule (1e),

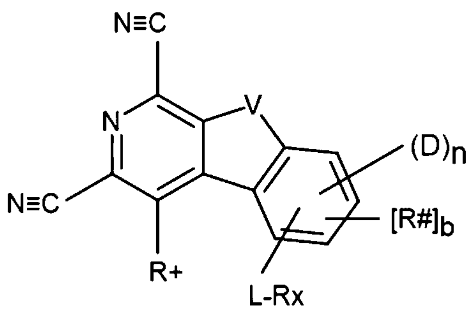

formule (1f),

dans laquelle les symboles utilisés V, R+, L, b, Rx, R# et n ont une signification telle que définie dans la revendication 1.

**3.** Composé selon la revendication 1 ou 2, dans lequel V représente O.

**4.** Mélange contenant au moins un composé selon une ou plusieurs des revendications 1 à 3 et au moins un autre composé choisi dans le groupe des matériaux de matrice, des émetteurs phosphorescents, des émetteurs fluorescents et/ou des émetteurs présentant une TADF (thermally activated delayed fluorescence).

**5.** Formulation contenant au moins un composé selon l'une ou plusieurs des revendications 1 à 3 ou un mélange selon la revendication 4 et au moins un solvant.

**6.** Dispositif organique électroluminescent comprenant une anode, une cathode et au moins une couche organique contenant au moins un composé selon une ou plusieurs des revendications 1 à 3.

**7.** Dispositif organique électroluminescent selon la revendication 6, dans lequel la couche organique contient au moins une couche émettrice de lumière contenant les composés selon l'une quelconque des revendications 1 à 3.

**8.** Dispositif électroluminescent organique selon la revendication 6 ou 7, **caractérisé en ce que** la couche émettrice de lumière contient un autre matériau de matrice.

**9.** Dispositif organique électroluminescent selon la revendication 8, **caractérisé en ce que** le second matériau de matrice correspond à un composé de formule (6), (7), (8), (9) ou (10),

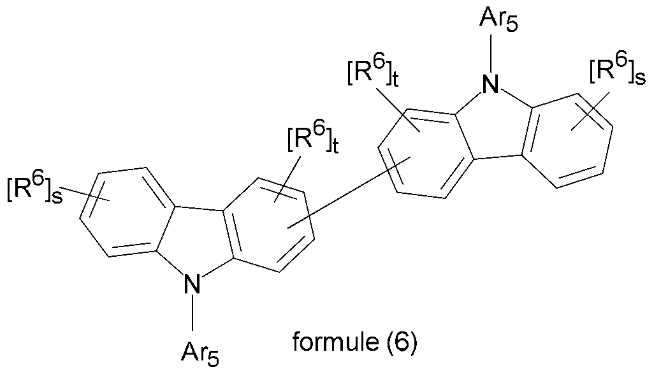

formule (6)

,

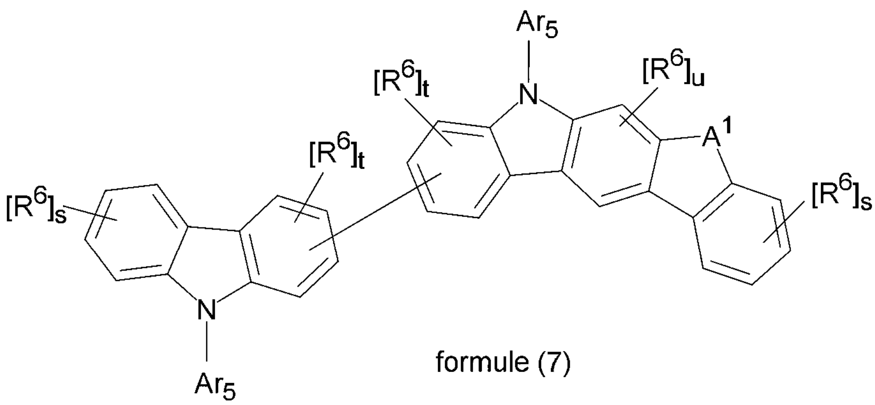

formule (7)

,

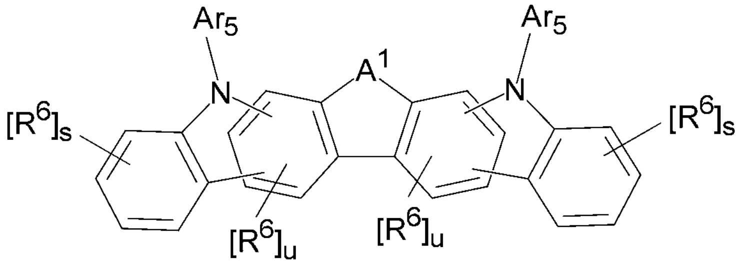

formule (8)

,

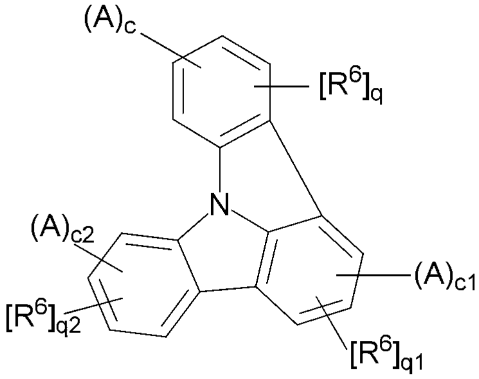

formule (9) ,

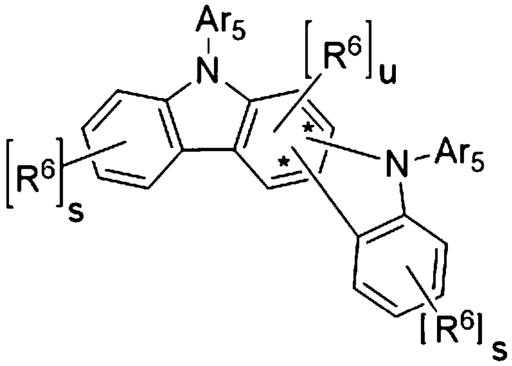

formule (10),

dans laquelle, pour les symboles et indices utilisés, on a:

$A^1$ est $C(R^7)_2$, $NR^7$, O ou S;
A est à chaque occurrence indépendamment un groupe de formule (3) ou (4),

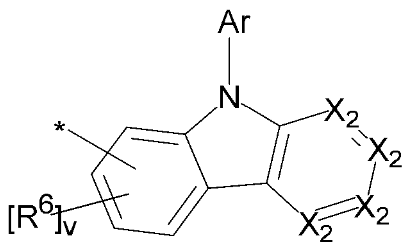

formule (3) ,

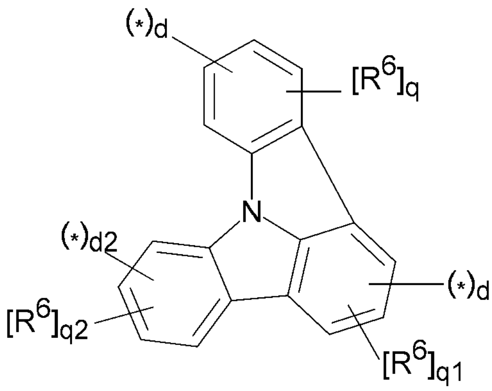

formule (4) ;

$X_2$ est, à chaque occurrence, identique ou différent de CH, $CR^6$ ou N, dans lequel au maximum 2 symboles $X_2$ peuvent représenter N;
* indique le site de liaison à la formule (9);
$R^6$ est à chaque occurrence identique ou différente D, CN, un groupe alkyle à chaîne droite avec 1 à 20 atomes de C ou un groupe alcényle ou alcynyle avec 2 à 20 atomes de C ou un groupe alkyle ramifié ou cyclique avec 3 à 20 atomes de C, dans lequel le groupe alkyle, alcényle ou alcynyle peut être substitué dans chaque cas par un ou plusieurs radicaux $R^7$ et dans lequel un ou plusieurs groupes $CH_2$ non adjacents peuvent être remplacés par $Si(R^7)_2$, C=O, $NR^7$, O, S ou $CONR^7$, ou un système cyclique aromatique ou hétéroaromatique ayant de 5 à 60

atomes dans le cycle, dont chacun peut être substitué par un ou plusieurs radicaux $R^7$ ; où deux radicaux $R^6$ peuvent également former ensemble un système cyclique aromatique, hétéroaromatique, aliphatique ou hétéroaliphatique;

Ar est à chaque occurrence, identique ou différente, un système cyclique aromatique ou hétéroaromatique ayant de 5 à 40 atomes dans le cycle, qui peut être substitué par un ou plusieurs radicaux $R^7$;

$Ar^5$ est identique ou différent à chaque occurrence et représente un système cyclique aromatique ou hétéroaromatique ayant de 5 à 40 atomes dans le cycle, qui peut être substitué par un ou plusieurs radicaux $R^7$;

$R^7$ est à chaque occurrence identique ou différente D, F, Cl, Br, I, $N(R^8)_2$, CN, $NO_2$, $OR^8$, $SR^8$, $Si(R^8)_3$, $B(OR^8)_2$, $C(=O)R^8$, $P(=O)(R^8)_2$, $S(=O)R^8$, $S(=O)_2R^8$, $OSO_2R^8$, un groupe alkyle à chaîne droite ayant de 1 à 20 atomes de carbone ou un groupe alcényle ou alcynyle ayant de 2 à 20 atomes de carbone ou un groupe alkyle ramifié ou cyclique ayant de 3 à 20 atomes de carbone, dans lequel chaque groupe alkyle, alcényle ou alcynyle peut être substitué par un ou plusieurs radicaux $R^8$, un ou plusieurs groupes $CH_2$ non adjacents étant remplacés par $Si(R^8)_2$, C=O, $NR^8$, O, S ou $CONR^8$, ou un système cyclique aromatique ou hétéroaromatique ayant de 5 à 40 atomes dans le cycle, dont chacun peut être substitué par un ou plusieurs radicaux $R^8$ ; où deux ou plusieurs radicaux $R^7$ peuvent former ensemble un système cyclique aromatique, hétéroaromatique, aliphatique ou hétéroaliphatique, de préférence les radicaux $R^7$ ne forment pas un tel système cyclique;

$R^8$ est à chaque occurrence identique ou différente H, D, F ou un radical organique aliphatique, aromatique ou hétéroaromatique, en particulier un radical hydrocarboné, ayant de 1 à 20 atomes de carbone, dans lequel un ou plusieurs atomes de H peuvent également être remplacés par F;

c, c1, c2 représentent chacun, indépendamment les uns des autres, 0 ou 1 à chaque occurrence, dans laquelle la somme des indices à chaque occurrence c+c1+c2 représente 1;

d, d1, d2 sont chacun indépendamment 0 ou 1 à chaque occurrence, dans lesquels la somme des indices à chaque occurrence d+d1+d2 est 1;

q, q1, q2 signifient chacun indépendamment 0 ou 1 à chaque occurrence;

s est identique ou différent de 0, 1, 2, 3 ou 4 à chaque occurrence;

t est égal ou différent de 0, 1, 2 ou 3 à chaque occurrence;

u est égal ou différent de 0, 1 ou 2 à chaque occurrence; et

v est 0 ou 1.

**10.** Dispositif organique électroluminescent selon la revendication 8, **caractérisé en ce que** le second matériau de matrice correspond à un composé de formule (11),

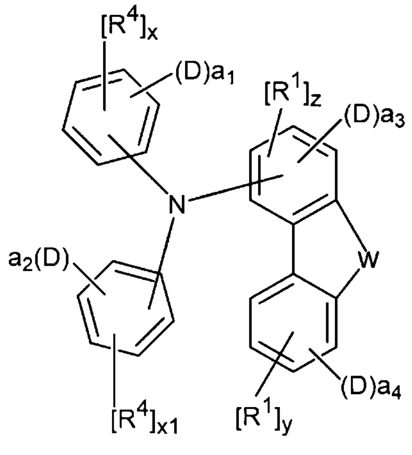

formule (11),

dans laquelle, pour les symboles et indices utilisés, on a :

W est O, S, $C(R)_2$, $N-Ar_1$;

R est dans chaque cas indépendamment un groupe alkyle à chaîne droite ou ramifiée ayant de 1 à 4 atomes de carbone, qui peut être partiellement ou totalement deutéré, ou un système cyclique aromatique non substitué ou partiellement ou totalement deutéré ayant de 6 à 18 atomes de carbone, dans lequel deux substituants R ayant l'atome de carbone, auquel ils sont liés, peuvent former un système cyclique mono- ou polycyclique, aliphatique ou aromatique ou hétéroaromatique, non substitué, partiellement deutéré ou complètement deutéré, qui peut être substitué par un ou plusieurs substituants $R^5$;

$Ar_1$ est à chaque occurrence, identique ou différente, un système cyclique aromatique ou hétéroaromatique

ayant de 5 à 30 atomes dans le cycle, qui peut être substitué par un ou plusieurs radicaux $R^5$; deux radicaux $Ar_1$, qui se lient au même atome N, P ou B, peuvent également être pontés l'un à l'autre par une liaison simple ou un pont choisi parmi $C(R^5)_2$, O ou S;

$R^1$ est, à chaque occurrence, identique ou différent, choisi dans le groupe constitué par F, Cl, Br, I, CN, $NO_2$, C(=O)R', $P(=O)(Ar_1)_2$, $P(Ar_1)_2$, $B(Ar_1)_2$, $Si(Ar_1)_3$, $Si(R')_3$, un groupe alkyle, alcoxy ou thioalkyle à chaîne droite ayant de 1 à 20 atomes de carbone ou un groupe alkyle, alcoxy ou thioalkyle ramifié ou cyclique, alcoxy ou thioalkyle ayant de 3 à 20 atomes de carbone ou un groupe alcényle ayant de 2 à 20 atomes de carbone, dont chacun peut être substitué par un ou plusieurs radicaux R', dans lequel un ou plusieurs groupes $CH_2$ non adjacents sont remplacés par R'C=CR', $Si(R')_2$, C=O, C=S, C=NR', P(=O)(R'), SO, $SO_2$, NR', O, S ou CONR' et dans lequel un ou plusieurs atomes de H peuvent être remplacés par D, F, Cl, Br, I, CN ou $NO_2$;

R' est à chaque occurrence, identique ou différente, un radical organique aliphatique, aromatique ou hétéroaromatique;

$R^4$ est, à chaque occurrence, identique ou différent, choisi dans le groupe constitué par F, Cl, Br, I, CN, $NO_2$, $N(Ar_1)_2$, $NH_2$, $N(R^5)_2$, $C(=O)Ar_1$, C(=O)H, $C(=O)R^5$, $P(=O)(Ar_1)_2$, un groupe alkyle, alcoxy ou thioalkyle à chaîne droite ayant de 1 à 40 atomes de carbone ou un groupe alkyle, alcoxy ou thioalkyle ramifié ou cyclique ayant de 1 à 40 atomes de carbone, alcoxy ou thioalkyle ayant de 3 à 40 atomes de carbone ou un groupe alcényle ou alcynyle ayant de 2 à 40 atomes de carbone, dont chacun peut être substitué par un ou plusieurs radicaux $R^5$, dans lequel un ou plusieurs groupes $CH_2$ non adjacents sont remplacés par HC=CH, $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, NH, $NR^5$, O, S, CONH ou $CONR^5$ et dans lequel un ou plusieurs atomes de H peuvent être remplacés par D, F, Cl, Br, I, CN ou $NO_2$, un système cyclique aromatique ou hétéroaromatique ayant de 5 à 60 atomes dans le cycle, dont chacun peut être substitué par un ou plusieurs radicaux $R^5$, un groupe aryloxy ou hétéroaryloxy ayant de 5 à 60 atomes dans le cycle, qui peut être substitué par un ou plusieurs radicaux $R^5$, ou une combinaison de ces systèmes, dans laquelle éventuellement deux ou plusieurs substituants $R^4$ adjacents peuvent former un système cyclique monocyclique ou polycyclique, aliphatique, aromatique ou hétéroaromatique, qui peut être substitué par un ou plusieurs radicaux $R^5$;

$R^5$ est choisi à chaque occurrence, de manière identique ou différente, dans le groupe constitué par D, F, CN, un groupe alkyle à chaîne linéaire ayant de 1 à 20 atomes de carbone ou un groupe alkyle ramifié ou cyclique ayant de 3 à 20 atomes de carbone, dans lequel un ou plusieurs groupes CH2 non adjacents peuvent être remplacés par O ou S et dans lequel un ou plusieurs atomes de H sont remplacés par D, F, ou CN, ou un système cyclique aromatique ou hétéroaromatique ayant de 5 à 30 atomes dans le cycle, dans lequel un ou plusieurs atomes de H peuvent être remplacés par D, F, Cl, Br, I ou CN et qui peut être substitué par un ou plusieurs groupes alkyle ayant chacun de 1 à 4 atomes de carbone ; où deux ou plusieurs substituants adjacents $R^5$ peuvent former ensemble un système cyclique aliphatique mono- ou polycyclique ;

x, x1 sont indépendamment 0, 1, 2, 3 ou 4 à chaque occurrence;

y, z sont chacun indépendamment 0, 1 ou 2;

a1, a2 sont chacun indépendamment 0, 1, 2, 3, 4 ou 5;

a3 est 0, 1, 2 ou 3;

a4 est 0, 1, 2, 3 ou 4.

**11.** Dispositif électroluminescent organique selon une ou plusieurs des revendications 6 à 9, **caractérisé en ce que** la couche émettrice de lumière contient un émetteur phosphorescent.

**12.** Dispositif organique électroluminescent selon une ou plusieurs des revendications 6 à 10, **caractérisé en ce qu'**il s'agit d'un dispositif électroluminescent choisi parmi les transistors organiques électroluminescents (OLET), les dispositifs organiques à champ quantifié (OFQD), les cellules électrochimiques organiques électroluminescentes (OLEC), les diodes laser organiques (O-Laser) et les diodes organiques électroluminescentes (OLED).

**13.** Procédé de fabrication d'un dispositif selon une ou plusieurs des revendications 6 à 11, **caractérisé en ce que** la couche organique est déposée par dépôt en phase vapeur ou à partir d'une solution.

**14.** Procédé selon la revendication 13, **caractérisé en ce que** la couche émettrice de lumière de la couche organique est appliquée par dépôt en phase vapeur, dans lequel le au moins un composé de formule (1) est déposé en phase vapeur successivement ou simultanément à partir d'au moins deux sources de matériau, conjointement avec les autres matériaux qui forment la couche émettrice de lumière.

**15.** Procédé selon la revendication 13 ou 14, **caractérisé en ce que** la couche émettrice de lumière de la couche organique est appliquée par dépôt en phase gazeuse, dans lequel le au moins un composé de formule (1) est déposé en phase gazeuse conjointement avec au moins un autre matériau de matrice en tant que prémélange, successivement ou simultanément avec les matériaux émetteurs de lumière choisis dans le groupe des émetteurs phosphorescents, des émetteurs fluorescents et/ou des émetteurs présentant une TADF (thermally activated delayed fluorescence).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014097866 A
- EP 2826781 A
- KR 20170068927
- CN 109912610
- WO 15105313 A
- WO 15108301 A
- WO 18060307 A
- WO 2018060218 A
- WO 2017115608 A
- WO 2019160315 A
- KR 20190141598
- US 2017186969 A
- WO 2015022051 A
- WO 2017148564 A
- WO 2018083053 A
- CN 112375053
- WO 2019192954 A
- WO 2021156323 A
- WO 21107728 A
- US 7294849 B
- WO 2021180614 A
- KR 2016041014
- WO 2017122988 A
- KR 202005282
- KR 101978651
- WO 2018110887 A
- WO 2019007867 A


### In der Beschreibung aufgeführte Nicht-Patentliteratur


- *Bulletin oft he Chemical Society of Japan*, 2021, vol. 94 (2), 600-605
- *Asian Journal of Organic Chemistry*, 2017, vol. 6 (8), 1063-1071
- **M. S. ARNOLD et al.** *Appl. Phys. Lett.*, 2008, vol. 92, 053301
- *CHEMICAL ABSTRACTS*, 1224447-88-4